(19) Europäisches Patentamt
European Patent Office
Office européen des brevets

(11) EP 3 106 521 B1

(12) **EUROPEAN PATENT SPECIFICATION**

(45) Date of publication and mention of the grant of the patent:
**26.12.2018 Bulletin 2018/52**

(51) Int Cl.:
***C12N 15/74*** *(2006.01)*

(21) Application number: **16175360.3**

(22) Date of filing: **20.06.2016**

(54) **A CONSTRUCT, A VECTOR AND A METHOD OF PRODUCTION OF A TARGET MOLECULE IN A CYANOBACTERIUM**

KONSTRUKT, VEKTOR UND VERFAHREN ZUR HERSTELLUNG EINES ZIELMOLEKÜLS IN EIN CYANOBAKTERIUM

CONSTRUCTION, VECTEUR ET PROCÉDÉ DE PRODUCTION D'UNE MOLÉCULE CIBLE DANS UNE CYANOBACTÉRIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.06.2015 PT 15108564**

(43) Date of publication of application:
**21.12.2016 Bulletin 2016/51**

(60) Divisional application:
**18204455.2**

(73) Proprietor: **Instituto de Biologia Molecular e Celular**
**4150-180 Porto (PT)**

(72) Inventors:
- **VILARINHO PINTO, Filipe José**
**4250-170 Porto (PT)**
- **SEQUEIRA TAMAGNINI BARBOSA OXELFELT, Paula Maria**
**4450-649 Matosinhos (PT)**

(74) Representative: **Teixeira de Carvalho, Anabela**
**Patentree**
**Edificio Net**
**Rua de Salazares 842**
**4149-002 Porto (PT)**

(56) References cited:
**CN-A- 103 361 274**

- **KUNERT A ET AL: "Construction of promoter probe vectors for Synechocystis sp. PCC 6803 using the light-emitting reporter systems Gfp and LuxAB.", JOURNAL OF MICROBIOLOGICAL METHODS AUG 2000, vol. 41, no. 3, August 2000 (2000-08), pages 185-194, XP002763424, ISSN: 0167-7012**
- **BENTLEY FIONA K ET AL: "Heterologous expression of the mevalonic acid pathway in cyanobacteria enhances endogenous carbon partitioning to isoprene.", MOLECULAR PLANT JAN 2014, vol. 7, no. 1, January 2014 (2014-01), pages 71-86, XP002763434, ISSN: 1752-9867**
- **PINTO FILIPE ET AL: "Improving a Synechocystis-based photoautotrophic chassis through systematic genome mapping and validation of neutral sites.", DNA RESEARCH : AN INTERNATIONAL JOURNAL FOR RAPID PUBLICATION OF REPORTS ON GENES AND GENOMES DEC 2015, vol. 22, no. 6, December 2015 (2015-12), pages 425-437, XP002763423, ISSN: 1756-1663**
- **TAN XIAOMING ET AL: "Role of Rbp1 in the acquired chill-light tolerance of cyanobacteria.", JOURNAL OF BACTERIOLOGY JUN 2011, vol. 193, no. 11, June 2011 (2011-06), pages 2675-2683, XP002763419, ISSN: 1098-5530**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 106 521 B1

- YADA T ET AL: "Gene recognition in cyanobacterium genomic sequence data using the hidden Markov model.", PROCEEDINGS / ... INTERNATIONAL CONFERENCE ON INTELLIGENT SYSTEMS FOR MOLECULAR BIOLOGY ; ISMB. INTERNATIONAL CONFERENCE ON INTELLIGENT SYSTEMS FOR MOLECULAR BIOLOGY 1996, vol. 4, 1996, pages 252-260, XP002763420, ISSN: 1553-0833
- HIHARA YUKAKO ET AL: "DNA microarray analysis of redox-responsive genes in the genome of the cyanobacterium Synechocystis sp. strain PCC 6803.", JOURNAL OF BACTERIOLOGY MAR 2003, vol. 185, no. 5, March 2003 (2003-03), pages 1719-1725, XP002763421, ISSN: 0021-9193
- DÖRRICH ANJA K ET AL: "Deletion of the Synechocystis sp. PCC 6803 kaiAB1C1 gene cluster causes impaired cell growth under light-dark conditions.", MICROBIOLOGY (READING, ENGLAND) NOV 2014, vol. 160, no. Pt 11, November 2014 (2014-11), pages 2538-2550, XP002763422, ISSN: 1465-2080

## Description

### Technical domain

The present disclosure relates to constructs for the integration of DNA into the genome of cyanobacteria, vectors comprising the constructs, cyanobacteria comprising the constructs or transformed by the vector and a method of transforming cyanobacteria.

The current disclosure described herein employs the use of five previously unidentified neutral sites into which foreign DNA can be integrated into the chromosome of *Synechocystis* without deleterious effects upon the growth and viability of the host cells. It is further described that foreign DNA may be integrated at such neutral sites so that *Synechocystis* expresses genes, which encode proteins that enable production of commercially important chemicals. The identification of a number of neutral sites which are spread throughout the *Synechocystis* chromosome, enables implementation of more complex synthetic circuits i.e. multiple genes may be expressed. New integrative vectors have been produced and are also disclosed herein. The present disclosure therefore relates to constructs for the integration of DNA into the genome of cyanobacteria, vectors comprising the constructs, cyanobacteria comprising the constructs or transformed by the vector and a method of transforming cyanobacteria.

### Technical background

Microorganisms have been widely used in biotechnological applications to produce added-value products (Madigan 2012). The Gram-negative bacterium *Escherichia coli* is the most commonly used, but others such as the Gram-positive bacterium *Bacillus subtilis,* and the eukaryotic yeast *Saccharomyces cerevisiae,* have also been successfully exploited (Harwood, Pohl et al. 2013, Li and Borodina 2014). Heterotrophic organisms require supplementation of the growth medium with external carbon sources increasing production costs therefore autotrophic organisms emerge as a valid alternative (Berla, Saha et al. 2013, Yu, You et al. 2013). In this context, cyanobacteria are promising "low-cost" cell factories since they can use $CO_2$ as carbon source, water as reducing power, light as energy source and some strains are even able to fix atmospheric $N_2$ (nitrogen source). These organisms are found in almost any ecological niche on Earth (Whitton and Potts 2000) which reflects their high degree of metabolic plasticity.

Among cyanobacteria, the unicellular non-$N_2$-fixing *Synechocystis* sp. PCC 6803 is the most studied strain, and the large amount of genomic, proteomic and physiological information generated over the years has allowed the construction of genome-scale models, enabling the prediction of the system behavior (Fu 2009, Montagud, Navarro et al. 2010, Steuer, Knoop et al. 2012). Moreover, it was the first photosynthetic organism to have its genome sequenced (Kaneko, Sato et al. 1996; (http://genome.microbedb.jp/cyanobase/Synechocystis) comprising a 3.6 Mb chromosome and 7 plasmids. It is also naturally transformable, and a number of molecular tools are available for its genetic manipulation (Vermaas 1996, Koksharova and Wolk 2002, Huang, Camsund et al. 2010, Heidorn, Camsund et al. 2011, Berla, Saha et al. 2013, Qi, Yao et al. 2013). Altogether, these characteristics potentially allow the exploitation of *Synechocystis* as a photoautotrophic biotechnological platform. However, in contrast to E. *coli,* for which several replicative plasmids from different compatibility groups are available, for *Synechocystis* these tools are limited (Koksharova and Wolk 2002, Huang, Camsund et al. 2010, Taton, Unglaub et al. 2014). Commonly used tools such as replicative vectors based on *E. coli* plasmids have some disadvantages in *Synechocystis* resulting in them exhibiting some instability.

The preferred approach for *Synechocystis* is for non-replicating suicidal integrative vectors to be used to implement new functionalities by integrating ectopic DNA into the genome, through homologous recombination (Williams 1988). However, the choice of the integration site is critical for use of the organism for biomanufacturing; such loci are required to be 'neutral' in that integration of ectopic DNA into these sites must not affect cellular viability or cause any distinguishable phenotype. A few 'neutral' integration sites in the *Synechocystis* genome have been reported in the literature, for example, to complement knockout mutants (Williams 1988, Burnap, Qian et al. 1994), to test promoters and study transcription regulation (Aoki, Kondo et al. 1995, Kunert, Hagemann et al. 2000), or for the heterologous expression of proteins (Zang, Liu et al. 2007). Although those loci have been described as "not appear to be deleterious" (Aoki, Kondo et al. 1995), "does not otherwise affect any observable functions" (Burnap, Qian et al. 1994), "did not show any detectable phenotype" (Aoki, Goto et al. 2011), or being "a neutral site of the chromosome" (Kunert, Hagemann et al. 2000), they have not been properly characterized as neutral sites - loci that can be disrupted without affecting cellular viability or cause any distinguishable phenotype (Clerico, Ditty et al. 2007).

KUNERT A ET AL: "Construction of promoter probe vectors for Synechocystis sp. PCC 6803 using the light-emitting reporter systems Gfp and LuxAB.", JOURNAL OF I\/IICROBIOLOGICAL METHODS AUG 2000, vol. 41, no. 3, August 2000, and BENTLEY FIONA K ET AL: "Heterologous expression of the mevalonic acid pathway in cyanobacteria enhances endogenous carbon partitioning to isoprene.", MOLECULAR PLANT JAN 2014, vol. 7, no. 1, January 2014, both disclose sites for integration of ectopic or heterologous sequences into the Synechocystis genome, which sites are not known to be associated with phenotypic changes following integration.

There is a need therefore to identify advantageous neutral sites in the chromosome of *Synechocystis* to enable development of methods and tools for transforming *Synechocystis* so that new functionalities can be introduced. Furthermore, such developments would mean that *Synechocystis* could be exploited as a cost-effective biomanufacturing system.

These facts are disclosed in order to illustrate the technical problem addressed by the present disclosure.

**Brief description**

The current disclosure described herein employs the use of previously unidentified neutral sites into which foreign DNA can be integrated into the chromosome of *Synechocystis* without deleterious effects upon the growth and viability of the host cells. It is further described that foreign DNA may be integrated at such neutral sites so that *Synechocystis* expresses genes, which encode proteins that enable production of commercially important chemicals The identification of a number of neutral sites which are spread throughout the *Synechocystis* chromosome, enables implementation of more complex synthetic circuits i.e. multiple genes may be expressed.

New integrative vectors have been produced and are also disclosed herein which are compatible with widely-available standard tools e.g. BioBrick™ RFC[10] standard (http://www.biobricks.org/) which will make easier to use *Synechocystis* in synthetic biology approaches.

In the present disclosure, foreign DNA was inserted into the chromosome of *Synechocystis,* and the cyanobacteria was transformed to produce a number of model chemicals without affecting the growth and viability of the host cells. Disclosed herein is a method of transforming *Synechocystis* comprising selection of one or more of the identified neutral sites, construction of integrative plasmids that enable foreign DNA to be integrated into the *Synechocystis* chromosome at the neutral sites and generation of mutant strains of *Synechocystis* capable of bioproduction. In addition, the present disclosure provides for vectors for integration at the five different neutral sites.

Further, the present disclosure provides a method of expression of exogenous genes in *Synechocystis* sp. PCC 6803 through use of a T/A cloning vector that contains a synthetic interface used to insulate the multiple cloning sites where ectopic DNA should be cloned for insertion in *Synechocystis* chromosome, whereby the insulation interface is flanked by the two regions for double homologous recombination on one of the neutral sites (n).

The disclosure further comprises a method of constructing integrative plasmids to generate a series of plasmids (pSN*n*) based upon a T/A cloning vector that contains a synthetic insulation interface flanked by the two regions for double homologous recombination on one of the neutral sites (n).

The disclosure also provides a method of generating a series of plasmids (pSNnKS) by cloning a PCR-amplified kanamycin resistance/sucrose sensitivity cassette, upstream of the insulation interface of each pSN*n* plasmid. The pSN*n*KS plasmids series are used to produce *Synechocystis* mutants that will allow the insertion of ectopic DNA cloned into the pSN*n* plasmids series (delivery vectors), resulting in mutants without selection markers.

The present disclosure relates to a genetic construct for the introduction of ectopic DNA in a cyanobacterium host cell, in particular *Synechocystis,* comprising two sequences selected from
an upstream and a downstream chromosomal regions of a locus in a cyanobacterium wherein the locus is selected from the following list: SEQ ID No 106, SEQ ID No 107, SEQ ID No 108, SEQ ID No 109, SEQ ID No 110,
and the sequences length vary between 400-1500 bp, preferably 430-800 bp, more preferably 430-560 bp.

In an embodiment, the genetic construct may comprise one of the following sequences: SEQ ID No 111, SEQ ID No 112, SEQ ID No 113, SEQ ID No 114, SEQ ID No 115.

In an embodiment, the genetic construct may further comprise the SEQ ID NO: 26.

The present disclosure also relates to a vector for the introduction of a genetic construct for the production of a target molecule in a cyanobacterium host cell comprising the genetic construct previously described.

In an embodiment, the vector for the introduction of a genetic construct for the production of a target molecule in a cyanobacterium host cell comprising the genetic construct previously described may further comprise a gene selected from the following list: a gene coding for antibiotic resistance, a gene coding for levansucrase, a gene coding for green fluorescence protein, in particular a gene coding for antibiotic kanamycin resistance.

In an embodiment, said vector may further comprise a constitutive promotor, preferably SEQ ID No 20.

In an embodiment, said vector is an integrative vector.

In an embodiment, said vector may comprise a gene or genes for coding in a host cell: an enzyme or enzymes for production of a target molecule, or a precursor of a target molecule or a target molecule.

In an embodiment, the genetic construct leads to the production of at least a precursor, an enzyme or the target molecule of the following list: a compatible solute, an organic compound, a polymer, an alcohol, a biofuel, an additive, an adhesive, a sealant, a pigment, an antibiotic, a medicament, a protein.

In an embodiment, the genetic construct encodes enzymes for the production of a compatible solute selected from the following list: ectoine, hydroxyectoine, or mixtures thereof.

In an embodiment, the genetic construct encodes enzymes for the production of the organic compound isoprene, glucoglycerol, betaine, or mixtures thereof.

In an embodiment, the genetic construct encodes proteins/enzymes for the production of the pigment phycocyanin.

The present disclosure also relates to a cyanobacterium host cell comprising the genetic construct now disclosed or comprising the vector now disclosed.

In an embodiment, the cyanobacterium host cell is *Synechocystis,* in particular *Synechocystis* sp. PCC6803.

This disclosure further relates to the use of a genetic construct comprising:

two sequences selected from an upstream and downstream chromosomal regions of a locus in a cyanobacterium wherein

the locus is selected from the following list: SEQ ID No 106 - N5 is *sll1476,* SEQ ID No 107 -N8 is *slr0573,* SEQ ID No 108 - N10 is *slr 1396*, SEQ ID No 109 -- N15 is slr0271, SEQ ID No 110 - N16 is slr0397,

and the sequences length vary between 400-1500 bp, preferably 430-800 bp, more preferably 430-560 bp,

as an enhancer of the production of an enzyme for production of the target molecule, or a precursor of a target molecule or a target molecule in a cyanobacterium host cell.

This disclosure also relates to a method for the expression of an enzyme for production of the target molecule, or a precursor of a target molecule or a target molecule with the following steps:

constructing a genetic construct as described in claim 1 - 3;

cloning the genetic construct in an appropriated vector;

cloning the genetic construct for the expression of the precursor, the enzyme or the target molecule, in the vector and obtaining a recombinant vector;

transforming *Synechocystis* host cell with the recombinant vector;

expressing the gene or genes for the production of a precursor, of the enzyme or of the target molecule.

This method is particularly relevant in the production of chemical produts, namely in the cosmetic industry or pharmaceutical industry.

Throughout the description and claims the word "comprise" and variations of the word, are not intended to exclude other technical features, additives, components, or steps. Additional objectives, advantages and features of the solution will become apparent to those skilled in the art upon examination of the description or may be learned by practice of the solution.

**Brief description of the drawings**

For a better understanding of the disclosure, the attached figures are joined, which represent preferred embodiments of the disclosure. The figures provide preferred embodiments for the present disclosure and should not be seen as limiting the scope of the disclosure.

**Fig. 1:** Chromosomal location and genomic context of the *Synechocystis'* putative neutral sites that were initially considered for transcriptional analysis. (**a**) In bold and marked with an asterisk are the sites chosen for mutants construction; the other sites fulfilled the neutral sites initial selection criteria, but were subsequently disregarded after additional analyses. (**b**) Genomic context of each putative neutral site (5,000 bp). Black - ORFs corresponding to the neutral sites, Gray - ORFs encoding proteins with assigned functions. tp - putative transposase, ' - partial ORFs.

**Fig. 2:** *Synechocystis* growth curves, sample collection and transcription analysis of eight selected loci (N5, N6, N7, N8, N10, N14, N15, N16). (**a**) Growth curves of three independent cultures of *Synechocystis* with sample collection points for RNA extraction indicated by arrows (1 - $OD_{730}$≈0.4, 2 - $OD_{730}$≈1.9,3 - $OD_{730}$≈9.0). (**b**) RT-PCR transcription analysis using total RNA extracted from cells collected at the time points indicated in **a**. These results are representative of three biological replicates and technical duplicates. - negative control (absence of template), + positive control (genomic DNA), and M - GeneRuler DNA ladder (Thermo Fisher Scientific).

**Fig. 3:** Growth curves of *Synechocystis* wild-type and mutants in the five neutral sites (SN5K, SN8K, SN10K, SN15K, SN16K) cultivated under different conditions. 10 mL of culture were grown in 25 mL flasks at 30 °C and $OD_{730}$ values were recorded daily for 10 consecutive days in continuous light (CL) or 12 h light (20 μmol photons/$m^2$/s)/12 h dark cycles (LD), in autotrophy (GO) or mixotrophy (5 mM glucose, G5).

**Fig. 4:** Detection of GFP expression in *Synechocystis* wild-type (WT) and insertion mutants containing a promoterless GFP generator (SN*n*K.gfp) or the GFP generator under the control of the constitutive minimal $P_{psba2*}$ synthetic promoter (SN*n*K.Cgfp). (**a**) Confocal micrographs of *Synechocystis:* autofluorescence is depicted in the left column, GFP signal in the middle column and the merged signals in the right column. (**b**) Normalized fluorescence of the cultures analyzed in **a.** Measurements were performed in triplicate, using 200 μL of each culture, and fluorescence was normalized to $OD_{790}$. Normalized fluorescence from the wild-type was used as baseline. Bars indicate mean ± SD.

**Fig. 5:** Normalized fold expression of *gfp* (RT-qPCR) in *Synechocystis* mutants harboring a synthetic device with *gfp* under the control of a synthetic constitutive promoter. WT - wild type (control). Data from 3 biological replicates and 3 technical replicates were normalized against three reference genes (16S, *petB,* and *rnpB*) and error bars represent ± SEM.

**Fig. 6:** Chromosomal location and genomic context of the five *Synechocystis'* neutral sites. (**a**) These sites were chosen for mutants construction. (**b**) Genomic context of each of the five neutral sites (5,000 bp). Show in black are the ORFs corresponding to the neutral sites.

**Fig. 7:** List of the 13 putative neutral sites initially identified and then subjected to further analysis. The five neutral sites that comprise this disclosure are identified as being N5, N8, N10, N15 and N16. * d - direct sequence, c - complement sequence.

**Fig. 8:** RT-PCR transcription analysis of thirteen loci for the putative neutral sites (N1, N3, N5, N6, N7, N8, N10, N11, N12, N13, N14, N15, N16). *Synechocystis* wild-type samples for RNA extraction were collected at OD730 ≈ 0.8-0.9. These results are representative of the three biological triplicates and technical duplicates. c - cDNA, - negative control (absence of template), + positive control (genomic DNA), M - GeneRuler DNA ladder (Thermo Fisher Scientific).

**Fig. 9:** List of Forward and Reverse Primers used for transcriptional analysis using RT-PCR of the 13 putative neutral sites

**Fig. 10:** List of other primers used in the present disclosure. *Restriction sites underlined; **Primers used to confirm genomic integrations and mutants full segregation; ***SDM - Site directed mutagenesis.

**Fig. 11:** List of all the mutant organisms and plasmids used or generated in this work

**Fig. 12:** List of plasmids constructed in this work. A range of plasmids were constructed for each of the 5 neutral sites.

**Fig. 13:** Schematic representation of a plasmid (pSN10K.Cgfp) that was constructed in respect of the neutral site identified as N10. Homologous regions flanking the neutral site were amplified by PCR (primers indicated by arrows) and fused by overlap-PCR (1). The fragment containing the two flanking regions was cloned into the pGEM-T easy vector; the restriction sites incompatible with the BioBrick standard RFC[10] were removed by digestion with *Xho*I and *Sal*I and vector re-circularization, and by site directed mutagenesis (SDM, mutagenic primers indicated by a crossed arrow) (2). The BioBrick-compatible cloning interface, flanked by two double BioBrick transcription terminators (TT), was synthesized and cloned into the *Xma*I site of the vector originating the pSN10 plasmid (3). A selection cassette conferring resistance to kanamycin was amplified by PCR (primers indicated by arrows) from the plasmid pK18mobsacB and cloned into the *Xma*I site (pSN10K) (4). Subsequently, the module containing the GFP encoding gene under the control of the minimal *psbA2* promoter ($P_{psA2*}$) was excised from the BioBrick vector pSB1A2-Cgfp and cloned into the cloning interface, originating the integrative plasmid pSN10K.Cgfp (5).

**Fig. 14:** ANOVA analysis of *Synechocystis* wild-type growth compared to mutants (SN*n*K mutants) into which a kanamycin resistance cassette is inserted into each of the five neutral sites. *P*-values of up to second order interactions produced by a three-way ANOVA analysis

**Fig. 15:** List of *Synechocystis* mutants constructed in the present disclosure.

**Fig. 16:** Detection of *gfp* transcripts and GFP in *Synechocystis* mutants harboring a promoterless GFP synthetic device (SN*n*K.gfp) or a device with *gfp* under the control of the synthetic constitutive promoter $P_{psba2*}$ (SN*n*K.Cgfp). *gfp* transcription was assessed by RT-PCR (**a**) and GFP expression was assessed by Western blot (**b**). Wild-type was included as control. M - GeneRuler DNA ladder (Thermo Fisher Scientific); C + - Positive control (wild-type gDNA for 16S rRNA and pSB1A2-E0240 for *gfp*); C - - Negative control (no template); - RT - PCRs performed using RNA as template; + RT - PCRs performed using cDNA as template.

**Fig. 17:** Detection of GFP expression in *Synechocystis* mutants harboring a promoterless GFP synthetic device (SNnK.gfp) or a device with *gfp* under the control of a synthetic constitutive promoter (SNnK.Cgfp). Total cell fluorescence was measured 2, 4, 7, 9 and 11 days after inoculation, using 200 μL of three independent culture replicates. Measurements were performed in triplicate and fluorescence was normalized to $OD_{790}$. Normalized fluorescence from the wild-type was used as baseline. Bars indicate mean ± SD.

**Fig. 18:** PCR confirmation of the full segregation of the *Synechocystis* mutants in the five neutral sites (N5, N8, N10, N15, N16). Schematic representation of the position of the primers used for the wild-type and mutants, exemplified for N10 (**a**). PCR reactions were performed using primers within the ORF corresponding to each neutral site (**b**), a primer external to each site and a primer within the selection cassette (**c**), primer amplifying the kanamycin resistance cassette (**d**), primers amplifying the within the gfp gene (**e**) or primers amplifying the double selection cassette (**f**) (listed in **Fig. 10**). No template controls were always included (c-) and the expected band sizes are within brackets. M - GeneRuler DNA ladder (Thermo Fisher Scientific).

**Fig. 19:** Southern blot confirmation of the full segregation of the *Synechocystis* mutants in the five neutral sites (N5, N8, N10, N15, N16). Expected band sizes are within brackets.

**Fig. 20:** Phenotypic characterization of mutants harboring the double selection cassette conferring kanamycin resistance and sucrose sensitivity (SNnKS). *Synechocystis* wild-type and mutants were grown in agar plates containing BG11 medium (BG11), BG11 medium supplemented with 100 μg/mL kanamycin (BG11 + $Km^{100}$) and BG11 medium supplemented with 5% (m/v) sucrose (BG11 + Suc 5%).

**Fig. 21:** Plasmid map of pSN5 (seq ID NO 1).

**Fig. 22:** Plasmid map of pSN8 (seq ID NO 2).

**Fig. 23:** Plasmid map of pSN10 (seq ID NO 3).

**Fig. 24:** Plasmid map of pSN15 (seq ID NO 4).

**Fig. 25:** Plasmid map of pSN16 (seq ID NO 5).

**Fig. 26:** Plasmid map of pSN5K (seq ID NO 6).

**Fig. 27:** Plasmid map of pSN8K (seq ID NO 7).

**Fig. 28:** Plasmid map of pSN10K (seq ID NO 8).

**Fig. 29:** Plasmid map of pSN15K (seq ID NO 9).

**Fig. 30:** Plasmid map of pSN16K (seq ID NO 10).

**Fig. 31:** Plasmid map of pSN5KS (seq ID NO 11).

**Fig. 32:** Plasmid map of pSN8KS (seq ID NO 12).

**Fig. 33:** Plasmid map of pSN10KS (seq ID NO 13).

**Fig. 34:** Plasmid map of pSN15KS (seq ID NO 14).

**Fig. 35:** Plasmid map of pSN16KS (seq ID NO 15).

**Description of the sequences**

SEQ ID NO: 1: Nucleotide sequence of plasmid for N5 neutral site (pSN5).
SEQ ID NO: 2: Nucleotide sequence of plasmid for neutral site N8 (pSN8).
SEQ ID NO: 3: Nucleotide sequence of plasmid for neutral site N10 (pSN10).
SEQ ID NO: 4: Nucleotide sequence of plasmid for neutral site N15 (pSN15).
SEQ ID NO: 5: Nucleotide sequence of plasmid for neutral site N16 (pSN16).
SEQ ID NO: 6: Nucleotide sequence of plasmid for neutral site N5 containing Kanamycin resistance cassette (pSN5K).
SEQ ID NO: 7: Nucleotide sequence of plasmid for neutral site N8 containing Kanamycin resistance cassette (pSN8K).
SEQ ID NO: 8: Nucleotide sequence of plasmid for neutral site N10 containing Kanamycin resistance cassette (pSN10K).
SEQ ID NO: 9: Nucleotide sequence of plasmid for neutral site N15 containing Kanamycin resistance cassette (pSN15K).
SEQ ID NO: 10: Nucleotide sequence of plasmid for neutral site N16 containing Kanamycin resistance cassette (pSN16K).
SEQ ID NO: 11: Nucleotide sequence of plasmid for neutral site N5 used to produce *Synechocystis* mutants without selection markers (pSN5KS).
SEQ ID NO: 12: Nucleotide sequence of plasmid for neutral site N8 used to produce *Synechocystis* mutants without selection markers (pSN8KS).
SEQ ID NO: 13: Nucleotide sequence of plasmid for neutral site N10 used to produce *Synechocystis* mutants without selection markers (pSN10KS).
SEQ ID NO: 14: Nucleotide sequence of plasmid for neutral site N15 used to produce *Synechocystis* mutants without selection markers (pSN15KS).
SEQ ID NO: 15: Nucleotide sequence of plasmid for neutral site N16 used to produce *Synechocystis* mutants without selection markers (pSN16KS).
SEQ ID NO: 16: Nucleotide sequence of T/A cloning vector (pGEM-T easy).
SEQ ID NO: 17: Nucleotide sequence of PCR amplified kanamycin resistance selection cassette (Km).
SEQ ID NO: 18: Nucleotide sequence of PCR amplified double selection cassette (KmSc).
SEQ ID NO: 19: Nucleotide sequence of GFP generator (BBa_E0840).
SEQ ID NO: 20: Nucleotide sequence of synthetic constitutive promoter (PpsbA2*).
SEQ ID NO: 21: Nucleotide sequence of recombination sequence for site N5 obtained by overlap PCR (N5.OL).
SEQ ID NO: 22: Nucleotide sequence of recombination sequence for site N8 obtained by overlap PCR (N8.OL).
SEQ ID NO: 23: Nucleotide sequence of recombination sequence for site N10 obtained by overlap PCR (N10.OL).
SEQ ID NO: 24: Nucleotide sequence of recombination sequence for site N15 obtained by overlap PCR (N15.OL).
SEQ ID NO: 25: Nucleotide sequence of recombination sequence for site N16 obtained by overlap PCR (N16.OL).
SEQ ID NO: 26: Nucleotide sequence of Bio-Brick compatible cloning interface (FP300).
SEQ ID NO: 27: Nucleotide sequence for DABA acetyltransferase (EctA) of *C. salexigens.*
SEQ ID NO: 28: Nucleotide sequence for diaminobutyric acid (DABA) aminotransferase (EctB) of *C. salexigens.*
SEQ ID NO: 29: Nucleotide sequence for ectoine synthase (EctC) of *C. salexigens.*
SEQ ID NO: 30: Nucleotide sequence for ectoine hydroxylase (EctD) of *C. salexigens.*
SEQ ID NO: 31: Nucleotide sequence for DABA acetyltransferase (EctA) of *M. alcaliphilum.*
SEQ ID NO: 32: Nucleotide sequence for diaminobutyric acid (DABA) aminotransferase (EctB) of M. *alcaliphilum.*
SEQ ID NO: 33: Nucleotide sequence for ectoine synthase (EctC) of *M. alcaliphilum.*
SEQ ID NO: 34: Nucleotide sequence for ectoine hydroxylase (EctD) of *M. alcaliphilum.*
SEQ ID NO: 35: Nucleotide sequence for aspartate kinase isozyme (Ask) of *M. alcaliphilum.*

**Primer Sequences for Transcriptional Analysis of the Putative Neutral Sites**

| Sequence ID Number | Primer name | Sequence 5' → 3' | Amplicon size (bp) |
|---|---|---|---|
| SEQ ID NO: 36: | N1F | CATGGATTGAAGGAAGGG | 100 |
| SEQ ID NO: 37: | N1R | TTTGGTGAACAATTAATGAAC | |
| SEQ ID NO: 38: | N3F | GCAGTCCCTCCTCCAAAC | 129 |
| SEQ ID NO: 39: | N3R | AAGCCGATATTCAGGTAAGATG | |
| SEQ ID NO: 40: | N5F | GGAAGTGTTGTTGCTGTC | 232 |
| SEQ ID NO: 41: | N5R | GTCCTTCGCCGTAGATTG | |
| SEQ ID NO: 42: | N6F | AAGGTAATCAAGCAGAAATG | 217 |
| SEQ ID NO: 43: | N6R | ATTGGTAACAGCACTTCC | |
| SEQ ID NO: 44: | N7F | TGACTGTAAGCAATCTACC | 500 |
| SEQ ID NO: 45: | N7R | ACTAACGATGGAATCTTGG | |
| SEQ ID NO: 46: | N8F | AACTTAACTTCTATTCGTGAG | 231 |
| SEQ ID NO: 47: | N8R | GAAACCTTGATAAGCAGTC | |
| SEQ ID NO: 48: | N10F | GATATTGATGTGTATTGC | 478 |
| SEQ ID NO: 49: | N10R | TTATTATCTTCTGTCTCC | |
| SEQ ID NO: 50: | N11F | TGAATACCACATCTCCCATTGAC | 200 |
| SEQ ID NO: 51: | N11R | TTTAACCTGCTGGCGTGAC | |
| SEQ ID NO: 52: | N12F | TTCTCACCGCTACTGTTAC | 227 |
| SEQ ID NO: 53: | N12R | CACTTCTCGCACTAATTCG | |
| SEQ ID NO: 54: | N13F | CCCAAATGATGACCGAAG | 216 |
| SEQ ID NO: 55: | N13R | CTAGTAACTGCTGTTCCTC | |
| SEQ ID NO: 56: | N14F | AAGTGTCCAAAGCCAAAC | 270 |
| SEQ ID NO: 57: | N14R | TCAACAATGCCATCTTCC | |
| SEQ ID NO: 58: | N15F | TTAATTGCCACTGCCTTAC | 293 |
| SEQ ID NO: 59: | N15R | TAGAGACAGCGGATATGC | |
| SEQ ID NO: 60: | N16F | TGATGTGGACGAGGATAC | 335 |
| SEQ ID NO: 61: | N16R | GGGCTAAGGGAATATGGG | |

**Other Primer Sequences**

| Sequence ID Number | Primer name | Sequence 5' → 3'* | Purpose |
|---|---|---|---|
| SEQ ID NO: 62: | Km.KmScFwd | CTGACCCCGGGTGAATGTCAGCTACTGG | Selection cassettes amplification |
| SEQ ID NO: 63: | KmRev | CAAACCCGGGCGATTTACTTTTCGACCTC | |
| SEQ ID NO: 64: | KmScRev | ACAGACCCGGGCAAGCGGATGGCTGATG | |

(continued)

| Sequence ID Number | Primer name | Sequence 5' → 3'* | Purpose |
|---|---|---|---|
| SEQ ID NO: 65: | N5.50** | GCGGCCTCGAGTGGGGGCATCTGCTAGGCAA TATTTG | Amplification of the DNA fragments flanking the neutral sites |
| SEQ ID NO: 66: | N5.5I | GATTACACCCGGGTGTAATCGGTTGATTATTT CAGTGGCCCGGCCGATGGATAATTAC | |
| SEQ ID NO: 67: | N5.3O** | GTCCAAATCAGCATTGCTCTGCCAGGTGAGAC | |
| SEQ ID NO: 68: | N5.3I | GATTACACCCGGGTGTAATCCTGAAGCCTTGA CAATCCCCGTTGGTGATTTATACTTAG | |
| SEQ ID NO: 69: | N8.5O** | GAACACTCGAGTGCCAGCAACGACAATG | |
| SEQ ID NO: 70: | N8.5I | GATTACACCCGGGTGTAATCCTTCATTCCCCTA TTGTTATTAATAGTGCTC | |
| SEQ ID NO: 71: | N8.3O** | GATGACCGCTGGCGGAGTTTAGTCCAG | |
| SEQ ID NO: 72: | N8.3I | GATTACACCCGGGTGTAATCTTGTCACCAATT TTTGTAGGGATGTTGGGCTAAATTG | |
| SEQ ID NO: 73: | N10.5O** | GATAACTCGAGTCGCCCGGTGGATTTAATGC | |
| SEQ ID NO: 74: | N10.5I | GATTACACCCGGGTGTAATCCCCATACTCAGC CTTCTAATGCTGAGAGAATG | |
| SEQ ID NO: 75: | N10.3O** | GGACTGACCCAAGATACAGTGGTAG | |
| SEQ ID NO: 76: | N10.3I | GATTACACCCGGGTGTAATCCGAGCGCAAACT ACAATGCGCTTCGTTTGC | |
| SEQ ID NO: 77: | N15.5O** | GGTTCTCGAGCCGCTGAATTTAGTCAACATCG | |
| SEQ ID NO: 78: | N15.51 | GATTACACCCGGGTGTAATCCATTGGGCACGA GAGTTAGTAAGGCAGTG | |
| SEQ ID NO: 79: | N15.3O* | CTAAACTTACGGCATTGGCATCAACGGGAG | |
| SEQ ID NO: 80: | N15.3I | GATTACACCCGGGTGTAATCTCTTTACAATGG CCAGGTCTTTAGGGAGCGGTGAC | |
| SEQ ID NO: 81: | N16.5O** | GAACTCGAGTAGTAACCACAGGCTTTTG | |
| SEQ ID NO: 82: | N16.5I | GATTACACCCGGGTGTAATCGCTGGAGGCGA ACTGGGTGAGAACCAT | |
| SEQ ID NO: 83: | N16.30** | GTGAGCTTGATGGTGATGGTGGGTAAAG | |
| SEQ ID NO: 84: | N16.3I | GATTACACCCGGGTGTAATCTGCTGGCTTTGT TGCCCTGTCAACCAAAGTTC | |
| SEQ ID NO: 85: | N5_SDM | CGAGGCGATCGCCCAGTTGGAAGAATTGGCC | SDM*** |

(continued)

| Sequence ID Number | Primer name | Sequence 5' → 3'* | Purpose |
|---|---|---|---|
| SEQ ID NO: 86: | N10_SDM | CTAAAAAGACAAGTCTGTGGCTAGTTACTATG ACGAGGC | SDM*** |
| SEQ ID NO: 87: | N5.FO | TCCTGGTAACTCACGCTATC | Mutants confirmation by PCR |
| SEQ ID NO: 88: | N5.FI | AGCCGATCCAGGGAAGTGTTGTTG | |
| SEQ ID NO: 89: | N5.RI | CCATCGTCCTTCGCCGTAGATTGTG | |
| SEQ ID NO: 90: | N8.FO | CCCAGTTAAACTGCGAAAGG | |
| SEQ ID NO: 91: | N8.FI | TCGCCAAGCTTTCAGAAC | |
| SEQ ID NO: 92: | N8.RI | CAAACTCCAGCCGATAAC | Mutants confirmation by PCR |
| SEQ ID NO: 93: | N10.FO | CCGGTTGCCCTTATCGGAACCGATG | |
| SEQ ID NO: 94: | N10.FI | GCTATGGCGTCACTTGTAGC | |
| SEQ ID NO: 95: | N10.RI | TTTGCGACCCATCGGATTGC | |
| SEQ ID NO: 96: | N15.FO | CTCCAAGGCGACTACCTTTC | |
| SEQ ID NO: 97: | N15.FI | CCCAGTGGGAATGCGATCAG | |
| SEQ ID NO: 98: | N15.RI | TAGGAGGGCGATCACCGAAG | |
| SEQ ID NO: 99: | N16.FO | ACCCATTTCTTGGGTGTAGG | |
| SEQ ID NO: 100: | N16.FI | GGCCTTGGTTGCCCTGACTGATGTG | |
| SEQ ID NO: 101: | N16.RI | GACCGATCGCCGCAGTAGTTCTTGG | |
| SEQ ID NO: 102: | GFP.F | TCTTGTTGAATTAGATGGTG | RT-PCR/RT-qPCR |
| SEQ ID NO: 103: | GFP.R | TGTGAGTTATAGTTGTATTCC | |
| *Restriction sites underlined<br>**Primers used to confirm genomic integrations and mutants full segregation.<br>***SDM - Site directed mutagenesis | | | |

SEQ ID NO: 104: Nucleotide sequence for BioBrick part BBa_B0030.
SEQ ID NO: 105: Nucleotide sequence for Ptrc1O (promoter, BioBrick part BBa_J153001).
SEQ ID NO: 106: Nucleotide sequence for N5 (sll1476)
SEQ ID NO: 107: - Nucleotide sequence for N8 (slr0573)
SEQ ID NO 108 - Nucleotide sequence for N10 (slr1396)

SEQ ID NO 109 - Nucletotide sequence for N15 (slr0271)
SEQ ID NO 110 - Nucletotide sequence for N16 (slr0397)
SEQ ID No 111 - Nucletotide sequence for genetic construct comprising the upstream and downstream sequences of site N5 (SEQ ID NO 106)
SEQ ID No 112 - Nucletotide sequence for genetic construct comprising the upstream and downstream sequences of site N8 (SEQ ID NO 107)
SEQ ID No 113- Nucletotide sequence for genetic construct comprising the upstream and downstream sequences of site N10 (SEQ ID NO 108)
SEQ ID No 114 - Nucletotide sequence for genetic construct comprising the upstream and downstream sequences of site N15 (SEQ ID NO 109)
SEQ ID No 115 - Nucletotide sequence for genetic construct comprising the upstream and downstream sequences of site N16 (SEQ ID NO 110)

**Detailed description**

*Synechocystis* is a well-studied cyanobacterium with some molecular tools developed for its genetic manipulation. Replicative plasmids have previously been used to introduce foreign DNA into this organism *via* conjugation or electroporation (Matsunaga and Takeyama 1995, Vioque 2007). These plasmids are usually based on the broad-host-range *E. coli* IncQ plasmid RSF1010, and are not specific for cyanobacteria (Scholz, Haring *et al.* 1989, Sode, Tatara *et al.* 1992, Mermet-Bouvier, Cassier-Chauvat *et al.* 1993, Huang, Camsund *et al.* 2010, Taton, Unglaub *et al.* 2014). Although presenting some advantages, such as being relatively easy to construct and quick to introduce in the cell, such broad-host-range replicative plasmids can exhibit some instability (David, Vielma et al. 1983, Priefer, Simon et al. 1985, Becker and Meyer 1997, De Gelder, Ponciano et al. 2007, Meyer 2009). In addition, (*i*) the use of large native DNA fragments may lead to the recombination of the replicative vectors with the chromosome, (*ii*) removal of selective pressure may lead to loss of the vector, and (*iii*) DNA structure of the vectors may have a strong influence on transcription. Altogether these aspects can compromise system predictions and control. In addition, it has been shown that *Synechocystis'* endogenous plasmids copy number per chromosome vary with nutritional conditions and growth phase (Berla and Pakrasi 2012). Taking into consideration that the number of chromosomes also varies throughout growth (Griese, Lange *et al.* 2011), plasmids copy number calculations may become even more complex. Therefore, stable integration of ectopic DNA into *Synechocystis'* chromosome enabled by this disclosure is the most viable alternative for the implementation of synthetic devices into this photoautotrophic chassis.

To date, only a few chromosomal loci have been used to introduce foreign DNA into *Synechocystis* but they have not been extensively characterized. The locus described by Williams and co-workers (Williams 1988) disrupts the gene *slr0168* that encodes a protein secreted by *Synechocystis* (Sergeyenko and Los 2000). Although its function is unknown, Slr0168 possesses a DUF4114 protein domain, found towards the C-terminal of various bacterial proteins, and suggested to carry enzymatic activity (Marchler-Bauer, Anderson *et al.* 2009). Burnap and co-workers (Burnap, Qian *et al.* 1994) describe a locus in a "nonessential, nontranscribed" region. However, recent RNA-seq works (Mitschke, Georg *et al.* 2011, Kopf, Klähn *et al.* 2014) show that the genes flanking that insertion locus are transcribed. The locus described by Aoki and co-workers (Aoki, Kondo *et al.* 1995) disrupts the *ssl0410* gene, which is located downstream of the gene encoding a subunit of the type I NAD(P)H dehydrogenase complex (*ndhB*) that is important for $CO_2$ uptake in *Synechocystis* (Ohkawa, Price *et al.* 2000). The other locus described by Aoki and co-workers (Aoki, Goto *et al.* 2011) is located in the intergenic region between *slr2030* and *slr2031,* which encodes the regulatory protein RsbU. The latter gene is constitutively transcribed and the respective mutant showed a defect in growth recovery after nitrogen- and sulfur-starvation-induced stationary phase (Huckauf, Nomura *et al.* 2000). Furthermore, *slr2030* and *slr2031* are transcribed as an operon (Huckauf, Nomura *et al.* 2000, Mitschke, Georg *et al.* 2011, Kopf, Klähn *et al.* 2014). Consequently, insertions in that intergenic region may disrupt the transcriptional unit and lead to the same phenotype described for *slr2031* mutants. None of these previously described integration sites are considered to be suitable for use of *Synechocystis* as an effective bioproduction system. Alternative and more advantageous integration loci are employed in this disclosure, described herein.

The method of transforming *Synechocystis* without deleterious effects on cell growth and viability has been enabled by the discovery of five neutral sites into which DNA can be stably integrated into the *Synechocystis* chromosome. In selecting a neutral site, the obvious choice would have been to choose non-coding DNA (ncDNA) regions, however it was further considered that these may possess *cis*-regulatory control sequences, which could affect the transcription profiles of the inserted genes. Therefore Open Reading Frames (ORF) that putatively encode unknown or hypothetical proteins were selected. A total of 16 candidate neutral sites were initially selected, with 13 of these selected for further transcriptional studies.

The cyanobacteria (also known as blue-green algae) in this disclosure preferably comprise a group of prokaryotic microorganisms capable of performing plant type oxygenic photosynthesis. Examples of cyanobacteria include transformable strains of the following strains: *Synechocystis* spp., *Synechococcus* spp., *Microcystis aeruginosa, Prochlorococcus marinus* and *Nostoc* spp.

The use of cyanobacteria for bioproduction may have the following advantages: (1) cyanobacteria are capable of absorbing solar energy and fixing carbon dioxide as carbon source for autotrophic growth, thereby having low cost for culturing; (2) cyanobacteria are ancient microorganisms and have lived on the earth for billions of years, so that they have remarkable adaptability to the environments, and they grow quickly compared to other autotrophic organisms; (3) cyanobacteria are convenient for genetic manipulations, because their genetic background is clear and genomic sequencing of many species of cyanobacteria has been completed which facilitates the genetic engineering of cyanobacteria. *Synechocystis* sp. PCC 6803 is a preferred unicellular cyanobacteria, because for *Synechocystis* sp. PCC 6803 the whole genome has been sequenced, plenty of molecular tools and genome-scale metabolic models are available.

The embodiments of the disclosure provide for a method of transforming *Synechocystis* that can be used to efficiently biomanufacture or bioproduce a range of chemical products. Biomanufacturing includes a whole range of industrial sectors and products that are produced using microbes; including the bioprocessing of proteins and peptides for therapeutic uses, biorefining to convert biomass into fuels, bioremediation to treat environmental waste and the use of microbial fermentation to produce commodity and specialty chemicals.

Further embodiments of the disclosure provide for a method of transforming *Synechocystis* that can be used to bioproduce 'drop-in' chemicals that can be used as direct replacements for petrochemical-based feedstocks. Such products include bioisoprene, a microbially-produced version of isoprene which is used predominantly in the tyre industry as a replacement for rubber. Isoprene is a petrochemical, usually produced as a by-product from the cracking of naphtha (a crude oil product) to produce ethylene or via C4 refinery stream synthesis. However, this requires a lot of crude oil and a greater move towards use of shale gas (ethane) for ethylene production means that the required by-products are no longer produced, resulting in a shortage of isoprene. The high costs and shortages of isoprene have motivated the tyre industry to develop ways to produce isoprene using different feedstocks e.g. biologically-produced isoprene using C5 sugars from renewable sources such as sugar cane or corn. Microbial production in this industry sector has solved the problem of reduced supplies and wildly fluctuating prices of a feedstock. Use of *Synechocystis* would provide for a more cost-effective means to bioproduce isoprene.

Another embodiment of the disclosure provides for a method of transforming *Synechocystis* that can be used to produce biofuels. Production of biofuels on a commercially viable scale has been fraught with difficulties. Examples include farnesene which is microbially-produced from sugar cane and which is converted to farnesane for use as jet-fuel. The major challenge faced by manufacturers has been development of a process that is cost-effective to scale up. Other examples include 'drop-in' fuels such as isobutanol which is used to replace ethanol as it contains more energy per equivalent volume than ethanol and it is less corrosive to pipelines. Currently, isobutanol and ethanol are mostly produced using yeast. Use of *Synechocystis* would provide for a more cost-effective means to bioproduce biofuels such as farnesene, isobutanol and ethanol.

A further embodiment of the disclosure provides for a method of transforming *Synechocystis* that can be used to produce specialty chemicals. Specialty chemicals are high value-added chemicals used in the manufacture of a wide variety of products, including but not limited to fine chemicals, additives, advanced polymers, adhesives, sealants and specialty paints, pigments and coatings. The main difference between specialty chemicals and commodity chemicals is that specialty chemicals are produced on a batch-to-batch basis, typically driven by specific demands from end-users.

Another embodiment of the disclosure provides for a method of transforming *Synechocystis* that can be used to produce phycocyanin. Phycocyanin is a cyanobacterial blue pigment and antioxidant that has applications in biotechnology (e.g. as a reporter dye) and in medicines and foods (as a colouring agent and antioxidant). Phycocyanin is currently commercially produced for use as a food dye in blue-coloured products like ice cream, confectionary and drinks. Phycocyanin is currently produced in *Spirulina plantensis,* another cyanobacterium.

A further embodiment of the disclosure provides for a method of transforming *Synechocystis* that can be used to produce ectoine and hydroxyectoine. Ectoine and hydroxyectoine are compatible solutes which help to protect extremophillic bacteria in high salt conditions, acting as osmolytes. Ectoine is used as a cosmetic ingredient as it it highly UV-protective and prevents skin dryness. However, ectoine cannot be chemically synthesised cost-effectively and at present is produced in halophillic bacteria e.g. *Halomonas elongata.*

Use of *Synechocystis* would potentially provide for the ability to increase the yield of bioproduced specialty chemicals.

Selection of neutral sites

In an embodiment, the selection of neutral sites was carried out as follows. To identify putative neutral sites in the chromosome of *Synechocystis,* the list of annotated open reading frames (ORFs) with the respective encoded proteins was retrieved from CyanoBase (http://genome.kazusa.or.jp/cyanobase). The list of the predicted/annotated ORFs of *Synechocystis* was retrieved from CyanoBase (Distribution of Sequence and Annotated Data Files, ftp://ftp.kazusa.or.jp/pub/CyanoBase/Synechocystis) (Nakamura, Kaneko et al. 1998, Nakamura, Kaneko et al. 2000). From the 3264 ORFs listed, only those putatively encoding unknown or hypothetical proteins were selected. From these, the final selection of 1532 ORFs was based on the following criteria: (*i*) length of the putatively encoded proteins ≤301 amino acids, (*ii*) encoded proteins with no transmembrane domains predicted by the TMHMM Server v. 2.0 (http://www.cbs.dtu.dk/services/TMHMM/), (*iii*) no interaction with other proteins as assessed by the two-hybrid system (CyanoBase data), and (*iv*) no relevant similarities found at the protein sequence level, when comparing with other sequences using BLASTP (http://blast.ncbi.nlm.nih.gov/Blast.cgi). The genomic context of the ORFs was also taken into consideration and sites were disregarded when in the vicinity of genes with assigned putative functions.

This analysis led to the selection of 16 candidate neutral sites - termed N1 to N16 (**Fig. 1** and **Fig 7**). Subsequently, a qualitative analysis of the genomic context of these ORFs was performed (**Fig. 1b**) and revealed that the N2 site superimposes the 5' region of an ORF (*slr0369*) putatively encoding a cation or drug efflux system protein, the N4 site overlaps the 5' region of an ORF (*slr0415*) putatively encoding a $Na^+/H^+$ antiporter, and the N9 site corresponds to the ORF located immediately upstream and in the same direction as an ORF (*sll0182)* putatively encoding an ABC-type transporter. Therefore, these 3 sites were disregarded, and transcription analyses were performed for the other 13 loci.

In an embodiment, the transcriptional analysis was carried out as follows. To assess transcription of the selected ORFs, primers were designed automatically from the genomic sequence (Kaneko, Sato et al. 1996) using the Beacon Designer 6 software (PREMIER Biosoft International). For other purposes, primers were designed manually and analyzed using the Integrated DNA Technologies web resource OligoAnalyzer v3.1 (http://eu.idtdna.com/analyzer/Applications/OligoAnalyzer/).

In an embodiment, to assess transcript levels of the identified loci, RT-PCRs were initially performed using RNA extracted from *Synechocystis* cells collected at the exponential growth phase ($OD_{730}$ of 0.8-0.9) under continuous light at 30 °C. Further transcriptional studies were performed with RNA extracted from samples collected in three different growth phases ($OD_{730}$ of approximately 0.4, 2 and 9, see **Fig. 2a**), from three independent cultures grown under the same conditions. Wild-type and mutants of the cyanobacterium *Synechocystis* sp. PCC 6803 were maintained in BG11 medium (Stanier, Kunisawa et al. 1971) at 25 °C, under a 16 h light /8 h dark regimen. Light intensity was 20 μmol photons/$m^2$/s in all experiments. For solid medium, BG11 was supplemented with 1.5% (w/v) agar noble (Difco), 0.3% (w/v) sodium thiosulfate and 10 mM TES-KOH buffer, pH 8.2. For the selection of mutants, BG11 medium was supplemented with kanamycin (Km, 10-600 μg/mL). For cloning purposes *Escherichia coli* strain DH5α (Stratagene) was used. *E. coli* cells were grown at 37 °C on LB medium (Sambrook and Russell 2001), supplemented with ampicillin (100 μg/mL) or kanamycin (50 μg/mL).

In an embodiment, the cyanobacterial genomic DNA (gDNA) extraction was carried out according to the procedure described previously (Tamagnini, Troshina et al. 1997). For RNA extraction *Synechocystis* cells were collected by centrifugation (10 min at 3850 g), and frozen at -80 °C. RNA was extracted using the TRIzol® Reagent (Invitrogen) according to the method described previously (Leitão, Pereira et al. 2006).

In an embodiment, RT-PCRs were carried out as described elsewhere (Ferreira, Pinto et al. 2009). Briefly, cDNA was synthesized in duplicate from 1 μg of total RNA using the iScript™ Select cDNA Synthesis Kit (Bio-Rad) and the random primers supplied, following the manufacturer's instructions. The two RT reactions from each sample were pooled together and each PCR reaction contained 1.5 μL of cDNA, 0.1 μM of each primer (see **Fig. 9**), 200 μM dNTPs mix, 1X PCR reaction buffer, and 0.025 U of illustra™ *Taq* DNA polymerase (GE Healthcare). The PCR profile was: 2 min at 94 °C followed by 40 cycles of 30 s at 94 °C, 30 s at 50 °C, and 30 s at 72 °C, concluding with a 7 min extension step at 72 °C. RNA contamination by gDNA prior to cDNA synthesis was assessed by PCR using primers specific for the *Synechocystis* 16S rDNA, and the RT reaction efficiency was assessed using the same primers pair. All the RT-PCRs included a negative control (omission of template) and a positive control (*Synechocystis* gDNA).

In an embodiment, PCRs to amplify the regions flanking the target loci contained 7 ng of gDNA, 0.2 μM of each primer, 250 μM dNTPs mix, 1X *Pfu* reaction buffer with $MgSO_4$, and 0.025 U of *Pfu* DNA polymerase (Fermentas). The PCR profile was: 1 min at 94 °C followed by 35 cycles of 30 s at 94 °C, 45 s at 48 °C, and 90 s at 72 °C, concluding with a 7 min extension step at 72 °C. Agarose gel electrophoresis was performed by standard protocols using 1× TAE buffer (Sambrook and Russell 2001), DNA fragments were purified using the GFX™ PCR DNA and Gel Band Purification Kit (GE Healthcare), according to the manufacturer's instructions. "Overlap-PCRs" (see below) contained 80 ng of each purified DNA fragment, 0.125 μM of each primer, 250 μM dNTPs mix, 1X PCR reaction buffer, and 0.025 U of illustra™ *Taq* DNA polymerase (GE Healthcare). The PCR profile was: 5 min at 95 °C followed by 10 cycles of 30 s at 95 °C, 45 s at 48 °C, and 90 s at 72 °C, and 30 cycles of 30 s at 95 °C, 45 s at 55 °C, and 90 s at 72 °C, concluding with a 7 min extension step at 72 °C. The oligonucleotide primers used to amplify the homologous regions are listed in **Fig. 10.**

The results revealed high transcript levels for N3, N11, N12 and N13 sites, low transcript levels for N6, N8, N14 and N15 sites, low primer efficiency for N1 site, and no detectable amplification for the remaining sites (N5, N7, N10 and N16) (**Fig. 8**). Further transcriptional studies were performed collecting cells at different phases (early exponential, exponential and stationary), to evaluate a possible differential transcription of sites N5, N6, N7, N8, N10, N14, N15 and N16 (**Fig. 2**). The results showed that there was residual transcription for N6 and N14 sites throughout the various growth phases and, therefore, these two sites were disregarded. Concerning the other sites, it was possible to detect residual transcription for N8 and N15 sites, but only on samples collected at the early exponential phase. Thus, six sites (N5, N7, N8, N10, N15 and N16) were selected to generate mutants in order to validate their "neutrality". Subsequent cloning difficulties in constructing N7-related plasmids resulted in this site also being disregarded. The final five target sites (N5, N8, N10, N15 and N16) are evenly distributed throughout the chromosome, with intervals in the order of 60-90º (72º corresponds to 1/5 of the chromosome, see **Fig. 1a**).

Construction of integrative plasmids

In an embodiment, the construction of integrative plasmids was carried out as follows. The integrative plasmids used to transform *Synechocystis* and characterize the neutral sites (**Fig. 11** and **Fig. 12)** were constructed following the design represented in **Fig. 13.** Briefly, the chromosomal regions up and downstream of the target loci (**Fig. 11**) were amplified by PCR using the *Pfu* polymerase and primers containing tags with restriction sites for cloning purposes (N*n*.5O/Nn.5I and N*n*.3O/N*n*.3I, **Fig. 10**). The corresponding up and downstream fragments of each locus were fused by "overlap-PCR", using the *Taq* polymerase (see above). The resulting products were cloned into the pGEM®-T easy vector (Promega), according to the manufacturer's instructions, originating the pSN*n** plasmids series (*n* for 'neutral site number'). The pGEM®-T easy vector restriction sites incompatible with the BioBrick RFC [10] standard were eliminated by digesting the vector with *Xho*I and *Sal*I and recircularizing the vector (originating the pSN*n*** series). The BioBrick RFC[10] standard incompatible restriction sites within the amplified chromosomal regions of pSN5** and pSN10** were eliminated using the QuikChange® Multi Site-Directed Mutagenesis Kit (Stratagene), according to the manufacturer's instructions, using the primers N5_SDM and N10_SDM, respectively (**Fig. 10**), originating the pSN5*** and the pSN10*** plasmids. A BioBrick-compatible cloning interface was synthesized (Epoch Life Science Inc.), containing the *Mun*I*, Xba*I*, Not*I*, Spe*I and *Pst*I restriction sites flanked by two double transcription terminators (based on the registry parts BBa_B1006, BBa_B0062, BBa_B0053 and BBa_B0020). This interface was excised from the delivery vector (pBSK-FP300) using *Xma*I and *Age*I and subsequently cloned into the *Xma*I site of the pSN*n*** or pSN*n**** plasmids, originating the pSN*n* plasmid series (see **Fig. 13**). Afterwards, two selection cassettes, one containing the *nptII* gene (encoding a neomycin phosphotransferase that confers resistance to neomycin and kanamycin) and the *sacB* gene (encoding a levansucrase that confers sucrose sensitivity), and another containing only the *nptII* gene, were amplified by PCR from the plasmid pK18mobsacB (Schäfer, Tauch et al. 1994), using the primer pairs Km.KmScFwd/KmScRev and Km.Km-ScFwd/KmRev, respectively (**Fig. 10**). These selection cassettes were cloned into the pGEM-T easy vector, excised with *Xma*I and cloned into the *Xma*I site of the pSN*n* plasmid series. The final plasmid series containing only the Km-resistance cassette was named pSN*n*K and the plasmid series containing the cassette with the two selective markers was named pSNnKS. Additionally, based on previous works (Eriksson, Salih et al. 2000, Shibato, Agrawal et al. 2002, Agrawal, Asayama et al. 2003) the minimal promoter sequence required for *Synechocystis psbA2* basal transcription (-38 to +14 bp; +1 corresponding to the transcription start site) was synthesized ($P_{psbA2*}$), meeting the BioBrick RFC[10] standard specifications (DNA 2.0 Inc.). This promoter region was cloned into the BioBrick vector pSB1A2 containing the part BBa_E0240 (composed by the RBS part BBa_B0032, the GFP-coding gene part BBa_E0040 and the double transcription terminator part BBa_B0015), according to the BioBrick standard cloning procedure. The BioBrick composite part BBa_E0240 (promoterless GFP generator) and the part with the synthetic constitutive promoter $P_{psbA2*}$ were digested with *Xba*I and *Spe*I and cloned into the *Xba*I site of both pSN*n* and pSN*n*K series, originating the pSN*n*K.gfp and pSN*n*K.Cgfp plasmid series (C for 'constitutive promoter'). These plasmids were used to assess proper cloning interface transcriptional insulation (promoterless *gfp*) and functionality of the neutral sites (*gfp* under the control of the constitutive promoter). The vectors were named as follows: p for 'plasmid', S for *'Synechocystis',* N for 'neutral site', *n* for the 'number of the neutral site' (5, 8, 10, 15 or 16), K for 'kanamycin resistance cassette', KS for the 'double selection cassette ($Km^R/Suc^S$)', C for 'constitutive $P_{psbA2*}$ promoter', and gfp for the 'GFP generator BioBrick part BBa_E0240'. Plasmids DNA was isolated from overnight grown *E. coli* cultures, using the GenElute™ Plasmid Miniprep Kit (Sigma-Aldrich), according to the manufacturer's instructions. All the BioBrick parts can be found at the Registry of Standard Biological Parts website (http://partsregistry.org/).

Generation of mutants

In an embodiment, the generation of mutants was carried out as follows. *Synechocystis* was transformed based on the procedure described previously (Williams 1988). Briefly, *Synechocystis* was grown in standard conditions to an $OD_{730}\approx0.5$. Cells were harvested by centrifugation (10 min at 3850 g) and resuspended in BG11 to a final $OD_{730}\approx2.5$. The purified plasmids were incubated for 5 h with 500 μL of those cells (final plasmid DNA concentration of 20 μg/mL), in light and at 25 °C. Cells were then spread onto Immobilon™-NC membranes (0.45 μm pore size, 82 mm, Millipore) resting on solid BG11 plates, grown in standard growth conditions, and transferred to selective plates after 24 h (Km, 10 μg/mL). Transformants were observed after 1-2 weeks. For complete segregation, Km-resistant colonies were grown at increasing Km concentrations (25 and 50 μg/mL) and finally transferred into liquid medium. The mutant full segregation was confirmed by PCR, using primers within and external to each site (N*n*F/N*n*R or N*n*.5O/N*n*.3O, respectively; see **Fig. 10**), and by Southern blot with probes labeled with digoxigenin using the DIG DNA labeling kit (Roche Molecular Biochemicals) and following the manufacturer's instructions. The probes were obtained by labeling the PCR-amplified 3' homologous regions for mutants in sites N5, N8, N10 and N16, and the 5' homologous region for mutants in site N15 (see **Fig. 10**). gDNA digestions were performed with *Mun*I*,* with the exception of N8 mutants for which the gDNA was digested with *Spe*I*.* When necessary antibiotic concentration was increased up to 600 μg/mL to fully segregate the mutants.

To assess the neutrality of the sites, *Synechocystis* mutants were produced exchanging each putative neutral site by a kanamycin resistance cassette, using the pSN*n*K plasmid series (**Fig. 11** and **Fig. 12**). The successful generation of fully segregated mutants in the five sites showed that the mutations did not affect cell viability under standard growth conditions. The growth of the wild-type and the five mutants was evaluated in standard growth conditions for *Synechocystis:*

For the growth experiments, 50 mL of BG11 medium were inoculated with *Synechocystis* wild-type or SN*n*K mutants to $OD_{730}$≈0.3. The cultures were grown to $OD_{730}$≈1-0, under the experimental light regimen of continuous light (20 μmol photons/$m^2$/s; CL) or 12h light (20 μmol photons/$m^2$/s)/12h dark cycles (LD), in autotrophy (no glucose; G0) or mixotrophy (5 mM glucose; G5), and at a constant temperature of 30 °C. The optical density of the cultures ($OD_{730}$) was monitored daily for ten consecutive days and the resulting growth curves for each organism and cultivation conditions are depicted in **Fig. 3** and subsequently diluted to a final $OD_{730}$≈0.1 in fresh BG11. 10 mL of each diluted culture was transferred to sterile 25 mL borosilicate glass Erlenmeyer-flasks, closed with cellulose stopper, and when necessary, glucose was added to a final concentration of 5 mM. The flasks were incubated at constant temperature (30 °C) and the indicated light regimens. All experiments were performed in triplicate and the $OD_{730}$ of the cultures was recorded daily for ten consecutive days.

In an embodiment, the analysis of growth was carried out as follows. Statistical analysis of variance (ANOVA) of the growth data was performed to compare the wild-type and the mutants, using the specific growth rate. The use of this parameter is reliable since the test duration was the same in all conditions and stationary growth was reached at similar moments. The quantitative analysis of *Synechocystis* wild-type and neutral sites mutant growth in various conditions was based on the OD measurements (see above). For each bin, *i*, a specific growth parameter, representing the exponent of exponential growth within the given time interval was calculated as:

$$\mu_i = \frac{log[x_{i+1}/x_i]}{t_{i+1} - t_i}$$

where $t_i$ is time, in units of days running from day 0 to $t_{i_{max}}$ and $x_i$ is the corresponding measured OD. As test duration was 10 days in all strains and stationary growth was reached at similar moments, the specific growth rate of all the exponential phase of each replica of each organism was considered to be a reliable parameter to assess growth patterns. ANOVA statistical analysis of the growth data was performed to compare the wild-type to each mutant organism, using the software Wolfram's Mathematica®. This ANOVA was three-way where a model with all the effects and interactions up to the third order was tested. Values of up to second order interactions can be studied in **Fig. 14.** Mutants SN5K, SN8K showed highly significant *P*-values compared to the wild-type, while for SN10K the *P*-value is approximately 0.04, and for SN15K and SN16K the *P*-values are not statistically significant (**Fig. 14**). Analysis of the growth data has shown that mutant SN5K grows slower than the wild-type (particularly in the presence of glucose), while mutant SN8K grows faster under continuous light (with or without glucose). Taking these results into consideration, the chromosomal loci neutrality may be qualitatively ordered as N15≈N16>N10>N8>>N5, from the most to the least similar to that of the wild-type. For the remaining variables considered in the ANOVA, irradiance and glucose presence were the major parameters affecting growth (highly significant *P*-values).

In an embodiment, the synthetic interface robustness and functionality of neutral sites was analysed. To use the constructed vectors as tools for the integration of synthetic devices, a BioBrick-compatible cloning interface was designed and synthesized. A DNA sequence containing the *Mun*I, *Not*I*, Xba*I, *Spe*I and *Pst*I restriction sites, compatible with the RFC[10] assembly standard, were flanked by two double transcription terminators (BioBricks BBa_B1006 and BBa_B0062 upstream, and BBa_B0053 and BBa_B0020 downstream), to prevent any potential promoter activity from backbone DNA sequences. The interface was synthesized and cloned between the two homologous recombination sequences of each vector. To evaluate the proper transcriptional insulation of the interface, a promoterless green fluorescent protein generator (GFP; BioBrick composite part BBa_E0240) was cloned in the pSN*n*K plasmid series, and *Synechocystis* mutants were produced (mutants SN*n*K.gfp, see **Fig. 11** and **Fig. 15**). Additionally, to assess the functionality of the neutral sites, *Synechocystis* mutants carrying the GFP generator under the control of a constitutive synthetic promoter (minimal *psbA2* promoter, $P_{psbA2*}$) were constructed (mutants SN*n*K.Cgfp, see **Fig. 11** and **Fig. 15**).

*gfp* transcription was assessed by RT-PCR (**Fig. 16a**).

In an embodiment, for total RNA extraction 400 mL of BG11 medium were inoculated to an $OD_{730}\approx0.2$ with pre-cultures of *Synechocystis* wild-type and mutants harboring the *gfp* gene (SN*n*K.gfp and SNnK.Cgfp mutants series). Three independent biological replicates of each strain were grown to an $OD_{730}\approx1$, at 28 °C in glass gas washing bottles with aeration, under continuous light. Cells from 100 mL of culture were collected by centrifugation (10 min at 5,000 rpm), pellets were treated with RNAprotect Bacteria Reagent (Qiagen) and stored at -80 °C. Total RNA was extracted as described previously (Pinto, Pacheco et al. 2012) and stored at -80 °C until further analysis. RNA quality and contamination verifications, cDNA synthesis, RT-PCR and RT-qPCR experiments were performed as described in Pinto and colleagues (Pinto, Pacheco et al. 2012) using primers listed in **Fig. 10.** The genes 16S, *petB,* and *rnpB* were amplified using the primers described elsewhere (Pinto, Pacheco et al. 2012) and used as reference genes for data normalization. All RT-qPCR parameters in these experiments were in agreement with the MIQE (minimum information for publication of quantitative real-time PCR experiments) guidelines (Bustin, Benes et al. 2009). RT-qPCR data from 3 biological replicates and 3 technical replicates were analyzed using qbase$^+$ v2.6 (Biogazelle) and the statistical analysis was performed by means of a one-way ANOVA, using GraphPad Prism v6 (GraphPad software Inc.).

In an embodiment, the GFP expression was analyzed by Western blot (**Fig. 16b**), confocal microscopy (**Fig. 4a**) and fluorimetry (**Fig. 4b** and **Fig. 17**).

In an embodiment, a Western blot was carried out as follows. For the Western blot, 20 mL of the same cultures used for *gfp* transcription analyses (by RT-PCR and RT-qPCR) were collected by centrifugation (10 min at 5,000 rpm) and stored at -80 °C until further use. Cells were resuspended in lysis buffer (10 mM HEPES pH 8.0, 10 mM EDTA pH 8.0, 0.5% (v/v) Triton X-100, 2 mM DTT) supplemented with a cocktail of protease inhibitors (cOmplete Mini, EDTA free, Roche) and disrupted by sonication on ice with a Branson Sonifier 250 using 5 cycles of 15 s (50% dutty cycle, output 3) intercalated with 1 min off duty. Cell debris was separated by two rounds of 10 min centrifugation at 10,000 *g* and 4 °C. Protein extracts were stored at -20 °C until further analysis. Protein quantification was performed using the BCA Protein Assay Kit (Thermo Fisher Scientific Inc.) and 15 μg of each extract was separated by electrophoresis on 12% (w/v) SDS-polyacrylamide gels. Protein extracts were visualized with colloidal Coomassie blue staining (Sigma) or transferred to a Hybond™-C extra nitrocellulose membrane (GE Healthcare). After the transfer, membranes were blocked for 3 h with 5% (w/v) milk powder in TTBS (Tris buffered saline (TBS) with 0.05% (v/v) Tween 20) and then incubated overnight at 4 °C with monoclonal mouse anti-GFP (Roche) in fresh blocking buffer. After washing with TTBS, blots were incubated for 1 h with goat-anti-mouse IgG (Invitrogen) linked to horseradish peroxidase. Membranes were washed with TTBS before immunodetection, which was performed by chemiluminescence using ECL Western Blotting Analysis System detection reagents.

In an embodiment, confocal microscopy was carried out as follows. 10 mL of BG11 medium were inoculated with *Synechocystis* wild-type or mutants harboring the *gfp* gene (SN*n*K.gfp and SN*n*K.Cgfp mutants series) to $OD_{730}\approx15$. The cultures were grown to $OD_{730}\approx1.0$, under continuous light at 30 °C and subsequently diluted to a final $OD_{730}\approx0.1$ in fresh BG11. 10 mL of each diluted culture were incubated under the same light and temperature conditions as above and grown until $OD_{730}\approx1.0$ 200 μL of each culture were collected by centrifugation, resuspended in 20 μL of fresh BG11 and 40 μL of 1% (w/v) low-melting-point agarose dissolved in BG11 medium were added. 10 μL of each mixture were immediately loaded on a glass slide and covered with a coverslip. The GFP emission (collected between 500 nm and 540 nm) was observed when the cells were exposed to a laser beam at 488 nm, using a Leica TCS SP5 II confocal microscope (Leica Microsystems). Cyanobacterial autofluorescence was collected between 640nm and 680 nm after excitation at 633 nm. Images were analyzed with the LAS AF Lite v3.3 software (Leica Microsystems). GFP fluorescence measurements were also performed for these samples as described below.

In an embodiment, GFP fluorescence measurements were carried out as follows. 50 mL of BG11 medium were inoculated with *Synechocystis* wild-type or mutants harboring the *gfp* gene (SN*n*K.gfp and SN*n*K.Cgfp mutants series) to $OD_{730}\approx0.1$. The cultures were grown to $OD_{730}\approx2.5$, under continuous light at 30 ºC and subsequently diluted to a final $OD_{730}\approx0.2$ in fresh BG11. 10 mL of each diluted culture were transferred to three sterile 25 mL borosilicate glass flasks and closed with cellulose stoppers. The flasks were incubated under the same light and temperature conditions and samples for total fluorescence measurements were collected after 2, 4, 7, 9 and 11 days after inoculation. For fluorescence measurements, three aliquots of 200 μL of each biological replicate were distributed in a Nunc™ MicroWell™ 96-Well Optical-Bottom Plates (Thermo Fisher Scientific Inc.) and GFP fluorescence and $OD_{790}$ were detected using the Synergy 2 Multi-Mode Microplate Reader and the Gen5™ software (BioTek Instruments, Inc.). For fluorescence detection an excitation filter of 485/20 nm and an emission filter of 528/20 nm were used (sensibility set for 110 nm). $OD_{790}$ was measured using an absorbance filter of 790 nm. Wells with BG11 were included for background fluorescence measurement and plate readings were carried out in duplicate. Absolute fluorescence values were normalized to the background values of the BG11 medium and the wild-type was used as fluorescence baseline. Mean GFP fluorescence per cell was normalized to the $OD_{790}$ and is expressed in arbitrary units

In all cases, *gfp* transcripts and GFP levels could only be detected in mutants when the promoter $P_{psbA2*}$ is present (SN*n*K.Cgfp); in these mutants GFP fluorescence is relatively constant over time (11 days). Moreover, to determine whether the same $P_{psbA2*}$ promoter-*gfp* construction is equivalent in terms of transcription strength at the different sites (chromosomal three-dimensional structure can affect promoter strength) RT-qPCR was performed using RNA extracted from the SN*n*K.Cgfp mutants (wild-type was used as negative control). All RT-qPCR parameters in these experiments were in agreement with the minimum information for publication of quantitative real-time PCR experiments, MIQE guidelines (Bustin, Benes et al. 2009). **Fig. 5** shows the normalized fold expression of *gfp* in the mutants and there are no statistically significant differences between the five sites, as calculated by one-way ANOVA.

In the present disclosure, a RT-PCR transcription study was performed for the five selected neutral sites throughout growth, and in the *Synechocystis,* in particular *Synechocystis* spp. 6803 strain the transcription levels appeared to be negligible. Moreover, the successful generation of fully segregated mutants, with each selected neutral site replaced by a kanamycin resistance cassette, showed that they do not encode proteins essential to the viability of the cyanobacterium under the growth conditions tested. Several studies reported that genes crucial to the viability of *Synechocystis* cannot be successfully and completely inactivated, resulting in heteroploid mutant cells, containing both chromosomes harboring the wild-type gene and the knockout locus (García-Domínguez, Reyes et al. 2000, Domain, Houot et al. 2004, Ishii and Hihara 2008, Oliveira and Lindblad 2008). Moreover, the neutrality of each site was assessed by analyzing the growth of mutants SN5K, SN8K, SN10K, SN15K and SN16K cultivated under different conditions. The results showed that the N15 and N16 mutants exhibit growth patterns similar to those of the wild-type, while the N5, N8 and N10 site mutants present statistically significant differences. Nonetheless, the mutant on site N8 exhibited growth rates slightly higher than the parental strain under continuous light, a feature that may be advantageous for biotechnological applications. As for the remaining variables considered in the ANOVA, and similarly to the results obtained by Lopo and co-workers (Lopo, Montagud et al. 2012), irradiance and the presence of glucose were the major parameters affecting growth (highly significant *P*-values).

All the integrative plasmids constructed in this disclosure possess a BioBrick-compatible synthetic interface for cloning purposes. The robustness of this synthetic interface was assessed using the promoterless GFP generator (BioBrick BBa_E0240), which showed no leakiness from the chromosomal backbone for each site, since no/negligible levels of *gfp* transcripts were detected by RT-PCR, and no GFP expression was observed by Western blot, confocal microscopy or whole cell fluorescence measurements. In contrast, using the same techniques, *gfp* transcripts or GFP signal could be detected in all the mutants generated bearing the GFP generator under the control of a constitutive synthetic promoter (based on the photosensitive promoter of the *psbA2* gene of *Synechocystis*). This shows that these five sites can effectively be used for the functional integration of synthetic devices. Moreover, the relative quantification of transcripts by RT-qPCR showed that *gfp* transcription is not affected by the position of the neutral site in the chromosome and consequently the five sites may be considered equivalent.

Altogether, these results show that the selected chromosomal sites are functional and suitable to receive ectopic DNA. In addition, the vectors constructed in this disclosure followed a rational design that allows the stable integration of synthetic devices into *Synechocystis.* Moreover, the possibility to generate markerless mutants enables the use of these vectors to introduce more than one synthetic device, and the existence of more than one integration site, spread throughout the chromosome of *Synechocystis,* allows the implementation of complex synthetic circuits. The vectors design also considered the possibility to use different resistance cassettes making the implementation faster. Finally, this disclosure contributes to the field of synthetic biology by allowing the use of *Synechocystis* as a photoautotrophic chassis, providing not only standard BioBrick-compatible vectors to integrate synthetic devices into fully characterized chromosomal neutral sites, but also chassis prone to receive a synthetic device without selective markers.

## DESCRIPTION OF THE PREFERRED EMBODIMENTS

The preferred embodiment comprises an improved method of transforming *Synechocystis* by integrating DNA at newly identified neutral sites (*n;* 5, 8, 10, 15 and 16) within the *Synechocystis* chromosome. The integration of DNA at these 5 neutral sites does not have a deleterious effect on growth of the *Synechocystis* and the inserted genes are insulated from transcriptional control by other promoters and enhancers.

In an embodiment, to transform the *Synechocystis* series of plasmids (pSN*n*; SEQ ID NO: 1:, SEQ ID NO: 2:, SEQ ID NO: 3:, SEQ ID NO: 4:, and SEQ ID NO: 5:) have been generated based on the delivery vector pGEM-T easy and containing the BioBrick compatible interface (FP300) flanked by the two regions for double homologous recombination at the five newly identified neutral sites (*n*).

In an embodiment, pGEM®-T easy (SEQ ID NO: 16:) is a commercially available plasmid (Promega) used for the T/A cloning of the PCR amplified recombination sequences. This is the delivery suicidal vector for all the plasmids used for transformation of *Synechocystis* to integrate DNA at the neutral sites. FP300 (SEQ ID NO: 26) is the synthetic interface used to insulate the multiple cloning site where ectopic DNA should be cloned for insertion in *Synechocystis* chromosome at the neutral sites. This interface contains a BioBrick compatible multiple cloning site, with recognition sequences for the restriction enzymes *Mun*I, *Not*I, *Xba*I, *Spe*I and *Pst*I, and is insulated with the BioBrick transcriptional terminators BBa_B1006 and BBa_B0062 (upstream), and BBa_B0053 and BBa_B020 (downstream). The interface was synthesized by Epoch Life Science Inc. and pBSK is the delivery vector.

Optionally, *Synechocystis* mutants that are resistant to kanamycin can be produced using a series of plasmids that have a kanamycin resistance cassette (Km; SEQ ID NO: 17:) inserted at the neutral sites (pSNnK; SEQ ID NO: 6:, SEQ ID NO: 7:, SEQ ID NO: 8:, SEQ ID NO: 9:, and SEQ ID NO: 10:,). The kanamycin resistance plasmids were generated by cloning the PCR-amplified kanamycin resistance cassette, from the plasmid pK18mobsacB, upstream of the BioBrick compatible interface of each pSN*n* plasmid. Generation of such mutants enables selection of transformed *Synechocystis* that have the cassette by exposure to kanamycin. A double selection cassette (KmSc; SEQ ID NO: 18:) can also be inserted at the neutral sites to confer both resistance to kanamycin (*nptII*) and sensitivity to sucrose (*sacB*). A plasmid series (pSN*n*KS; SEQ ID NO: 11:, SEQ ID NO: 12:, SEQ ID NO: 13:, SEQ ID NO: 14:, and SEQ ID NO: 15:) was obtained by cloning the PCR-amplified kanamycin resistance/sucrose sensitivity cassette, from the plasmid pK18mobsacB, upstream of the BioBrick compatible interface of each pSN*n* plasmid. These plasmids are used to produce *Synechocystis* mutants that are resistant to kanamycin and sensitive to sucrose. Such mutants will allow the insertion of ectopic DNA using the pSN*n* plasmids, resulting in mutants without selection markers (sensitive to kanamycin and tolerant to sucrose). Exposure to sucrose enables selection of transformed *Synechocystis* that lost the KmSc cassette.

In an embodiment, *Synechocystis* mutants that carry a Green Fluorescent Protein (GFP) generator as a reporter can also be generated. The BioBrick™ plasmid (pSB1A2-E0240) contains the GFP generator (BBa_E0240; SEQ ID NO: 19:), which comprises the GFP encoding sequence preceded by a ribosome binding site and followed by transcriptional terminators, and is cloned in the pSB1A2 plasmid (delivery vector). A promoterless series of plasmids (pSN*n*K.gfp) were generated to assess the insulation of the synthetic cloning interface (FP300), by the use of a promoterless GFP generator. The plasmids in this series were obtained by cloning the GFP generator (BioBrick BBa_E0240) in the multiple cloning site of the synthetic interface of each pSN*n*K plasmid. A promoter-controlled series of plasmids (pSN*n*K.Cgfp) were also generated. The promoter (pJ201-$P_{psbA2}$*), comprises a synthetic minimal promoter region based on *Synechocystis* native *psbA2* promoter (termed as $P_{psbA2*}$; SEQ ID NO: 20), and uses pJ201 as the delivery vector. This plasmid series was used to assess the functionality of the neutral sites by insertion of a synthetic construct constitutively promoting GFP expression. For this purpose the GFP generator (BioBrick BBa_E0240) was cloned under the control of the synthetic constitutive promoter $P_{psbA2*}$. The plasmids in this series were obtained by cloning this synthetic construct in the multiple cloning site of the synthetic interface of each pSN*n*K plasmid.

Production of ectoine and hydroxyectoine. Ectoine and hydroxyectoine belong to the family of compatible solutes and are among the most abundant osmolytes in nature, protecting and maintaining the native function of biomolecules under non-optimal conditions. Ectoine (1,4,5,6-tetrahydro-2-methyl-4-pyrimidine carboxylic acid) is a widely distributed compatible solute accumulated by halophilic and halotolerant microorganisms to prevent osmotic stress in highly saline environments. Ectoine is a highly water keeping compound stabilizing biomolecules and whole cells and can be used in research, cosmetics, and medicine (Reshetnikov et al., 2011). Although *Synechocystis* does not naturally synthesize ectoine nor its derivative hydroxyectoine, this organism produces L-aspartate 4-semialdehyde, which is the precursor for ectoine/hydroxyectoine. These compounds are produced by several organisms including *Chromohalobacter salexigens* and *Methylomicrobium alcaliphilum,* for which the genome sequences are publically available. *M. alcaliphilum 20Z* is a halotolerant alkaliphilic methanotroph, able to grow using methane as the sole carbon and energy source under conditions of high salinity (up to 10% (w/v) NaCl) and extremely alkaline pH values (pH>9). The halophilic and highly halotolerant bacterium *C. salexigens* is able to grow between 0.9% and 32% (w/v) NaCl in rich media and between 2.9% and 19% (w/v) NaCl in minimal media. These organisms, have the genes coding for the specific enzymes of ectoine biosynthesis:diaminobutyric acid (DABA) aminotransferase (EctB; SEQ ID NO: 28: and SEQ ID NO: 32:), DABA acetyltransferase (EctA; SEQ ID NO: 27: and SEQ ID NO: 31), and ectoine synthase (EctC; SEQ ID NO: 29: and SEQ ID NO: 33). These genes are organized in clusters: *ectABC* in *C. salexigens* and *ectABC-ask* in *M. alcaliphilum. ask* encodes an alternative aspartate kinase isozyme (Ask; SEQ ID NO: 35) that increases ectoine accumulation (Reshetnikov et al., 2011). For the synthesis of hydroxyectoine, the additional enzyme ectoine hydroxylase (EctD; SEQ ID NO: 30: and SEQ ID NO: 34:) is required. The enzymes described above catalyze the following reactions:

1. L-2-amino-4-oxobutanoate + L-glutamate = L-2,4-diaminobutanoate + 2-oxoglutarate (catalyzed by EctA)
2. acetyl-CoA + L-2,4-diaminobutanoate = CoA + N4-acetyl-L-2,4-diaminobutanoate (EctB)
3. 4-N-acetyl-L-2,4-diaminobutanoate = L-ectoine + $H_2O$ (EctC)
4. L-ectoine + 2-Oxoglutarate + $O_2$ = Hydroxyectoine + succinate + $CO_2$ (EctD)

To produce ectoine using the *Synechocystis,* it is necessary to introduce the 3 genes (*ectABC*) into the chassis, and an additional gene is required for hydroxyectoine production (*ectD*). These genes/gene clusters will be preferably introduced in one or more chromosomal neutral sites. In addition, the gene encoding an alternative aspartate kinase (*ask*) present in the *ect* cluster of *M. alcaliphilum* will also be considered, since it is reported to increase ectoine (and possibly hydroxyectoine) accumulation. Both the genes from *C. salexigens* and *M. alcaliphilum* were considered for ectoine and hydroxyectoine production. For this purpose, the original gene sequences were optimized for *Synechocystis* using the software Gene Designer (DNA 2.0 Inc.) and two gene clusters were designed *in silico:* (i) containing *ectABCD* from *C. salexigens* and (ii) containing *ectABCD-ask* from *M. alcaliphilum.* These gene clusters were designed as single operons containing ribosome binding sites for all genes (BioBrick BBa_B0030; SEQ ID NO: 104:) and a double transcription terminator downstream the last gene of the operon (BioBrick BBa_B0015). Restriction sites to remove *ectD* and/or *ask* from the cluster were included (*Avril* and *Nhe*I). The sequences were adapted to the BioBrick RFC[10] standard, and were synthesized at DNA 2.0 Inc. The synthetic clusters are cloned under the control of two different constitutive promoters ($P_{trc1O}$ [SEQ ID NO: 105:] and $P_{psba2*}$) and introduced into the chromosomal neutral sites described, using the vectors constructed.

The present disclosure is not, obviously, in any way restricted to the herein described embodiments and a person with average knowledge in the area can predict many possibilities of modification of the same solution and substitutions of technical characteristics by others equivalent, depending on the requirements of each situation, as defined in the appended claims.

The embodiments described above can be combined with each other. The following claims further define the preferred embodiments of the present disclosure.

**SEQUENCE LISTING**

**SEQ ID NO: 1**

```
   1 tgtccaaatc agcattgctc tgccaggtga gacggagttt ttgtcccact aatccctgtt
  61 ggatagaagg cgcttgatac agttccaacc aaacccaatc tcctgtcctg gctttagttt
 121 cttccaccgt cggcaggatt aaacgaccca accactctcc caaaggccga taatacgcct
 181 catctaaatt ctgttgcaga ggataataat ctacctggtt ggcgggcaac tgactgctaa
 241 tttgataatc cgggatgcgg ccagaggttt cctgaggttg aaacagcgta gctagggaaa
 301 tcaccaccag agcggcgatc gccaccaata ccatccgcca gcgccattgc ttcatctgtg
 361 gactgcccta aaaattgcca taaaaaaaca cctggggcgt ctttccttgt ttcgactcca
 421 gatgtttttc taatttcctc acacctgtcc taagtataaa tcaccaacgg ggattgtcaa
 481 ggcttcagga ttacacccgg gaaaaaaaaa ccccgcccct gacagggcgg ggtttttttt
 541 cagataaaaa aaatccttag ctttcgctaa ggatgatttc tgcaattggc ggccgcttct
 601 agaatgcact agtagcggcc gctgcagtcc ggcaaaaaaa cgggcaaggt gtcaccaccc
 661 tgcccttttt ctttaaaacc gaaaagatta cttcgcgttg gagagcgttc accgacaaac
 721 aacagataaa acgaaaggcc cagtctttcg actgagcctt tcgttttatt tgatgcctgg
 781 taccgggtgt aatcggttga ttatttcagt ggcccggccg atggataatt accatggccc
 841 gaccgaccgg gatttccaga ccagtttgaa cagtattaaa ttagcctttc cggacaaggc
 901 ctctgtgaaa atcttaaaaa agtattaaga ttttaaaagc tagcttattt tcggaggaaa
 961 tgtgtttact cgacttgccc agcaacaccg cgatttcgtg aaggatttag tcatgagctt
1021 gagggctttg gccactgtgc tcgaaaatcg ggctacatt gcttcttgct acacctgtgg
1081 cgaccaactc aacagcgcct ccttcatggt gagcttgggg gaaaatcatc tgatccgctt
1141 tttggtatcg gactacggca tcacctggac agaaatgcgg gatgaccgag aattaatgaa
1201 attagaagga gccgaggcga tcgcccagtt ggaagaattg gccaatgtgg tcaaatattg
1261 cctagcagat gcccccactc gaccatatgg gagagctccc aacgcgttgg atgcatagct
1321 tgagtattct atagtgtcac ctaaatagct tggcgtaatc atggtcatag ctgtttcctg
1381 tgtgaaattg ttatccgctc acaattccac acaacatacg agccggaagc ataaagtgta
1441 aagcctgggg tgcctaatga gtgagctaac tcacattaat tgcgttgcgc tcactgcccg
1501 ctttccagtc gggaaacctg tcgtgccagc tgcattaatg aatcggccaa cgcgcgggga
1561 gaggcggttt gcgtattggg cgctcttccg cttcctcgct cactgactcg ctgcgctcgg
1621 tcgttcggct gcggcgagcg gtatcagctc actcaaaggc ggtaatacgg ttatccacag
1681 aatcagggga taacgcagga aagaacatgt gagcaaaagg ccagcaaaag gccaggaacc
1741 gtaaaaaggc cgcgttgctg gcgtttttcc ataggctccg cccccctgac gagcatcaca
1801 aaaatcgacg ctcaagtcag aggtggcgaa acccgacagg actataaaga taccaggcgt
1861 ttccccctgg aagctccctc gtgcgctctc ctgttccgac cctgccgctt accggatacc
1921 tgtccgcctt tctcccttcg ggaagcgtgg cgctttctca tagctcacgc tgtaggtatc
1981 tcagttcggt gtaggtcgtt cgctccaagc tgggctgtgt gcacgaaccc cccgttcagc
2041 ccgaccgctg cgccttatcc ggtaactatc gtcttgagtc caacccggta agacacgact
2101 tatcgccact ggcagcagcc actggtaaca ggattagcag agcgaggtat gtaggcggtg
2161 ctacagagtt cttgaagtgg tggcctaact acggctacac tagaagaaca gtatttggta
2221 tctgcgctct gctgaagcca gttaccttcg gaaaaagagt tggtagctct tgatccggca
2281 aacaaaccac cgctggtagc ggtggttttt ttgtttgcaa gcagcagatt acgcgcagaa
2341 aaaaaggatc tcaagaagat cctttgatct tttctacggg gtctgacgct cagtggaacg
2401 aaaactcacg ttaagggatt ttggtcatga gattatcaaa aaggatcttc acctagatcc
2461 ttttaaatta aaaatgaagt tttaaatcaa tctaaagtat atatgagtaa acttggtctg
2521 acagttacca atgcttaatc agtgaggcac ctatctcagc gatctgtcta tttcgttcat
2581 ccatagttgc ctgactcccc gtcgtgtaga taactacgat acgggagggc ttaccatctg
2641 gccccagtgc tgcaatgata ccgcgagacc cacgctcacc ggctccagat ttatcagcaa
2701 taaaccagcc agccggaagg gccgagcgca gaagtggtcc tgcaacttta tccgcctcca
2761 tccagtctat taattgttgc cgggaagcta gagtaagtag ttcgccagtt aatagtttgc
2821 gcaacgttgt tgccattgct acaggcatcg tggtgtcacg ctcgtcgttt ggtatggctt
```

```
2881 cattcagctc cggttcccaa cgatcaaggc gagttacatg atcccccatg ttgtgcaaaa
2941 aagcggttag ctccttcggt cctccgatcg ttgtcagaag taagttggcc gcagtgttat
3001 cactcatggt tatggcagca ctgcataatt ctcttactgt catgccatcc gtaagatgct
3061 tttctgtgac tggtgagtac tcaaccaagt cattctgaga atagtgtatg cggcgaccga
3121 gttgctcttg cccggcgtca atacgggata ataccgcgcc acatagcaga actttaaaag
3181 tgctcatcat tggaaaacgt tcttcggggc gaaaactctc aaggatctta ccgctgttga
3241 gatccagttc gatgtaaccc actcgtgcac ccaactgatc ttcagcatct tttactttca
3301 ccagcgtttc tgggtgagca aaaacaggaa ggcaaaatgc cgcaaaaaag ggaataaggg
3361 cgacacggaa atgttgaata ctcatactct tcctttttca atattattga agcatttatc
3421 agggttattg tctcatgagc ggatacatat ttgaatgtat ttagaaaaat aaacaaatag
3481 gggttccgcg cacatttccc cgaaaagtgc cacctgatgc ggtgtgaaat accgcacaga
3541 tgcgtaagga gaaaataccg catcaggaaa ttgtaagcgt taatattttg ttaaaattcg
3601 cgttaaattt ttgttaaatc agctcatttt ttaaccaata ggccgaaatc ggcaaaatcc
3661 cttataaatc aaaagaatag accgagatag ggttgagtgt tgttccagtt tggaacaaga
3721 gtccactatt aaagaacgtg gactccaacg tcaaagggcg aaaaaccgtc tatcagggcg
3781 atggcccact acgtgaacca tcaccctaat caagtttttt ggggtcgagg tgccgtaaag
3841 cactaaatcg gaaccctaaa gggagccccc gatttagagc ttgacgggga aagccggcga
3901 acgtggcgag aaaggaaggg aagaaagcga aaggagcggg cgctagggcg ctggcaagtg
3961 tagcggtcac gctgcgcgta accaccacac ccgccgcgct taatgcgccg ctacagggcg
4021 cgtccattcg ccattcaggc tgcgcaactg ttgggaaggg cgatcggtgc gggcctcttc
4081 gctattacgc cagctggcga aagggggatg tgctgcaagg cgattaagtt gggtaacgcc
4141 agggttttcc cagtcacgac gttgtaaaac gacggccagt gaattgtaat acgactcact
4201 atagggcgaa ttgggcccga cgtcgcatgc tcccggccgc catggcggcc gcgggaattc
4261 gat
```

**SEQ ID NO: 2:**

1 cacccgccgc gcttaatgcg ccgctacagg gcgcgtccat tcgccattca ggctgcgcaa
61 ctgttgggaa gggcgatcgg tgcgggcctc ttcgctatta cgccagctgg cgaaaggggg
121 atgtgctgca aggcgattaa gttgggtaac gccagggttt tcccagtcac gacgttgtaa
181 aacgacggcc agtgaattgt aatacgactc actatagggc gaattgggcc cgacgtcgca
241 tgctcccggc cgccatggcg gccgcgggaa ttcgattgat gaccgctggc ggagtttagt
301 ccagggttat ttgtaccgtc aaggttacat tgccacccca gatttaacta cgctgcgagt
361 ccgtaccatt ggcgatcggg cctatctcac cattaagggt aaaaatgcca gcattgcccg
421 cttggatttg agtatgaaat tcttgcagtg gaagccaatt aaattttgac agaactctgt
481 tcaccgcccc tgattgaaaa tatcgttatt gcctcgatta ccatggtaaa acctgagaag
541 tagacgagtt tttgggggat aaccagggtc taattttagc ggaagtggaa ttaacctaca
601 ctggtgaaaa aataagtcta cttccctgga tcggggaaga ggtaacggat gatgcccgct
661 attacaacgt caatttagcc caacatccct acaaaaattg gtgacaagat tacacccggg
721 aaaaaaaaac cccgcccctg acagggcggg gtttttttc agataaaaaa aatccttagc
781 tttcgctaag gatgatttct gcaattggcg gccgcttcta gaatgcacta gtagcggccg
841 ctgcagtccg gcaaaaaaac gggcaaggtg tcaccaccct gccctttttc tttaaaaccg
901 aaaagattac ttcgcgttgg agagcgttca ccgacaaaca acagataaaa cgaaaggccc
961 agtctttcga ctgagccttt cgttttattt gatgcctggt accgggtgta atccttcatt
1021 cccctattgt tattaatagt gctctgaaac taacaaataa gcaagatcaa attgtgaaga
1081 aaacgcaaca aaatgaacca ttaagataat ttctgtctat cctggctggt atattccagt
1141 ctactcagaa atgagtttgt tttatgacaa atcagtatgt accaaattac ttggcgcaat
1201 cgatcctggt aactctattt tgttgcttac ccctgggtat tgtggccatt atcaaagcat
1261 cggaagttaa ttctcgttta gcttcagggg actatgaagg cgctgtaaag gcttccaagg
1321 aagcgaaaaa gttttgttgg tggtcctttg gtgccggcat aattttcatt gccatctatt
1381 ttgtgctagt ggttattgcc gccgtctttg gtcagtaatt aaagttacat tttttgactt
1441 tgccttgttc accattcatt aacgaatacc atgtttagtt tgaaaaatta ttccccatct
1501 ccattactat ccgctaaggc caaggaatat ttagtattca ctttggtaac cctaaccatt

```
1561 gtcgttgctg gcactcgacc atatgggaga gctcccaacg cgttggatgc atagcttgag
1621 tattctatag tgtcacctaa atagcttggc gtaatcatgg tcatagctgt ttcctgtgtg
1681 aaattgttat ccgctcacaa ttccacacaa catacgagcc ggaagcataa agtgtaaagc
1741 ctggggtgcc taatgagtga gctaactcac attaattgcg ttgcgctcac tgcccgcttt
1801 ccagtcggga aacctgtcgt gccagctgca ttaatgaatc ggccaacgcg cggggagagg
1861 cggtttgcgt attgggcgct cttccgcttc ctcgctcact gactcgctgc gctcggtcgt
1921 tcggctgcgg cgagcggtat cagctcactc aaaggcggta atacggttat ccacagaatc
1981 aggggataac gcaggaaaga acatgtgagc aaaaggccag caaaaggcca ggaaccgtaa
2041 aaaggccgcg ttgctggcgt ttttccatag gctccgcccc cctgacgagc atcacaaaaa
2101 tcgacgctca agtcagaggt ggcgaaaccc gacaggacta taaagatacc aggcgtttcc
2161 ccctggaagc tccctcgtgc gctctcctgt tccgaccctg ccgcttaccg gatacctgtc
2221 cgcctttctc ccttcgggaa gcgtggcgct ttctcatagc tcacgctgta ggtatctcag
2281 ttcggtgtag gtcgttcgct ccaagctggg ctgtgtgcac gaaccccccg ttcagcccga
2341 ccgctgcgcc ttatccggta actatcgtct tgagtccaac ccggtaagac acgacttatc
2401 gccactggca gcagccactg gtaacaggat tagcagagcg aggtatgtag gcggtgctac
2461 agagttcttg aagtggtggc ctaactacgg ctacactaga agaacagtat ttggtatctg
2521 cgctctgctg aagccagtta ccttcggaaa aagagttggt agctcttgat ccggcaaaca
2581 aaccaccgct ggtagcggtg gtttttttgt ttgcaagcag cagattacgc gcagaaaaaa
2641 aggatctcaa gaagatcctt tgatcttttc tacggggtct gacgctcagt ggaacgaaaa
2701 ctcacgttaa gggattttgg tcatgagatt atcaaaaagg atcttcacct agatcctttt
2761 aaattaaaaa tgaagtttta aatcaatcta aagtatatat gagtaaactt ggtctgacag
2821 ttaccaatgc ttaatcagtg aggcacctat ctcagcgatc tgtctatttc gttcatccat
2881 agttgcctga ctccccgtcg tgtagataac tacgatacgg gagggcttac catctggccc
2941 cagtgctgca atgataccgc gagacccacg ctcaccggct ccagatttat cagcaataaa
3001 ccagccagcc ggaagggccg agcgcagaag tggtcctgca actttatccg cctccatcca
3061 gtctattaat tgttgccggg aagctagagt aagtagttcg ccagttaata gtttgcgcaa
3121 cgttgttgcc attgctacag gcatcgtggt gtcacgctcg tcgtttggta tggcttcatt
3181 cagctccggt tcccaacgat caaggcgagt tacatgatcc cccatgttgt gcaaaaaagc
3241 ggttagctcc ttcggtcctc cgatcgttgt cagaagtaag ttggccgcag tgttatcact
3301 catggttatg gcagcactgc ataattctct tactgtcatg ccatccgtaa gatgcttttc
3361 tgtgactggt gagtactcaa ccaagtcatt ctgagaatag tgtatgcggc gaccgagttg
3421 ctcttgcccg gcgtcaatac gggataatac cgcgccacat agcagaactt taaaagtgct
3481 catcattgga aaacgttctt cggggcgaaa actctcaagg atcttaccgc tgttgagatc
3541 cagttcgatg taacccactc gtgcacccaa ctgatcttca gcatctttta ctttcaccag
3601 cgtttctggg tgagcaaaaa caggaaggca aaatgccgca aaaaagggaa taagggcgac
3661 acggaaatgt tgaatactca tactcttcct ttttcaatat tattgaagca tttatcaggg
3721 ttattgtctc atgagcggat acatatttga atgtatttag aaaaataaac aaataggggt
3781 tccgcgcaca tttccccgaa aagtgccacc tgatgcggtg tgaaataccg cacagatgcg
3841 taaggagaaa ataccgcatc aggaaattgt aagcgttaat attttgttaa aattcgcgtt
3901 aaatttttgt taaatcagct cattttttaa ccaataggcc gaaatcggca aaatccctta
3961 taaatcaaaa gaatagaccg agatagggtt gagtgttgtt ccagtttgga acaagagtcc
4021 actattaaag aacgtggact ccaacgtcaa agggcgaaaa accgtctatc agggcgatgg
4081 cccactacgt gaaccatcac cctaatcaag ttttttgggg tcgaggtgcc gtaaagcact
4141 aaatcggaac cctaaaggga gcccccgatt tagagcttga cggggaaagc cggcgaacgt
4201 ggcgagaaag gaagggaaga aagcgaaagg agcgggcgct agggcgctgg caagtgtagc
4261 ggtcacgctg cgcgtaacca cca
```

**SEQ ID NO: 3:**

```
1 cacccgccgc gcttaatgcg ccgctacagg gcgcgtccat tcgccattca ggctgcgcaa
61 ctgttgggaa gggcgatcgg tgcgggcctc ttcgctatta cgccagctgg cgaaaggggg
121 atgtgctgca aggcgattaa gttgggtaac gccagggttt tcccagtcac gacgttgtaa
181 aacgacggcc agtgaattgt aatacgactc actatagggc gaattgggcc cgacgtcgca
```

241 tgctcccggc cgccatggcg gccgcgggaa ttcgattgga ctgacccaag atacagtggt
301 agcttcaagt tcgtcaatca accccaatgg aaagctgaga taatctgttg ttgatgcgac
361 cgtcttcatt ttagctaaaa agacaagtct gtggctagtt actatgacga ggccatcggg
421 tctatcccag cggtcttgct gattccaaaa cttggcgatt ttctgaagta gtatcttttc
481 accgcttaag ccgccaaggt cttggatgag cttggtcgaa tcatcttttg atgcctcgac
541 atcgcgaata actttgtaaa cgaccttccc gagcttgaat gcaaagtcgg ttagattatc
601 catcagagtt gaatgccgat aatacagact gctatactat tttaatgaaa agagtgctgg
661 caagcaagcc cgccgaacgt tgaagctgtg cagcgcaaac gaagcgcatt gtagtttgcg
721 ctcggattac acccgggaaa aaaaaacccc gcccctgaca gggcggggtt ttttttcaga
781 taaaaaaaat ccttagcttt cgctaaggat gatttctgca attggcggcc gcttctagaa
841 tgcactagta gcggccgctg cagtccggca aaaaaacggg caaggtgtca ccaccctgcc
901 ctttttcttt aaaaccgaaa agattacttc gcgttggaga gcgttcaccg acaaacaaca
961 gataaaacga aaggcccagt ctttcgactg agcctttcgt tttatttgat gcctggtacc
1021 gggtgtaatc cccatactca gccttctaat gctgagagaa tgcctgaaaa gaaccactaa
1081 gtcaaattat ttggattaag ttttattaag tcaacggagg tgtcagatag catttagatc
1141 gtaaaagcat acggcatgaa caaccatgaa cggcttccca gaggttaacc ccaggtttat
1201 ggtttaccaa aatcccctga ctttttccga gttgggggac aaaatcaact aatttgggcg
1261 acgatttttt ctgctatctg gcgatcgcca tgtttgtcta acttctgatc caacctaggt
1321 ttttgggggc tttccaacaa aaaatcccag ttgccggcga aaaattgctg gtggggcaca
1381 atttggtgct ctccgtagtt ctgcaaacct gccagtaaaa cttgcgcttc cgcaaaacca
1441 tccctggtga gggaaataat cgggacttct aactttaacg cttcagcaaa ggtaccaaaa
1501 ccaggcttgg aaaatacccg tccgcaaagg ggcattaaat ccaccgggcg actcgaccat
1561 atgggagagc tcccaacgcg ttggatgcat agcttgagta ttctatagtg tcacctaaat
1621 agcttggcgt aatcatggtc atagctgttt cctgtgtgaa attgttatcc gctcacaatt
1681 ccacacaaca tacgagccgg aagcataaag tgtaaagcct ggggtgccta atgagtgagc
1741 taactcacat taattgcgtt gcgctcactg cccgctttcc agtcgggaaa cctgtcgtgc
1801 cagctgcatt aatgaatcgg ccaacgcgcg gggagaggcg gtttgcgtat tgggcgctct
1861 tccgcttcct cgctcactga ctcgctgcgc tcggtcgttc ggctgcggcg agcggtatca
1921 gctcactcaa aggcggtaat acggttatcc acagaatcag gggataacgc aggaaagaac
1981 atgtgagcaa aaggccagca aaaggccagg aaccgtaaaa aggccgcgtt gctggcgttt
2041 ttccataggc tccgcccccc tgacgagcat cacaaaaatc gacgctcaag tcagaggtgg
2101 cgaaacccga caggactata aagataccag gcgtttcccc ctggaagctc cctcgtgcgc
2161 tctcctgttc cgaccctgcc gcttaccgga tacctgtccg cctttctccc ttcgggaagc
2221 gtggcgcttt ctcatagctc acgctgtagg tatctcagtt cggtgtaggt cgttcgctcc
2281 aagctgggct gtgtgcacga accccccgtt cagcccgacc gctgcgcctt atccggtaac
2341 tatcgtcttg agtccaaccc ggtaagacac gacttatcgc cactggcagc agccactggt
2401 aacaggatta gcagagcgag gtatgtaggc ggtgctacag agttcttgaa gtggtggcct
2461 aactacggct acactagaag aacagtattt ggtatctgcg ctctgctgaa gccagttacc
2521 ttcggaaaaa gagttggtag ctcttgatcc ggcaaacaaa ccaccgctgg tagcggtggt
2581 ttttttgttt gcaagcagca gattacgcgc agaaaaaaag gatctcaaga agatcctttg
2641 atcttttcta cggggtctga cgctcagtgg aacgaaaact cacgttaagg gattttggtc
2701 atgagattat caaaaaggat cttcacctag atccttttaa attaaaaatg aagttttaaa
2761 tcaatctaaa gtatatatga gtaaacttgg tctgacagtt accaatgctt aatcagtgag
2821 gcacctatct cagcgatctg tctatttcgt tcatccatag ttgcctgact ccccgtcgtg
2881 tagataacta cgatacggga gggcttacca tctggcccca gtgctgcaat gataccgcga
2941 gacccacgct caccggctcc agatttatca gcaataaacc agccagccgg aagggccgag
3001 cgcagaagtg gtcctgcaac tttatccgcc tccatccagt ctattaattg ttgccgggaa
3061 gctagagtaa gtagttcgcc agttaatagt ttgcgcaacg ttgttgccat tgctacaggc
3121 atcgtggtgt cacgctcgtc gtttggtatg gcttcattca gctccggttc ccaacgatca
3181 aggcgagtta catgatcccc catgttgtgc aaaaaagcgg ttagctcctt cggtcctccg
3241 atcgttgtca gaagtaagtt ggccgcagtg ttatcactca tggttatggc agcactgcat
3301 aattctctta ctgtcatgcc atccgtaaga tgcttttctg tgactggtga gtactcaacc
3361 aagtcattct gagaatagtg tatgcggcga ccgagttgct cttgcccggc gtcaatacgg

3421 gataataccg cgccacatag cagaacttta aaagtgctca tcattggaaa acgttcttcg
3481 gggcgaaaac tctcaaggat cttaccgctg ttgagatcca gttcgatgta acccactcgt
3541 gcacccaact gatcttcagc atcttttact ttcaccagcg tttctgggtg agcaaaaaca
3601 ggaaggcaaa atgccgcaaa aaagggaata agggcgacac ggaaatgttg aatactcata
3661 ctcttccttt ttcaatatta ttgaagcatt tatcagggtt attgtctcat gagcggatac
3721 atatttgaat gtatttagaa aaataaacaa ataggggttc cgcgcacatt tccccgaaaa
3781 gtgccacctg atgcggtgtg aaataccgca cagatgcgta aggagaaaat accgcatcag
3841 gaaattgtaa gcgttaatat tttgttaaaa ttcgcgttaa atttttgtta aatcagctca
3901 ttttttaacc aataggccga aatcggcaaa atcccttata aatcaaaaga atagaccgag
3961 atagggttga gtgttgttcc agtttggaac aagagtccac tattaaagaa cgtggactcc
4021 aacgtcaaag ggcgaaaaac cgtctatcag ggcgatggcc cactacgtga accatcaccc
4081 taatcaagtt ttttggggtc gaggtgccgt aaagcactaa atcggaaccc taaagggagc
4141 ccccgattta gagcttgacg gggaaagccg gcgaacgtgg cgagaaagga agggaagaaa
4201 gcgaaaggag cgggcgctag ggcgctggca agtgtagcgg tcacgctgcg cgtaaccacc
4261 a

**SEQ ID NO: 4:**

1 cacccgccgc gcttaatgcg ccgctacagg gcgcgtccat tcgccattca ggctgcgcaa
61 ctgttgggaa gggcgatcgg tgcgggcctc ttcgctatta cgccagctgg cgaaaggggg
121 atgtgctgca aggcgattaa gttgggtaac gccagggttt tcccagtcac gacgttgtaa
181 aacgacggcc agtgaattgt aatacgactc actatagggc gaattgggcc cgacgtcgca
241 tgctcccggc cgccatggcg gccgcgggaa ttcgattcta aacttacggc attggcatca
301 acgggagcca ccgtcggagt aggggaagta acaacggggg aactggtatt ggtgggcaaa
361 atttttacta gccattgatt ccattggggc agattgaacg ctcccaacca ccaaagtccc
421 cctaatacaa cgacagaact aaataacagg aaaaaaggaa tccaaggagt taccccctctt
481 ttatgggatg gaacttcatc gacattaaag gtgggagggg gaggaggcaa tggggacaat
541 ggtggtccag aaaaggaagg tggctccgac gtcaaggcaa cgggacatcc acaggattca
601 cagaaacgaa cctgggggct aaggcggttg ccacaattag tacaaaaaac gggagtagtc
661 ataggtgaaa accccgacta tagaattaga aaaatttaac tttttatccg aattttattc
721 gtccatgttc cccaaataac tatcaaaata attggaaaaa ttaaattatt tggtcgttgg
781 tcaccgctcc ctaaagacct ggccattgta aagagattac acccgggaaa aaaaaacccc
841 gcccctgaca gggcggggtt ttttttcaga taaaaaaaat ccttagcttt cgctaaggat
901 gatttctgca attggcggcc gcttctagaa tgcactagta gcggccgctg cagtccggca
961 aaaaaacggg caaggtgtca ccaccctgcc ctttttcttt aaaaccgaaa agattacttc
1021 gcgttggaga gcgttcaccg acaaacaaca gataaaacga aaggcccagt ctttcgactg
1081 agcctttcgt tttatttgat gcctggtacc gggtgtaatc cattgggcac gagagttagt
1141 aaggcagtgg caattaatag aggcttatgg ttgattcgca ttgttttgct cctgaaattt
1201 tcggcaaata caaatacttc gctcttctag ccctattaac cattttaacg acaaattgat
1261 ggggcaacga ttaacaaata atgaataaat tttatgtttt tcaagatgaa aatttgaaaa
1321 tttgatttcc ttatatttct actatagaag actaatacaa ttagatctaa aatttgcaag
1381 tataaaaatc agcaaatagt tatattgtta ataattcaat gacccaataa ctcgtactgt
1441 tatctacgtg gtgaaagcca aaaagacgaa cagtttagcc tcctcctcct cggcgatcgc
1501 caagcgaaat gtcatgggag atgttcagat tgagcatttt tttctaaaag cccttgctaa
1561 aacaaaccac atgtgcaggg tgtccccgat gttgactaaa ttcagcggct cgaccatatg
1621 ggagagctcc caacgcgttg gatgcatagc ttgagtattc tatagtgtca cctaaatagc
1681 ttggcgtaat catggtcata gctgtttcct gtgtgaaatt gttatccgct cacaattcca
1741 cacaacatac gagccggaag cataaagtgt aaagcctggg gtgcctaatg agtgagctaa
1801 ctcacattaa ttgcgttgcg ctcactgccc gctttccagt cgggaaacct gtcgtgccag
1861 ctgcattaat gaatcggcca acgcgcgggg agaggcggtt tgcgtattgg gcgctcttcc
1921 gcttcctcgc tcactgactc gctgcgctcg gtcgttcggc tgcggcgagc ggtatcagct
1981 cactcaaagg cggtaatacg gttatccaca gaatcagggg ataacgcagg aaagaacatg
2041 tgagcaaaag gccagcaaaa ggccaggaac cgtaaaaagg ccgcgttgct ggcgtttttc

2101 cataggctcc gcccccctga cgagcatcac aaaaatcgac gctcaagtca gaggtggcga
2161 aacccgacag gactataaag ataccaggcg tttccccctg gaagctccct cgtgcgctct
2221 cctgttccga ccctgccgct taccggatac ctgtccgcct ttctcccttc gggaagcgtg
2281 gcgctttctc atagctcacg ctgtaggtat ctcagttcgg tgtaggtcgt tcgctccaag
2341 ctgggctgtg tgcacgaacc ccccgttcag cccgaccgct gcgccttatc cggtaactat
2401 cgtcttgagt ccaacccggt aagacacgac ttatcgccac tggcagcagc cactggtaac
2461 aggattagca gagcgaggta tgtaggcggt gctacagagt tcttgaagtg gtggcctaac
2521 tacggctaca ctagaagaac agtatttggt atctgcgctc tgctgaagcc agttaccttc
2581 ggaaaaagag ttggtagctc ttgatccggc aaacaaacca ccgctggtag cggtggtttt
2641 tttgtttgca agcagcagat tacgcgcaga aaaaaaggat ctcaagaaga tcctttgatc
2701 ttttctacgg ggtctgacgc tcagtggaac gaaaactcac gttaagggat tttggtcatg
2761 agattatcaa aaaggatctt cacctagatc cttttaaatt aaaaatgaag ttttaaatca
2821 atctaaagta tatatgagta aacttggtct gacagttacc aatgcttaat cagtgaggca
2881 cctatctcag cgatctgtct atttcgttca tccatagttg cctgactccc cgtcgtgtag
2941 ataactacga tacgggaggg cttaccatct ggccccagtg ctgcaatgat accgcgagac
3001 ccacgctcac cggctccaga tttatcagca ataaaccagc cagccggaag ggccgagcgc
3061 agaagtggtc ctgcaacttt atccgcctcc atccagtcta ttaattgttg ccgggaagct
3121 agagtaagta gttcgccagt taatagtttg cgcaacgttg ttgccattgc tacaggcatc
3181 gtggtgtcac gctcgtcgtt tggtatggct tcattcagct ccggttccca acgatcaagg
3241 cgagttacat gatcccccat gttgtgcaaa aaagcggtta gctccttcgg tcctccgatc
3301 gttgtcagaa gtaagttggc cgcagtgtta tcactcatgg ttatggcagc actgcataat
3361 tctcttactg tcatgccatc cgtaagatgc ttttctgtga ctggtgagta ctcaaccaag
3421 tcattctgag aatagtgtat gcggcgaccg agttgctctt gcccggcgtc aatacgggat
3481 aataccgcgc cacatagcag aactttaaaa gtgctcatca ttggaaaacg ttcttcgggg
3541 cgaaaactct caaggatctt accgctgttg agatccagtt cgatgtaacc cactcgtgca
3601 cccaactgat cttcagcatc ttttactttc accagcgttt ctgggtgagc aaaaacagga
3661 aggcaaaatg ccgcaaaaaa gggaataagg gcgacacgga aatgttgaat actcatactc
3721 ttcctttttc aatattattg aagcatttat cagggttatt gtctcatgag cggatacata
3781 tttgaatgta tttagaaaaa taaacaaata ggggttccgc gcacatttcc ccgaaaagtg
3841 ccacctgatg cggtgtgaaa taccgcacag atgcgtaagg agaaaatacc gcatcaggaa
3901 attgtaagcg ttaatatttt gttaaaattc gcgttaaatt tttgttaaat cagctcattt
3961 tttaaccaat aggccgaaat cggcaaaatc ccttataaat caaaagaata gaccgagata
4021 gggttgagtg ttgttccagt ttggaacaag agtccactat taaagaacgt ggactccaac
4081 gtcaaagggc gaaaaaccgt ctatcagggc gatggcccac tacgtgaacc atcaccctaa
4141 tcaagttttt tggggtcgag gtgccgtaaa gcactaaatc ggaaccctaa agggagcccc
4201 cgatttagag cttgacgggg aaagccggcg aacgtggcga gaaaggaagg gaagaaagcg
4261 aaaggagcgg gcgctagggc gctggcaagt gtagcggtca cgctgcgcgt aaccacca

**SEQ ID NO: 5:**

1 cacccgccgc gcttaatgcg ccgctacagg gcgcgtccat tcgccattca ggctgcgcaa
61 ctgttgggaa gggcgatcgg tgcgggcctc ttcgctatta cgccagctgg cgaaaggggg
121 atgtgctgca aggcgattaa gttgggtaac gccagggttt tcccagtcac gacgttgtaa
181 aacgacggcc agtgaattgt aatacgactc actatagggc gaattgggcc cgacgtcgca
241 tgctcccggc cgccatggcg gccgcgggaa ttcgattgtg agcttgatgg tgatggtggg
301 taaagcttca ttgtccatgc gccggagctt gacggtaaaa tccgtgtcct ggggccaatc
361 attgggaacg ctcacacaga taccgttgcg agttaagtcg aaggataccc ccaccaattc
421 cccttccata tcgaacactt ggcaaaaggc caacatccgc tcttcctgcc gtcgctcctg
481 cacccccctgg agatgggcaa tggttagggc ttcttgggag agggaagatg gagaatcttg
541 agccatggag attatttcct cggttaaatt aggtttacct agtaaatggg ccaggttgac
601 cattattgta gtcattcact gggtcgatct tccttccatc agggtcattt ggttaattgt
661 tgatgagaaa tgggaaggag taatccatag atatttgccc agttaactcg atttgagcag
721 aattgggaag gacgtattgg gaactttggt tgacagggca acaaagccag cagattacac

781 ccgggaaaaa aaaaccccgc ccctgacagg gcggggtttt ttttcagata aaaaaaatcc
841 ttagctttcg ctaaggatga tttctgcaat tggcggccgc ttctagaatg cactagtagc
901 ggccgctgca gtccggcaaa aaaacgggca aggtgtcacc accctgccct ttttctttaa
961 aaccgaaaag attacttcgc gttggagagc gttcaccgac aaacaacaga taaaacgaaa
1021 ggcccagtct ttcgactgag cctttcgttt tatttgatgc ctggtaccgg gtgtaatcgc
1081 tggaggcgaa ctgggtgaga accataaaat cttggggaac aggggaatgt taatgaacac
1141 atgacatgca taggatagcg aattgattta aagcaatgat tccccctgag ataaatatat
1201 tttgacccac tgctccgctt aattttggcg ctaatctggg caaaaattcg ttggttttcg
1261 gaaaggtgaa cccgtagcta gcttgtattg cccaaaccta actcatcatt gctccatggc
1321 ggccaaaaat ctttaggaca aaactcgcca gaggtcaagg cttgattttt tcagagggaa
1381 aaattcctcg gctaaataat caaaaagttg aaaaaaaaag tttttcagct aaggtttgat
1441 gttatatttt atcttatgta gacttttgaa aaaaacaagt ccccgcaatc aagcatcatt
1501 caacggaaaa aataatccta tgccaaacgc ctccaccgca ctaaccggca aagcattact
1561 caataaagtt aaagaactat cccatctgcc ccgtcgagaa acggcaaaag cctgtggtta
1621 ctactcgacc atatgggaga gctcccaacg cgttggatgc atagcttgag tattctatag
1681 tgtcacctaa atagcttggc gtaatcatgg tcatagctgt ttcctgtgtg aaattgttat
1741 ccgctcacaa ttccacacaa catacgagcc ggaagcataa agtgtaaagc ctggggtgcc
1801 taatgagtga gctaactcac attaattgcg ttgcgctcac tgcccgcttt ccagtcggga
1861 aacctgtcgt gccagctgca ttaatgaatc ggccaacgcg cggggagagg cggtttgcgt
1921 attgggcgct cttccgcttc ctcgctcact gactcgctgc gctcggtcgt tcggctgcgg
1981 cgagcggtat cagctcactc aaaggcggta atacggttat ccacagaatc aggggataac
2041 gcaggaaaga acatgtgagc aaaaggccag caaaaggcca ggaaccgtaa aaaggccgcg
2101 ttgctggcgt ttttccatag gctccgcccc cctgacgagc atcacaaaaa tcgacgctca
2161 agtcagaggt ggcgaaaccc gacaggacta taaagatacc aggcgtttcc ccctggaagc
2221 tccctcgtgc gctctcctgt tccgaccctg ccgcttaccg gatacctgtc cgcctttctc
2281 ccttcgggaa gcgtggcgct ttctcatagc tcacgctgta ggtatctcag ttcggtgtag
2341 gtcgttcgct ccaagctggg ctgtgtgcac gaaccccccg ttcagcccga ccgctgcgcc
2401 ttatccggta actatcgtct tgagtccaac ccggtaagac acgacttatc gccactggca
2461 gcagccactg gtaacaggat tagcagagcg aggtatgtag gcggtgctac agagttcttg
2521 aagtggtggc ctaactacgg ctacactaga agaacagtat ttggtatctg cgctctgctg
2581 aagccagtta ccttcggaaa aagagttggt agctcttgat ccggcaaaca aaccaccgct
2641 ggtagcggtg gtttttttgt ttgcaagcag cagattacgc gcagaaaaaa aggatctcaa
2701 gaagatcctt tgatcttttc tacggggtct gacgctcagt ggaacgaaaa ctcacgttaa
2761 gggattttgg tcatgagatt atcaaaaagg atcttcacct agatcctttt aaattaaaaa
2821 tgaagtttta aatcaatcta aagtatatat gagtaaactt ggtctgacag ttaccaatgc
2881 ttaatcagtg aggcacctat ctcagcgatc tgtctatttc gttcatccat agttgcctga
2941 ctccccgtcg tgtagataac tacgatacgg gagggcttac catctggccc cagtgctgca
3001 atgataccgc gagacccacg ctcaccggct ccagatttat cagcaataaa ccagccagcc
3061 ggaagggccg agcgcagaag tggtcctgca actttatccg cctccatcca gtctattaat
3121 tgttgccggg aagctagagt aagtagttcg ccagttaata gtttgcgcaa cgttgttgcc
3181 attgctacag gcatcgtggt gtcacgctcg tcgtttggta tggcttcatt cagctccggt
3241 tcccaacgat caaggcgagt tacatgatcc cccatgttgt gcaaaaaagc ggttagctcc
3301 ttcggtcctc cgatcgttgt cagaagtaag ttggccgcag tgttatcact catggttatg
3361 gcagcactgc ataattctct tactgtcatg ccatccgtaa gatgcttttc tgtgactggt
3421 gagtactcaa ccaagtcatt ctgagaatag tgtatgcggc gaccgagttg ctcttgcccg
3481 gcgtcaatac gggataatac cgcgccacat agcagaactt taaaagtgct catcattgga
3541 aaacgttctt cggggcgaaa actctcaagg atcttaccgc tgttgagatc cagttcgatg
3601 taacccactc gtgcacccaa ctgatcttca gcatctttta ctttcaccag cgtttctggg
3661 tgagcaaaaa caggaaggca aaatgccgca aaaaagggaa taagggcgac acggaaatgt
3721 tgaatactca tactcttcct ttttcaatat tattgaagca tttatcaggg ttattgtctc
3781 atgagcggat acatatttga atgtatttag aaaaataaac aaataggggt tccgcgcaca
3841 tttccccgaa aagtgccacc tgatgcggtg tgaaataccg cacagatgcg taaggagaaa
3901 ataccgcatc aggaaattgt aagcgttaat attttgttaa aattcgcgtt aaatttttgt

```
3961 taaatcagct catttttttaa ccaataggcc gaaatcggca aaatccctta taaatcaaaa
4021 gaatagaccg agatagggtt gagtgttgtt ccagtttgga acaagagtcc actattaaag
4081 aacgtggact ccaacgtcaa agggcgaaaa accgtctatc agggcgatgg cccactacgt
4141 gaaccatcac cctaatcaag ttttttgggg tcgaggtgcc gtaaagcact aaatcggaac
4201 cctaaaggga gcccccgatt tagagcttga cggggaaagc cggcgaacgt ggcgagaaag
4261 gaagggaaga aagcgaaagg agcgggcgct agggcgctgg caagtgtagc ggtcacgctg
4321 cgcgtaacca cca
```

**SEQ ID NO: 6:**

```
   1 tgtccaaatc agcattgctc tgccaggtga gacggagttt ttgtcccact aatccctgtt
  61 ggatagaagg cgcttgatac agttccaacc aaacccaatc tcctgtcctg gctttagttt
 121 cttccaccgt cggcaggatt aaacgaccca accactctcc caaaggccga taatacgcct
 181 catctaaatt ctgttgcaga ggataataat ctacctggtt ggcgggcaac tgactgctaa
 241 tttgataatc cgggatgcgg ccagaggttt cctgaggttg aaacagcgta gctagggaaa
 301 tcaccaccag agcggcgatc gccaccaata ccatccgcca gcgccattgc ttcatctgtg
 361 gactgcccta aaaattgcca taaaaaaaca cctggggcgt ctttccttgt ttcgactcca
 421 gatgtttttc taatttcctc acacctgtcc taagtataaa tcaccaacgg ggattgtcaa
 481 ggcttcagga ttacacccgg gcgatttact tttcgacctc attctattag actctcgttt
 541 ggattgcaac tggtctattt tcctcttttg tttgatagaa aatcataaaa ggatttgcag
 601 actacgggcc taaagaacta aaaaatctat ctgtttcttt tcattctctg tattttttat
 661 agtttctgtt gcatgggcat aaagttgcct ttttaatcac aattcagaaa atatcataat
 721 atctcatttc actaaataat agtgaacggc aggtatatgt gatgggttaa aaaggatcga
 781 tcctctagcg aaccccagag tcccgctcag aagaactcgt caagaaggcg atagaaggcg
 841 atgcgctgcg aatcgggagc ggcgataccg taaagcacga ggaagcggtc agcccattcg
 901 ccgccaagct cttcagcaat atcacgggta gccaacgcta tgtcctgata gcggtccgcc
 961 acacccagcc ggccacagtc gatgaatcca gaaaagcggc cattttccac catgatattc
1021 ggcaagcagg catcgccatg ggtcacgacg agatcctcgc cgtcgggcat ccgcgccttg
1081 agcctggcga acagttcggc tggcgcgagc ccctgatgct cttcgtccag atcatcctga
1141 tcgacaagac cggcttccat ccgagtacgt gctcgctcga tgcgatgttt cgcttggtgg
1201 tcgaatgggc aggtagccgg atcaagcgta tgcagccgcc gcattgcatc agccatgatg
1261 gatactttct cggcaggagc aaggtgagat gacaggagat cctgccccgg cacttcgccc
1321 aatagcagcc agtcccttcc cgcttcagtg acaacgtcga gcacagctgc gcaaggaacg
1381 cccgtcgtgg ccagccacga tagccgcgct gcctcgtctt ggagttcatt cagggcaccg
1441 gacaggtcgg tcttgacaaa aagaaccggg cgcccctgcg ctgacagccg gaacacggcg
1501 gcatcagagc agccgattgt ctgttgtgcc cagtcatagc cgaatagcct ctccacccaa
1561 gcggccggag aacctgcgtg caatccatct tgttcaatca tgcgaaacga tcctcatcct
1621 gtctcttgat cagatcttga tcccctgcgc catcagatcc ttggcggcaa gaaagccatc
1681 cagtttactt tgcagggctt cccaacctta ccagagggcg ccccagctgg caattccggt
1741 tcgcttgctg tccataaaac cgcccagtct agctatcgcc atgtaagccc actgcaagct
1801 acctgctttc tctttgcgct tgcgttttcc cttgtccaga tagcccagta gctgacattc
1861 acccgggaaa aaaaaacccc gcccctgaca gggcggggtt ttttttcaga taaaaaaaat
1921 ccttagcttt cgctaaggat gatttctgca attggcggcc gcttctagaa tgcactagta
1981 gcggccgctg cagtccggca aaaaaacggg caaggtgtca ccaccctgcc ctttttcttt
2041 aaaaccgaaa agattacttc gcgttggaga gcgttcaccg acaaacaaca gataaaacga
2101 aaggcccagt ctttcgactg agcctttcgt tttatttgat gcctggtacc gggtgtaatc
2161 ggttgattat ttcagtggcc cggccgatgg ataattacca tggcccgacc gaccgggatt
2221 tccagaccag tttgaacagt attaaattag cctttccgga caaggcctct gtgaaaatct
2281 taaaaaagta ttaagatttt aaaagctagc ttattttcgg aggaaatgtg tttactcgac
2341 ttgcccagca acaccgcgat ttcgtgaagg atttagtcat gagcttgagg gctttggcca
2401 ctgtgctcga aaatcggggc tacattgctt cttgctacac ctgtggcgac caactcaaca
2461 gcgcctcctt catggtgagc ttgggggaaa atcatctgat ccgctttttg gtatcggact
2521 acggcatcac ctggacagaa atgcgggatg accgagaatt aatgaaatta gaaggagccg
```

2581 aggcgatcgc ccagttggaa gaattggcca atgtggtcaa atattgccta gcagatgccc
2641 ccactcgacc atatgggaga gctcccaacg cgttggatgc atagcttgag tattctatag
2701 tgtcacctaa atagcttggc gtaatcatgg tcatagctgt ttcctgtgtg aaattgttat
2761 ccgctcacaa ttccacacaa catacgagcc ggaagcataa agtgtaaagc ctggggtgcc
2821 taatgagtga gctaactcac attaattgcg ttgcgctcac tgcccgcttt ccagtcggga
2881 aacctgtcgt gccagctgca ttaatgaatc ggccaacgcg cggggagagg cggtttgcgt
2941 attgggcgct cttccgcttc ctcgctcact gactcgctgc gctcggtcgt tcggctgcgg
3001 cgagcggtat cagctcactc aaaggcggta atacggttat ccacagaatc aggggataac
3061 gcaggaaaga acatgtgagc aaaaggccag caaaaggcca ggaaccgtaa aaaggccgcg
3121 ttgctggcgt ttttccatag gctccgcccc cctgacgagc atcacaaaaa tcgacgctca
3181 agtcagaggt ggcgaaaccc gacaggacta taaagatacc aggcgtttcc ccctggaagc
3241 tccctcgtgc gctctcctgt tccgaccctg ccgcttaccg gatacctgtc cgcctttctc
3301 ccttcgggaa gcgtggcgct ttctcatagc tcacgctgta ggtatctcag ttcggtgtag
3361 gtcgttcgct ccaagctggg ctgtgtgcac gaaccccccg ttcagcccga ccgctgcgcc
3421 ttatccggta actatcgtct tgagtccaac ccggtaagac acgacttatc gccactggca
3481 gcagccactg gtaacaggat tagcagagcg aggtatgtag gcggtgctac agagttcttg
3541 aagtggtggc ctaactacgg ctacactaga agaacagtat ttggtatctg cgctctgctg
3601 aagccagtta ccttcggaaa aagagttggt agctcttgat ccggcaaaca aaccaccgct
3661 ggtagcggtg gtttttttgt ttgcaagcag cagattacgc gcagaaaaaa aggatctcaa
3721 gaagatcctt tgatcttttc tacggggtct gacgctcagt ggaacgaaaa ctcacgttaa
3781 gggattttgg tcatgagatt atcaaaaagg atcttcacct agatcctttt aaattaaaaa
3841 tgaagtttta aatcaatcta aagtatatat gagtaaactt ggtctgacag ttaccaatgc
3901 ttaatcagtg aggcacctat ctcagcgatc tgtctatttc gttcatccat agttgcctga
3961 ctccccgtcg tgtagataac tacgatacgg gagggcttac catctggccc cagtgctgca
4021 atgataccgc gagacccacg ctcaccggct ccagatttat cagcaataaa ccagccagcc
4081 ggaagggccg agcgcagaag tggtcctgca actttatccg cctccatcca gtctattaat
4141 tgttgccggg aagctagagt aagtagttcg ccagttaata gtttgcgcaa cgttgttgcc
4201 attgctacag gcatcgtggt gtcacgctcg tcgtttggta tggcttcatt cagctccggt
4261 tcccaacgat caaggcgagt tacatgatcc cccatgttgt gcaaaaaagc ggttagctcc
4321 ttcggtcctc cgatcgttgt cagaagtaag ttggccgcag tgttatcact catggttatg
4381 gcagcactgc ataattctct tactgtcatg ccatccgtaa gatgcttttc tgtgactggt
4441 gagtactcaa ccaagtcatt ctgagaatag tgtatgcggc gaccgagttg ctcttgcccg
4501 gcgtcaatac gggataatac cgcgccacat agcagaactt taaaagtgct catcattgga
4561 aaacgttctt cggggcgaaa actctcaagg atcttaccgc tgttgagatc cagttcgatg
4621 taacccactc gtgcacccaa ctgatcttca gcatctttta ctttcaccag cgtttctggg
4681 tgagcaaaaa caggaaggca aaatgccgca aaaaagggaa taagggcgac acggaaatgt
4741 tgaatactca tactcttcct ttttcaatat tattgaagca tttatcaggg ttattgtctc
4801 atgagcggat acatatttga atgtatttag aaaaataaac aaataggggt tccgcgcaca
4861 tttccccgaa aagtgccacc tgatgcggtg tgaaataccg cacagatgcg taaggagaaa
4921 ataccgcatc aggaaattgt aagcgttaat attttgttaa aattcgcgtt aaatttttgt
4981 taaatcagct cattttttaa ccaataggcc gaaatcggca aaatccctta taaatcaaaa
5041 gaatagaccg agatagggtt gagtgttgtt ccagtttgga acaagagtcc actattaaag
5101 aacgtggact ccaacgtcaa agggcgaaaa accgtctatc agggcgatgg cccactacgt
5161 gaaccatcac cctaatcaag ttttttgggg tcgaggtgcc gtaaagcact aaatcggaac
5221 cctaaaggga gcccccgatt tagagcttga cggggaaagc cggcgaacgt ggcgagaaag
5281 gaagggaaga aagcgaaagg agcgggcgct agggcgctgg caagtgtagc ggtcacgctg
5341 cgcgtaacca ccacacccgc cgcgcttaat gcgccgctac agggcgcgtc cattcgccat
5401 tcaggctgcg caactgttgg gaagggcgat cggtgcgggc ctcttcgcta ttacgccagc
5461 tggcgaaagg gggatgtgct gcaaggcgat taagttgggt aacgccaggg ttttcccagt
5521 cacgacgttg taaaacgacg gccagtgaat tgtaatacga ctcactatag ggcgaattgg
5581 gcccgacgtc gcatgctccc ggccgccatg gcggccgcgg gaattcgat

**SEQ ID NO: 7:**

```
   1 cacccgccgc gcttaatgcg ccgctacagg gcgcgtccat tcgccattca ggctgcgcaa
  61 ctgttgggaa gggcgatcgg tgcgggcctc ttcgctatta cgccagctgg cgaaaggggg
 121 atgtgctgca aggcgattaa gttgggtaac gccagggttt tcccagtcac gacgttgtaa
 181 aacgacggcc agtgaattgt aatacgactc actatagggc gaattgggcc cgacgtcgca
 241 tgctcccggc cgccatggcg gccgcgggaa ttcgattgat gaccgctggc ggagtttagt
 301 ccagggttat ttgtaccgtc aaggttacat tgccacccca gatttaacta cgctgcgagt
 361 ccgtaccatt ggcgatcggg cctatctcac cattaagggt aaaaatgcca gcattgcccg
 421 cttggatttg agtatgaaat tcttgcagtg gaagccaatt aaattttgac agaactctgt
 481 tcaccgcccc tgattgaaaa tatcgttatt gcctcgatta ccatggtaaa acctgagaag
 541 tagacgagtt tttgggggat aaccagggtc taattttagc ggaagtggaa ttaacctaca
 601 ctggtgaaaa aataagtcta cttccctgga tcggggaaga ggtaacggat gatgcccgct
 661 attacaacgt caatttagcc caacatccct acaaaaattg gtgacaagat tacacccggg
 721 cgatttactt ttcgacctca ttctattaga ctctcgtttg gattgcaact ggtctatttt
 781 cctcttttgt ttgatagaaa atcataaaag gatttgcaga ctacgggcct aaagaactaa
 841 aaaatctatc tgtttctttt cattctctgt atttttata gtttctgttg catgggcata
 901 aagttgcctt tttaatcaca attcagaaaa tatcataata tctcatttca ctaaataata
 961 gtgaacggca ggtatatgtg atgggttaaa aaggatcgat cctctagcga accccagagt
1021 cccgctcaga agaactcgtc aagaaggcga tagaaggcga tgcgctgcga atcgggagcg
1081 gcgataccgt aaagcacgag gaagcggtca gcccattcgc cgccaagctc ttcagcaata
1141 tcacgggtag ccaacgctat gtcctgatag cggtccgcca cacccagccg gccacagtcg
1201 atgaatccag aaaagcggcc attttccacc atgatattcg gcaagcaggc atcgccatgg
1261 gtcacgacga gatcctcgcc gtcgggcatc cgcgccttga gcctggcgaa cagttcggct
1321 ggcgcgagcc cctgatgctc ttcgtccaga tcatcctgat cgacaagacc ggcttccatc
1381 cgagtacgtg ctcgctcgat gcgatgtttc gcttggtggt cgaatgggca ggtagccgga
1441 tcaagcgtat gcagccgccg cattgcatca gccatgatgg atactttctc ggcaggagca
1501 aggtgagatg acaggagatc ctgccccggc acttcgccca atagcagcca gtcccttccc
1561 gcttcagtga caacgtcgag cacagctgcg caaggaacgc ccgtcgtggc cagccacgat
1621 agccgcgctg cctcgtcttg gagttcattc agggcaccgg acaggtcggt cttgacaaaa
1681 agaaccgggc gcccctgcgc tgacagccgg aacacggcgg catcagagca gccgattgtc
1741 tgttgtgccc agtcatagcc gaatagcctc tccacccaag cggccggaga acctgcgtgc
1801 aatccatctt gttcaatcat gcgaaacgat cctcatcctg tctcttgatc agatcttgat
1861 cccctgcgcc atcagatcct tggcggcaag aaagccatcc agtttacttt gcagggcttc
1921 ccaaccttac cagagggcgc cccagctggc aattccggtt cgcttgctgt ccataaaacc
1981 gcccagtcta gctatcgcca tgtaagccca ctgcaagcta cctgctttct ctttgcgctt
2041 gcgttttccc ttgtccagat agcccagtag ctgacattca cccgggaaaa aaaaaccccg
2101 cccctgacag ggcggggttt tttttcagat aaaaaaaatc cttagctttc gctaaggatg
2161 atttctgcaa ttggcggccg cttctagaat gcactagtag cggccgctgc agtccggcaa
2221 aaaaacgggc aaggtgtcac caccctgccc tttttcttta aaaccgaaaa gattacttcg
2281 cgttggagag cgttcaccga caaacaacag ataaaacgaa aggcccagtc tttcgactga
2341 gcctttcgtt ttatttgatg cctggtaccg ggtgtaatcc ttcattcccc tattgttatt
2401 aatagtgctc tgaaactaac aaataagcaa gatcaaattg tgaagaaaac gcaacaaaat
2461 gaaccattaa gataatttct gtctatcctg gctggtatat tccagtctac tcagaaatga
2521 gtttgtttta tgacaaatca gtatgtacca aattacttgg cgcaatcgat cctggtaact
2581 ctattttgtt gcttacccct gggtattgtg gccattatca aagcatcgga agttaattct
2641 cgtttagctt caggggacta tgaaggcgct gtaaaggctt ccaaggaagc gaaaaagttt
2701 tgttggtggt cctttggtgc cggcataatt ttcattgcca tctattttgt gctagtggtt
2761 attgccgccg tctttggtca gtaattaaag ttacattttt tgactttgcc ttgttcacca
2821 ttcattaacg aataccatgt ttagtttgaa aaattattcc ccatctccat tactatccgc
2881 taaggccaag gaatatttag tattcacttt ggtaacccta accattgtcg ttgctggcac
2941 tcgaccatat gggagagctc ccaacgcgtt ggatgcatag cttgagtatt ctatagtgtc
3001 acctaaatag cttggcgtaa tcatggtcat agctgtttcc tgtgtgaaat tgttatccgc
3061 tcacaattcc acacaacata cgagccggaa gcataaagtg taaagcctgg ggtgcctaat
```

3121 gagtgagcta actcacatta attgcgttgc gctcactgcc cgctttccag tcgggaaacc
3181 tgtcgtgcca gctgcattaa tgaatcggcc aacgcgcggg gagaggcggt ttgcgtattg
3241 ggcgctcttc cgcttcctcg ctcactgact cgctgcgctc ggtcgttcgg ctgcggcgag
3301 cggtatcagc tcactcaaag gcggtaatac ggttatccac agaatcaggg gataacgcag
3361 gaaagaacat gtgagcaaaa ggccagcaaa aggccaggaa ccgtaaaaag gccgcgttgc
3421 tggcgttttt ccataggctc cgcccccctg acgagcatca caaaaatcga cgctcaagtc
3481 agaggtggcg aaacccgaca ggactataaa gataccaggc gtttccccct ggaagctccc
3541 tcgtgcgctc tcctgttccg accctgccgc ttaccggata cctgtccgcc tttctccctt
3601 cgggaagcgt ggcgctttct catagctcac gctgtaggta tctcagttcg gtgtaggtcg
3661 ttcgctccaa gctgggctgt gtgcacgaac cccccgttca gcccgaccgc tgcgccttat
3721 ccggtaacta tcgtcttgag tccaacccgg taagacacga cttatcgcca ctggcagcag
3781 ccactggtaa caggattagc agagcgaggt atgtaggcgg tgctacagag ttcttgaagt
3841 ggtggcctaa ctacggctac actagaagaa cagtatttgg tatctgcgct ctgctgaagc
3901 cagttacctt cggaaaaaga gttggtagct cttgatccgg caaacaaacc accgctggta
3961 gcggtggttt ttttgtttgc aagcagcaga ttacgcgcag aaaaaaagga tctcaagaag
4021 atcctttgat cttttctacg gggtctgacg ctcagtggaa cgaaaactca cgttaaggga
4081 ttttggtcat gagattatca aaaaggatct tcacctagat ccttttaaat taaaaatgaa
4141 gttttaaatc aatctaaagt atatatgagt aaacttggtc tgacagttac caatgcttaa
4201 tcagtgaggc acctatctca gcgatctgtc tatttcgttc atccatagtt gcctgactcc
4261 ccgtcgtgta gataactacg atacgggagg gcttaccatc tggccccagt gctgcaatga
4321 taccgcgaga cccacgctca ccggctccag atttatcagc aataaaccag ccagccggaa
4381 gggccgagcg cagaagtggt cctgcaactt tatccgcctc catccagtct attaattgtt
4441 gccgggaagc tagagtaagt agttcgccag ttaatagttt gcgcaacgtt gttgccattg
4501 ctacaggcat cgtggtgtca cgctcgtcgt ttggtatggc ttcattcagc tccggttccc
4561 aacgatcaag gcgagttaca tgatccccca tgttgtgcaa aaaagcggtt agctccttcg
4621 gtcctccgat cgttgtcaga agtaagttgg ccgcagtgtt atcactcatg gttatggcag
4681 cactgcataa ttctcttact gtcatgccat ccgtaagatg cttttctgtg actggtgagt
4741 actcaaccaa gtcattctga gaatagtgta tgcggcgacc gagttgctct tgcccggcgt
4801 caatacggga taataccgcg ccacatagca gaactttaaa agtgctcatc attggaaaac
4861 gttcttcggg gcgaaaactc tcaaggatct taccgctgtt gagatccagt tcgatgtaac
4921 ccactcgtgc acccaactga tcttcagcat cttttacttt caccagcgtt tctgggtgag
4981 caaaaacagg aaggcaaaat gccgcaaaaa agggaataag ggcgacacgg aaatgttgaa
5041 tactcatact cttccttttt caatattatt gaagcattta tcagggttat tgtctcatga
5101 gcggatacat atttgaatgt atttagaaaa ataaacaaat aggggttccg cgcacatttc
5161 cccgaaaagt gccacctgat gcggtgtgaa ataccgcaca gatgcgtaag gagaaaatac
5221 cgcatcagga aattgtaagc gttaatattt tgttaaaatt cgcgttaaat ttttgttaaa
5281 tcagctcatt ttttaaccaa taggccgaaa tcggcaaaat cccttataaa tcaaaagaat
5341 agaccgagat agggttgagt gttgttccag tttggaacaa gagtccacta ttaaagaacg
5401 tggactccaa cgtcaaaggg cgaaaaaccg tctatcaggg cgatggccca ctacgtgaac
5461 catcacccta atcaagtttt ttggggtcga ggtgccgtaa agcactaaat cggaacccta
5521 aagggagccc ccgatttaga gcttgacggg gaaagccggc gaacgtggcg agaaaggaag
5581 ggaagaaagc gaaaggagcg ggcgctaggg cgctggcaag tgtagcggtc acgctgcgcg
5641 taaccacca

**SEQ ID NO: 8:**

1 cacccgccgc gcttaatgcg ccgctacagg gcgcgtccat tcgccattca ggctgcgcaa
61 ctgttgggaa gggcgatcgg tgcgggcctc ttcgctatta cgccagctgg cgaaaggggg
121 atgtgctgca aggcgattaa gttgggtaac gccagggttt tcccagtcac gacgttgtaa
181 aacgacggcc agtgaattgt aatacgactc actatagggc gaattgggcc cgacgtcgca
241 tgctcccggc cgccatggcg gccgcgggaa ttcgattgga ctgacccaag atacagtggt
301 agcttcaagt tcgtcaatca accccaatgg aaagctgaga taatctgttg ttgatgcgac
361 cgtcttcatt ttagctaaaa agacaagtct gtggctagtt actatgacga ggccatcggg

421 tctatcccag cggtcttgct gattccaaaa cttggcgatt ttctgaagta gtatcttttc
481 accgcttaag ccgccaaggt cttggatgag cttggtcgaa tcatcttttg atgcctcgac
541 atcgcgaata actttgtaaa cgaccttccc gagcttgaat gcaaagtcgg ttagattatc
601 catcagagtt gaatgccgat aatacagact gctatactat tttaatgaaa agagtgctgg
661 caagcaagcc cgccgaacgt tgaagctgtg cagcgcaaac gaagcgcatt gtagtttgcg
721 ctcggattac acccgggcga tttacttttc gacctcattc tattagactc tcgtttggat
781 tgcaactggt ctattttcct cttttgtttg atagaaaatc ataaaaggat ttgcagacta
841 cgggcctaaa gaactaaaaa atctatctgt ttcttttcat tctctgtatt ttttatagtt
901 tctgttgcat gggcataaag ttgccttttt aatcacaatt cagaaaatat cataatatct
961 catttcacta aataatagtg aacggcaggt atatgtgatg ggttaaaaag gatcgatcct
1021 ctagcgaacc ccagagtccc gctcagaaga actcgtcaag aaggcgatag aaggcgatgc
1081 gctgcgaatc gggagcggcg ataccgtaaa gcacgaggaa gcggtcagcc cattcgccgc
1141 caagctcttc agcaatatca cgggtagcca acgctatgtc ctgatagcgg tccgccacac
1201 ccagccggcc acagtcgatg aatccagaaa agcggccatt ttccaccatg atattcggca
1261 agcaggcatc gccatgggtc acgacgagat cctcgccgtc gggcatccgc gccttgagcc
1321 tggcgaacag ttcggctggc gcgagcccct gatgctcttc gtccagatca tcctgatcga
1381 caagaccggc ttccatccga gtacgtgctc gctcgatgcg atgtttcgct tggtggtcga
1441 atgggcaggt agccggatca agcgtatgca gccgccgcat tgcatcagcc atgatggata
1501 ctttctcggc aggagcaagg tgagatgaca ggagatcctg ccccggcact tcgcccaata
1561 gcagccagtc ccttcccgct tcagtgacaa cgtcgagcac agctgcgcaa ggaacgcccg
1621 tcgtggccag ccacgatagc cgcgctgcct cgtcttggag ttcattcagg gcaccggaca
1681 ggtcggtctt gacaaaaaga accgggcgcc cctgcgctga cagccggaac acggcggcat
1741 cagagcagcc gattgtctgt tgtgcccagt catagccgaa tagcctctcc acccaagcgg
1801 ccggagaacc tgcgtgcaat ccatcttgtt caatcatgcg aaacgatcct catcctgtct
1861 cttgatcaga tcttgatccc ctgcgccatc agatccttgg cggcaagaaa gccatccagt
1921 ttactttgca gggcttccca accttaccag agggcgcccc agctggcaat tccggttcgc
1981 ttgctgtcca taaaaccgcc cagtctagct atcgccatgt aagcccactg caagctacct
2041 gctttctctt tgcgcttgcg ttttcccttg tccagatagc ccagtagctg acattcaccc
2101 gggaaaaaaa aaccccgccc ctgacagggc ggggtttttt ttcagataaa aaaaatcctt
2161 agctttcgct aaggatgatt tctgcaattg gcggccgctt ctagaatgca ctagtagcgg
2221 ccgctgcagt ccggcaaaaa aacgggcaag gtgtcaccac cctgcccttt ttctttaaaa
2281 ccgaaaagat tacttcgcgt tggagagcgt tcaccgacaa acaacagata aaacgaaagg
2341 cccagtcttt cgactgagcc tttcgtttta tttgatgcct ggtaccgggt gtaatcccca
2401 tactcagcct tctaatgctg agagaatgcc tgaaaagaac cactaagtca aattatttgg
2461 attaagtttt attaagtcaa cggaggtgtc agatagcatt tagatcgtaa aagcatacgg
2521 catgaacaac catgaacggc ttcccagagg ttaaccccag gtttatggtt taccaaaatc
2581 ccctgacttt ttccgagttg ggggacaaaa tcaactaatt tgggcgacga ttttttctgc
2641 tatctggcga tcgccatgtt tgtctaactt ctgatccaac ctaggttttt gggggctttc
2701 caacaaaaaa tcccagttgc cggcgaaaaa ttgctggtgg ggcacaattt ggtgctctcc
2761 gtagttctgc aaacctgcca gtaaaacttg cgcttccgca aaaccatccc tggtgaggga
2821 aataatcggg acttctaact ttaacgcttc agcaaaggta ccaaaaccag gcttggaaaa
2881 tacccgtccg caaaggggca ttaaatccac cgggcgactc gaccatatgg gagagctccc
2941 aacgcgttgg atgcatagct tgagtattct atagtgtcac ctaaatagct tggcgtaatc
3001 atggtcatag ctgtttcctg tgtgaaattg ttatccgctc acaattccac acaacatacg
3061 agccggaagc ataaagtgta aagcctgggg tgcctaatga gtgagctaac tcacattaat
3121 tgcgttgcgc tcactgcccg ctttccagtc gggaaacctg tcgtgccagc tgcattaatg
3181 aatcggccaa cgcgcgggga gaggcggttt gcgtattggg cgctcttccg cttcctcgct
3241 cactgactcg ctgcgctcgg tcgttcggct gcggcgagcg gtatcagctc actcaaaggc
3301 ggtaatacgg ttatccacag aatcagggga taacgcagga aagaacatgt gagcaaaagg
3361 ccagcaaaag gccaggaacc gtaaaaaggc cgcgttgctg gcgtttttcc ataggctccg
3421 cccccctgac gagcatcaca aaaatcgacg ctcaagtcag aggtggcgaa acccgacagg
3481 actataaaga taccaggcgt ttccccctgg aagctccctc gtgcgctctc ctgttccgac
3541 cctgccgctt accggatacc tgtccgcctt tctcccttcg ggaagcgtgg cgctttctca

3601 tagctcacgc tgtaggtatc tcagttcggt gtaggtcgtt cgctccaagc tgggctgtgt
3661 gcacgaaccc cccgttcagc ccgaccgctg cgccttatcc ggtaactatc gtcttgagtc
3721 caacccggta agacacgact tatcgccact ggcagcagcc actggtaaca ggattagcag
3781 agcgaggtat gtaggcggtg ctacagagtt cttgaagtgg tggcctaact acggctacac
3841 tagaagaaca gtatttggta tctgcgctct gctgaagcca gttaccttcg gaaaaagagt
3901 tggtagctct tgatccggca aacaaaccac cgctggtagc ggtggttttt ttgtttgcaa
3961 gcagcagatt acgcgcagaa aaaaaggatc tcaagaagat cctttgatct tttctacggg
4021 gtctgacgct cagtggaacg aaaactcacg ttaagggatt ttggtcatga gattatcaaa
4081 aaggatcttc acctagatcc ttttaaatta aaaatgaagt tttaaatcaa tctaaagtat
4141 atatgagtaa acttggtctg acagttacca atgcttaatc agtgaggcac ctatctcagc
4201 gatctgtcta tttcgttcat ccatagttgc ctgactcccc gtcgtgtaga taactacgat
4261 acgggagggc ttaccatctg gccccagtgc tgcaatgata ccgcgagacc cacgctcacc
4321 ggctccagat ttatcagcaa taaaccagcc agccggaagg gccgagcgca gaagtggtcc
4381 tgcaacttta tccgcctcca tccagtctat taattgttgc cgggaagcta gagtaagtag
4441 ttcgccagtt aatagtttgc gcaacgttgt tgccattgct acaggcatcg tggtgtcacg
4501 ctcgtcgttt ggtatggctt cattcagctc cggttcccaa cgatcaaggc gagttacatg
4561 atcccccatg ttgtgcaaaa aagcggttag ctccttcggt cctccgatcg ttgtcagaag
4621 taagttggcc gcagtgttat cactcatggt tatggcagca ctgcataatt ctcttactgt
4681 catgccatcc gtaagatgct tttctgtgac tggtgagtac tcaaccaagt cattctgaga
4741 atagtgtatg cggcgaccga gttgctcttg cccggcgtca atacgggata ataccgcgcc
4801 acatagcaga actttaaaag tgctcatcat tggaaaacgt tcttcggggc gaaaactctc
4861 aaggatctta ccgctgttga gatccagttc gatgtaaccc actcgtgcac ccaactgatc
4921 ttcagcatct tttactttca ccagcgtttc tgggtgagca aaaacaggaa ggcaaaatgc
4981 cgcaaaaaag ggaataaggg cgacacggaa atgttgaata ctcatactct tcctttttca
5041 atattattga agcatttatc agggttattg tctcatgagc ggatacatat ttgaatgtat
5101 ttagaaaaat aaacaaatag gggttccgcg cacatttccc cgaaaagtgc cacctgatgc
5161 ggtgtgaaat accgcacaga tgcgtaagga gaaaataccg catcaggaaa ttgtaagcgt
5221 taatattttg ttaaaattcg cgttaaattt ttgttaaatc agctcatttt ttaaccaata
5281 ggccgaaatc ggcaaaatcc cttataaatc aaaagaatag accgagatag ggttgagtgt
5341 tgttccagtt tggaacaaga gtccactatt aaagaacgtg gactccaacg tcaaagggcg
5401 aaaaaccgtc tatcagggcg atggcccact acgtgaacca tcaccctaat caagtttttt
5461 ggggtcgagg tgccgtaaag cactaaatcg gaaccctaaa gggagccccc gatttagagc
5521 ttgacgggga aagccggcga acgtggcgag aaaggaaggg aagaaagcga aaggagcggg
5581 cgctagggcg ctggcaagtg tagcggtcac gctgcgcgta accacca

**SEQ ID NO:9:**

1 cacccgccgc gcttaatgcg ccgctacagg gcgcgtccat tcgccattca ggctgcgcaa
61 ctgttgggaa gggcgatcgg tgcgggcctc ttcgctatta cgccagctgg cgaaaggggg
121 atgtgctgca aggcgattaa gttgggtaac gccagggttt tcccagtcac gacgttgtaa
181 aacgacggcc agtgaattgt aatacgactc actatagggc gaattgggcc cgacgtcgca
241 tgctcccggc cgccatggcg gccgcgggaa ttcgattcta aacttacggc attggcatca
301 acgggagcca ccgtcggagt aggggaagta acaacggggg aactggtatt ggtgggcaaa
361 atttttacta gccattgatt ccattggggc agattgaacg ctcccaacca ccaaagtccc
421 cctaatacaa cgacagaact aaataacagg aaaaaaggaa tccaaggagt taccccctctt
481 ttatgggatg gaacttcatc gacattaaag gtgggagggg gaggaggcaa tggggacaat
541 ggtggtccag aaaaggaagg tggctccgac gtcaaggcaa cgggacatcc acaggattca
601 cagaaacgaa cctgggggct aaggcggttg ccacaattag tacaaaaaac gggagtagtc
661 ataggtgaaa accccgacta tagaattaga aaaatttaac tttttatccg aattttattc
721 gtccatgttc cccaaataac tatcaaaata attggaaaaa ttaaattatt tggtcgttgg
781 tcaccgctcc ctaaagacct ggccattgta aagagattac acccgggcga tttactttc
841 gacctcattc tattagactc tcgtttggat tgcaactggt ctattttcct cttttgtttg
901 atagaaaatc ataaaaggat ttgcagacta cgggcctaaa gaactaaaaa atctatctgt

961 ttcttttcat tctctgtatt ttttatagtt tctgttgcat gggcataaag ttgccttttt
1021 aatcacaatt cagaaaatat cataatatct catttcacta aataatagtg aacggcaggt
1081 atatgtgatg ggttaaaaag gatcgatcct ctagcgaacc ccagagtccc gctcagaaga
1141 actcgtcaag aaggcgatag aaggcgatgc gctgcgaatc gggagcggcg ataccgtaaa
1201 gcacgaggaa gcggtcagcc cattcgccgc caagctcttc agcaatatca cgggtagcca
1261 acgctatgtc ctgatagcgg tccgccacac ccagccggcc acagtcgatg aatccagaaa
1321 agcggccatt ttccaccatg atattcggca agcaggcatc gccatgggtc acgacgagat
1381 cctcgccgtc gggcatccgc gccttgagcc tggcgaacag ttcggctggc gcgagcccct
1441 gatgctcttc gtccagatca tcctgatcga caagaccggc ttccatccga gtacgtgctc
1501 gctcgatgcg atgtttcgct tggtggtcga atgggcaggt agccggatca agcgtatgca
1561 gccgccgcat tgcatcagcc atgatggata ctttctcggc aggagcaagg tgagatgaca
1621 ggagatcctg ccccggcact tcgcccaata gcagccagtc ccttcccgct tcagtgacaa
1681 cgtcgagcac agctgcgcaa ggaacgcccg tcgtggccag ccacgatagc cgcgctgcct
1741 cgtcttggag ttcattcagg gcaccggaca ggtcggtctt gacaaaaaga accgggcgcc
1801 cctgcgctga cagccggaac acggcggcat cagagcagcc gattgtctgt tgtgcccagt
1861 catagccgaa tagcctctcc acccaagcgg ccggagaacc tgcgtgcaat ccatcttgtt
1921 caatcatgcg aaacgatcct catcctgtct cttgatcaga tcttgatccc ctgcgccatc
1981 agatccttgg cggcaagaaa gccatccagt ttactttgca gggcttccca accttaccag
2041 agggcgcccc agctggcaat tccggttcgc ttgctgtcca taaaaccgcc cagtctagct
2101 atcgccatgt aagcccactg caagctacct gctttctctt tgcgcttgcg ttttcccttg
2161 tccagatagc ccagtagctg acattcaccc gggaaaaaaa aaccccgccc ctgacagggc
2221 ggggtttttt ttcagataaa aaaaatcctt agctttcgct aaggatgatt tctgcaattg
2281 gcggccgctt ctagaatgca ctagtagcgg ccgctgcagt ccggcaaaaa aacgggcaag
2341 gtgtcaccac cctgcccttt ttctttaaaa ccgaaaagat tacttcgcgt tggagagcgt
2401 tcaccgacaa acaacagata aaacgaaagg cccagtcttt cgactgagcc tttcgtttta
2461 tttgatgcct ggtaccgggt gtaatccatt gggcacgaga gttagtaagg cagtggcaat
2521 taatagaggc ttatggttga ttcgcattgt tttgctcctg aaattttcgg caaatacaaa
2581 tacttcgctc ttctagccct attaaccatt ttaacgacaa attgatgggg caacgattaa
2641 caaataatga ataaatttta tgtttttcaa gatgaaaatt tgaaaatttg atttccttat
2701 atttctacta tagaagacta atacaattag atctaaaatt tgcaagtata aaaatcagca
2761 aatagttata ttgttaataa ttcaatgacc caataactcg tactgttatc tacgtggtga
2821 aagccaaaaa gacgaacagt ttagcctcct cctcctcggc gatcgccaag cgaaatgtca
2881 tgggagatgt tcagattgag catttttttc taaaagccct tgctaaaaca aaccacatgt
2941 gcagggtgtc cccgatgttg actaaattca gcggctcgac catatgggag agctcccaac
3001 gcgttggatg catagcttga gtattctata gtgtcaccta aatagcttgg cgtaatcatg
3061 gtcatagctg tttcctgtgt gaaattgtta tccgctcaca attccacaca acatacgagc
3121 cggaagcata aagtgtaaag cctggggtgc ctaatgagtg agctaactca cattaattgc
3181 gttgcgctca ctgcccgctt tccagtcggg aaacctgtcg tgccagctgc attaatgaat
3241 cggccaacgc gcggggagag gcggtttgcg tattgggcgc tcttccgctt cctcgctcac
3301 tgactcgctg cgctcggtcg ttcggctgcg gcgagcggta tcagctcact caaaggcggt
3361 aatacggtta tccacagaat caggggataa cgcaggaaag aacatgtgag caaaaggcca
3421 gcaaaaggcc aggaaccgta aaaaggccgc gttgctggcg tttttccata ggctccgccc
3481 ccctgacgag catcacaaaa atcgacgctc aagtcagagg tggcgaaacc cgacaggact
3541 ataaagatac caggcgtttc cccctggaag ctccctcgtg cgctctcctg ttccgaccct
3601 gccgcttacc ggatacctgt ccgcctttct cccttcggga agcgtggcgc tttctcatag
3661 ctcacgctgt aggtatctca gttcggtgta ggtcgttcgc tccaagctgg gctgtgtgca
3721 cgaacccccc gttcagcccg accgctgcgc cttatccggt aactatcgtc ttgagtccaa
3781 cccggtaaga cacgacttat cgccactggc agcagccact ggtaacagga ttagcagagc
3841 gaggtatgta ggcggtgcta cagagttctt gaagtggtgg cctaactacg gctacactag
3901 aagaacagta tttggtatct gcgctctgct gaagccagtt accttcggaa aaagagttgg
3961 tagctcttga tccggcaaac aaaccaccgc tggtagcggt ggtttttttg tttgcaagca
4021 gcagattacg cgcagaaaaa aaggatctca agaagatcct ttgatctttt ctacggggtc
4081 tgacgctcag tggaacgaaa actcacgtta agggattttg gtcatgagat tatcaaaaag

```
4141 gatcttcacc tagatccttt taaattaaaa atgaagtttt aaatcaatct aaagtatata
4201 tgagtaaact tggtctgaca gttaccaatg cttaatcagt gaggcaccta tctcagcgat
4261 ctgtctattt cgttcatcca tagttgcctg actccccgtc gtgtagataa ctacgatacg
4321 ggagggctta ccatctggcc ccagtgctgc aatgataccg cgagacccac gctcaccggc
4381 tccagattta tcagcaataa accagccagc cggaagggcc gagcgcagaa gtggtcctgc
4441 aactttatcc gcctccatcc agtctattaa ttgttgccgg gaagctagag taagtagttc
4501 gccagttaat agtttgcgca acgttgttgc cattgctaca ggcatcgtgg tgtcacgctc
4561 gtcgtttggt atggcttcat tcagctccgg ttcccaacga tcaaggcgag ttacatgatc
4621 ccccatgttg tgcaaaaaag cggttagctc cttcggtcct ccgatcgttg tcagaagtaa
4681 gttggccgca gtgttatcac tcatggttat ggcagcactg cataattctc ttactgtcat
4741 gccatccgta agatgctttt ctgtgactgg tgagtactca accaagtcat tctgagaata
4801 gtgtatgcgg cgaccgagtt gctcttgccc ggcgtcaata cgggataata ccgcgccaca
4861 tagcagaact ttaaaagtgc tcatcattgg aaaacgttct tcggggcgaa aactctcaag
4921 gatcttaccg ctgttgagat ccagttcgat gtaacccact cgtgcaccca actgatcttc
4981 agcatctttt actttcacca gcgtttctgg gtgagcaaaa acaggaaggc aaaatgccgc
5041 aaaaaaggga ataagggcga cacggaaatg ttgaatactc atactcttcc tttttcaata
5101 ttattgaagc atttatcagg gttattgtct catgagcgga tacatatttg aatgtattta
5161 gaaaaataaa caaatagggg ttccgcgcac atttccccga aaagtgccac ctgatgcggt
5221 gtgaaatacc gcacagatgc gtaaggagaa aataccgcat caggaaattg taagcgttaa
5281 tattttgtta aaattcgcgt taaatttttg ttaaatcagc tcatttttta accaataggc
5341 cgaaatcggc aaaatccctt ataaatcaaa agaatagacc gagatagggt tgagtgttgt
5401 tccagtttgg aacaagagtc cactattaaa gaacgtggac tccaacgtca aagggcgaaa
5461 aaccgtctat cagggcgatg gcccactacg tgaaccatca ccctaatcaa gtttttttggg
5521 gtcgaggtgc cgtaaagcac taaatcggaa ccctaaaggg agcccccgat ttagagcttg
5581 acggggaaag ccggcgaacg tggcgagaaa ggaagggaag aaagcgaaag gagcgggcgc
5641 tagggcgctg gcaagtgtag cggtcacgct gcgcgtaacc acca
```

**SEQ ID NO: 10:**

```
1 cacccgccgc gcttaatgcg ccgctacagg gcgcgtccat tcgccattca ggctgcgcaa
61 ctgttgggaa gggcgatcgg tgcgggcctc ttcgctatta cgccagctgg cgaaaggggg
121 atgtgctgca aggcgattaa gttgggtaac gccagggttt tcccagtcac gacgttgtaa
181 aacgacggcc agtgaattgt aatacgactc actatagggc gaattgggcc cgacgtcgca
241 tgctcccggc cgccatggcg gccgcgggaa ttcgattgtg agcttgatgg tgatggtggg
301 taaagcttca ttgtccatgc gccggagctt gacggtaaaa tccgtgtcct ggggccaatc
361 attgggaacg ctcacacaga taccgttgcg agttaagtcg aaggataccc ccaccaattc
421 cccttccata tcgaacactt ggcaaaaggc caacatccgc tcttcctgcc gtcgctcctg
481 caccccctgg agatgggcaa tggttagggc ttcttgggag agggaagatg gagaatcttg
541 agccatggag attatttcct cggttaaatt aggtttacct agtaaatggg ccaggttgac
601 cattattgta gtcattcact gggtcgatct tccttccatc agggtcattt ggttaattgt
661 tgatgagaaa tgggaaggag taatccatag atatttgccc agttaactcg atttgagcag
721 aattgggaag gacgtattgg gaactttggt tgacagggca acaaagccag cagattacac
781 ccgggcgatt tacttttcga cctcattcta ttagactctc gtttggattg caactggtct
841 attttcctct tttgtttgat agaaaatcat aaaaggattt gcagactacg ggcctaaaga
901 actaaaaaat ctatctgttt cttttcattc tctgtatttt ttatagtttc tgttgcatgg
961 gcataaagtt gccttttttaa tcacaattca gaaaatatca taatatctca tttcactaaa
1021 taatagtgaa cggcaggtat atgtgatggg ttaaaaagga tcgatcctct agcgaacccc
1081 agagtcccgc tcagaagaac tcgtcaagaa ggcgatagaa ggcgatgcgc tgcgaatcgg
1141 gagcggcgat accgtaaagc acgaggaagc ggtcagccca ttcgccgcca agctcttcag
1201 caatatcacg ggtagccaac gctatgtcct gatagcggtc cgccacaccc agccggccac
1261 agtcgatgaa tccagaaaag cggccatttt ccaccatgat attcggcaag caggcatcgc
1321 catgggtcac gacgagatcc tcgccgtcgg gcatccgcgc cttgagcctg gcgaacagtt
1381 cggctggcgc gagcccctga tgctcttcgt ccagatcatc ctgatcgaca agaccggctt
```

1441 ccatccgagt acgtgctcgc tcgatgcgat gtttcgcttg gtggtcgaat gggcaggtag
1501 ccggatcaag cgtatgcagc cgccgcattg catcagccat gatggatact ttctcggcag
1561 gagcaaggtg agatgacagg agatcctgcc ccggcacttc gcccaatagc agccagtccc
1621 ttcccgcttc agtgacaacg tcgagcacag ctgcgcaagg aacgcccgtc gtggccagcc
1681 acgatagccg cgctgcctcg tcttggagtt cattcagggc accggacagg tcggtcttga
1741 caaaaagaac cgggcgcccc tgcgctgaca gccggaacac ggcggcatca gagcagccga
1801 ttgtctgttg tgcccagtca tagccgaata gcctctccac ccaagcggcc ggagaacctg
1861 cgtgcaatcc atcttgttca atcatgcgaa acgatcctca tcctgtctct tgatcagatc
1921 ttgatcccct gcgccatcag atccttggcg gcaagaaagc catccagttt actttgcagg
1981 gcttcccaac cttaccagag ggcgccccag ctggcaattc cggttcgctt gctgtccata
2041 aaaccgccca gtctagctat cgccatgtaa gcccactgca agctacctgc tttctctttg
2101 cgcttgcgtt ttcccttgtc cagatagccc agtagctgac attcacccgg gaaaaaaaaa
2161 ccccgcccct gacagggcgg ggtttttttt cagataaaaa aaatccttag ctttcgctaa
2221 ggatgatttc tgcaattggc ggccgcttct agaatgcact agtagcggcc gctgcagtcc
2281 ggcaaaaaaa cgggcaaggt gtcaccaccc tgcccttttt ctttaaaacc gaaaagatta
2341 cttcgcgttg gagagcgttc accgacaaac aacagataaa acgaaaggcc cagtctttcg
2401 actgagcctt tcgttttatt tgatgcctgg taccgggtgt aatcgctgga ggcgaactgg
2461 gtgagaacca taaaatcttg gggaacaggg gaatgttaat gaacacatga catgcatagg
2521 atagcgaatt gatttaaagc aatgattccc cctgagataa atatattttg acccactgct
2581 ccgcttaatt ttggcgctaa tctgggcaaa aattcgttgg ttttcggaaa ggtgaacccg
2641 tagctagctt gtattgccca aacctaactc atcattgctc catggcggcc aaaaatcttt
2701 aggacaaaac tcgccagagg tcaaggcttg attttttcag agggaaaaat tcctcggcta
2761 aataatcaaa aagttgaaaa aaaaagtttt tcagctaagg tttgatgtta tattttatct
2821 tatgtagact tttgaaaaaa acaagtcccc gcaatcaagc atcattcaac ggaaaaaata
2881 atcctatgcc aaacgcctcc accgcactaa ccggcaaagc attactcaat aaagttaaag
2941 aactatccca tctgccccgt cgagaaacgg caaaagcctg tggttactac tcgaccatat
3001 gggagagctc ccaacgcgtt ggatgcatag cttgagtatt ctatagtgtc acctaaatag
3061 cttggcgtaa tcatggtcat agctgtttcc tgtgtgaaat tgttatccgc tcacaattcc
3121 acacaacata cgagccggaa gcataaagtg taaagcctgg ggtgcctaat gagtgagcta
3181 actcacatta attgcgttgc gctcactgcc cgctttccag tcgggaaacc tgtcgtgcca
3241 gctgcattaa tgaatcggcc aacgcgcggg gagaggcggt ttgcgtattg ggcgctcttc
3301 cgcttcctcg ctcactgact cgctgcgctc ggtcgttcgg ctgcggcgag cggtatcagc
3361 tcactcaaag gcggtaatac ggttatccac agaatcaggg gataacgcag gaaagaacat
3421 gtgagcaaaa ggccagcaaa aggccaggaa ccgtaaaaag gccgcgttgc tggcgttttt
3481 ccataggctc cgcccccctg acgagcatca caaaaatcga cgctcaagtc agaggtggcg
3541 aaacccgaca ggactataaa gataccaggc gtttccccct ggaagctccc tcgtgcgctc
3601 tcctgttccg accctgccgc ttaccggata cctgtccgcc tttctccctt cgggaagcgt
3661 ggcgctttct catagctcac gctgtaggta tctcagttcg gtgtaggtcg ttcgctccaa
3721 gctgggctgt gtgcacgaac cccccgttca gcccgaccgc tgcgccttat ccggtaacta
3781 tcgtcttgag tccaacccgg taagacacga cttatcgcca ctggcagcag ccactggtaa
3841 caggattagc agagcgaggt atgtaggcgg tgctacagag ttcttgaagt ggtggcctaa
3901 ctacggctac actagaagaa cagtatttgg tatctgcgct ctgctgaagc cagttacctt
3961 cggaaaaaga gttggtagct cttgatccgg caaacaaacc accgctggta gcggtggttt
4021 ttttgtttgc aagcagcaga ttacgcgcag aaaaaaagga tctcaagaag atcctttgat
4081 cttttctacg gggtctgacg ctcagtggaa cgaaaactca cgttaaggga ttttggtcat
4141 gagattatca aaaaggatct tcacctagat ccttttaaat taaaaatgaa gttttaaatc
4201 aatctaaagt atatatgagt aaacttggtc tgacagttac caatgcttaa tcagtgaggc
4261 acctatctca gcgatctgtc tatttcgttc atccatagtt gcctgactcc ccgtcgtgta
4321 gataactacg atacgggagg gcttaccatc tggccccagt gctgcaatga taccgcgaga
4381 cccacgctca ccggctccag atttatcagc aataaaccag ccagccggaa gggccgagcg
4441 cagaagtggt cctgcaactt tatccgcctc catccagtct attaattgtt gccgggaagc
4501 tagagtaagt agttcgccag ttaatagttt gcgcaacgtt gttgccattg ctacaggcat
4561 cgtggtgtca cgctcgtcgt ttggtatggc ttcattcagc tccggttccc aacgatcaag

```
4621 gcgagttaca tgatccccca tgttgtgcaa aaaagcggtt agctccttcg gtcctccgat
4681 cgttgtcaga agtaagttgg ccgcagtgtt atcactcatg gttatggcag cactgcataa
4741 ttctcttact gtcatgccat ccgtaagatg cttttctgtg actggtgagt actcaaccaa
4801 gtcattctga gaatagtgta tgcggcgacc gagttgctct tgcccggcgt caatacggga
4861 taataccgcg ccacatagca gaactttaaa agtgctcatc attggaaaac gttcttcggg
4921 gcgaaaactc tcaaggatct taccgctgtt gagatccagt tcgatgtaac ccactcgtgc
4981 acccaactga tcttcagcat cttttacttt caccagcgtt tctgggtgag caaaaacagg
5041 aaggcaaaat gccgcaaaaa agggaataag ggcgacacgg aaatgttgaa tactcatact
5101 cttccttttt caatattatt gaagcattta tcagggttat tgtctcatga gcggatacat
5161 atttgaatgt atttagaaaa ataaacaaat aggggttccg cgcacatttc cccgaaaagt
5221 gccacctgat gcggtgtgaa ataccgcaca gatgcgtaag gagaaaatac cgcatcagga
5281 aattgtaagc gttaatattt tgttaaaatt cgcgttaaat ttttgttaaa tcagctcatt
5341 ttttaaccaa taggccgaaa tcggcaaaat cccttataaa tcaaaagaat agaccgagat
5401 agggttgagt gttgttccag tttggaacaa gagtccacta ttaaagaacg tggactccaa
5461 cgtcaaaggg cgaaaaaccg tctatcaggg cgatggccca ctacgtgaac catcacccta
5521 atcaagtttt ttggggtcga ggtgccgtaa agcactaaat cggaacccta aagggagccc
5581 ccgatttaga gcttgacggg gaaagccggc gaacgtggcg agaaaggaag ggaagaaagc
5641 gaaaggagcg ggcgctaggg cgctggcaag tgtagcggtc acgctgcgcg taaccacca
```

**SEQ ID NO: 11:**

```
   1 tgtccaaatc agcattgctc tgccaggtga gacggagttt ttgtcccact aatccctgtt
  61 ggatagaagg cgcttgatac agttccaacc aaacccaatc tcctgtcctg gctttagttt
 121 cttccaccgt cggcaggatt aaacgaccca accactctcc caaaggccga taatacgcct
 181 catctaaatt ctgttgcaga ggataataat ctacctggtt ggcgggcaac tgactgctaa
 241 tttgataatc cgggatgcgg ccagaggttt cctgaggttg aaacagcgta gctagggaaa
 301 tcaccaccag agcggcgatc gccaccaata ccatccgcca gcgccattgc ttcatctgtg
 361 gactgcccta aaaattgcca taaaaaaaca cctggggcgt ctttccttgt ttcgactcca
 421 gatgtttttc taatttcctc acacctgtcc taagtataaa tcaccaacgg ggattgtcaa
 481 ggcttcagga ttacacccgg gcaagcggat ggctgatgaa accaagccaa ccaggaaggg
 541 cagcccacct atcaaggtgt actgccttcc agacgaacga agagcgattg aggaaaaggc
 601 ggcggcggcc ggcatgagcc tgtcggccta cctgctggcc gtcggccagg gctacaaaat
 661 cacgggcgtc gtggactatg agcacgtccg cgagggcgtc ccggaaaacg attccgaagc
 721 ccaacctttc atagaaggcg gcggtggaat cgaaatctcg tgatggcagg ttgggcgtcg
 781 cttggtcggt catttcgctc ggtaccatcg gcattttctt ttgcgttttt atttgttaac
 841 tgttaattgt ccttgttcaa ggatgctgtc tttgacaaca gatgttttct tgcctttgat
 901 gttcagcagg aagctcggcg caaacgttga ttgtttgtct gcgtagaatc ctctgtttgt
 961 catatagctt gtaatcacga cattgtttcc tttcgcttga ggtacagcga agtgtgagta
1021 agtaaaggtt acatcgttag gatcaagatc catttttaac acaaggccag ttttgttcag
1081 cggcttgtat gggccagtta aagaattaga aacataacca agcatgtaaa tatcgttaga
1141 cgtaatgccg tcaatcgtca tttttgatcc gcgggagtca gtgaacaggt accatttgcc
1201 gttcatttta aagacgttcg cgcgttcaat ttcatctgtt actgtgttag atgcaatcag
1261 cggtttcatc acttttttca gtgtgtaatc atcgtttagc tcaatcatac cgagagcgcc
1321 gtttgctaac tcagccgtgc gtttttttatc gctttgcaga agtttttgac tttcttgacg
1381 gaagaatgat gtgcttttgc catagtatgc tttgttaaat aaagattctt cgccttggta
1441 gccatcttca gttccagtgt ttgcttcaaa tactaagtat ttgtggcctt tatcttctac
1501 gtagtgagga tctctcagcg tatggttgtc gcctgagctg tagttgcctt catcgatgaa
1561 ctgctgtaca ttttgatacg tttttccgtc accgtcaaag attgatttat aatcctctac
1621 accgttgatg ttcaaagagc tgtctgatgc tgatacgtta acttgtgcag ttgtcagtgt
1681 ttgtttgccg taatgtttac cggagaaatc agtgtagaat aaacggattt ttccgtcaga
1741 tgtaaatgtg gctgaacctg accattcttg tgtttggtct tttaggatag aatcatttgc
1801 atcgaatttg tcgctgtctt taaagacgcg gccagcgttt ttccagctgt caatagaagt
1861 ttcgccgact ttttgataga acatgtaaat cgatgtgtca tccgcatttt taggatctcc
```

```
1921 ggctaatgca aagacgatgt ggtagccgtg atagtttgcg acagtgccgt cagcgttttg
1981 taatggccag ctgtcccaaa cgtccaggcc ttttgcagaa gagatatttt taattgtgga
2041 cgaatcaaat tcagaaactt gatatttttc atttttttgc tgttcaggga tttgcagcat
2101 atcatggcgt gtaatatggg aaatgccgta tgtttcctta tatggctttt ggttcgtttc
2161 tttcgcaaac gcttgagttg cgcctcctgc cagcagtgcg gtagtaaagg ttaatactgt
2221 tgcttgtttt gcaaactttt tgatgttcat cgttcatgtc tcctttttta tgtactgtgt
2281 tagcggtctg cttcttccag ccctcctgtt tgaagatggc aagttagtta cgcacaataa
2341 aaaaagacct aaaatatgta aggggtgacg ccaaagtata cactttgccc tttacacatt
2401 ttaggtcttg cctgctttat cagtaacaaa cccgcgcgat ttacttttcg acctcattct
2461 attagactct cgtttggatt gcaactggtc tattttcctc ttttgtttga tagaaaatca
2521 taaaaggatt tgcagactac gggcctaaag aactaaaaaa tctatctgtt tcttttcatt
2581 ctctgtattt tttatagttt ctgttgcatg ggcataaagt tgccttttta atcacaattc
2641 agaaaatatc ataatatctc atttcactaa ataatagtga acggcaggta tatgtgatgg
2701 gttaaaaagg atcgatcctc tagcgaaccc cagagtcccg ctcagaagaa ctcgtcaaga
2761 aggcgataga aggcgatgcg ctgcgaatcg ggagcggcga taccgtaaag cacgaggaag
2821 cggtcagccc attcgccgcc aagctcttca gcaatatcac gggtagccaa cgctatgtcc
2881 tgatagcggt ccgccacacc cagccggcca cagtcgatga atccagaaaa gcggccattt
2941 tccaccatga tattcggcaa gcaggcatcg ccatgggtca cgacgagatc ctcgccgtcg
3001 ggcatccgcg ccttgagcct ggcgaacagt tcggctggcg cgagcccctg atgctcttcg
3061 tccagatcat cctgatcgac aagaccggct tccatccgag tacgtgctcg ctcgatgcga
3121 tgtttcgctt ggtggtcgaa tgggcaggta gccggatcaa gcgtatgcag ccgccgcatt
3181 gcatcagcca tgatggatac tttctcggca ggagcaaggt gagatgacag gagatcctgc
3241 cccggcactt cgcccaatag cagccagtcc cttcccgctt cagtgacaac gtcgagcaca
3301 gctgcgcaag gaacgcccgt cgtggccagc cacgatagcc gcgctgcctc gtcttggagt
3361 tcattcaggg caccggacag gtcggtcttg acaaaaagaa ccgggcgccc ctgcgctgac
3421 agccggaaca cggcggcatc agagcagccg attgtctgtt gtgcccagtc atagccgaat
3481 agcctctcca cccaagcggc cggagaacct gcgtgcaatc catcttgttc aatcatgcga
3541 aacgatcctc atcctgtctc ttgatcagat cttgatcccc tgcgccatca gatccttggc
3601 ggcaagaaag ccatccagtt tactttgcag ggcttcccaa ccttaccaga gggcgcccca
3661 gctggcaatt ccggttcgct tgctgtccat aaaaccgccc agtctagcta tcgccatgta
3721 agcccactgc aagctacctg ctttctcttt gcgcttgcgt tttcccttgt ccagatagcc
3781 cagtagctga cattcacccg ggaaaaaaaa accccgcccc tgacagggcg gggttttttt
3841 tcagataaaa aaaatcctta gctttcgcta aggatgattt ctgcaattgg cggccgcttc
3901 tagaatgcac tagtagcggc cgctgcagtc cggcaaaaaa acgggcaagg tgtcaccacc
3961 ctgccctttt tctttaaaac cgaaaagatt acttcgcgtt ggagagcgtt caccgacaaa
4021 caacagataa aacgaaaggc ccagtctttc gactgagcct ttcgttttat ttgatgcctg
4081 gtaccgggtg taatcggttg attatttcag tggcccggcc gatggataat taccatggcc
4141 cgaccgaccg ggatttccag accagtttga acagtattaa attagccttt ccggacaagg
4201 cctctgtgaa aatcttaaaa aagtattaag attttaaaag ctagcttatt ttcggaggaa
4261 atgtgtttac tcgacttgcc cagcaacacc gcgatttcgt gaaggattta gtcatgagct
4321 tgagggcttt ggccactgtg ctcgaaaatc ggggctacat tgcttcttgc tacacctgtg
4381 gcgaccaact caacagcgcc tccttcatgg tgagcttggg ggaaaatcat ctgatccgct
4441 ttttggtatc ggactacggc atcacctgga cagaaatgcg ggatgaccga gaattaatga
4501 aattagaagg agccgaggcg atcgcccagt tggaagaatt ggccaatgtg gtcaaatatt
4561 gcctagcaga tgccccccact cgaccatatg ggagagctcc caacgcgttg gatgcatagc
4621 ttgagtattc tatagtgtca cctaaatagc ttggcgtaat catggtcata gctgtttcct
4681 gtgtgaaatt gttatccgct cacaattcca cacaacatac gagccggaag cataaagtgt
4741 aaagcctggg gtgcctaatg agtgagctaa ctcacattaa ttgcgttgcg ctcactgccc
4801 gctttccagt cgggaaacct gtcgtgccag ctgcattaat gaatcggcca acgcgcgggg
4861 agaggcggtt tgcgtattgg gcgctcttcc gcttcctcgc tcactgactc gctgcgctcg
4921 gtcgttcggc tgcggcgagc ggtatcagct cactcaaagg cggtaatacg gttatccaca
4981 gaatcagggg ataacgcagg aaagaacatg tgagcaaaag gccagcaaaa ggccaggaac
5041 cgtaaaaagg ccgcgttgct ggcgtttttc cataggctcc gcccccctga cgagcatcac
```

5101 aaaaatcgac gctcaagtca gaggtggcga aacccgacag gactataaag ataccaggcg
5161 tttccccctg gaagctccct cgtgcgctct cctgttccga ccctgccgct taccggatac
5221 ctgtccgcct ttctcccttc gggaagcgtg gcgctttctc atagctcacg ctgtaggtat
5281 ctcagttcgg tgtaggtcgt tcgctccaag ctgggctgtg tgcacgaacc ccccgttcag
5341 cccgaccgct gcgccttatc cggtaactat cgtcttgagt ccaacccggt aagacacgac
5401 ttatcgccac tggcagcagc cactggtaac aggattagca gagcgaggta tgtaggcggt
5461 gctacagagt tcttgaagtg gtggcctaac tacggctaca ctagaagaac agtatttggt
5521 atctgcgctc tgctgaagcc agttaccttc ggaaaaagag ttggtagctc ttgatccggc
5581 aaacaaacca ccgctggtag cggtggtttt tttgtttgca agcagcagat tacgcgcaga
5641 aaaaaaggat ctcaagaaga tcctttgatc ttttctacgg ggtctgacgc tcagtggaac
5701 gaaaactcac gttaagggat tttggtcatg agattatcaa aaaggatctt cacctagatc
5761 cttttaaatt aaaaatgaag ttttaaatca atctaaagta tatatgagta aacttggtct
5821 gacagttacc aatgcttaat cagtgaggca cctatctcag cgatctgtct atttcgttca
5881 tccatagttg cctgactccc cgtcgtgtag ataactacga tacgggaggg cttaccatct
5941 ggccccagtg ctgcaatgat accgcgagac ccacgctcac cggctccaga tttatcagca
6001 ataaaccagc cagccggaag ggccgagcgc agaagtggtc ctgcaacttt atccgcctcc
6061 atccagtcta ttaattgttg ccgggaagct agagtaagta gttcgccagt taatagtttg
6121 cgcaacgttg ttgccattgc tacaggcatc gtggtgtcac gctcgtcgtt tggtatggct
6181 tcattcagct ccggttccca acgatcaagg cgagttacat gatcccccat gttgtgcaaa
6241 aaagcggtta gctccttcgg tcctccgatc gttgtcagaa gtaagttggc cgcagtgtta
6301 tcactcatgg ttatggcagc actgcataat tctcttactg tcatgccatc cgtaagatgc
6361 ttttctgtga ctggtgagta ctcaaccaag tcattctgag aatagtgtat gcggcgaccg
6421 agttgctctt gcccggcgtc aatacgggat aataccgcgc cacatagcag aactttaaaa
6481 gtgctcatca ttggaaaacg ttcttcgggg cgaaaactct caaggatctt accgctgttg
6541 agatccagtt cgatgtaacc cactcgtgca cccaactgat cttcagcatc ttttactttc
6601 accagcgttt ctgggtgagc aaaaacagga aggcaaaatg ccgcaaaaaa gggaataagg
6661 gcgacacgga aatgttgaat actcatactc ttcctttttc aatattattg aagcatttat
6721 cagggttatt gtctcatgag cggatacata tttgaatgta tttagaaaaa taaacaaata
6781 ggggttccgc gcacatttcc ccgaaaagtg ccacctgatg cggtgtgaaa taccgcacag
6841 atgcgtaagg agaaaatacc gcatcaggaa attgtaagcg ttaatatttt gttaaaattc
6901 gcgttaaatt tttgttaaat cagctcattt tttaaccaat aggccgaaat cggcaaaatc
6961 ccttataaat caaaagaata gaccgagata gggttgagtg ttgttccagt ttggaacaag
7021 agtccactat taaagaacgt ggactccaac gtcaaagggc gaaaaaccgt ctatcagggc
7081 gatggcccac tacgtgaacc atcaccctaa tcaagttttt tggggtcgag gtgccgtaaa
7141 gcactaaatc ggaaccctaa agggagcccc cgatttagag cttgacgggg aaagccggcg
7201 aacgtggcga gaaaggaagg gaagaaagcg aaaggagcgg gcgctagggc gctggcaagt
7261 gtagcggtca cgctgcgcgt aaccaccaca cccgccgcgc ttaatgcgcc gctacagggc
7321 gcgtccattc gccattcagg ctgcgcaact gttgggaagg gcgatcggtg cgggcctctt
7381 cgctattacg ccagctggcg aaagggggat gtgctgcaag gcgattaagt tgggtaacgc
7441 cagggttttc ccagtcacga cgttgtaaaa cgacggccag tgaattgtaa tacgactcac
7501 tatagggcga attgggcccg acgtcgcatg ctcccggccg ccatggcggc cgcgggaatt
7561 cgat

**SEQ ID NO: 12:**

ORIGIN

1 cacccgccgc gcttaatgcg ccgctacagg gcgcgtccat tcgccattca ggctgcgcaa
61 ctgttgggaa gggcgatcgg tgcgggcctc ttcgctatta cgccagctgg cgaaaggggg
121 atgtgctgca aggcgattaa gttgggtaac gccagggttt tcccagtcac gacgttgtaa
181 aacgacggcc agtgaattgt aatacgactc actatagggc gaattgggcc cgacgtcgca
241 tgctcccggc cgccatggcg gccgcgggaa ttcgattgat gaccgctggc ggagtttagt
301 ccagggttat ttgtaccgtc aaggttacat tgccacccca gatttaacta cgctgcgagt
361 ccgtaccatt ggcgatcggg cctatctcac cattaagggt aaaaatgcca gcattgcccg

```
421 cttggatttg agtatgaaat tcttgcagtg gaagccaatt aaattttgac agaactctgt
481 tcaccgcccc tgattgaaaa tatcgttatt gcctcgatta ccatggtaaa acctgagaag
541 tagacgagtt tttgggggat aaccagggtc taattttagc ggaagtggaa ttaacctaca
601 ctggtgaaaa aataagtcta cttccctgga tcggggaaga ggtaacggat gatgcccgct
661 attacaacgt caatttagcc caacatccct acaaaaattg gtgacaagat tacacccggg
721 caagcggatg gctgatgaaa ccaagccaac caggaagggc agcccaccta tcaaggtgta
781 ctgccttcca gacgaacgaa gagcgattga ggaaaaggcg gcggcggccg gcatgagcct
841 gtcggcctac ctgctggccg tcggccaggg ctacaaaatc acgggcgtcg tggactatga
901 gcacgtccgc gagggcgtcc cggaaaacga ttccgaagcc caacctttca tagaaggcgg
961 cggtggaatc gaaatctcgt gatggcaggt tgggcgtcgc ttggtcggtc atttcgctcg
1021 gtaccatcgg cattttcttt tgcgttttta tttgttaact gttaattgtc cttgttcaag
1081 gatgctgtct ttgacaacag atgttttctt gcctttgatg ttcagcagga agctcggcgc
1141 aaacgttgat tgtttgtctg cgtagaatcc tctgtttgtc atatagcttg taatcacgac
1201 attgtttcct ttcgcttgag gtacagcgaa gtgtgagtaa gtaaaggtta catcgttagg
1261 atcaagatcc atttttaaca caaggccagt tttgttcagc ggcttgtatg ggccagttaa
1321 agaattagaa acataaccaa gcatgtaaat atcgttagac gtaatgccgt caatcgtcat
1381 ttttgatccg cgggagtcag tgaacaggta ccatttgccg ttcattttaa agacgttcgc
1441 gcgttcaatt tcatctgtta ctgtgttaga tgcaatcagc ggtttcatca ctttttttcag
1501 tgtgtaatca tcgtttagct caatcatacc gagagcgccg tttgctaact cagccgtgcg
1561 tttttttatcg ctttgcagaa gtttttgact ttcttgacgg aagaatgatg tgcttttgcc
1621 atagtatgct ttgttaaata aagattcttc gccttggtag ccatcttcag ttccagtgtt
1681 tgcttcaaat actaagtatt tgtggccttt atcttctacg tagtgaggat ctctcagcgt
1741 atggttgtcg cctgagctgt agttgccttc atcgatgaac tgctgtacat tttgatacgt
1801 ttttccgtca ccgtcaaaga ttgatttata atcctctaca ccgttgatgt tcaaagagct
1861 gtctgatgct gatacgttaa cttgtgcagt tgtcagtgtt tgtttgccgt aatgtttacc
1921 ggagaaatca gtgtagaata aacggatttt tccgtcagat gtaaatgtgg ctgaacctga
1981 ccattcttgt gtttggtctt ttaggataga atcatttgca tcgaatttgt cgctgtcttt
2041 aaagacgcgg ccagcgtttt tccagctgtc aatagaagtt tcgccgactt tttgatagaa
2101 catgtaaatc gatgtgtcat ccgcattttt aggatctccg gctaatgcaa agacgatgtg
2161 gtagccgtga tagtttgcga cagtgccgtc agcgttttgt aatggccagc tgtcccaaac
2221 gtccaggcct tttgcagaag agatattttt aattgtggac gaatcaaatt cagaaacttg
2281 atatttttca ttttttgct gttcagggat ttgcagcata tcatggcgtg taatatggga
2341 aatgccgtat gtttccttat atggcttttg gttcgtttct ttcgcaaacg cttgagttgc
2401 gcctcctgcc agcagtgcgg tagtaaaggt taatactgtt gcttgttttg caaacttttt
2461 gatgttcatc gttcatgtct ccttttttat gtactgtgtt agcggtctgc ttcttccagc
2521 cctcctgttt gaagatggca agttagttac gcacaataaa aaaagaccta aaatatgtaa
2581 ggggtgacgc caaagtatac actttgccct ttacacattt taggtcttgc ctgctttatc
2641 agtaacaaac ccgcgcgatt tacttttcga cctcattcta ttagactctc gtttggattg
2701 caactggtct attttcctct tttgtttgat agaaaatcat aaaaggattt gcagactacg
2761 ggcctaaaga actaaaaaat ctatctgttt cttttcattc tctgtatttt ttatagtttc
2821 tgttgcatgg gcataaagtt gccttttttaa tcacaattca gaaaatatca taatatctca
2881 tttcactaaa taatagtgaa cggcaggtat atgtgatggg ttaaaaagga tcgatcctct
2941 agcgaacccc agagtcccgc tcagaagaac tcgtcaagaa ggcgatagaa ggcgatgcgc
3001 tgcgaatcgg gagcggcgat accgtaaagc acgaggaagc ggtcagccca ttcgccgcca
3061 agctcttcag caatatcacg ggtagccaac gctatgtcct gatagcggtc cgccacaccc
3121 agccggccac agtcgatgaa tccagaaaag cggccatttt ccaccatgat attcggcaag
3181 caggcatcgc catgggtcac gacgagatcc tcgccgtcgg gcatccgcgc cttgagcctg
3241 gcgaacagtt cggctggcgc gagcccctga tgctcttcgt ccagatcatc ctgatcgaca
3301 agaccggctt ccatccgagt acgtgctcgc tcgatgcgat gtttcgcttg gtggtcgaat
3361 gggcaggtag ccggatcaag cgtatgcagc cgccgcattg catcagccat gatggatact
3421 ttctcggcag gagcaaggtg agatgacagg agatcctgcc ccggcacttc gcccaatagc
3481 agccagtccc ttcccgcttc agtgacaacg tcgagcacag ctgcgcaagg aacgcccgtc
3541 gtggccagcc acgatagccg cgctgcctcg tcttggagtt cattcagggc accggacagg
```

3601 tcggtcttga caaaaagaac cgggcgcccc tgcgctgaca gccggaacac ggcggcatca
3661 gagcagccga ttgtctgttg tgcccagtca tagccgaata gcctctccac ccaagcggcc
3721 ggagaacctg cgtgcaatcc atcttgttca atcatgcgaa acgatcctca tcctgtctct
3781 tgatcagatc ttgatcccct gcgccatcag atccttggcg gcaagaaagc catccagttt
3841 actttgcagg gcttcccaac cttaccagag ggcgccccag ctggcaattc cggttcgctt
3901 gctgtccata aaaccgccca gtctagctat cgccatgtaa gcccactgca agctacctgc
3961 tttctctttg cgcttgcgtt ttcccttgtc cagatagccc agtagctgac attcacccgg
4021 gaaaaaaaaa ccccgcccct gacagggcgg ggtttttttt cagataaaaa aaatccttag
4081 ctttcgctaa ggatgatttc tgcaattggc ggccgcttct agaatgcact agtagcggcc
4141 gctgcagtcc ggcaaaaaaa cgggcaaggt gtcaccaccc tgcccttttt ctttaaaacc
4201 gaaaagatta cttcgcgttg gagagcgttc accgacaaac aacagataaa acgaaaggcc
4261 cagtctttcg actgagcctt tcgttttatt tgatgcctgg taccgggtgt aatccttcat
4321 tcccctattg ttattaatag tgctctgaaa ctaacaaata agcaagatca aattgtgaag
4381 aaaacgcaac aaaatgaacc attaagataa tttctgtcta tcctggctgg tatattccag
4441 tctactcaga aatgagtttg ttttatgaca aatcagtatg taccaaatta cttggcgcaa
4501 tcgatcctgg taactctatt ttgttgctta cccctgggta ttgtggccat tatcaaagca
4561 tcggaagtta attctcgttt agcttcaggg gactatgaag gcgctgtaaa ggcttccaag
4621 gaagcgaaaa agttttgttg gtggtccttt ggtgccggca taattttcat tgccatctat
4681 tttgtgctag tggttattgc cgccgtcttt ggtcagtaat taaagttaca ttttttgact
4741 ttgccttgtt caccattcat taacgaatac catgtttagt ttgaaaaatt attccccatc
4801 tccattacta tccgctaagg ccaaggaata tttagtattc actttggtaa ccctaaccat
4861 tgtcgttgct ggcactcgac catatgggag agctcccaac gcgttggatg catagcttga
4921 gtattctata gtgtcaccta aatagcttgg cgtaatcatg gtcatagctg tttcctgtgt
4981 gaaattgtta tccgctcaca attccacaca acatacgagc cggaagcata aagtgtaaag
5041 cctggggtgc ctaatgagtg agctaactca cattaattgc gttgcgctca ctgcccgctt
5101 tccagtcggg aaacctgtcg tgccagctgc attaatgaat cggccaacgc gcggggagag
5161 gcggtttgcg tattgggcgc tcttccgctt cctcgctcac tgactcgctg cgctcggtcg
5221 ttcggctgcg gcgagcggta tcagctcact caaaggcggt aatacggtta tccacagaat
5281 caggggataa cgcaggaaag aacatgtgag caaaaggcca gcaaaaggcc aggaaccgta
5341 aaaaggccgc gttgctggcg tttttccata ggctccgccc ccctgacgag catcacaaaa
5401 atcgacgctc aagtcagagg tggcgaaacc cgacaggact ataaagatac caggcgtttc
5461 cccctggaag ctccctcgtg cgctctcctg ttccgaccct gccgcttacc ggatacctgt
5521 ccgcctttct cccttcggga agcgtggcgc tttctcatag ctcacgctgt aggtatctca
5581 gttcggtgta ggtcgttcgc tccaagctgg gctgtgtgca cgaacccccc gttcagcccg
5641 accgctgcgc cttatccggt aactatcgtc ttgagtccaa cccggtaaga cacgacttat
5701 cgccactggc agcagccact ggtaacagga ttagcagagc gaggtatgta ggcggtgcta
5761 cagagttctt gaagtggtgg cctaactacg gctacactag aagaacagta tttggtatct
5821 gcgctctgct gaagccagtt accttcggaa aaagagttgg tagctcttga tccggcaaac
5881 aaaccaccgc tggtagcggt ggtttttttg tttgcaagca gcagattacg cgcagaaaaa
5941 aaggatctca agaagatcct ttgatctttt ctacggggtc tgacgctcag tggaacgaaa
6001 actcacgtta agggattttg gtcatgagat tatcaaaaag gatcttcacc tagatccttt
6061 taaattaaaa atgaagtttt aaatcaatct aaagtatata tgagtaaact tggtctgaca
6121 gttaccaatg cttaatcagt gaggcaccta tctcagcgat ctgtctattt cgttcatcca
6181 tagttgcctg actccccgtc gtgtagataa ctacgatacg ggagggctta ccatctggcc
6241 ccagtgctgc aatgataccg cgagacccac gctcaccggc tccagattta tcagcaataa
6301 accagccagc cggaagggcc gagcgcagaa gtggtcctgc aactttatcc gcctccatcc
6361 agtctattaa ttgttgccgg gaagctagag taagtagttc gccagttaat agtttgcgca
6421 acgttgttgc cattgctaca ggcatcgtgg tgtcacgctc gtcgtttggt atggcttcat
6481 tcagctccgg ttcccaacga tcaaggcgag ttacatgatc ccccatgttg tgcaaaaaag
6541 cggttagctc cttcggtcct ccgatcgttg tcagaagtaa gttggccgca gtgttatcac
6601 tcatggttat ggcagcactg cataattctc ttactgtcat gccatccgta agatgctttt
6661 ctgtgactgg tgagtactca accaagtcat tctgagaata gtgtatgcgg cgaccgagtt
6721 gctcttgccc ggcgtcaata cgggataata ccgcgccaca tagcagaact ttaaaagtgc

6781 tcatcattgg aaaacgttct tcggggcgaa aactctcaag gatcttaccg ctgttgagat
6841 ccagttcgat gtaacccact cgtgcaccca actgatcttc agcatctttt actttcacca
6901 gcgtttctgg gtgagcaaaa acaggaaggc aaaatgccgc aaaaaaggga ataagggcga
6961 cacggaaatg ttgaatactc atactcttcc tttttcaata ttattgaagc atttatcagg
7021 gttattgtct catgagcgga tacatatttg aatgtattta gaaaaataaa caaatagggg
7081 ttccgcgcac atttccccga aaagtgccac ctgatgcggt gtgaaatacc gcacagatgc
7141 gtaaggagaa aataccgcat caggaaattg taagcgttaa tattttgtta aaattcgcgt
7201 taaatttttg ttaaatcagc tcatttttta accaataggc cgaaatcggc aaaatccctt
7261 ataaatcaaa agaatagacc gagatagggt tgagtgttgt tccagtttgg aacaagagtc
7321 cactattaaa gaacgtggac tccaacgtca aagggcgaaa aaccgtctat cagggcgatg
7381 gcccactacg tgaaccatca ccctaatcaa gtttttggg gtcgaggtgc cgtaaagcac
7441 taaatcggaa ccctaaaggg agcccccgat ttagagcttg acggggaaag ccggcgaacg
7501 tggcgagaaa ggaagggaag aaagcgaaag gagcgggcgc tagggcgctg gcaagtgtag
7561 cggtcacgct gcgcgtaacc acca

**SEQ ID NO: 13:**

1 cacccgccgc gcttaatgcg ccgctacagg gcgcgtccat tcgccattca ggctgcgcaa
61 ctgttgggaa gggcgatcgg tgcgggcctc ttcgctatta cgccagctgg cgaaaggggg
121 atgtgctgca aggcgattaa gttgggtaac gccagggttt tcccagtcac gacgttgtaa
181 aacgacggcc agtgaattgt aatacgactc actatagggc gaattgggcc cgacgtcgca
241 tgctcccggc cgccatggcg gccgcgggaa ttcgattgga ctgacccaag atacagtggt
301 agcttcaagt tcgtcaatca accccaatgg aaagctgaga taatctgttg ttgatgcgac
361 cgtcttcatt ttagctaaaa agacaagtct gtggctagtt actatgacga ggccatcggg
421 tctatcccag cggtcttgct gattccaaaa cttggcgatt ttctgaagta gtatcttttc
481 accgcttaag ccgccaaggt cttggatgag cttggtcgaa tcatcttttg atgcctcgac
541 atcgcgaata actttgtaaa cgaccttccc gagcttgaat gcaaagtcgg ttagattatc
601 catcagagtt gaatgccgat aatacagact gctatactat tttaatgaaa agagtgctgg
661 caagcaagcc cgccgaacgt tgaagctgtg cagcgcaaac gaagcgcatt gtagtttgcg
721 ctcggattac acccgggcaa gcggatggct gatgaaacca agccaaccag gaagggcagc
781 ccacctatca aggtgtactg ccttccagac gaacgaagag cgattgagga aaaggcggcg
841 gcggccggca tgagcctgtc ggcctacctg ctggccgtcg gccagggcta caaaatcacg
901 ggcgtcgtgg actatgagca cgtccgcgag ggcgtcccgg aaaacgattc cgaagcccaa
961 cctttcatag aaggcggcgg tggaatcgaa atctcgtgat ggcaggttgg gcgtcgcttg
1021 gtcggtcatt tcgctcggta ccatcggcat ttcttttgc gtttttattt gttaactgtt
1081 aattgtcctt gttcaaggat gctgtctttg acaacagatg ttttcttgcc tttgatgttc
1141 agcaggaagc tcggcgcaaa cgttgattgt ttgtctgcgt agaatcctct gtttgtcata
1201 tagcttgtaa tcacgacatt gtttcctttc gcttgaggta cagcgaagtg tgagtaagta
1261 aaggttacat cgttaggatc aagatccatt tttaacacaa ggccagtttt gttcagcggc
1321 ttgtatgggc cagttaaaga attagaaaca taaccaagca tgtaaatatc gttagacgta
1381 atgccgtcaa tcgtcatttt tgatccgcgg gagtcagtga acaggtacca tttgccgttc
1441 attttaaaga cgttcgcgcg ttcaatttca tctgttactg tgttagatgc aatcagcggt
1501 ttcatcactt ttttcagtgt gtaatcatcg tttagctcaa tcataccgag agcgccgttt
1561 gctaactcag ccgtgcgttt tttatcgctt tgcagaagtt tttgactttc ttgacggaag
1621 aatgatgtgc ttttgccata gtatgctttg ttaaataaag attcttcgcc ttggtagcca
1681 tcttcagttc cagtgtttgc ttcaaatact aagtatttgt ggcctttatc ttctacgtag
1741 tgaggatctc tcagcgtatg gttgtcgcct gagctgtagt tgccttcatc gatgaactgc
1801 tgtacatttt gatacgtttt tccgtcaccg tcaaagattg atttataatc ctctacaccg
1861 ttgatgttca aagagctgtc tgatgctgat acgttaactt gtgcagttgt cagtgtttgt
1921 ttgccgtaat gtttaccgga gaaatcagtg tagaataaac ggatttttcc gtcagatgta
1981 aatgtggctg aacctgacca ttcttgtgtt tggtctttta ggatagaatc atttgcatcg
2041 aatttgtcgc tgtctttaaa gacgcggcca gcgtttttcc agctgtcaat agaagtttcg
2101 ccgacttttt gatagaacat gtaaatcgat gtgtcatccg catttttagg atctccggct

2161 aatgcaaaga cgatgtggta gccgtgatag tttgcgacag tgccgtcagc gttttgtaat
2221 ggccagctgt cccaaacgtc caggcctttt gcagaagaga tatttttaat tgtggacgaa
2281 tcaaattcag aaacttgata tttttcattt ttttgctgtt cagggatttg cagcatatca
2341 tggcgtgtaa tatgggaaat gccgtatgtt tccttatatg gcttttggtt cgtttctttc
2401 gcaaacgctt gagttgcgcc tcctgccagc agtgcggtag taaaggttaa tactgttgct
2461 tgttttgcaa actttttgat gttcatcgtt catgtctcct tttttatgta ctgtgttagc
2521 ggtctgcttc ttccagccct cctgtttgaa gatggcaagt tagttacgca caataaaaaa
2581 agacctaaaa tatgtaaggg gtgacgccaa agtatacact ttgcccttta cacattttag
2641 gtcttgcctg ctttatcagt aacaaacccg cgcgatttac ttttcgacct cattctatta
2701 gactctcgtt tggattgcaa ctggtctatt ttcctctttt gtttgataga aaatcataaa
2761 aggatttgca gactacgggc ctaaagaact aaaaaatcta tctgtttctt ttcattctct
2821 gtattttttta tagtttctgt tgcatgggca taaagttgcc tttttaatca caattcagaa
2881 aatatcataa tatctcattt cactaaataa tagtgaacgg caggtatatg tgatgggtta
2941 aaaaggatcg atcctctagc gaaccccaga gtcccgctca gaagaactcg tcaagaaggc
3001 gatagaaggc gatgcgctgc gaatcgggag cggcgatacc gtaaagcacg aggaagcggt
3061 cagcccattc gccgccaagc tcttcagcaa tatcacgggt agccaacgct atgtcctgat
3121 agcggtccgc cacacccagc cggccacagt cgatgaatcc agaaaagcgg ccattttcca
3181 ccatgatatt cggcaagcag gcatcgccat gggtcacgac gagatcctcg ccgtcgggca
3241 tccgcgcctt gagcctggcg aacagttcgg ctggcgcgag cccctgatgc tcttcgtcca
3301 gatcatcctg atcgacaaga ccggcttcca tccgagtacg tgctcgctcg atgcgatgtt
3361 tcgcttggtg gtcgaatggg caggtagccg gatcaagcgt atgcagccgc cgcattgcat
3421 cagccatgat ggatactttc tcggcaggag caaggtgaga tgacaggaga tcctgccccg
3481 gcacttcgcc caatagcagc cagtcccttc ccgcttcagt gacaacgtcg agcacagctg
3541 cgcaaggaac gcccgtcgtg gccagccacg atagccgcgc tgcctcgtct tggagttcat
3601 tcagggcacc ggacaggtcg gtcttgacaa aaagaaccgg gcgcccctgc gctgacagcc
3661 ggaacacggc ggcatcagag cagccgattg tctgttgtgc ccagtcatag ccgaatagcc
3721 tctccaccca agcggccgga gaacctgcgt gcaatccatc ttgttcaatc atgcgaaacg
3781 atcctcatcc tgtctcttga tcagatcttg atcccctgcg ccatcagatc cttggcggca
3841 agaaagccat ccagtttact ttgcagggct tcccaacctt accagagggc gccccagctg
3901 gcaattccgg ttcgcttgct gtccataaaa ccgcccagtc tagctatcgc catgtaagcc
3961 cactgcaagc tacctgcttt ctctttgcgc ttgcgttttc ccttgtccag atagcccagt
4021 agctgacatt cacccgggaa aaaaaaaccc cgcccctgac agggcggggt tttttttcag
4081 ataaaaaaaa tccttagctt tcgctaagga tgatttctgc aattggcggc cgcttctaga
4141 atgcactagt agcggccgct gcagtccggc aaaaaaacgg gcaaggtgtc accaccctgc
4201 ccttttttctt taaaaccgaa aagattactt cgcgttggag agcgttcacc gacaaacaac
4261 agataaaacg aaaggcccag tctttcgact gagcctttcg ttttatttga tgcctggtac
4321 cgggtgtaat ccccatactc agccttctaa tgctgagaga atgcctgaaa agaaccacta
4381 agtcaaatta tttggattaa gttttattaa gtcaacggag gtgtcagata gcatttagat
4441 cgtaaaagca tacggcatga acaaccatga acggcttccc agaggttaac cccaggttta
4501 tggtttacca aaatcccctg actttttccg agttgggga caaaatcaac taatttgggc
4561 gacgattttt tctgctatct ggcgatcgcc atgtttgtct aacttctgat ccaacctagg
4621 tttttggggg ctttccaaca aaaaatccca gttgccggcg aaaaattgct ggtggggcac
4681 aatttggtgc tctccgtagt tctgcaaacc tgccagtaaa acttgcgctt ccgcaaaacc
4741 atccctggtg agggaaataa tcgggacttc taactttaac gcttcagcaa aggtaccaaa
4801 accaggcttg gaaaataccc gtccgcaaag gggcattaaa tccaccgggc gactcgacca
4861 tatgggagag ctcccaacgc gttggatgca tagcttgagt attctatagt gtcacctaaa
4921 tagcttggcg taatcatggt catagctgtt tcctgtgtga aattgttatc cgctcacaat
4981 tccacacaac atacgagccg gaagcataaa gtgtaaagcc tggggtgcct aatgagtgag
5041 ctaactcaca ttaattgcgt tgcgctcact gcccgctttc cagtcgggaa acctgtcgtg
5101 ccagctgcat taatgaatcg gccaacgcgc ggggagaggc ggtttgcgta ttgggcgctc
5161 ttccgcttcc tcgctcactg actcgctgcg ctcggtcgtt cggctgcggc gagcggtatc
5221 agctcactca aaggcggtaa tacggttatc cacagaatca ggggataacg caggaaagaa
5281 catgtgagca aaaggccagc aaaaggccag gaaccgtaaa aaggccgcgt tgctggcgtt

5341 tttccatagg ctccgccccc ctgacgagca tcacaaaaat cgacgctcaa gtcagaggtg
5401 gcgaaacccg acaggactat aaagatacca ggcgtttccc cctggaagct ccctcgtgcg
5461 ctctcctgtt ccgaccctgc cgcttaccgg atacctgtcc gcctttctcc cttcgggaag
5521 cgtggcgctt tctcatagct cacgctgtag gtatctcagt tcggtgtagg tcgttcgctc
5581 caagctgggc tgtgtgcacg aaccccccgt tcagcccgac cgctgcgcct tatccggtaa
5641 ctatcgtctt gagtccaacc cggtaagaca cgacttatcg ccactggcag cagccactgg
5701 taacaggatt agcagagcga ggtatgtagg cggtgctaca gagttcttga agtggtggcc
5761 taactacggc tacactagaa gaacagtatt tggtatctgc gctctgctga agccagttac
5821 cttcggaaaa agagttggta gctcttgatc cggcaaacaa accaccgctg gtagcggtgg
5881 tttttttgtt tgcaagcagc agattacgcg cagaaaaaaa ggatctcaag aagatccttt
5941 gatcttttct acggggtctg acgctcagtg gaacgaaaac tcacgttaag ggattttggt
6001 catgagatta tcaaaaagga tcttcaccta gatcctttta aattaaaaat gaagttttaa
6061 atcaatctaa agtatatatg agtaaacttg gtctgacagt taccaatgct taatcagtga
6121 ggcacctatc tcagcgatct gtctatttcg ttcatccata gttgcctgac tccccgtcgt
6181 gtagataact acgatacggg agggcttacc atctggcccc agtgctgcaa tgataccgcg
6241 agacccacgc tcaccggctc cagatttatc agcaataaac cagccagccg gaagggccga
6301 gcgcagaagt ggtcctgcaa ctttatccgc ctccatccag tctattaatt gttgccggga
6361 agctagagta agtagttcgc cagttaatag tttgcgcaac gttgttgcca ttgctacagg
6421 catcgtggtg tcacgctcgt cgtttggtat ggcttcattc agctccggtt cccaacgatc
6481 aaggcgagtt acatgatccc ccatgttgtg caaaaaagcg gttagctcct tcggtcctcc
6541 gatcgttgtc agaagtaagt tggccgcagt gttatcactc atggttatgg cagcactgca
6601 taattctctt actgtcatgc catccgtaag atgcttttct gtgactggtg agtactcaac
6661 caagtcattc tgagaatagt gtatgcggcg accgagttgc tcttgcccgg cgtcaatacg
6721 ggataatacc gcgccacata gcagaacttt aaaagtgctc atcattggaa aacgttcttc
6781 ggggcgaaaa ctctcaagga tcttaccgct gttgagatcc agttcgatgt aacccactcg
6841 tgcacccaac tgatcttcag catcttttac tttcaccagc gtttctgggt gagcaaaaac
6901 aggaaggcaa aatgccgcaa aaaagggaat aagggcgaca cggaaatgtt gaatactcat
6961 actcttcctt tttcaatatt attgaagcat ttatcagggt tattgtctca tgagcggata
7021 catatttgaa tgtatttaga aaaataaaca aataggggtt ccgcgcacat ttccccgaaa
7081 agtgccacct gatgcggtgt gaaataccgc acagatgcgt aaggagaaaa taccgcatca
7141 ggaaattgta agcgttaata ttttgttaaa attcgcgtta aatttttgtt aaatcagctc
7201 attttttaac caataggccg aaatcggcaa aatcccttat aaatcaaaag aatagaccga
7261 gataggggtg agtgttgttc cagtttggaa caagagtcca ctattaaaga acgtggactc
7321 caacgtcaaa gggcgaaaaa ccgtctatca gggcgatggc ccactacgtg aaccatcacc
7381 ctaatcaagt tttttggggt cgaggtgccg taaagcacta aatcggaacc ctaaagggag
7441 cccccgattt agagcttgac ggggaaagcc ggcgaacgtg gcgagaaagg aagggaagaa
7501 agcgaaagga gcgggcgcta gggcgctggc aagtgtagcg gtcacgctgc gcgtaaccac
7561 ca

**SEQ ID NO: 14:**

1 cacccgccgc gcttaatgcg ccgctacagg gcgcgtccat tcgccattca ggctgcgcaa
61 ctgttgggaa gggcgatcgg tgcgggcctc ttcgctatta cgccagctgg cgaaaggggg
121 atgtgctgca aggcgattaa gttgggtaac gccagggttt tcccagtcac gacgttgtaa
181 aacgacggcc agtgaattgt aatacgactc actatagggc gaattgggcc cgacgtcgca
241 tgctcccggc cgccatggcg gccgcgggaa ttcgattcta aacttacggc attggcatca
301 acgggagcca ccgtcggagt aggggaagta acaacggggg aactggtatt ggtgggcaaa
361 atttttacta gccattgatt ccattggggc agattgaacg ctcccaacca ccaaagtccc
421 cctaatacaa cgacagaact aaataacagg aaaaaaggaa tccaaggagt tacccctctt
481 ttatgggatg gaacttcatc gacattaaag gtgggagggg gaggaggcaa tggggacaat
541 ggtggtccag aaaaggaagg tggctccgac gtcaaggcaa cgggacatcc acaggattca
601 cagaaacgaa cctgggggct aaggcggttg ccacaattag tacaaaaaac gggagtagtc
661 ataggtgaaa accccgacta tagaattaga aaaatttaac tttttatccg aattttattc

721 gtccatgttc cccaaataac tatcaaaata attggaaaaa ttaaattatt tggtcgttgg
781 tcaccgctcc ctaaagacct ggccattgta aagagattac acccgggcaa gcggatggct
841 gatgaaacca agccaaccag gaagggcagc ccacctatca aggtgtactg ccttccagac
901 gaacgaagag cgattgagga aaaggcggcg gcggccggca tgagcctgtc ggcctacctg
961 ctggccgtcg gccagggcta caaaatcacg ggcgtcgtgg actatgagca cgtccgcgag
1021 ggcgtcccgg aaaacgattc cgaagcccaa cctttcatag aaggcggcgg tggaatcgaa
1081 atctcgtgat ggcaggttgg gcgtcgcttg gtcggtcatt tcgctcggta ccatcggcat
1141 tttcttttgc gtttttattt gttaactgtt aattgtcctt gttcaaggat gctgtctttg
1201 acaacagatg ttttcttgcc tttgatgttc agcaggaagc tcggcgcaaa cgttgattgt
1261 ttgtctgcgt agaatcctct gtttgtcata tagcttgtaa tcacgacatt gtttcctttc
1321 gcttgaggta cagcgaagtg tgagtaagta aaggttacat cgttaggatc aagatccatt
1381 tttaacacaa ggccagtttt gttcagcggc ttgtatgggc cagttaaaga attagaaaca
1441 taaccaagca tgtaaatatc gttagacgta atgccgtcaa tcgtcatttt tgatccgcgg
1501 gagtcagtga acaggtacca tttgccgttc attttaaaga cgttcgcgcg ttcaatttca
1561 tctgttactg tgttagatgc aatcagcggt ttcatcactt ttttcagtgt gtaatcatcg
1621 tttagctcaa tcataccgag agcgccgttt gctaactcag ccgtgcgttt tttatcgctt
1681 tgcagaagtt tttgactttc ttgacggaag aatgatgtgc ttttgccata gtatgctttg
1741 ttaaataaag attcttcgcc ttggtagcca tcttcagttc cagtgtttgc ttcaaatact
1801 aagtatttgt ggcctttatc ttctacgtag tgaggatctc tcagcgtatg gttgtcgcct
1861 gagctgtagt tgccttcatc gatgaactgc tgtacatttt gatacgtttt tccgtcaccg
1921 tcaaagattg atttataatc ctctacaccg ttgatgttca aagagctgtc tgatgctgat
1981 acgttaactt gtgcagttgt cagtgtttgt ttgccgtaat gtttaccgga gaaatcagtg
2041 tagaataaac ggatttttcc gtcagatgta aatgtggctg aacctgacca ttcttgtgtt
2101 tggtctttta ggatagaatc atttgcatcg aatttgtcgc tgtctttaaa gacgcggcca
2161 gcgtttttcc agctgtcaat agaagtttcg ccgacttttt gatagaacat gtaaatcgat
2221 gtgtcatccg cattttttagg atctccggct aatgcaaaga cgatgtggta gccgtgatag
2281 tttgcgacag tgccgtcagc gttttgtaat ggccagctgt cccaaacgtc caggcctttt
2341 gcagaagaga tatttttaat tgtggacgaa tcaaattcag aaacttgata tttttcattt
2401 ttttgctgtt cagggatttg cagcatatca tggcgtgtaa tatgggaaat gccgtatgtt
2461 tccttatatg gcttttggtt cgtttctttc gcaaacgctt gagttgcgcc tcctgccagc
2521 agtgcggtag taaaggttaa tactgttgct tgttttgcaa actttttgat gttcatcgtt
2581 catgtctcct tttttatgta ctgtgttagc ggtctgcttc ttccagccct cctgtttgaa
2641 gatggcaagt tagttacgca caataaaaaa agacctaaaa tatgtaaggg gtgacgccaa
2701 agtatacact ttgcccttta cacattttag gtcttgcctg ctttatcagt aacaaacccg
2761 cgcgatttac ttttcgacct cattctatta gactctcgtt tggattgcaa ctggtctatt
2821 ttcctctttt gtttgataga aaatcataaa aggatttgca gactacgggc ctaaagaact
2881 aaaaaatcta tctgtttctt ttcattctct gtattttttta tagtttctgt tgcatgggca
2941 taaagttgcc tttttaatca caattcagaa aatatcataa tatctcattt cactaaataa
3001 tagtgaacgg caggtatatg tgatgggtta aaaaggatcg atcctctagc gaaccccaga
3061 gtcccgctca gaagaactcg tcaagaaggc gatagaaggc gatgcgctgc gaatcgggag
3121 cggcgatacc gtaaagcacg aggaagcggt cagcccattc gccgccaagc tcttcagcaa
3181 tatcacgggt agccaacgct atgtcctgat agcggtccgc cacacccagc cggccacagt
3241 cgatgaatcc agaaaagcgg ccattttcca ccatgatatt cggcaagcag gcatcgccat
3301 gggtcacgac gagatcctcg ccgtcgggca tccgcgcctt gagcctggcg aacagttcgg
3361 ctggcgcgag cccctgatgc tcttcgtcca gatcatcctg atcgacaaga ccggcttcca
3421 tccgagtacg tgctcgctcg atgcgatgtt tcgcttggtg gtcgaatggg caggtagccg
3481 gatcaagcgt atgcagccgc cgcattgcat cagccatgat ggatactttc tcggcaggag
3541 caaggtgaga tgacaggaga tcctgccccg gcacttcgcc caatagcagc cagtcccttc
3601 ccgcttcagt gacaacgtcg agcacagctg cgcaaggaac gcccgtcgtg gccagccacg
3661 atagccgcgc tgcctcgtct tggagttcat tcagggcacc ggacaggtcg gtcttgacaa
3721 aaagaaccgg gcgcccctgc gctgacagcc ggaacacggc ggcatcagag cagccgattg
3781 tctgttgtgc ccagtcatag ccgaatagcc tctccaccca agcggccgga gaacctgcgt
3841 gcaatccatc ttgttcaatc atgcgaaacg atcctcatcc tgtctcttga tcagatcttg

3901 atcccctgcg ccatcagatc cttggcggca agaaagccat ccagtttact ttgcagggct
3961 tcccaacctt accagagggc gccccagctg gcaattccgg ttcgcttgct gtccataaaa
4021 ccgcccagtc tagctatcgc catgtaagcc cactgcaagc tacctgcttt ctctttgcgc
4081 ttgcgttttc ccttgtccag atagcccagt agctgacatt cacccgggaa aaaaaaaccc
4141 cgcccctgac agggcggggt tttttttcag ataaaaaaaa tccttagctt tcgctaagga
4201 tgatttctgc aattggcggc cgcttctaga atgcactagt agcggccgct gcagtccggc
4261 aaaaaaacgg gcaaggtgtc accaccctgc cctttttctt taaaaccgaa aagattactt
4321 cgcgttggag agcgttcacc gacaaacaac agataaaacg aaaggcccag tctttcgact
4381 gagcctttcg ttttatttga tgcctggtac cgggtgtaat ccattgggca cgagagttag
4441 taaggcagtg gcaattaata gaggcttatg gttgattcgc attgttttgc tcctgaaatt
4501 ttcggcaaat acaaatactt cgctcttcta gccctattaa ccattttaac gacaaattga
4561 tggggcaacg attaacaaat aatgaataaa ttttatgttt ttcaagatga aaatttgaaa
4621 atttgatttc cttatatttc tactatagaa gactaataca attagatcta aaatttgcaa
4681 gtataaaaat cagcaaatag ttatattgtt aataattcaa tgacccaata actcgtactg
4741 ttatctacgt ggtgaaagcc aaaaagacga acagtttagc ctcctcctcc tcggcgatcg
4801 ccaagcgaaa tgtcatggga gatgttcaga ttgagcattt ttttctaaaa gcccttgcta
4861 aaacaaacca catgtgcagg gtgtccccga tgttgactaa attcagcggc tcgaccatat
4921 gggagagctc ccaacgcgtt ggatgcatag cttgagtatt ctatagtgtc acctaaatag
4981 cttggcgtaa tcatggtcat agctgtttcc tgtgtgaaat tgttatccgc tcacaattcc
5041 acacaacata cgagccggaa gcataaagtg taaagcctgg ggtgcctaat gagtgagcta
5101 actcacatta attgcgttgc gctcactgcc cgctttccag tcgggaaacc tgtcgtgcca
5161 gctgcattaa tgaatcggcc aacgcgcggg gagaggcggt ttgcgtattg ggcgctcttc
5221 cgcttcctcg ctcactgact cgctgcgctc ggtcgttcgg ctgcggcgag cggtatcagc
5281 tcactcaaag gcggtaatac ggttatccac agaatcaggg gataacgcag gaaagaacat
5341 gtgagcaaaa ggccagcaaa aggccaggaa ccgtaaaaag gccgcgttgc tggcgttttt
5401 ccataggctc cgcccccctg acgagcatca caaaaatcga cgctcaagtc agaggtggcg
5461 aaacccgaca ggactataaa gataccaggc gtttccccct ggaagctccc tcgtgcgctc
5521 tcctgttccg accctgccgc ttaccggata cctgtccgcc tttctccctt cgggaagcgt
5581 ggcgctttct catagctcac gctgtaggta tctcagttcg gtgtaggtcg ttcgctccaa
5641 gctgggctgt gtgcacgaac cccccgttca gcccgaccgc tgcgccttat ccggtaacta
5701 tcgtcttgag tccaacccgg taagacacga cttatcgcca ctggcagcag ccactggtaa
5761 caggattagc agagcgaggt atgtaggcgg tgctacagag ttcttgaagt ggtggcctaa
5821 ctacggctac actagaagaa cagtatttgg tatctgcgct ctgctgaagc cagttacctt
5881 cggaaaaaga gttggtagct cttgatccgg caaacaaacc accgctggta gcggtggttt
5941 ttttgtttgc aagcagcaga ttacgcgcag aaaaaaagga tctcaagaag atcctttgat
6001 cttttctacg gggtctgacg ctcagtggaa cgaaaactca cgttaaggga ttttggtcat
6061 gagattatca aaaaggatct tcacctagat ccttttaaat taaaaatgaa gttttaaatc
6121 aatctaaagt atatatgagt aaacttggtc tgacagttac caatgcttaa tcagtgaggc
6181 acctatctca gcgatctgtc tatttcgttc atccatagtt gcctgactcc ccgtcgtgta
6241 gataactacg atacgggagg gcttaccatc tggccccagt gctgcaatga taccgcgaga
6301 cccacgctca ccggctccag atttatcagc aataaaccag ccagccggaa gggccgagcg
6361 cagaagtggt cctgcaactt tatccgcctc catccagtct attaattgtt gccgggaagc
6421 tagagtaagt agttcgccag ttaatagttt gcgcaacgtt gttgccattg ctacaggcat
6481 cgtggtgtca cgctcgtcgt ttggtatggc ttcattcagc tccggttccc aacgatcaag
6541 gcgagttaca tgatccccca tgttgtgcaa aaaagcggtt agctccttcg gtcctccgat
6601 cgttgtcaga agtaagttgg ccgcagtgtt atcactcatg gttatggcag cactgcataa
6661 ttctcttact gtcatgccat ccgtaagatg cttttctgtg actggtgagt actcaaccaa
6721 gtcattctga gaatagtgta tgcggcgacc gagttgctct tgcccggcgt caatacggga
6781 taataccgcg ccacatagca gaactttaaa agtgctcatc attggaaaac gttcttcggg
6841 gcgaaaactc tcaaggatct taccgctgtt gagatccagt tcgatgtaac ccactcgtgc
6901 acccaactga tcttcagcat cttttacttt caccagcgtt tctgggtgag caaaaacagg
6961 aaggcaaaat gccgcaaaaa agggaataag ggcgacacgg aaatgttgaa tactcatact
7021 cttccttttt caatattatt gaagcattta tcagggttat tgtctcatga gcggatacat

7081 atttgaatgt atttagaaaa ataaacaaat aggggttccg cgcacatttc cccgaaaagt
7141 gccacctgat gcggtgtgaa ataccgcaca gatgcgtaag gagaaaatac cgcatcagga
7201 aattgtaagc gttaatattt tgttaaaatt cgcgttaaat ttttgttaaa tcagctcatt
7261 ttttaaccaa taggccgaaa tcggcaaaat cccttataaa tcaaaagaat agaccgagat
7321 agggttgagt gttgttccag tttggaacaa gagtccacta ttaaagaacg tggactccaa
7381 cgtcaaaggg cgaaaaaccg tctatcaggg cgatggccca ctacgtgaac catcacccta
7441 atcaagtttt ttggggtcga ggtgccgtaa agcactaaat cggaacccta aagggagccc
7501 ccgatttaga gcttgacggg gaaagccggc gaacgtggcg agaaaggaag ggaagaaagc
7561 gaaaggagcg ggcgctaggg cgctggcaag tgtagcggtc acgctgcgcg taaccacca

**SEQ ID NO: 15:**

1 cacccgccgc gcttaatgcg ccgctacagg gcgcgtccat tcgccattca ggctgcgcaa
61 ctgttgggaa gggcgatcgg tgcgggcctc ttcgctatta cgccagctgg cgaaaggggg
121 atgtgctgca aggcgattaa gttgggtaac gccagggttt tcccagtcac gacgttgtaa
181 aacgacggcc agtgaattgt aatacgactc actatagggc gaattgggcc cgacgtcgca
241 tgctcccggc cgccatggcg gccgcgggaa ttcgattgtg agcttgatgg tgatggtggg
301 taaagcttca ttgtccatgc gccggagctt gacggtaaaa tccgtgtcct ggggccaatc
361 attgggaacg ctcacacaga taccgttgcg agttaagtcg aaggataccc ccaccaattc
421 cccttccata tcgaacactt ggcaaaaggc caacatccgc tcttcctgcc gtcgctcctg
481 caccccctgg agatgggcaa tggttagggc ttcttgggag agggaagatg gagaatcttg
541 agccatggag attatttcct cggttaaatt aggtttacct agtaaatggg ccaggttgac
601 cattattgta gtcattcact gggtcgatct tccttccatc agggtcattt ggttaattgt
661 tgatgagaaa tgggaaggag taatccatag atatttgccc agttaactcg atttgagcag
721 aattgggaag gacgtattgg gaactttggt tgacagggca acaaagccag cagattacac
781 ccgggcaagc ggatggctga tgaaaccaag ccaaccagga agggcagccc acctatcaag
841 gtgtactgcc ttccagacga acgaagagcg attgaggaaa aggcggcggc ggccggcatg
901 agcctgtcgg cctacctgct ggccgtcggc cagggctaca aaatcacggg cgtcgtggac
961 tatgagcacg tccgcgaggg cgtcccggaa aacgattccg aagcccaacc tttcatagaa
1021 ggcggcggtg gaatcgaaat ctcgtgatgg caggttgggc gtcgcttggt cggtcatttc
1081 gctcggtacc atcggcattt tcttttgcgt tttttatttgt taactgttaa ttgtccttgt
1141 tcaaggatgc tgtctttgac aacagatgtt ttcttgcctt tgatgttcag caggaagctc
1201 ggcgcaaacg ttgattgttt gtctgcgtag aatcctctgt ttgtcatata gcttgtaatc
1261 acgacattgt ttcctttcgc ttgaggtaca gcgaagtgtg agtaagtaaa ggttacatcg
1321 ttaggatcaa gatccatttt taacacaagg ccagttttgt tcagcggctt gtatgggcca
1381 gttaaagaat tagaaacata accaagcatg taaatatcgt tagacgtaat gccgtcaatc
1441 gtcatttttg atccgcggga gtcagtgaac aggtaccatt tgccgttcat tttaaagacg
1501 ttcgcgcgtt caatttcatc tgttactgtg ttagatgcaa tcagcggttt catcactttt
1561 ttcagtgtgt aatcatcgtt tagctcaatc ataccgagag cgccgtttgc taactcagcc
1621 gtgcgttttt tatcgctttg cagaagtttt tgactttctt gacggaagaa tgatgtgctt
1681 ttgccatagt atgctttgtt aaataaagat tcttcgcctt ggtagccatc ttcagttcca
1741 gtgtttgctt caaatactaa gtatttgtgg cctttatctt ctacgtagtg aggatctctc
1801 agcgtatggt tgtcgcctga gctgtagttg ccttcatcga tgaactgctg tacattttga
1861 tacgtttttc cgtcaccgtc aaagattgat ttataatcct ctacaccgtt gatgttcaaa
1921 gagctgtctg atgctgatac gttaacttgt gcagttgtca gtgtttgttt gccgtaatgt
1981 ttaccggaga aatcagtgta gaataaacgg atttttccgt cagatgtaaa tgtggctgaa
2041 cctgaccatt cttgtgtttg gtcttttagg atagaatcat ttgcatcgaa tttgtcgctg
2101 tctttaaaga cgcggccagc gtttttccag ctgtcaatag aagtttcgcc gactttttga
2161 tagaacatgt aaatcgatgt gtcatccgca tttttaggat ctccggctaa tgcaaagacg
2221 atgtggtagc cgtgatagtt tgcgacagtg ccgtcagcgt tttgtaatgg ccagctgtcc
2281 caaacgtcca ggccttttgc agaagagata tttttaattg tggacgaatc aaattcagaa
2341 acttgatatt tttcattttt ttgctgttca gggatttgca gcatatcatg gcgtgtaata
2401 tgggaaatgc cgtatgtttc cttatatggc ttttggttcg tttctttcgc aaacgcttga

2461 gttgcgcctc ctgccagcag tgcggtagta aaggttaata ctgttgcttg ttttgcaaac
2521 tttttgatgt tcatcgttca tgtctccttt tttatgtact gtgttagcgg tctgcttctt
2581 ccagccctcc tgtttgaaga tggcaagtta gttacgcaca ataaaaaaag acctaaaata
2641 tgtaaggggt gacgccaaag tatacacttt gccctttaca cattttaggt cttgcctgct
2701 ttatcagtaa caaacccgcg cgatttactt ttcgacctca ttctattaga ctctcgtttg
2761 gattgcaact ggtctatttt cctcttttgt ttgatagaaa atcataaaag gatttgcaga
2821 ctacgggcct aaagaactaa aaaatctatc tgtttctttt cattctctgt atttttttata
2881 gtttctgttg catgggcata aagttgcctt tttaatcaca attcagaaaa tatcataata
2941 tctcatttca ctaaataata gtgaacggca ggtatatgtg atgggttaaa aaggatcgat
3001 cctctagcga accccagagt cccgctcaga agaactcgtc aagaaggcga tagaaggcga
3061 tgcgctgcga atcgggagcg gcgataccgt aaagcacgag gaagcggtca gcccattcgc
3121 cgccaagctc ttcagcaata tcacgggtag ccaacgctat gtcctgatag cggtccgcca
3181 cacccagccg gccacagtcg atgaatccag aaaagcggcc attttccacc atgatattcg
3241 gcaagcaggc atcgccatgg gtcacgacga gatcctcgcc gtcgggcatc cgcgccttga
3301 gcctggcgaa cagttcggct ggcgcgagcc cctgatgctc ttcgtccaga tcatcctgat
3361 cgacaagacc ggcttccatc cgagtacgtg ctcgctcgat gcgatgtttc gcttggtggt
3421 cgaatgggca ggtagccgga tcaagcgtat gcagccgccg cattgcatca gccatgatgg
3481 atactttctc ggcaggagca aggtgagatg acaggagatc ctgccccggc acttcgccca
3541 atagcagcca gtcccttccc gcttcagtga caacgtcgag cacagctgcg caaggaacgc
3601 ccgtcgtggc cagccacgat agccgcgctg cctcgtcttg gagttcattc agggcaccgg
3661 acaggtcggt cttgacaaaa agaaccgggc gcccctgcgc tgacagccgg aacacggcgg
3721 catcagagca gccgattgtc tgttgtgccc agtcatagcc gaatagcctc tccacccaag
3781 cggccggaga acctgcgtgc aatccatctt gttcaatcat gcgaaacgat cctcatcctg
3841 tctcttgatc agatcttgat cccctgcgcc atcagatcct tggcggcaag aaagccatcc
3901 agtttacttt gcagggcttc ccaaccttac cagagggcgc cccagctggc aattccggtt
3961 cgcttgctgt ccataaaacc gcccagtcta gctatcgcca tgtaagccca ctgcaagcta
4021 cctgctttct ctttgcgctt gcgttttccc ttgtccagat agcccagtag ctgacattca
4081 cccgggaaaa aaaaaccccg cccctgacag ggcggggttt tttttcagat aaaaaaaatc
4141 cttagctttc gctaaggatg atttctgcaa ttggcggccg cttctagaat gcactagtag
4201 cggccgctgc agtccggcaa aaaaacgggc aaggtgtcac caccctgccc tttttcttta
4261 aaaccgaaaa gattacttcg cgttggagag cgttcaccga caaacaacag ataaaacgaa
4321 aggcccagtc tttcgactga gcctttcgtt ttatttgatg cctggtaccg ggtgtaatcg
4381 ctggaggcga actgggtgag aaccataaaa tcttggggaa caggggaatg ttaatgaaca
4441 catgacatgc ataggatagc gaattgattt aaagcaatga ttccccctga gataaatata
4501 ttttgaccca ctgctccgct taattttggc gctaatctgg gcaaaaattc gttggttttc
4561 ggaaaggtga acccgtagct agcttgtatt gcccaaacct aactcatcat tgctccatgg
4621 cggccaaaaa tctttaggac aaaactcgcc agaggtcaag gcttgatttt ttcagaggga
4681 aaaattcctc ggctaaataa tcaaaaagtt gaaaaaaaaa gtttttcagc taaggtttga
4741 tgttatattt tatcttatgt agacttttga aaaaaacaag tccccgcaat caagcatcat
4801 tcaacggaaa aaataatcct atgccaaacg cctccaccgc actaaccggc aaagcattac
4861 tcaataaagt taaagaacta tcccatctgc cccgtcgaga aacggcaaaa gcctgtggtt
4921 actactcgac catatgggag agctcccaac gcgttggatg catagcttga gtattctata
4981 gtgtcaccta aatagcttgg cgtaatcatg gtcatagctg tttcctgtgt gaaattgtta
5041 tccgctcaca attccacaca acatacgagc cggaagcata aagtgtaaag cctggggtgc
5101 ctaatgagtg agctaactca cattaattgc gttgcgctca ctgcccgctt tccagtcggg
5161 aaacctgtcg tgccagctgc attaatgaat cggccaacgc gcggggagag gcggtttgcg
5221 tattgggcgc tcttccgctt cctcgctcac tgactcgctg cgctcggtcg ttcggctgcg
5281 gcgagcggta tcagctcact caaaggcggt aatacggtta tccacagaat caggggataa
5341 cgcaggaaag aacatgtgag caaaaggcca gcaaaaggcc aggaaccgta aaaaggccgc
5401 gttgctggcg tttttccata ggctccgccc ccctgacgag catcacaaaa atcgacgctc
5461 aagtcagagg tggcgaaacc cgacaggact ataaagatac caggcgtttc cccctggaag
5521 ctccctcgtg cgctctcctg ttccgaccct gccgcttacc ggatacctgt ccgcctttct
5581 cccttcggga agcgtggcgc tttctcatag ctcacgctgt aggtatctca gttcggtgta

5641 ggtcgttcgc tccaagctgg gctgtgtgca cgaacccccc gttcagcccg accgctgcgc
5701 cttatccggt aactatcgtc ttgagtccaa cccggtaaga cacgacttat cgccactggc
5761 agcagccact ggtaacagga ttagcagagc gaggtatgta ggcggtgcta cagagttctt
5821 gaagtggtgg cctaactacg gctacactag aagaacagta tttggtatct gcgctctgct
5881 gaagccagtt accttcggaa aaagagttgg tagctcttga tccggcaaac aaaccaccgc
5941 tggtagcggt ggtttttttg tttgcaagca gcagattacg cgcagaaaaa aaggatctca
6001 agaagatcct ttgatctttt ctacggggtc tgacgctcag tggaacgaaa actcacgtta
6061 agggattttg gtcatgagat tatcaaaaag gatcttcacc tagatccttt taaattaaaa
6121 atgaagtttt aaatcaatct aaagtatata tgagtaaact tggtctgaca gttaccaatg
6181 cttaatcagt gaggcaccta tctcagcgat ctgtctattt cgttcatcca tagttgcctg
6241 actccccgtc gtgtagataa ctacgatacg ggagggctta ccatctggcc ccagtgctgc
6301 aatgataccg cgagacccac gctcaccggc tccagattta tcagcaataa accagccagc
6361 cggaagggcc gagcgcagaa gtggtcctgc aactttatcc gcctccatcc agtctattaa
6421 ttgttgccgg gaagctagag taagtagttc gccagttaat agtttgcgca acgttgttgc
6481 cattgctaca ggcatcgtgg tgtcacgctc gtcgtttggt atggcttcat tcagctccgg
6541 ttcccaacga tcaaggcgag ttacatgatc ccccatgttg tgcaaaaaag cggttagctc
6601 cttcggtcct ccgatcgttg tcagaagtaa gttggccgca gtgttatcac tcatggttat
6661 ggcagcactg cataattctc ttactgtcat gccatccgta agatgctttt ctgtgactgg
6721 tgagtactca accaagtcat tctgagaata gtgtatgcgg cgaccgagtt gctcttgccc
6781 ggcgtcaata cgggataata ccgcgccaca tagcagaact ttaaaagtgc tcatcattgg
6841 aaaacgttct tcggggcgaa aactctcaag gatcttaccg ctgttgagat ccagttcgat
6901 gtaacccact cgtgcaccca actgatcttc agcatctttt actttcacca gcgtttctgg
6961 gtgagcaaaa acaggaaggc aaaatgccgc aaaaaaggga ataagggcga cacggaaatg
7021 ttgaatactc atactcttcc tttttcaata ttattgaagc atttatcagg gttattgtct
7081 catgagcgga tacatatttg aatgtattta gaaaaataaa caaatagggg ttccgcgcac
7141 atttccccga aaagtgccac ctgatgcggt gtgaaatacc gcacagatgc gtaaggagaa
7201 aataccgcat caggaaattg taagcgttaa tattttgtta aaattcgcgt taaatttttg
7261 ttaaatcagc tcatttttta accaataggc cgaaatcggc aaaatccctt ataaatcaaa
7321 agaatagacc gagatagggt tgagtgttgt tccagtttgg aacaagagtc cactattaaa
7381 gaacgtggac tccaacgtca aagggcgaaa aaccgtctat cagggcgatg gcccactacg
7441 tgaaccatca ccctaatcaa gttttttggg gtcgaggtgc cgtaaagcac taaatcggaa
7501 ccctaaaggg agcccccgat ttagagcttg acggggaaag ccggcgaacg tggcgagaaa
7561 ggaagggaag aaagcgaaag gagcgggcgc tagggcgctg gcaagtgtag cggtcacgct
7621 gcgcgtaacc acca

**SEQ ID NO:16**

AATCACTAGTGAATTCGCGGCCGCCTGCAGGTCGACCATATGGGAGAGCTCCCAACGCGTTGGATGCATAGCTTGAGTATTCTATAGTGTCACCTAAATAGCTT
GGCGTAATCATGGTCATAGCTGTTTCCTGTGTGAAATTGTTATCCGCTCACAATTCCACACAACATACGAGCCGGAAGCATAAAGTGTAAAGCCTGGGGTGCCT
AATGAGTGAGCTAACTCACATTAATTGCGTTGCGCTCACTGCCCGCTTTCCAGTCGGGAAACCTGTCGTGCCAGCTGCATTAATGAATCGGCCAACGCGCGGGG
AGAGGCGGTTTGCGTATTGGGCGCTCTTCCGCTTCCTCGCTCACTGACTCGCTGCGCTCGGTCGTTCGGCTGCGGCGAGCGGTATCAGCTCACTCAAAGGCGGT
AATACGGTTATCCACAGAATCAGGGGATAACGCAGGAAAGAACATGTGAGCAAAAGGCCAGCAAAAGGCCAGGAACCGTAAAAAGGCCGCGTTGCTGGCGTT
TTTCCATAGGCTCCGCCCCCCTGACGAGCATCACAAAAATCGACGCTCAAGTCAGAGGTGGCGAAACCCGACAGGACTATAAAGATACCAGGCGTTTCCCCCTG
GAAGCTCCCTCGTGCGCTCTCCTGTTCCGACCCTGCCGCTTACCGGATACCTGTCCGCCTTTCTCCCTTCGGGAAGCGTGGCGCTTTCTCATAGCTCACGCTGTAG
GTATCTCAGTTCGGTGTAGGTCGTTCGCTCCAAGCTGGGCTGTGTGCACGAACCCCCCGTTCAGCCCGACCGCTGCGCCTTATCCGGTAACTATCGTCTTGAGTC
CAACCCGGTAAGACACGACTTATCGCCACTGGCAGCAGCCACTGGTAACAGGATTAGCAGAGCGAGGTATGTAGGCGGTGCTACAGAGTTCTTGAAGTGGTGG
CCTAACTACGGCTACACTAGAAGAACAGTATTTGGTATCTGCGCTCTGCTGAAGCCAGTTACCTTCGGAAAAAGAGTTGGTAGCTCTTGATCCGGCAAACAAAC
CACCGCTGGTAGCGGTGGTTTTTTTGTTTGCAAGCAGCAGATTACGCGCAGAAAAAAAGGATCTCAAGAAGATCCTTTGATCTTTTCTACGGGGTCTGACGCTCA
GTGGAACGAAAACTCACGTTAAGGGATTTTGGTCATGAGATTATCAAAAAGGATCTTCACCTAGATCCTTTTAAATTAAAAATGAAGTTTTAAATCAATCTAAAG
TATATATGAGTAAACTTGGTCTGACAGTTACCAATGCTTAATCAGTGAGGCACCTATCTCAGCGATCTGTCTATTTCGTTCATCCATAGTTGCCTGACTCCCCGTC
GTGTAGATAACTACGATACGGGAGGGCTTACCATCTGGCCCCAGTGCTGCAATGATACCGCGAGACCCACGCTCACCGGCTCCAGATTTATCAGCAATAAACCA
GCCAGCCGGAAGGGCCGAGCGCAGAAGTGGTCCTGCAACTTTATCCGCCTCCATCCAGTCTATTAATTGTTGCCGGGAAGCTAGAGTAAGTAGTTCGCCAGTTA
ATAGTTTGCGCAACGTTGTTGCCATTGCTACAGGCATCGTGGTGTCACGCTCGTCGTTTGGTATGGCTTCATTCAGCTCCGGTTCCCAACGATCAAGGCGAGTTA
CATGATCCCCCATGTTGTGCAAAAAAGCGGTTAGCTCCTTCGGTCCTCCGATCGTTGTCAGAAGTAAGTTGGCCGCAGTGTTATCACTCATGGTTATGGCAGCAC
TGCATAATTCTCTTACTGTCATGCCATCCGTAAGATGCTTTTCTGTGACTGGTGAGTACTCAACCAAGTCATTCTGAGAATAGTGTATGCGGCGACCGAGTTGCTC
TTGCCCGGCGTCAATACGGGATAATACCGCGCCACATAGCAGAACTTTAAAAGTGCTCATCATTGGAAAACGTTCTTCGGGGCGAAAACTCTCAAGGATCTTAC
CGCTGTTGAGATCCAGTTCGATGTAACCCACTCGTGCACCCAACTGATCTTCAGCATCTTTTACTTTCACCAGCGTTTCTGGGTGAGCAAAAACAGGAAGGCAAA
ATGCCGCAAAAAAGGGAATAAGGGCGACACGGAAATGTTGAATACTCATACTCTTCCTTTTTCAATATTATTGAAGCATTTATCAGGGTTATTGTCTCATGAGCG
GATACATATTTGAATGTATTTAGAAAAATAAACAAATAGGGGTTCCGCGCACATTTCCCCGAAAAGTGCCACCTGATGCGGTGTGAAATACCGCACAGATGCGT
AAGGAGAAAATACCGCATCAGGAAATTGTAAGCGTTAATATTTTGTTAAAATTCGCGTTAAATTTTTGTTAAATCAGCTCATTTTTTAACCAATAGGCCGAAATCG

GCAAAATCCCTTATAAATCAAAAGAATAGACCGAGATAGGGTTGAGTGTTGTTCCAGTTTGGAACAAGAGTCCACTATTAAAGAACGTGGACTCCAACGTCAAA
GGGCGAAAAACCGTCTATCAGGGCGATGGCCCACTACGTGAACCATCACCCTAATCAAGTTTTTTGGGGTCGAGGTGCCGTAAAGCACTAAATCGGAACCCTAA
AGGGAGCCCCCGATTTAGAGCTTGACGGGGAAAGCCGGCGAACGTGGCGAGAAAGGAAGGGAAGAAAGCGAAAGGAGCGGGCGCTAGGGCGCTGGCAAGT
GTAGCGGTCACGCTGCGCGTAACCACCACACCCGCCGCGCTTAATGCGCCGCTACAGGGCGCGTCCATTCGCCATTCAGGCTGCGCAACTGTTGGGAAGGGCG
ATCGGTGCGGGCCTCTTCGCTATTACGCCAGCTGGCGAAAGGGGGATGTGCTGCAAGGCGATTAAGTTGGGTAACGCCAGGGTTTTCCCAGTCACGACGTTGT
AAAACGACGGCCAGTGAATTGTAATACGACTCACTATAGGGCGAATTGGGCCCGACGTCGCATGCTCCCGGCCGCCATGGCGGCCGCGGGAATTCGATT

**SEQ ID NO:17**

CCCGGGTGAATGTCAGCTACTGGGCTATCTGGACAAGGGAAAACGCAAGCGCAAAGAGAAAGCAGGTAGCTTGCAGTGGGCTTACATGGCGATAGCTAGACT
GGGCGGTTTTATGGACAGCAAGCGAACCGGAATTGCCAGCTGGGGCGCCCTCTGGTAAGGTTGGGAAGCCCTGCAAAGTAAACTGGATGGCTTTCTTGCCGCC
AAGGATCTGATGGCGCAGGGGATCAAGATCTGATCAAGAGACAGGATGAGGATCGTTTCGCATGATTGAACAAGATGGATTGCACGCAGGTTCTCCGGCCGCT
TGGGTGGAGAGGCTATTCGGCTATGACTGGGCACAACAGACAATCGGCTGCTCTGATGCCGCCGTGTTCCGGCTGTCAGCGCAGGGGCGCCCGGTTCTTTTTGT
CAAGACCGACCTGTCCGGTGCCCTGAATGAACTCCAAGACGAGGCAGCGCGGCTATCGTGGCTGGCCACGACGGGCGTTCCTTGCGCAGCTGTGCTCGACGTT
GTCACTGAAGCGGGAAGGGACTGGCTGCTATTGGGCGAAGTGCCGGGGCAGGATCTCCTGTCATCTCACCTTGCTCCTGCCGAGAAAGTATCCATCATGGCTG
ATGCAATGCGGCGGCTGCATACGCTTGATCCGGCTACCTGCCCATTCGACCACCAAGCGAAACATCGCATCGAGCGAGCACGTACTCGGATGGAAGCCGGTCTT
GTCGATCAGGATGATCTGGACGAAGAGCATCAGGGGCTCGCGCCAGCCGAACTGTTCGCCAGGCTCAAGGCGCGGATGCCCGACGGCGAGGATCTCGTCGTG
ACCCATGGCGATGCCTGCTTGCCGAATATCATGGTGGAAAATGGCCGCTTTTCTGGATTCATCGACTGTGGCCGGCTGGGTGTGGCGGACCGCTATCAGGACAT
AGCGTTGGCTACCCGTGATATTGCTGAAGAGCTTGGCGGCGAATGGGCTGACCGCTTCCTCGTGCTTTACGGTATCGCCGCTCCCGATTCGCAGCGCATCGCCTT
CTATCGCCTTCTTGACGAGTTCTTCTGAGCGGGACTCTGGGGTTCGCTAGAGGATCGATCCTTTTTAACCCATCACATATACCTGCCGTTCACTATTATTTAGTGA
AATGAGATATTATGATATTTTCTGAATTGTGATTAAAAAGGCAACTTTATGCCCATGCAACAGAAACTATAAAAAATACAGAGAATGAAAAGAAACAGATAGAT
TTTTTAGTTCTTTAGGCCCGTAGTCTGCAAATCCTTTTATGATTTTCTATCAAACAAAAGAGGAAAATAGACCAGTTGCAATCCAAACGAGAGTCTAATAGAATGA
GGTCGAAAAGTAAATCGCCCGGG

**SEQ ID NO:18**

CCGGGTGAATGTCAGCTACTGGGCTATCTGGACAAGGGAAAACGCAAGCGCAAAGAGAAAGCAGGTAGCTTGCAGTGGGCTTACATGGCGATAGCTAGACTG
GGCGGTTTTATGGACAGCAAGCGAACCGGAATTGCCAGCTGGGGCGCCCTCTGGTAAGGTTGGGAAGCCCTGCAAAGTAAACTGGATGGCTTTCTTGCCGCCA
AGGATCTGATGGCGCAGGGGATCAAGATCTGATCAAGAGACAGGATGAGGATCGTTTCGCATGATTGAACAAGATGGATTGCACGCAGGTTCTCCGGCCGCTT
GGGTGGAGAGGCTATTCGGCTATGACTGGGCACAACAGACAATCGGCTGCTCTGATGCCGCCGTGTTCCGGCTGTCAGCGCAGGGGCGCCCGGTTCTTTTTGT
CAAGACCGACCTGTCCGGTGCCCTGAATGAACTCCAAGACGAGGCAGCGCGGCTATCGTGGCTGGCCACGACGGGCGTTCCTTGCGCAGCTGTGCTCGACGTT
GTCACTGAAGCGGGAAGGGACTGGCTGCTATTGGGCGAAGTGCCGGGGCAGGATCTCCTGTCATCTCACCTTGCTCCTGCCGAGAAAGTATCCATCATGGCTG
ATGCAATGCGGCGGCTGCATACGCTTGATCCGGCTACCTGCCCATTCGACCACCAAGCGAAACATCGCATCGAGCGAGCACGTACTCGGATGGAAGCCGGTCTT
GTCGATCAGGATGATCTGGACGAAGAGCATCAGGGGCTCGCGCCAGCCGAACTGTTCGCCAGGCTCAAGGCGCGGATGCCCGACGGCGAGGATCTCGTCGTG
ACCCATGGCGATGCCTGCTTGCCGAATATCATGGTGGAAAATGGCCGCTTTTCTGGATTCATCGACTGTGGCCGGCTGGGTGTGGCGGACCGCTATCAGGACAT
AGCGTTGGCTACCCGTGATATTGCTGAAGAGCTTGGCGGCGAATGGGCTGACCGCTTCCTCGTGCTTTACGGTATCGCCGCTCCCGATTCGCAGCGCATCGCCTT
CTATCGCCTTCTTGACGAGTTCTTCTGAGCGGGACTCTGGGGTTCGCTAGAGGATCGATCCTTTTTAACCCATCACATATACCTGCCGTTCACTATTATTTAGTGA
AATGAGATATTATGATATTTTCTGAATTGTGATTAAAAAGGCAACTTTATGCCCATGCAACAGAAACTATAAAAAATACAGAGAATGAAAAGAAACAGATAGAT
TTTTTAGTTCTTTAGGCCCGTAGTCTGCAAATCCTTTTATGATTTTCTATCAAACAAAAGAGGAAAATAGACCAGTTGCAATCCAAACGAGAGTCTAATAGAATGA
GGTCGAAAAGTAAATCGCGCGGGTTTGTTACTGATAAAGCAGGCAAGACCTAAAATGTGTAAAGGGCAAAGTGTATACTTTGGCGTCACCCCTTACATATTTTA
GGTCTTTTTTTATTGTGCGTAACTAACTTGCCATCTTCAAACAGGAGGGCTGGAAGAAGCAGACCGCTAACACAGTACATAAAAAAGGAGACATGAACGATGAA
CATCAAAAAGTTTGCAAAACAAGCAACAGTATTAACCTTTACTACCGCACTGCTGGCAGGAGGCGCAACTCAAGCGTTTGCGAAAGAAACGAACCAAAAGCCAT
ATAAGGAAACATACGGCATTTCCCATATTACACGCCATGATATGCTGCAAATCCCTGAACAGCAAAAAAATGAAAAATATCAAGTTTCTGAATTTGATTCGTCCA
CAATTAAAAATATCTCTTCTGCAAAAGGCCTGGACGTTTGGGACAGCTGGCCATTACAAAACGCTGACGGCACTGTCGCAAACTATCACGGCTACCACATCGTCT
TTGCATTAGCCGGAGATCCTAAAAATGCGGATGACACATCGATTTACATGTTCTATCAAAAAGTCGGCGAAACTTCTATTGACAGCTGGAAAAACGCTGGCCGC
GTCTTTAAAGACAGCGACAAATTCGATGCAAATGATTCTATCCTAAAAGACCAAACACAAGAATGGTCAGGTTCAGCCACATTTACATCTGACGGAAAAATCCGT
TTATTCTACACTGATTTCTCCGGTAAACATTACGGCAAACAAACACTGACAACTGCACAAGTTAACGTATCAGCATCAGACAGCTCTTTGAACATCAACGGTGTA
GAGGATTATAAATCAATCTTTGACGGTGACGGAAAAACGTATCAAAATGTACAGCAGTTCATCGATGAAGGCAACTACAGCTCAGGCGACAACCATACGCTGA
GAGATCCTCACTACGTAGAAGATAAAGGCCACAAATACTTAGTATTTGAAGCAAACACTGGAACTGAAGATGGCTACCAAGGCGAAGAATCTTTATTTAACAAA
GCATACTATGGCAAAAGCACATCATTCTTCCGTCAAGAAAGTCAAAAACTTCTGCAAAGCGATAAAAAACGCACGGCTGAGTTAGCAAACGGCGCTCTCGGTAT
GATTGAGCTAAACGATGATTACACACTGAAAAAAGTGATGAAACCGCTGATTGCATCTAACACAGTAACAGATGAAATTGAACGCGCGAACGTCTTTAAAATGA
ACGGCAAATGGTACCTGTTCACTGACTCCCGCGGATCAAAAATGACGATTGACGGCATTACGTCTAACGATATTTACATGCTTGGTTATGTTTCTAATTCTTTAAC
TGGCCCATACAAGCCGCTGAACAAAACTGGCCTTGTGTTAAAAATGGATCTTGATCCTAACGATGTAACCTTTACTTACTCACACTTCGCTGTACCTCAAGCGAAA
GGAAACAATGTCGTGATTACAAGCTATATGACAAACAGAGGATTCTACGCAGACAAACAATCAACGTTTGCGCCGAGCTTCCTGCTGAACATCAAAGGCAAGAA
AACATCTGTTGTCAAAGACAGCATCCTTGAACAAGGACAATTAACAGTTAACAAATAAAAACGCAAAAGAAAATGCCGATGGTACCGAGCGAAATGACCGACC
AAGCGACGCCCAACCTGCCATCACGAGATTTCGATTCCACCGCCGCCTTCTATGAAAGGTTGGGCTTCGGAATCGTTTTCCGGGACGCCCTCGCGGACGTGCTC
ATAGTCCACGACGCCCGTGATTTTGTAGCCCTGGCCGACGGCCAGCAGGTAGGCCGACAGGCTCATGCCGGCCGCCGCCGCCTTTTCCTCAATCGCTCTTCGTTC
GTCTGGAAGGCAGTACACCTTGATAGGTGGGCTGCCCTTCCTGGTTGGCTTGGTTTCATCAGCCATCCGCTTGC

**SEQ ID NO:19**

ATTAAAGAGGAGAAATACTAGATGCGTAAAGGAGAAGAACTTTTCACTGGAGTTGTCCCAATTCTTGTTGAATTAGATGGTGATGTTAATGGGCACAAATTTTC
TGTCAGTGGAGAGGGTGAAGGTGATGCAACATACGGAAAACTTACCCTTAAATTTATTTGCACTACTGGAAAACTACCTGTTCCATGGCCAACACTTGTCACTAC
TTTCGGTTATGGTGTTCAATGCTTTGCGAGATACCCAGATCATATGAAACAGCATGACTTTTTCAAGAGTGCCATGCCCGAAGGTTATGTACAGGAAAGAACTAT
ATTTTTCAAAGATGACGGGAACTACAAGACACGTGCTGAAGTCAAGTTTGAAGGTGATACCCTTGTTAATAGAATCGAGTTAAAAGGTATTGATTTTAAAGAAG
ATGGAAACATTCTTGGACACAAATTGGAATACAACTATAACTCACACAATGTATACATCATGGCAGACAAACAAAAGAATGGAATCAAAGTTAACTTCAAAATT
AGACACAACATTGAAGATGGAAGCGTTCAACTAGCAGACCATTATCAACAAAATACTCCAATTGGCGATGGCCCTGTCCTTTTACCAGACAACCATTACCTGTCC
ACACAATCTGCCCTTTCGAAAGATCCCAACGAAAAGAGAGACCACATGGTCCTTCTTGAGTTTGTAACAGCTGCTGGGATTACACATGGCATGGATGAACTATA
CAAATAATAATACTAGAGCCAGGCATCAAATAAAACGAAAGGCTCAGTCGAAAGACTGGGCCTTTCGTTTTATCTGTTGTTTGTCGGTGAACGCTCTCTACTAGA
GTCACACTGGCTCACCTTCGGGTGGGCCTTTCTGCGTTTATA

**SEQ ID NO:20**
AGCTTTACAAAACTCTCATTAATCCTTTAGACTAAGTTTAGTCAGTTCCAATCTG
**SEQ ID NO:21**

GCGGCCTCGAGTGGGGGCATCTGCTAGGCAATATTTGACCACATTGGCCAATTCTTCCAATTGGGCGATCGCCTCGGCTCCTTCTAATTTCATTAATTCTCGGTCA
TCCCGCATTTCTGTCCAGGTGATGCCGTAGTCCGATACCAAAAAGCGGATCAGATGATTTTCCCCCAAGCTCACCATGAAGGAGGCGCTGTTGAGTTGGTCGCC
ACAGGTGTAGCAAGAAGCAATGTAGCCCCGATTTTCGAGCACAGTGGCCAAAGCCCTCAAGCTCATGACTAAATCCTTCACGAAATCGCGGTGTTGCTGGGCAA
GTCGAGTAAACACATTTCCTCCGAAAATAAGCTAGCTTTTAAAATCTTAATACTTTTTTAAGATTTTCACAGAGGCCTTGTCCGGAAAGGCTAATTTAATACTGTTC
AAACTGGTCTGGAAATCCCGGTCGGTCGGGCCATGGTAATTATCCATCGGCCGGGCCACTGAAATAATCAACCGATTACACCCGGGTGTAATCCTGAAGCCTTG
ACAATCCCCGTTGGTGATTTATACTTAGGACAGGTGTGAGGAAATTAGAAAAACATCTGGAGTCGAAACAAGGAAAGACGCCCCAGGTGTTTTTTTATGGCAAT
TTTTAGGGCAGTCCACAGATGAAGCAATGGCGCTGGCGGATGGTATTGGTGGCGATCGCCGCTCTGGTGGTGATTTCCCTAGCTACGCTGTTTCAACCTCAGGA
AACCTCTGGCCGCATCCCGGATTATCAAATTAGCAGTCAGTTGCCCGCCAACCAGGTAGATTATTATCCTCTGCAACAGAATTTAGATGAGGCGTATTATCGGCC
TTTGGGAGAGTGGTTGGGTCGTTTAATCCTGCCGACGGTGGAAGAAACTAAAGCCAGGACAGGAGATTGGGTTTGGTTGGAACTGTATCAAGCGCCTTCTATC
CAACAGGGATTAGTGGGACAAAAACTCCGTCTCACCTGGCAGAGCAATGCTGATTTGGAC

**SEQ ID NO: 22**

GAACACTCGAGTGCCAGCAACGACAATGGTTAGGGTTACCAAAGTGAATACTAAATATTCCTTGGCCTTAGCGGATAGTAATGGAGATGGGGAATAATTTTTCA
AACTAAACATGGTATTCGTTAATGAATGGTGAACAAGGCAAAGTCAAAAAATGTAACTTTAATTACTGACCAAAGACGGCGGCAATAACCACTAGCACAAAATA
GATGGCAATGAAAATTATGCCGGCACCAAAGGACCACCAACAAAACTTTTTCGCTTCCTTGGAAGCCTTTACAGCGCCTTCATAGTCCCCTGAAGCTAAACGAGA
ATTAACTTCCGATGCTTTGATAATGGCCACAATACCCAGGGGTAAGCAACAAAATAGAGTTACCAGGATCGATTGCGCCAAGTAATTTGGTACATACTGATTTGT
CATAAAACAAACTCATTTCTGAGTAGACTGGAATATACCAGCCAGGATAGACAGAAATTATCTTAATGGTTCATTTTGTTGCGTTTTCTTCACAATTTGATCTTGC
TTATTTGTTAGTTTCAGAGCACTATTAATAACAATAGGGGAATGAAGGATTACACCCGGGTGTAATCTTGTCACCAATTTTTGTAGGGATGTTGGGCTAAATTGA
CGTTGTAATAGCGGGCATCATCCGTTACCTCTTCCCCGATCCAGGGAAGTAGACTTATTTTTTCACCAGTGTAGGTTAATTCCACTTCCGCTAAAATTAGACCCTG
GTTATCCCCCAAAAACTCGTCTACTTCTCAGGTTTTACCATGGTAATCGAGGCAATAACGATATTTTCAATCAGGGGCGGTGAACAGAGTTCTGTCAAAATTTAAT
TGGCTTCCACTGCAAGAATTTCATACTCAAATCCAAGCGGGCAATGCTGGCATTTTTACCCTTAATGGTGAGATAGGCCCGATCGCCAATGGTACGGACTCGCAG
CGTAGTTAAATCTGGGGTGGCAATGTAACCTTGACGGTACAAATAACCCTGGACTAAACTCCGCCAGCGGTCATC

**SEQ ID NO: 23**

GATAACTCGAGTCGCCCGGTGGATTTAATGCCCCTTTGCGGACGGGTATTTTCCAAGCCTGGTTTTGGTACCTTTGCTGAAGCGTTAAAGTTAGAAGTCCCGATT
ATTTCCCTCACCAGGGATGGTTTTGCGGAAGCGCAAGTTTTACTGGCAGGTTTGCAGAACTACGGAGAGCACCAAATTGTGCCCCACCAGCAATTTTTCGCCGG
CAACTGGGATTTTTTGTTGGAAAGCCCCCAAAAACCTAGGTTGGATCAGAAGTTAGACAAACATGGCGATCGCCAGATAGCAGAAAAAATCGTCGCCCAAATTA
GTTGATTTTGTCCCCCAACTCGGAAAAAGTCAGGGGATTTTGGTAAACCATAAACCTGGGGTTAACCTCTGGGAAGCCGTTCATGGTTGTTCATGCCGTATGCTT
TTACGATCTAAATGCTATCTGACACCTCCGTTGACTTAATAAAACTTAATCCAAATAATTTGACTTAGTGGTTCTTTTCAGGCATTCTCTCAGCATTAGAAGGCTGA
GTATGGGGATTACACCCGGGTGTAATCCGAGCGCAAACTACAATGCGCTTCGTTTGCGCTGCACAGCTTCAACGTTCGGCGGGCTTGCTTGCCAGCACTCTTTTC
ATTAAAATAGTATAGCAGTCTGTATTATCGGCATTCAACTCTGATGGATAATCTAACCGACTTTGCATTCAAGCTCGGGAAGGTCGTTTACAAAGTTATTCGCGA
TGTCGAGGCATCAAAAGATGATTCGACCAAGCTCATCCAAGACCTTGGCGGCTTAAGCGGTGAAAAGATACTACTTCAGAAAATCGCCAAGTTTTGGAATCAGC
AAGACCGCTGGGATAGACCCGATGGCCTCGTCATAGTAACTAGTCACAGACTTGTCTTTTTAGCTAAAATGAAGACGGTCGCATCAACAACAGATTATCTCAGCT
TTCCATTGGGGTTGATTGACGAACTTGAAGCTACCACTGTATCTTGGGTCAGTCC

**SEQ ID NO: 24**

GGTTCTCGAGCCGCTGAATTTAGTCAACATCGGGGACACCCTGCACATGTGGTTTGTTTTAGCAAGGGCTTTTAGAAAAAAATGCTCAATCTGAACATCTCCCAT
GACATTTCGCTTGGCGATCGCCGAGGAGGAGGAGGCTAAACTGTTCGTCTTTTTGGCTTTCACCACGTAGATAACAGTACGAGTTATTGGGTCATTGAATTATTA
ACAATATAACTATTTGCTGATTTTTATACTTGCAAATTTTAGATCTAATTGTATTAGTCTTCTATAGTAGAAATATAAGGAAATCAAATTTTCAAATTTTCATCTTGA
AAAACATAAAATTTATTCATTATTTGTTAATCGTTGCCCCATCAATTTGTCGTTAAAATGGTTAATAGGGCTAGAAGAGCGAAGTATTTGTATTTGCCGAAAATTT
CAGGAGCAAAACAATGCGAATCAACCATAAGCCTCTATTAATTGCCACTGCCTTACTAACTCTCGTGCCCAATGGATTACACCCGGGTGTAATCTCTTTACAATG
GCCAGGTCTTTAGGGAGCGGTGACCAACGACCAAATAATTTAATTTTTCCAATTATTTTGATAGTTATTTGGGGAACATGGACGAATAAAATTCGGATAAAAAGT
TAAATTTTTCTAATTCTATAGTCGGGGTTTTCACCTATGACTACTCCCGTTTTTTGTACTAATTGTGGCAACCGCCTTAGCCCCCAGGTTCGTTTCTGTGAATCCTGT
GGATGTCCCGTTGCCTTGACGTCGGAGCCACCTTCCTTTTCTGGACCACCATTGTCCCCATTGCCTCCTCCCCCTCCCACCTTTAATGTCGATGAAGTTCCATCCCA
TAAAAGAGGGGTAACTCCTTGGATTCCTTTTTTCCTGTTATTTAGTTCTGTCGTTGTATTAGGGGGACTTTGGTGGTTGGGAGCGTTCAATCTGCCCCAATGGAAT
CAATGGCTAGTAAAAATTTTGCCCACCAATACCAGTTCCCCCGTTGTTACTTCCCCTACTCCGACGGTGGCTCCCGTTGATGCCAATGCCGTAAGTTTAG

**SEQ ID NO: 25**

GAACTCGAGTAGTAACCACAGGCTTTTGCCGTTTCTCGACGGGGCAGATGGGATAGTTCTTTAACTTTATTGAGTAATGCTTTGCCGGTTAGTGCGGTGGAGGC
GTTTGGCATAGGATTATTTTTTCCGTTGAATGATGCTTGATTGCGGGGACTTGTTTTTTTCAAAAGTCTACATAAGATAAAATATAACATCAAACCTTAGCTGAAA
AACTTTTTTTTTCAACTTTTTGATTATTTAGCCGAGGAATTTTTCCCTCTGAAAAAATCAAGCCTTGACCTCTGGCGAGTTTTGTCCTAAAGATTTTTGGCCGCCAT
GGAGCAATGATGAGTTAGGTTTGGGCAATACAAGCTAGCTACGGGTTCACCTTTCCGAAAACCAACGAATTTTTGCCCAGATTAGCGCCAAAATTAAGCGGAGC
AGTGGGTCAAAATATATTTATCTCAGGGGGAATCATTGCTTTAAATCAATTCGCTATCCTATGCATGTCATGTGTTCATTAACATTCCCCTGTTCCCCAAGATTTTA
TGGTTCTCACCCAGTTCGCCTCCAGCGATTACACCCGGGTGTAATCTGCTGGCTTTGTTGCCCTGTCAACCAAAGTTCCCAATACGTCCTTCCCAATTCTGCTCAAA
TCGAGTTAACTGGGCAAATATCTATGGATTACTCCTTCCCATTTCTCATCAACAATTAACCAAATGACCCTGATGGAAGGAAGATCGACCCAGTGAATGACTACA
ATAATGGTCAACCTGGCCCATTTACTAGGTAAACCTAATTTAACCGAGGAAATAATCTCCATGGCTCAAGATTCTCCATCTTCCCTCTCCCAAGAAGCCCTAACCA
TTGCCCATCTCCAGGGGGTGCAGGAGCGACGGCAGGAAGAGCGGATGTTGGCCTTTTGCCAAGTGTTCGATATGGAAGGGGAATTGGTGGGGGTATCCTTCG
ACTTAACTCGCAACGGTATCTGTGTGAGCGTTCCCAATGATTGGCCCCAGGACACGGATTTTACCGTCAAGCTCCGGCGCATGGACAATGAAGCTTTACCCACCA
TCACCATCAAGCTCAC

**SEQ ID NO: 26**

ATGCCCCGGGAAAAAAAAACCCCGCCCCTGACAGGGCGGGGTTTTTTTTCAGATAAAAAAAATCCTTAGCTTTCGCTAAGGATGATTTCTGCAATTGGCGGCCG
CTTCTAGAATGCACTAGTAGCGGCCGCTGCAGTCCGGCAAAAAAACGGGCAAGGTGTCACCACCCTGCCCTTTTTCTTTAAAACCGAAAAGATTACTTCGCGTTG
GAGAGCGTTCACCGACAAACAACAGATAAAACGAAAGGCCCAGTCTTTCGACTGAGCCTTTCGTTTTATTTGATGCCTGGTACCGGTATGC

**SEQ ID NO: 27**

ATGACCCCAACCACTGAGAACTTTACCCCAAGTGCCGATTTAGCCCGCCCCTCGGTGGCGGACACCGTGATTGGTTCCGCTAAAAAAACTTTATTTATTCGCAAA
CCCACCACTGACGATGGTTGGGGTATTTACGAACTGGTTAAAGCGTGTCCACCTTTAGACGTGAACTCTGGCTACGCTTACCTTTTGCTCGCCACCCAATTTCGTG
ACACCTGTGCGGTGGCGACCGATGAGGAGGGGGAAATTGTTGGTTTTGTGAGTGGTTATGTAAAGCGTAACGCGCCCGATACCTATTTCTTGTGGCAAGTGGC
GGTGGGGGAAAAAGCACGGGGAACCGGGCTCGCCCGGAGATTAGTGGAAGCTGTGCTAATGCGCCCCGGAATGGGGGATGTTCGCCATCTCGAAACGACTAT

TACCCCGGACAACGAAGCTAGTTGGGGGCTTTTTAAAAGATTGGCCGATCGCTGGCAGGCCCCACTGAATAGTCGCGAATATTTTTCCACAGGTCAGCTAGGCG
GCGAACATGATCCTGAAAACCTGGTACGGATAGGACCCTTCGAACCCCAGCAAATCTAATAA

**SEQ ID NO: 28**

ATGCAGACGCAAATCTTAGAACGTATGGAGAGTGAAGTGCGTACCTATTCTCGTTCTTTCCCCACCGTATTTACCGAGGCCAAAGGTGCGCGGTTGCACGCCGA
GGACGGCAATCAGTACATTGATTTTTTAGCGGGGGCTGGAACTCTAAATTATGGTCACAACCACCCCAAATTGAAGCAAGCCTTGGCCGATTATATCGCCTCTGA
TGGTATTGTTCATGGCCTGGACATGTGGTCCGCCGCTAAACGCGACTATTTAGAAACATTAGAAGAAGTTATTCTAAAACCGCGCGGGTTAGACTACAAAGTGC
ATTTGCCCGGCCCCACCGGGACTAATGCGGTGGAAGCCGCCATCCGCTTGGCCAGAAATGCCAAAGGCAGACATAATATTGTCACCTTTACCAATGGGTTCCAC
GGTGTTACTATGGGCGCACTCGCCACCACAGGCAACCGCAAGTTTCGGGAAGCTACTGGCGGCATTCCAACTCAAGGAGCCTCTTTTATGCCCTTCGACGGTTA
TATGGGTGAAGGGGTAGACACCTTATCCTATTTTGAAAAGCTCTTGGGCGATAATTCCGGGGGTTTAGACGTTCCCGCCGCCGTAATTATCGAGACTGTCCAAG
GGGAAGGGGGCATAAATCCGGCTGGTATTCCTTGGTTGCAAAGACTAGAAAAGATTTGTCGTGACCATGATATGCTACTTATTGTAGACGACATTCAAGCGGGC
TGTGGTCGTACCGGCAAGTTTTTTAGTTTTGAGCATGCGGGCATTACCCCTGACATTGTGACGAACAGCAAGTCCCTATCCGGGTTTGGTTTGCCATTTGCTCAT
GTGCTTATGAGACCAGAACTGGATATTTGGAAACCAGGTCAGTATAATGGGACCTTTAGAGGCTTCAACTTGGCCTTTGTTACCGCTGCGGCGGCCATGCGACA
TTTCTGGTCCGACGACACTTTTGAACGCGACGTGCAACGAAAAGGGCGTGTCGTGGAAGATAGATTTCAGAAACTGGCGAGTTTTATGACGGAAAAAGGCCAC
CCAGCTTCCGAGCGAGGCCGTGGCTTGATGCGGGGGTTAGATGTTGGCGATGGCGATATGGCCGATAAGATTACTGCGCAGGCATTTAAAAATGGGTTAATTA
TTGAAACGTCCGGTCATTCCGGCCAGGTCATTAAGTGTCTGTGCCCCTTGACAATTACCGACGAAGATCTAGTCGGTGGCTTAGATATTTTGGAACAGAGTGTG
AAAGAAGTGTTTGGTCAAGCGTAATAA

**SEQ ID NO: 29**

ATGATTGTTCGCAACCTGGAAGAATGTCGGAAAACTGAGCGGTTCGTGGAGGCCGAGAATGGGAATTGGGACAGTACCCGGCTCGTGTTAGCCGACGATAAT
GTGGGTTTTTCGTTCAACATTACCCGTATACATCCAGGTACCGAAACACATATTCACTACAAGCATCACTTTGAAGCGGTGTTCTGCTATGAAGGCGAAGGAGAG
GTGGAAACCTTGGCCGATGGTAAGATTCACCCCATCAAGGCCGGAGATATGTATCTACTGGACCAGCATGATGAACATCTCTTGCGGGGTAAAGAAAAAGGCA
TGACAGTCGCCTGCGTGTTCAACCCAGCTCTGACCGGCCGCGAAGTCCATCGTGAAGACGGGTCCTACGCTCCCGTGGATTAATAA

**SEQ ID NO: 30**

ATGTCTGTGCAATCAGCTTTTAGGCATGAAGATGTTACGTTTGGGAACGCTTTTGACGCCTATCCTACTCGGCTCTCTGACGCCACGGGCTCCCTGTGGCAGGAT
CGAAAAGATGCAGTAGTCAAAGGTAGGGCCGAAGATGGCCCGTTGTCCCGCGATCAACTAGCCCGCTTTGAGCGTGACGGGTTTCTTTTTGAGCCCCACTTCCT
GGCCGATGATGAAGTCGGGGAATTACGTAGAGAACTTGCCGCCCTGCTAGAACGCGACGATTTTCGGGGTCGTGACTTCTCCATTACGGAACCCGATTCTCAAG
AGATCCGGTCATTGTTTGCAGTGCATTTTTTATCCGAGCGGTTTCGTAGATTGGCCGAAGATCCCAGGTTGACCGGGCGTGCACGACAAATTGTCGGTGGGGAC
GTGTATGTACATCAAAGTCGTATTAACTATAAACCGGGCTTTCACGGCAAGGGGTTCAACTGGCATTCTGACTTCGAAACCTGGCATGCGGAAGATGGCATGCC
CGAAATGCACGCGGTGTCCGCCTCGATTGTATTAACCGACAATCATCATTATAATGGGCCCTTAATGTTGATCCCAGGTTCCCATAAAGTATTCGTACACTGCTTA
GGTGAAACCCCAGAAGATCACCACAAGCAGAGTTTGAAGTCCCAAGAGTTCGGAGTGCCTAGTCACGAAGCCTTGCGCCGCTTAATCGGCGCTAATGGGATTG
AAGCGCCCACCGGGGCAGCGGGAGGGCTGTTATTATTCGATTGCAATACCCTACACGGAAGCAATGCCAATATGAGCCCGGACCCGCGCAGTAATGCCTTCTTT
GTGTATAACCGCAGAGATAATGCCTGCCACGCACCATACGGCGCAAAACGCCCTCGACCCAGCTTTTTAGCCCATGCCCCCGACGAAGTGTGGACACCTGATAC
CCATTAATAA

**SEQ ID NO: 31**

ATGCTTCCTGATAAAACAGCCTTGCCTATTATTACCCTTAGCCAACCAACCGCGGAAGTCGGTGCGCAAGTGCATCGACTGATCTCCAAATGTCCACCCTTAGATC
CGAACTCCATGTACTGCAATCTGCTTCAAAGCAGTCACTTCTCAGAAACTGCCGTGGCTGCCAAAATTGGAGATGAATTAGTTGGCTTCGTTTCAGGATATCGTA
TTCCCCAGAGGCCCGACACTTTATTTGTGTGGCAAGTAGCCGTTGGGGAAAAGGCGCGGGGCCAGGGGTTAGCCACTCGTATGCTGAAAGCCATTTTAGCCAG
GCCCGTCAACCAGGACATTAACCGAATTGAAACGACCATTACCCCCAATAATAAAGCGTCGTGGGCTCTGTTTGAAGGGCTGGCCAAAAAGTTGGACACACAG
ATTGGTAGCGCTGTTATGTTTGATAAAACTCGTCACTTTGCAGATCAACATGAGACCGAAATGCTCGTTAAAGTGGGTCCATTTAAAGCAGTGCAAGCATAATAA

**SEQ ID NO: 32**

ATGAAGATTTTTGAGGAGATTGAAAGTGAAGTTAGGTCCTACGCCCGCGCCTTCCCGCGCGTATTTGATCGGGCTCAGGGCGCTAAGATTTACGACCACGAAG
GAAACGAATACCTGGATTTTTTGGCTGGTGCGGGCAGCCTGAATTATGGGCATAACCATCCCGTGTTTAAGGAAAAGTTACTAGAATATATTCAAAATGATGGC
ATTACCCAGGGTCTTGATCTGCATACGCGCGCGAAAGGGGAGTTCTTAGAATCGTTTAATGAACACATTCTCAAGCCCCGTAACTTGGACTATATCGTGATGTTT
ACTGGACCGACAGGGACTAATGCTGTTGAAGCCGCCCTGAAAATAGCGCGCAAAAAAACAGGGCGGGAGAATATTATTTCCTTCACCAACGGTTGGCATGGGC
AAACGCTCGGCAGTTTAAGCGTGACCGGAAACTCAACCCACCGGGGCGGCGCTGGAATTGCTTTGCATGGAAGTACGCGAATACCCTATGATGGTTACCTGGG
TGATGATTTTGATACCACTAATCTATTAGACAAGATGCTGTCAGATAGCAGTTCCGGAGTGGATAAACCCGCGGCTGTGATCGTTGAAACTGTGCAGGGGGAG
GGTGGCATTAACGCTGCCAGCATGACCTGGTTACGATCGCTATCCGAAATCTGTAAGCGGCATGATGTGCTCCTTATCATTGACGATATACAGGCTGGCTGCGG
AAGAACTGGTACCTTTTTTTCCTTTGAGGAAGCCGGTATTTACCCCGATATCGTAACGTTGAGTAAAAGTTTATCCGGATATGGGCTGCCCTTAGCCATCGTGTTA
ATGAAGCCAGAAATAGACCAATGGTCCCCAGGCGAACATAATGGTACTTTTCGAGGGAATAATCACGCCTTTGTGACCGCCAAAGCTGCGATTGATCATTTCTG
GAAAGACGATTCCTTTGCGAAAGATGTGCAACGGAAAGGGCGATACATTGCCGACCGTGTTGATGTAATTGTTGCCAAGTATGGAGAGGGAAACTTTAATTCA
CACGGCCGAGGCATGTTTCGTGGCATTAACTGTGTGTCCGGCGATTTGGCGGGGCAAATCACGCGCAGATGTTTCCAAAAGGGCCTCATCATTGAAACCTCCGG
TGCTGATGATCATGTGGTTAAGTTCCTGTGTCCCCTTATTATTACCGATGAGGAACTCGAAAAAGGTATTGATATTTTGGAACAGGCAATTAAGGAAGTGTGCG
ATAAGGCAGACACGATTCCGGAAGGTAAGGATTTCTTCGAAGGGGATTATAGCGTTAGCAAAGAAGTGAGCAAAGCTGGACACTAATAA

**SEQ ID NO: 33**

ATGATTGTGCGCCAACTCCAAGACGCCCAACAAACCAACCGCCGCATTGTGTCTCCCGATGGGAATTGGGAATCCACAAGATTGTTACTAAAGGATGATGGCAT
GGGTTACTCCTTTCATATTACCACCATTTTTGCAGGAGCTGATTTTCGCATGCACTATCAAAATCACTTAGAAAGCGTATATTGTATCTCCGGCGAGGGCGAAGTA
GAGACCCTTGACGATGGCAAGAAATATCCTATTACTCCTGGCACCTTGTATAATCTCGACCAGCACGACAGGCACATCTTGCGGGCCTTTAAAGAACTTCAACTC
GCCTGCGTTTTCAATCCTCCCCTAAATGGCAAGGAAGTACACAACGCCGAAGGCGCATACGAATTAGAAGCGGAAACTGTGTCAGATTAATAA

**SEQ ID NO: 34**

ATGATTACCGAAAATGCCGCACAATCCGAACAGAGTGAAGATTTTTATCAGTCCCGGAATGGAAGCAAACCCAAAATTATTCCTCGCGTCGATCCTGTGGTCTAT
GCGCAGACGGCAAATCCCGGACTAATCGCCGAGGACTTGCAGGCCCGTTACGAACAGCAAGGTTTTTTGGTTATTGATAATGTGTTTAACGAACGCGAAGTGG
ATTGTTTCAAACAAGAATTAAAGCGGTTGAACGATGATGAAAAGATCAAAGCCAGCGCCGAAGCCATTACAGAGCTGTCGTCCGATGAATTACGCTCCCTATTT
AAAATACATGAAGTGTCACCTGTGTTCAAACGCCTGGCCGCCGATAACCGTCTGGCCGGTCTCGCTCAACACTTATTAAATGATCGAGTATACATTCACCAAAGC
CGTTTGAATTACAAACCCGGTTTCCGTGGCAAGGAATTTTATTGGCACTCCGACTTTGAAACCTGGCACGTAGAGGACGGTATGCCTCGCATGCGCGCTTTATCT
ATGAGTATCATTTTGACCGAAAACGATCAACATAACGGACCACTGATGTTGGTGCCCGGCAGTCACAAGAAATTTGTCGTGTGCGAAGAAGAAACTCCCGAAAA
CCATTATTCAGTTAGTTTAAAAAAACAAGAATATGGGATTCCCTCAGACGAATGTTTAGCCTCATTGGTTGCCGATGGAGGGATAGTAAGCGCCAATGGCAAAC
CTGGTAGTGTGCTTATTTTTGATAGTAACGTGATGCATGGTTCCAATTCAAACATTACCCCGTGGCCTCGCTCCAACTTATTCTTTGTGTATAACGCCATAAATAAT
CGTGTTACCTGGCCCTTTTGCGGTCTGTTACCCCGCCCAGAATACTTGTGTAGCCGGAAAAATATTCGAGTAATCGAACCTAGACCCTTTATAGCCGCCGCGGAC
CAACTAATTTACGCCTAATAA

**SEQ ID NO: 35**

ATGACGTTCCATACCGTAGAAAAGATCGGAGGCACCTCTATGAGTGACTACGTGGCTGTTCGTGACAACATTATCTTGAAACCTGTCCACCGCGAAGATCTGTAC
CAACGTATTTTTGTGGTGTCCGCGTACGGCGGAATTACCGACATGTTATTGGAGCATAAAAAAAATGGGCAAGCCGGGATTTATGGACAGTTTGCGAATAGTGT
GAATGACGACGGATGGCAAGCCAGCCTCGTTGCTTTAAAACAAGCCATGTTTGCCATTAATGCCAATATTTTCGATACCGCAGATGCCATTAAAAAAGCCGATAC
ATTTATTGGCGAGCGGTTAGATGATGCGGAACGGTGTTTAGCTGATCTGCAACACTTGTGTCGTCACGGGCATTTCTCGTTAGATGCGCACTTGTCCACCGTGAG
GGAGATGCTCGCTAGTCTTGGGGAAGCGCATAGCGCCTGGAACACAGTGCAACTCTTGCAACAAGATGGTATTAACGCCGTTTTTGTGGATCTAACCGGGTGG
CAAACCGATAAACACATGACCTTAGATGAACGTATTAAAAATGCTTTTGCAGAAATTGACTTACGTCAGCAACTGCCGATCGCCACAGGCTATGCTCACAGCATT
GACGGTCTGATGAGTACCTTCGACCGGGGTTATTCAGAAATGACCTTTAGCCGTATCGCAGTCCTGACCAAGGCTGATGAGGCTATCATTCATAAGGAATTTCA
CTTGAGTAGTGCCGATCCTCGGTTGGTCGGAGAAGATAAAGCCGTTCCCATTGGTCGCACCAATTATGATGTAGCGGATCAACTCGCCAACCTGGGTATGGAGG
CTATCCACCCCAAAGCCGCGAAGGGCCTCCGCCAAAACGATATAGCCCTACGTGTGCGTAATACGTTTGAACCCGATCATGCAGGGACACTAATTACCGGAGAT
TACGTGTCCAGTAAACCATGTGTTGAAATTATAGCAGGCTGTAAAGGGGTCTATGCGTTCGAAGTTTTCGACCAAAACATGGCCGGTGAAATACACAATTACGA
TCGCGAGATTCTGGGCCAGATTCGCCGTTTTCGCGCTCATATTGTAAGTAAGGACATTAATGCAAACACGATTACACACTACTTATCTGCCTCATTAAAGACGATT
CGTCGGATCCGAGATGCCTTGCAAGAGATTTATCCAGACGCGGAAATCAACCAACAAAAGGTGAGTATTGTGAGTGCCATTGGGTCTGATATGAAGATCCCCG
GTATTCTAGCCAAAACCGTGAGTGCTCTGGCTGAGAAACAAATCTCGGTTTTGGCCATGCATCAATCCATGCGTCAGGTTGATATGCAGTTTGTAATTGATGAGG
ACGCGTACACTGATGCCATGAAAAGTTTACATTGTCACTTGGTGGAAGTTCATGATCATGGGATTGCGATTTGCCTAGCGAGCTAATAA

**SEQ ID NO: 36:** CATGGATTGAAGGAAGGG
**SEQ ID NO: 37:** TTTGGTGAACAATTAATGAAC
**SEQ ID NO: 38:** GCAGTCCCTCCTCCAAAC
**SEQ ID NO: 39:** AAGCCGATATTCAGGTAAGATG
**SEQ ID NO: 40:** GGAAGTGTTGTTGCTGTC
**SEQ ID NO: 41:** GTCCTTCGCCGTAGATTG
**SEQ ID NO: 42:** AAGGTAATCAAGCAGAAATG
**SEQ ID NO: 43:** ATTGGTAACAGCACTTCC
**SEQ ID NO: 44:** TGACTGTAAGCAATCTACC
**SEQ ID NO: 45:** ACTAACGATGGAATCTTGG
**SEQ ID NO: 46:** AACTTAACTTCTATTCGTGAG
**SEQ ID NO: 47:** GAAACCTTGATAAGCAGTC
**SEQ ID NO: 48:** GATATTGATGTGTATTGC
**SEQ ID NO: 49:** TTATTATCTTCTGTCTCC
**SEQ ID NO: 50:** TGAATACCACATCTCCCATTGAC
**SEQ ID NO: 51:** TTTAACCTGCTGGCGTGAC
**SEQ ID NO: 52:** TTCTCACCGCTACTGTTAC
**SEQ ID NO: 53:** CACTTCTCGCACTAATTCG
**SEQ ID NO: 54:** CCCAAATGATGACCGAAG
**SEQ ID NO: 55:** CTAGTAACTGCTGTTCCTC
**SEQ ID NO: 56:** AAGTGTCCAAAGCCAAAC
**SEQ ID NO: 57:** TCAACAATGCCATCTTCC
**SEQ ID NO: 58:** TTAATTGCCACTGCCTTAC
**SEQ ID NO: 59:** TAGAGACAGCGGATATGC
**SEQ ID NO: 60:** TGATGTGGACGAGGATAC

**SEQ ID NO: 61:** GGGCTAAGGGAATATGGG
**SEQ ID NO: 62:** CTGACCCCGGGTGAATGTCAGCTACTGG
**SEQ ID NO: 63:** CAAACCCGGGCGATTTACTTTTCGACCTC
**SEQ ID NO: 64:** ACAGACCCGGGCAAGCGGATGGCTGATG
**SEQ ID NO: 65:** GCGGCCTCGAGTGGGGGCATCTGCTAGGCAATATTTG
**SEQ ID NO: 66:** GATTACACCCGGGTGTAATCGGTTGATTATTTCAGTGGCCCGGCCGATGGATAATTAC
**SEQ ID NO: 67:** GTCCAAATCAGCATTGCTCTGCCAGGTGAGAC
**SEQ ID NO: 68:** GATTACACCCGGGTGTAATCCTGAAGCCTTGACAATCCCCGTTGGTGATTTATACTTAG
**SEQ ID NO: 69:** GAACACTCGAGTGCCAGCAACGACAATG
**SEQ ID NO: 70:** GATTACACCCGGGTGTAATCCTTCATTCCCCTATTGTTATTAATAGTGCTC
**SEQ ID NO: 71:** GATGACCGCTGGCGGAGTTTAGTCCAG
**SEQ ID NO: 72:** GATTACACCCGGGTGTAATCTTGTCACCAATTTTTGTAGGGATGTTGGGCTAAATTG
**SEQ ID NO: 73:** GATAACTCGAGTCGCCCGGTGGATTTAATGC
**SEQ ID NO: 74:** GATTACACCCGGGTGTAATCCCCATACTCAGCCTTCTAATGCTGAGAGAATG
**SEQ ID NO: 75:** GGACTGACCCAAGATACAGTGGTAG
**SEQ ID NO: 76:** GATTACACCCGGGTGTAATCCGAGCGCAAACTACAATGCGCTTCGTTTGC
**SEQ ID NO: 77:** GGTTCTCGAGCCGCTGAATTTAGTCAACATCG
**SEQ ID NO: 78:** GATTACACCCGGGTGTAATCCATTGGGCACGAGAGTTAGTAAGGCAGTG
**SEQ ID NO: 79:** CTAAACTTACGGCATTGGCATCAACGGGAG
**SEQ ID NO: 80:** GATTACACCCGGGTGTAATCTCTTTACAATGGCCAGGTCTTTAGGGAGCGGTGAC
**SEQ ID NO: 81:** GAACTCGAGTAGTAACCACAGGCTTTTG
**SEQ ID NO: 82:** GATTACACCCGGGTGTAATCGCTGGAGGCGAACTGGGTGAGAACCAT
**SEQ ID NO: 83:** GTGAGCTTGATGGTGATGGTGGGTAAAG
**SEQ ID NO: 84:** GATTACACCCGGGTGTAATCTGCTGGCTTTGTTGCCCTGTCAACCAAAGTTC
**SEQ ID NO: 85:** CGAGGCGATCGCCCAGTTGGAAGAATTGGCC
**SEQ ID NO: 86:** CTAAAAAGACAAGTCTGTGGCTAGTTACTATGACGAGGC
**SEQ ID NO: 87:** TCCTGGTAACTCACGCTATC
**SEQ ID NO: 88:** AGCCGATCCAGGGAAGTGTTGTTG
**SEQ ID NO: 89:** CCATCGTCCTTCGCCGTAGATTGTG
**SEQ ID NO: 90:** CCCAGTTAAACTGCGAAAGG
**SEQ ID NO: 91:** TCGCCAAGCTTTCAGAAC
**SEQ ID NO: 92:** CAAACTCCAGCCGATAAC
**SEQ ID NO: 93:** CCGGTTGCCCTTATCGGAACCGATG
**SEQ ID NO: 94:** GCTATGGCGTCACTTGTAGC
**SEQ ID NO: 95:** TTTGCGACCCATCGGATTGC
**SEQ ID NO: 96:** CTCCAAGGCGACTACCTTTC
**SEQ ID NO: 97:** CCCAGTGGGAATGCGATCAG
**SEQ ID NO: 98:** TAGGAGGGCGATCACCGAAG
**SEQ ID NO: 99:** ACCCATTTCTTGGGTGTAGG
**SEQ ID NO: 100:** GGCCTTGGTTGCCCTGACTGATGTG
**SEQ ID NO: 101:** GACCGATCGCCGCAGTAGTTCTTGG
**SEQ ID NO: 102:** TCTTGTTGAATTAGATGGTG
**SEQ ID NO: 103:** TGTGAGTTATAGTTGTATTCC
**SEQ ID NO: 104:** ATTAAAGAGGAGAAA
**SEQ ID NO: 105:** TTGACAATTAATCATCCGGCTCGTATAATGTGTGGAATTGTGAGCGGATAACAATTTCACACA
**SEQ ID NO 106**

ATGGGGAAAGGCAAAGCCGATCCAGGGAAGTGTTGTTGCTGTCCAAAAAAGTCACCAATCAGCCTGGGCTTTTTGCCAATCCTCCGTGATCTTAGCG
TGATTATTAAAATGGCGATGGCCTTTATACAACTCTTAACCGAAAACAATCCGAGTGGGGAATTTGTTTTTGGTCTATGGAGTGGTGCTGGGCCAGG
GGAAACCCTGATTGAAGAATTTTTAAAAATCTTAGAAAAAATTCACAATCTACGGCGAAGGACGATGGAAAAAGCTTCGGAGTTCTGCTTTAGGTCA
AAGTTTCTTGAAAATTAA

**SEQ ID NO 107**

ATGATTTTTATGAAATACAAAGCTGAACAATTTATCAGCTCTTCTGAATTTAGTCCTAGTTTCGCCAAGCTTTCAGAACACGGCAAAAACTCTTTGT
CATGCAAAATAGTCAGAAATTACTATGTGATGGCTTCTACAAACTTAACTTCTATTCGTGAGTCTCTTAGGAGATGTCGCGATGAGAGTGGACAGCC
ACTTAGCTTAAGCCCTAGCGAATTAAAACCATCAAGTTATCGGCTGGAGTTTGAACTAGTAGCACTAATTGCTTCAAATTTGACACAGATGCCAGAT
TTAGAGAATTTTATCAAAGATGATAAAGAAACTACTAATGAAGATAAATTAATTTTGTTGACTGCTTATCAAGGTTTCAAAAATAAAGTTGATGGAT
TGTTTAAATTATTTGGTTGGCCATGCTTTGGATTTAAACAAGAAAAAGAAACTTAA

## SEQ ID NO 108

ATGAAAAAACCAGATATTGATGTGTATTGCAATGTAATGGAAGAAATCAAGCGACGAACAGGCGTTATCGATTTTTTTCTATCTGGTAATGGTCACG
CTTTATGTGAACCTACAACACTGGAATCAATTGCGCTGCAAATCAGGAAAATCCTAGAGCTAATAGCTATGGCGTCACTTGTAGCAAACGAAAAAGA
ATACAAAAAGGTATATTCCAATTTTGCCAGCGCATGGAATGCAGAATATTTACTAAAAGACTTAGGCAGAATTAATCCTGATTTTTACCCTAAACCA
GTCGTAGAGAAACTTTCTGATGACCCTAAAGTAAAAAATCGCCTAGCAGATCGTGACGAAGACTATCTAACCAAAAATGAATTTATAAAAGTTTACA
AGAAATGTGGCGCAATCATGCATGCGAGCAATCCGATGGGTCGCAAAATAGGCTATGAATACTACAAAAAGAACATTCCAGAATGGAGACAGAAGAT
AATAAACCTCCTAAACAACCACCAAATTAAGTTATTTAATCACTCTGGCTTTTATCTAATTCATATGAAAGAAGATCGTGATGATAAGGTACATTTT
TATGAATTCGACCTTGTGCAAACCACAAGCAGCTAA

## SEQ ID NO 109

ATGCGAATCAACCATAAGCCTCTATTAATTGCCACTGCCTTACTAACTCTCGTGCCCAATGTTGTTTGGTCTTCTCCCTTTGATGAAGATCAGGAGA
TTTGTCCCCAGTGGGAATGCGATCAGGCGGGAAATTGCCGCAAACTTGATCTTACCTGCGGTTCTTCCCCAGATTCTTCTGGTGCTCAGGGCTATCA
ATTATATTTTGATGGGGAATTAGTGTCTGGCCCCGACGCCCAGGGTTATACATCCCAACAAGCGAAAGAAAATTGTACTTGGAATATGCAAACTAAA
CCGCAAATGCATATCCGCTGTCTCTACAATGGCCAGGTCTTCGGTGATCGCCCTCCTAGTAACTCTCCTGGGTATGAACTATATTTTGATGGGGAAT
TAGTGTCTGGCGCCGACGCCCAAGGTTATACGTTCCAACAAGCGGAAGAAAATTGCATTTGGAATATGCAAACCAAACCGCAAATTCACATTCGCTG
TCTTTACAATGGCCAGGTCTTTAGGGAGCGGTGA

## SEQ ID NO 110

ATGGTTCTCACCCAGTTCGCCTCCAGCCCAGCTCCTCCCCAGTCCTTTGCTGTTGCCCCCAACCGTCAACAATTGCGACAAATGAATTGCTACCTGG
ACCAGCTTTTGTTGGCCTTGGTTGCCCTGACTGATGTGGACGAGGATACTTGGACGGCAATCCTAGAAAAAATTTCGTCATCTACGACAACAGGCCA
ATCTTTGACTAGGAAATACCCCATTCCTGCTGTTGCCCCCGGCCAGCTAGATGTGGAGGGAATTCGACTTTTGGCCATGGCGATCGCCTATTTAGGG
GCCCATTACCAAGAACTACTGCGGCGATCGGTCAGTTTAGTTGAACAGACCAAGGAACAGGGTAAAAATCCTTTACAAACCGTACTTTTAAGCGAGT
ACGCCCGAAAATTTACCTCAGCTTGGCAAGCCTATGGAGAGAAACAAGGGGGAGAAGCCCATATTCCCTTAGCCCCCATTACCTCTGATCGCCTGCT
TTCTTTGCTGACAGAATTATTTTTCTTTAGCAGTCAAGACGGCCCCCGCCGACTCTGGGGAGTATTGGCCACCATGGTGTCCCCCGCTTAG

## SEQ ID No 111

GTCCAAATCAGCATTGCTCTGCCAGGTGAGACGGAGTTTTTGTCCCACTAATCCCTGTTGGATAGAAGGCGCTTGATACAGTTCCAACCAAACCCAATCTCCTGT
CCTGGCTTTAGTTTCTTCCACCGTCGGCAGGATTAAACGACCCAACCACTCTCCCAAAGGCCGATAATACGCCTCATCTAAATTCTGTTGCAGAGGATAATAATCT
ACCTGGTTGGCGGGCAACTGACTGCTAATTTGATAATCCGGGATGCGGCCAGAGGTTTCCTGAGGTTGAAACAGCGTAGCTAGGGAAATCACCACCAGAGCGG
CGATCGCCACCAATACCATCCGCCAGCGCCATTGCTTCATCTGTGGACTGCCCTAAAAATTGCCATAAAAAAACACCTGGGGCGTCTTTCCTTGTTTCGACTCCAG
ATGTTTTTCTAATTTCCTCACACCTGTCCTAAGTATAAATCACCAACGGGGATTGTCAAGGCTTCAGGATTACACCCGGGTGTAATCGGTTGATTATTTCAGTGGC
CCGGCCGATGGATAATTACCATGGCCCGACCGACCGGGATTTCCAGACCAGTTTGAACAGTATTAAATTAGCCTTTCCGGACAAGGCCTCTGTGAAAATCTTAAA
AAAGTATTAAGATTTTAAAAGCTAGCTTATTTTCGGAGGAAATGTGTTTACTCGACTTGCCCAGCAACACCGCGATTTCGTGAAGGATTTAGTCATGAGCTTGAG
GGCTTTGGCCACTGTGCTCGAAAATCGGGGCTACATTGCTTCTTGCTACACCTGTGGCGACCAACTCAACAGCGCCTCCTTCATGGTGAGCTTGGGGGAAAATC
ATCTGATCCGCTTTTTGGTATCGGACTACGGCATCACCTGGACAGAAATGCGGGATGACCGAGAATTAATGAAATTAGAAGGAGCCGAGGCGATCGCCCAATT
GGAAGAATTGGCCAATGTGGTCAAATATTGCCTAGCAGATGCCCCCACTCGAGGCCGC

## SEQ ID No 112

GATGACCGCTGGCGGAGTTTAGTCCAGGGTTATTTGTACCGTCAAGGTTACATTGCCACCCCAGATTTAACTACGCTGCGAGTCCGTACCATTGGCGATCGGGC
CTATCTCACCATTAAGGGTAAAAATGCCAGCATTGCCCGCTTGGATTTGAGTATGAAATTCTTGCAGTGGAAGCCAATTAAATTTTGACAGAACTCTGTTCACCG
CCCCTGATTGAAAATATCGTTATTGCCTCGATTACCATGGTAAAACCTGAGAAGTAGACGAGTTTTTGGGGGATAACCAGGGTCTAATTTTAGCGGAAGTGGAA
TTAACCTACACTGGTGAAAAAATAAGTCTACTTCCCTGGATCGGGGAAGAGGTAACGGATGATGCCCGCTATTACAACGTCAATTTAGCCCAACATCCCTACAAA
AATTGGTGACAAGATTACACCCGGGTGTAATCCTTCATTCCCCTATTGTTATTAATAGTGCTCTGAAACTAACAAATAAGCAAGATCAAATTGTGAAGAAAACGC
AACAAAATGAACCATTAAGATAATTTCTGTCTATCCTGGCTGGTATATTCCAGTCTACTCAGAAATGAGTTTGTTTTATGACAAATCAGTATGTACCAAATTACTT
GGCGCAATCGATCCTGGTAACTCTATTTTGTTGCTTACCCCTGGGTATTGTGGCCATTATCAAAGCATCGGAAGTTAATTCTCGTTTAGCTTCAGGGGACTATGAA
GGCGCTGTAAAGGCTTCCAAGGAAGCGAAAAAGTTTTGTTGGTGGTCCTTTGGTGCCGGCATAATTTTCATTGCCATCTATTTTGTGCTAGTGGTTATTGCCGCC

GTCTTTGGTCAGTAATTAAAGTTACATTTTTTGACTTTGCCTTGTTCACCATTCATTAACGAATACCATGTTTAGTTTGAAAAATTATTCCCCATCTCCATTACTATC
CGCTAAGGCCAAGGAATATTTAGTATTCACTTTGGTAACCCTAACCATTGTCGTTGCTGGCACTCGAGTGTTC

## SEQ ID No 113

GGACTGACCCAAGATACAGTGGTAGCTTCAAGTTCGTCAATCAACCCCAATGGAAAGCTGAGATAATCTGTTGTTGATGCGACCGTCTTCATTTTAGCTAAAAAG
ACAAGTCTGTGACTAGTTACTATGACGAGGCCATCGGGTCTATCCCAGCGGTCTTGCTGATTCCAAAACTTGGCGATTTTCTGAAGTAGTATCTTTTCACCGCTTA
AGCCGCCAAGGTCTTGGATGAGCTTGGTCGAATCATCTTTTGATGCCTCGACATCGCGAATAACTTTGTAAACGACCTTCCCGAGCTTGAATGCAAAGTCGGTTA
GATTATCCATCAGAGTTGAATGCCGATAATACAGACTGCTATACTATTTTAATGAAAAGAGTGCTGGCAAGCAAGCCCGCCGAACGTTGAAGCTGTGCAGCGCA
AACGAAGCGCATTGTAGTTTGCGCTCGGATTACACCCGGGTGTAATCCCCATACTCAGCCTTCTAATGCTGAGAGAATGCCTGAAAAGAACCACTAAGTCAAATT
ATTTGGATTAAGTTTTATTAAGTCAACGGAGGTGTCAGATAGCATTTAGATCGTAAAAGCATACGGCATGAACAACCATGAACGGCTTCCCAGAGGTTAACCCC
AGGTTTATGGTTTACCAAAATCCCCTGACTTTTTCCGAGTTGGGGGACAAAATCAACTAATTTGGGCGACGATTTTTTCTGCTATCTGGCGATCGCCATGTTTGTC
TAACTTCTGATCCAACCTAGGTTTTTGGGGGCTTTCCAACAAAAAATCCCAGTTGCCGGCGAAAAATTGCTGGTGGGGCACAATTTGGTGCTCTCCGTAGTTCTG
CAAACCTGCCAGTAAAACTTGCGCTTCCGCAAAACCATCCCTGGTGAGGGAAATAATCGGGACTTCTAACTTTAACGCTTCAGCAAAGGTACCAAAACCAGGCT
TGGAAAATACCCGTCCGCAAAGGGGCATTAAATCCACCGGGCGACTCGAGTTATC

**SEQ ID No 114**

CTAAACTTACGGCATTGGCATCAACGGGAGCCACCGTCGGAGTAGGGGAAGTAACAACGGGGGAACTGGTATTGGTGGGCAAAATTTTTACTAGCCATTGATT
CCATTGGGGCAGATTGAACGCTCCCAACCACCAAAGTCCCCCTAATACAACGACAGAACTAAATAACAGGAAAAAAGGAATCCAAGGAGTTACCCCTCTTTTAT
GGGATGGAACTTCATCGACATTAAAGGTGGGAGGGGGAGGAGGCAATGGGGACAATGGTGGTCCAGAAAAGGAAGGTGGCTCCGACGTCAAGGCAACGGG
ACATCCACAGGATTCACAGAAACGAACCTGGGGGCTAAGGCGGTTGCCACAATTAGTACAAAAAACGGGAGTAGTCATAGGTGAAAACCCCGACTATAGAATT
AGAAAAATTTAACTTTTTATCCGAATTTTATTCGTCCATGTTCCCCAAATAACTATCAAAATAATTGGAAAAATTAAATTATTTGGTCGTTGGTCACCGCTCCCTAA
AGACCTGGCCATTGTAAAGAGATTACACCCGGGTGTAATCCATTGGGCACGAGAGTTAGTAAGGCAGTGGCAATTAATAGAGGCTTATGGTTGATTCGCATTGT
TTTGCTCCTGAAATTTTCGGCAAATACAAATACTTCGCTCTTCTAGCCCTATTAACCATTTTAACGACAAATTGATGGGGCAACGATTAACAAATAATGAATAAAT
TTTATGTTTTTCAAGATGAAAATTTGAAAATTTGATTTCCTTATATTTCTACTATAGAAGACTAATACAATTAGATCTAAAATTTGCAAGTATAAAAATCAGCAAAT
AGTTATATTGTTAATAATTCAATGACCCAATAACTCGTACTGTTATCTACGTGGTGAAAGCCAAAAAGACGAACAGTTTAGCCTCCTCCTCCTCGGCGATCGCCAA
GCGAAATGTCATGGGAGATGTTCAGATTGAGCATTTTTTTCTAAAAGCCCTTGCTAAAACAAACCACATGTGCAGGGTGTCCCCGATGTTGACTAAATTCAGCGG
CTCGAGAACC

**SEQ ID No 115**

GTGAGCTTGATGGTGATGGTGGGTAAAGCTTCATTGTCCATGCGCCGGAGCTTGACGGTAAAATCCGTGTCCTGGGGCCAATCATTGGGAACGCTCA
CACAGATACCGTTGCGAGTTAAGTCGAAGGATACCCCCACCAATTCCCCTTCCATATCGAACACTTGGCAAAAGGCCAACATCCGCTCTTCCTGCCG
TCGCTCCTGCACCCCCTGGAGATGGGCAATGGTTAGGGCTTCTTGGGAGAGGGAAGATGGAGAATCTTGAGCCATGGAGATTATTTCCTCGGTTAAA
TTAGGTTTACCTAGTAAATGGGCCAGGTTGACCATTATTGTAGTCATTCACTGGGTCGATCTTCCTTCCATCAGGGTCATTTGGTTAATTGTTGATG
AGAAATGGGAAGGAGTAATCCATAGATATTTGCCCAGTTAACTCGATTTGAGCAGAATTGGGAAGGACGTATTGGGAACTTTGGTTGACAGGGCAAC
AAAGCCAGCAGATTACACCCGGGTGTAATCGCTGGAGGCGAACTGGGTGAGAACCATAAAATCTTGGGGAACAGGGGAATGTTAATGAACACATGAC
ATGCATAGGATAGCGAATTGATTTAAAGCAATGATTCCCCCTGAGATAAATATATTTTGACCCACTGCTCCGCTTAATTTTGGCGCTAATCTGGGCA
AAAATTCGTTGGTTTTCGGAAAGGTGAACCCGTAGCTAGCTTGTATTGCCCAAACCTAACTCATCATTGCTCCATGGCGGCCAAAAATCTTTAGGAC
AAAACTCGCCAGAGGTCAAGGCTTGATTTTTTCAGAGGGAAAAATTCCTCGGCTAAATAATCAAAAAGTTGAAAAAAAAAGTTTTTCAGCTAAGGTT
TGATGTTATATTTTATCTTATGTAGACTTTTGAAAAAAACAAGTCCCCGCAATCAAGCATCATTCAACGGAAAAAATAATCCTATGCCAAACGCCTC
CACCGCACTAACCGGCAAAGCATTACTCAATAAAGTTAAAGAACTATCCCATCTGCCCCGTC

## Claims

1. A genetic construct for the introduction of ectopic DNA by double homologous recombination in *Synechocystis* PCC6803, comprising two sequences
wherein the first sequence is selected from an upstream and the second sequence is selected from a downstream chromosomal regions of a locus in *Synechocystis* sp. PCC6803 wherein the locus is SEQ ID No 107,
and the sequences length vary between 400-1500 bp, preferably 430-800 bp, more preferably 430-560 bp and wherein the genetic construct comprises the sequence SEQ ID No 112.

2. The genetic construct according to the preceding claims further comprising the SEQ ID NO: 26.

3. A vector for the introduction of a genetic construct for the production of a target molecule in a *Synechocystis* sp. PCC6803host cell comprising the genetic construct described in any one of previous claims.

4. The vector according to the preceding Claim 3 further comprising a gene selected from the following list: a gene coding for antibiotic resistance, a gene coding for levansucrase, a gene coding for green fluorescence protein, in particular a gene coding for antibiotic kanamycin resistance.

5. The vector according to the preceding claims 3-4 further comprising a constitutive promotor, preferably SEQ ID No 20.

6. The vector according to the preceding claims 3-5 wherein the vector is an integrative vector.

7. The vector according to the preceding claims 3-6 further comprising a gene or genes for coding in a host cell: an enzyme or enzymes for production of a target molecule, or a precursor of a target molecule or a target molecule.

8. The vector according to the preceding claims 3-7 wherein the genetic construct leads to the production of at least a precursor, an enzyme or the target molecule of the following list: a compatible solute, an organic compound, a polymer, an alcohol, a biofuel, an additive, an adhesive, a sealant, a pigment, an antibiotic, a medicament, a protein.

9. The vector according to the preceding claims 3-8 wherein the genetic construct encodes enzymes for the production of a compatible solute selected from the following list: ectoine, hydroxyectoine, or mixtures thereof.

10. The vector according to the preceding claims 3-9 wherein the genetic construct encodes enzymes for the production of the organic compound isoprene, glucoglycerol, betaine or mixtures thereof.

11. The vector according to the preceding claims 3-10 wherein the genetic construct encodes proteins/enzymes for the production of the pigment phycocyanin.

12. A cyanobacterium host cell comprising the construct according to any of the claims 1-2 or comprising the vector according to any of the claims 3-11, wherein the cyanobacterium is *Synechocystis,* in particular *Synechocystis* sp. PCC6803.

13. A use of a genetic construct as described in any of the claims 1-2, as an enhancer of the production of an enzyme for production of the target molecule, or a precursor of a target molecule or a target molecule in a cyanobacterium host cell.

14. A method for the expression of an enzyme for production of the target molecule, or a precursor of a target molecule or a target molecule with the following steps:

constructing a genetic construct as described in claim 1 - 2;
cloning the genetic construct in an appropriate vector;
cloning the genetic construct for the expression of the precursor, the enzyme or the target molecule, in the vector and obtaining a recombinant vector;
transforming *Synechocystis* host cell with the recombinant vector;
expressing the gene or genes for the production of a precursor, of the enzyme or of the target molecule.

**Patentansprüche**

1. Ein genetisches Konstrukt zur Einführung von ektopischer DNA durch doppelt homologe Rekombination bei *Synechocystis* PCC6803, umfassend zwei Sequenzen,
wobei die erste Sequenz aus einem stromaufwärts gerichteten und die zweite Sequenz aus einem stromabwärts gerichteten chromosomalen Bereich eines Locus bei *Synechocystis* sp. PCC6803 ausgewählt ist,
wobei der Locus SEQ ID Nr. 107 ist
und die Sequenzlänge zwischen 400-1500 bp, bevorzugt zwischen 430-800 bp, besonders bevorzugt zwischen 430-560 bp variiert und
wobei das genetische Konstrukt die Sequenz SEQ ID Nr. 112 umfasst.

2. Das genetische Konstrukt nach den vorangehenden Ansprüchen, das ferner die SEQ ID Nr. 26 umfasst.

3. Ein Vektor für die Einführung eines genetischen Konstrukts für die Herstellung eines Zielmoleküls in einer *Synechocystis* sp. PCC6803-Wirtszelle, umfassend das in einem der vorherigen Ansprüche beschriebene genetische Konstrukt.

4. Der Vektor nach dem vorangehenden Anspruch 3, der ferner ein aus folgender Liste ausgewähltes Gen umfasst: ein Gen, das für Antibiotikaresistenz kodiert, ein Gen, das für Levansucrase kodiert, ein Gen, das für grünes Fluoreszenzprotein kodiert, insbesondere ein Gen, das für Kanamycin-Antibiotikaresistenz kodiert ist.

5. Der Vektor nach den vorangehenden Ansprüchen 3-4 ferner umfassend einen konstitutiven Promotor, bevorzugt SEQ ID Nr. 20.

6. Der Vektor nach den vorangehenden Ansprüchen 3-5, wobei der Vektor ein integrativer Vektor ist.

7. Der Vektor nach den vorangehenden Ansprüchen 3-6, ferner umfassend ein oder mehrere Gene zur Codierung in einer Wirtszelle: ein oder mehrere Enzyme zur Herstellung eines Zielmoleküls oder einen Vorläufer eines Zielmoleküls oder ein Zielmolekül.

8. Der Vektor nach den vorangehenden Ansprüchen 3-7, wobei das genetische Konstrukt zur Herstellung von mindestens einem Vorläufer, einem Enzym oder dem Zielmolekül der folgenden Liste führt: einem kompatiblen gelösten Stoff, einer organischen Verbindung, einem Polymer, einem Alkohol, einem Biokraftstoff, einem Additiv, einem Klebstoff, einem Dichtungsmittel, einem Pigment, einem Antibiotikum, einem Medikament, einem Protein.

9. Der Vektor nach den vorangehenden Ansprüchen 3-8, wobei das genetische Konstrukt Enzyme für die Herstellung eines kompatiblen gelösten Stoffes kodiert, der aus folgender Liste ausgewählt wird: Ectoin, Hydroxyectoin oder Mischungen davon.

10. Der Vektor nach den vorangehenden Ansprüchen 3-9, wobei das genetische Konstrukt Enzyme für die Herstellung der organischen Verbindung Isopren, Glucoglycerin, Betain oder Mischungen davon kodiert.

11. Der Vektor nach den vorangehenden Ansprüchen 3-10, wobei das genetische Konstrukt Proteine/Enzyme für die Herstellung des Pigments Phycocyanin kodiert.

12. Eine Cyanobacterium-Wirtszelle, umfassend das Konstrukt nach einem der Ansprüche 1-2 oder umfassend den Vektor nach einem der Ansprüche 3-11, wobei das Cyanobacterium *Synechocystis,* insbesondere *Synechocystis* sp. PCC6803 ist.

13. Eine Verwendung eines genetischen Konstrukts, wie in einem der Ansprüche 1-2 beschrieben, als ein Enhancer der Produktion eines Enzyms zur Herstellung des Zielmoleküls oder eines Vorläufers eines Zielmoleküls oder eines Zielmoleküls in einer Cyanobakterien-Wirtszelle.

14. Ein Verfahren zur Expression eines Enzyms zur Herstellung des Zielmoleküls oder eines Vorläufers eines Zielmoleküls oder eines Zielmoleküls mit folgenden Schritten:

Konstruieren eines genetischen Konstrukts nach Anspruch 1 - 2;
Klonen des genetischen Konstrukts in einem geeigneten Vektor;
Klonen des genetischen Konstrukts für die Expression des Vorläufers, des Enzyms oder des Zielmoleküls in dem Vektor und Erhalten eines rekombinanten Vektors;
Transformieren der *Synechocystis*-Wirtszelle mit dem rekombinanten Vektor;
Exprimieren des Gens oder der Gene für die Herstellung eines Vorläufers, des Enzyms oder des Zielmoleküls.

**Revendications**

1. Une construction génétique pour l'introduction d'ADN ectopique par recombinaison homologue double de *Synechocystis* PCC6803, comprenant deux séquences
dans laquelle la première séquence est sélectionnée à partir d'une région chromosomique en amont et la seconde séquence est sélectionnee à partir d'une région chromosomique en aval d'un locus de *Synechocystis* sp. PCC6803 dans laquelle le locus est SEQ ID № 107,
et la longueur des séquences varie entre 400-1500 bp, de préférence entre 430-800 bp, plus préférablement entre 430-560 bp et
dans laquelle la construction génétique comprend la séquence SEQ ID № 112.

2. La construction génétique selon les revendications précédentes comprenant également la SEQ ID NO :26.

3. Un vecteur pour l'introduction d'une construction génétique pour la production d'une molécule cible dans une cellule hôte *Synechocystis* sp. PCC6803 comprenant la construction génétique décrite dans l'une quelconque des revendications précédentes.

4. Le vecteur selon la revendication 3 précédente comprenant également un gène sélectionné à partir de la liste suivante : un gène codant pour résistance antibiotique, un gène codant pour lévane-sucrase, un gène codant pour protéine fluorescente verte, en particulier un gène codant pour résistance à la kanamycine antibiotique.

5. Le vecteur selon les revendications 3-4 précédentes comprenant également un promoteur constitutif, de préférence SEQ ID № 20.

6. Le vecteur selon les revendications 3-5 précédentes dans lequel le vecteur est un vecteur intégratif.

7. Le vecteur selon les revendications 3-6 précédentes comprenant également un gène ou des gènes pour la codification dans une cellule hôte : une enzyme ou des enzymes pour la production d'une molécule cible, ou un précurseur d'une molécule cible ou une molécule cible.

8. Le vecteur selon les revendications 3-7 précédentes dans lequel la construction génétique mène à la production d'au moins un précurseur, une enzyme ou la molécule cible de la liste suivante : un soluté compatible, un composé organique, un polymère, un alcool, un biocarburant, un additif, un adhésif, un scellant, un pigment, un antibiotique, un médicament, une protéine.

9. Le vecteur selon les revendications 3-8 précédentes dans lequel la construction génétique encode des enzymes pour la production d'un soluté compatible sélectionné à partir de la liste suivante : ectoïne, hydroxyectoïne, ou un mélange de ceux-ci.

10. Le vecteur selon les revendications 3-9 précédentes dans lequel la construction génétique encode des enzymes pour la production du composé organique isoprène, gluco-glycérol, bétaïne ou un mélange de ceux-ci.

11. Le vecteur selon les revendications 3-10 précédentes dans lequel la construction génétique encode des protéines/enzymes pour la production du pigment phycocyanine.

12. Une cellule hôte de type cyanobactérie comprenant la construction selon l'une quelconque des revendications 1-2 ou comprenant le vecteur selon l'une quelconque des revendications 3-11, dans laquelle la cyanobactérie est *Synechocystis,* en particulier *Synechocystis* sp. PCC6803.

13. Une utilisation d'une construction génétique telle que décrite dans l'une quelconque des revendications 1-2 en tant qu'optimisateur de la production d'une enzyme pour la production d'une molécule cible, ou un précurseur d'une molécule cible ou une molécule cible dans une cellule hôte de type cyanobactérie.

14. Une méthode pour l'expression d'une enzyme pour la production de la molécule cible, ou un précurseur d'une molécule cible ou une molécule cible avec les étapes suivantes :

    construire une construction génétique telle que décrite dans la revendication 1-2 ;
    cloner la construction génétique dans un vecteur approprié ;
    cloner la construction génétique pour l'expression du précurseur, de l'enzyme ou de la molécule cible, dans le vecteur et obtenir un vecteur recombinant ;
    transformer la cellule hôte *Synechocystis* avec le vecteur recombinant ;
    exprimer le gène ou les gènes pour la production d'un précurseur, de l'enzyme ou de la molécule cible.

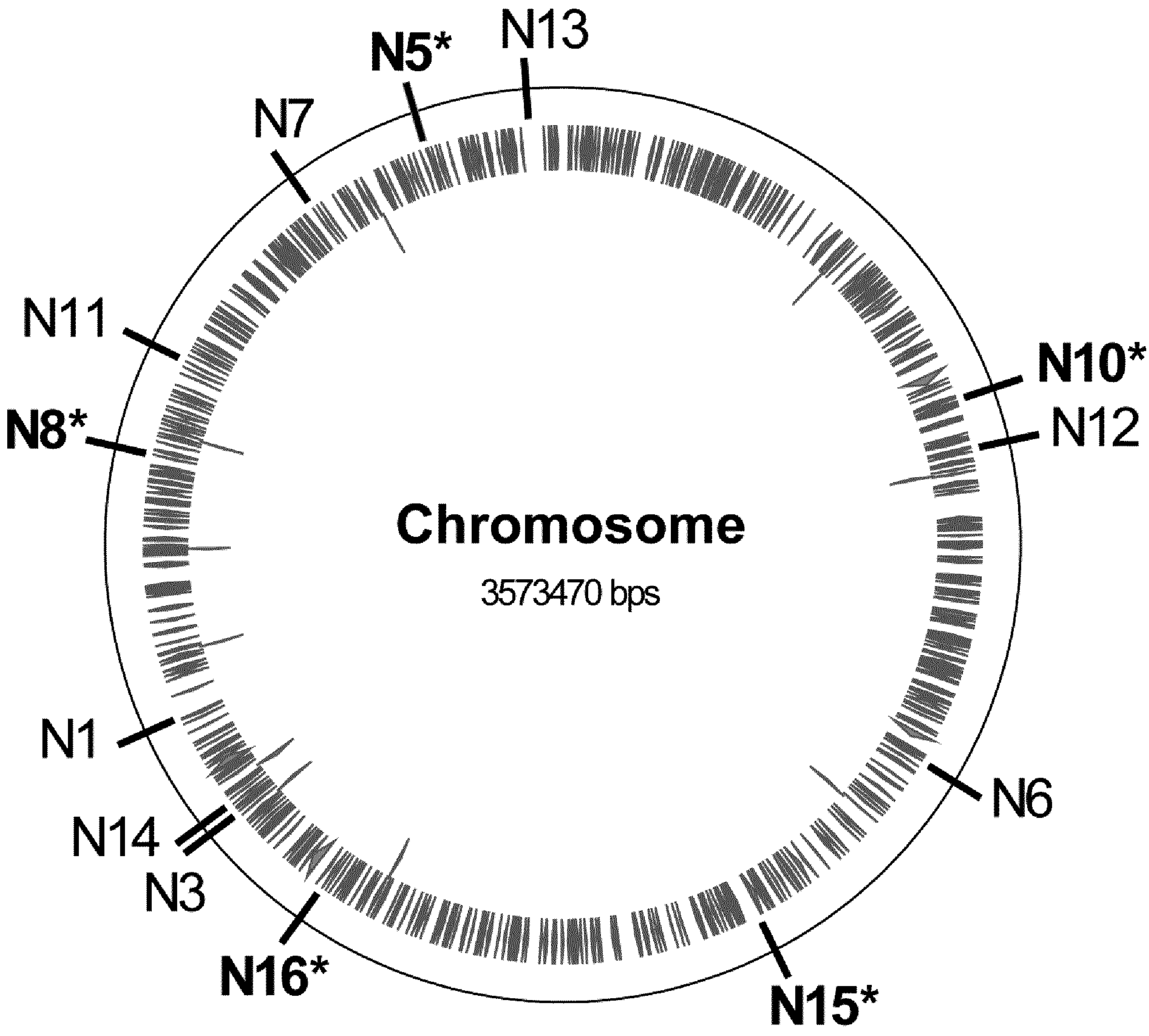
N13
N5*
N7
N11
N10*
N8*
N12
Chromosome
3573470 bps
N1
N6
N14
N3
N16*
N15*

Fig. 1A

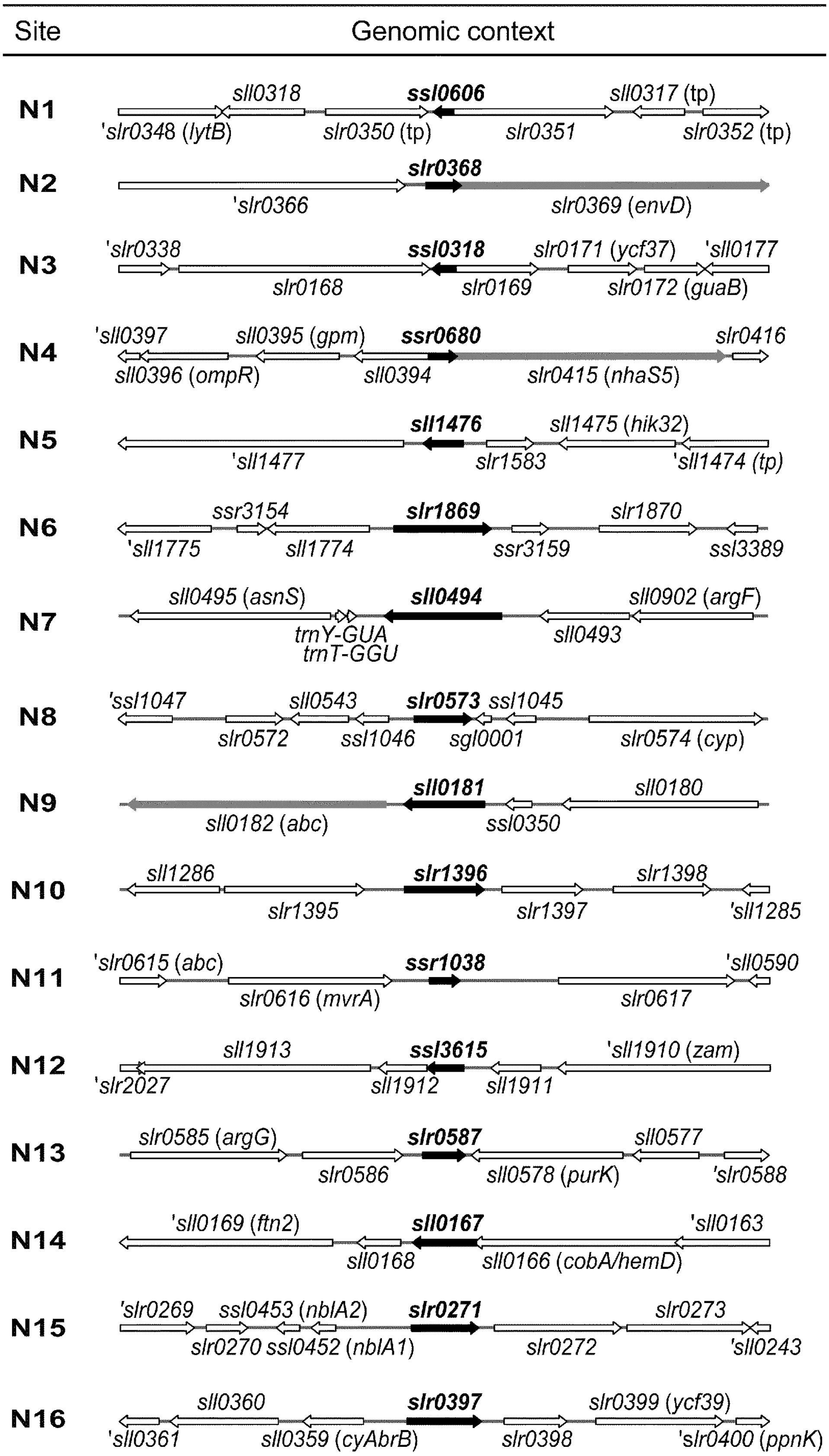
Site
Genomic context
N1
sll0318
ssl0606
sll0317 (tp)
'slr0348 (lytB)
slr0350 (tp)
slr0351
slr0352 (tp)
N2
slr0368
'slr0366
slr0369 (envD)
N3
'slr0338
ssl0318
slr0171 (ycf37)
'sll0177
slr0168
slr0169
slr0172 (guaB)
N4
'sll0397
sll0395 (gpm)
ssr0680
slr0416
sll0396 (ompR)
sll0394
slr0415 (nhaS5)
N5
sll1476
sll1475 (hik32)
'sll1477
slr1583
'sll1474 (tp)
N6
ssr3154
slr1869
slr1870
'sll1775
sll1774
ssr3159
ssl3389
N7
sll0495 (asnS)
sll0494
sll0902 (argF)
trnY-GUA
trnT-GGU
sll0493
N8
'ssl1047
sll0543
slr0573
ssl1045
slr0572
ssl1046
sgl0001
slr0574 (cyp)
N9
sll0181
sll0180
sll0182 (abc)
ssl0350
N10
sll1286
slr1396
slr1398
slr1395
slr1397
'sll1285
N11
'slr0615 (abc)
ssr1038
'sll0590
slr0616 (mvrA)
slr0617
N12
sll1913
ssl3615
'sll1910 (zam)
'slr2027
sll1912
sll1911
N13
slr0585 (argG)
slr0587
sll0577
slr0586
sll0578 (purK)
'slr0588
N14
'sll0169 (ftn2)
sll0167
'sll0163
sll0168
sll0166 (cobA/hemD)
N15
'slr0269
ssl0453 (nblA2)
slr0271
slr0273
slr0270 ssl0452 (nblA1)
slr0272
'sll0243
N16
sll0360
slr0397
slr0399 (ycf39)
'sll0361
sll0359 (cyAbrB)
slr0398
'slr0400 (ppnK)

Fig. 1B

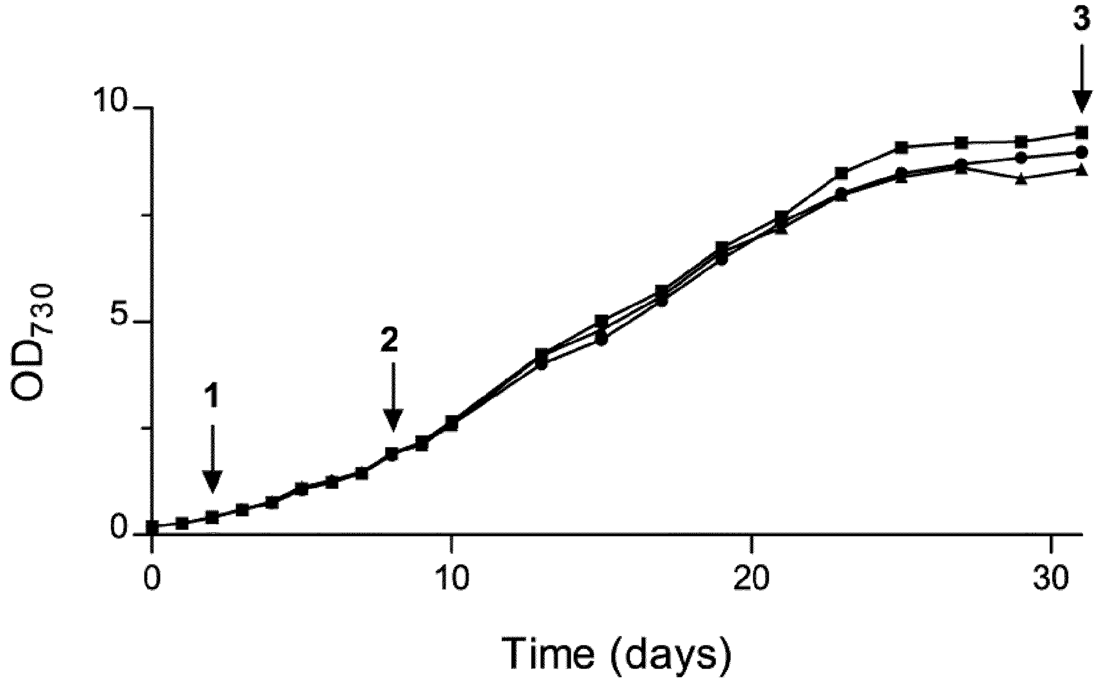

3
10
2
5
1
0
0
10
20
30
OD730
Time (days)


Fig. 2A

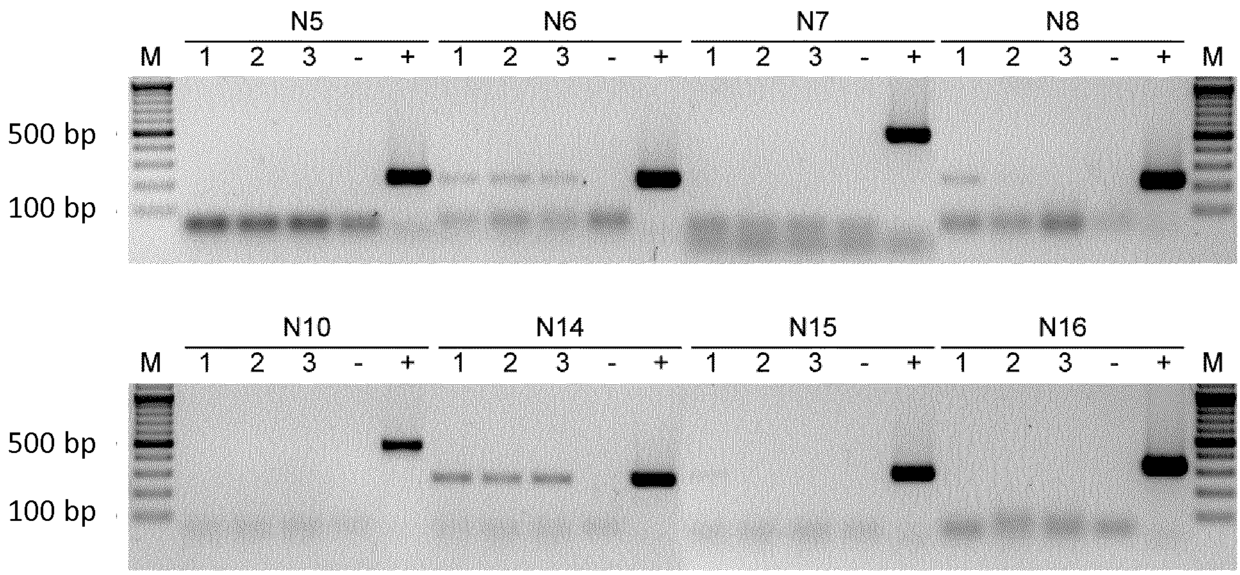

N5
N6
N7
N8
M 1 2 3 - + 1 2 3 - + 1 2 3 - + 1 2 3 - + M
500 bp
100 bp
N10
N14
N15
N16
M 1 2 3 - + 1 2 3 - + 1 2 3 - + 1 2 3 - + M
500 bp
100 bp


Fig. 2B

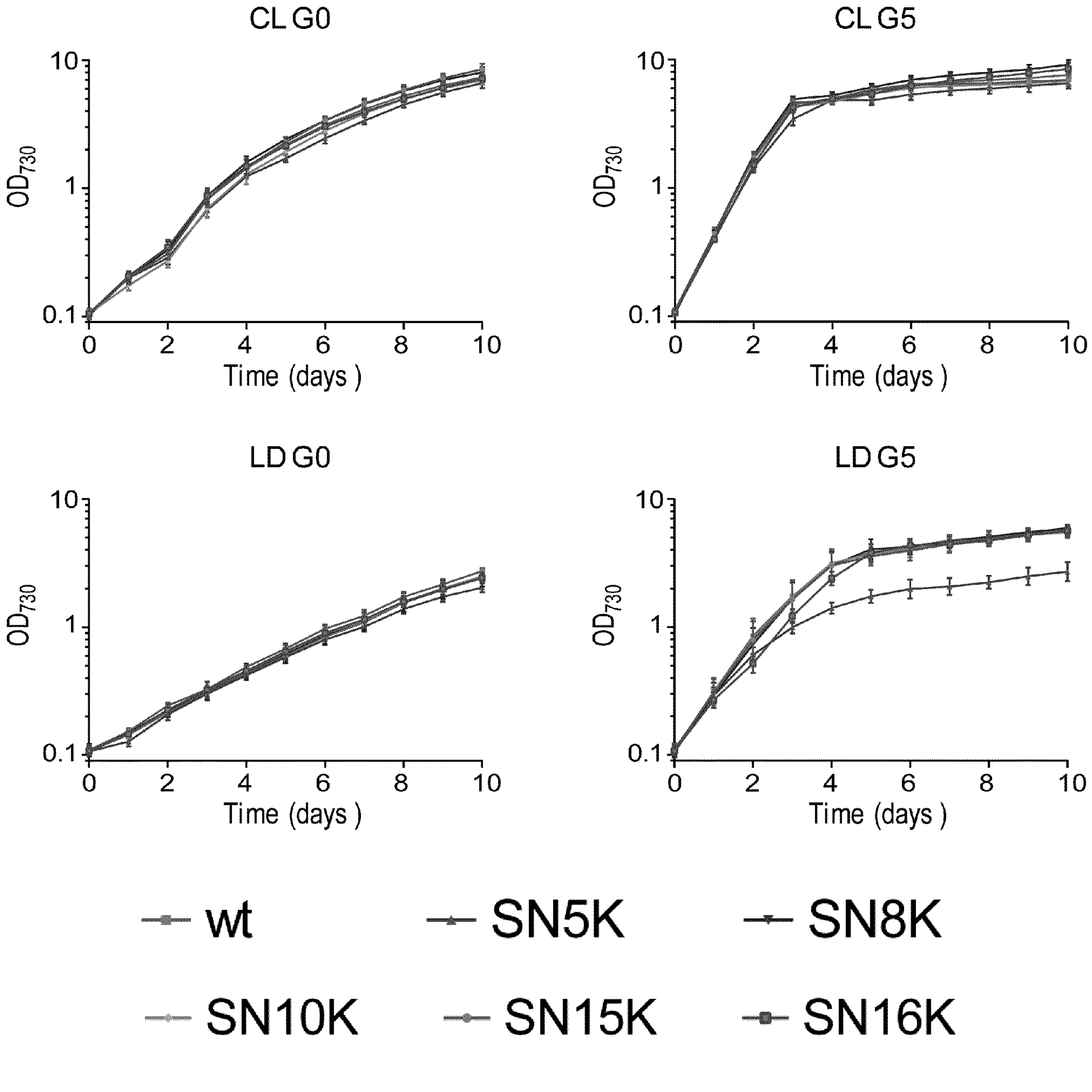
CL G0
CL G5
LD G0
LD G5
$OD_{730}$
10
1
0.1
0
2
4
6
8
10
Time (days )
wt
SN5K
SN8K
SN10K
SN15K
SN16K

Fig. 3

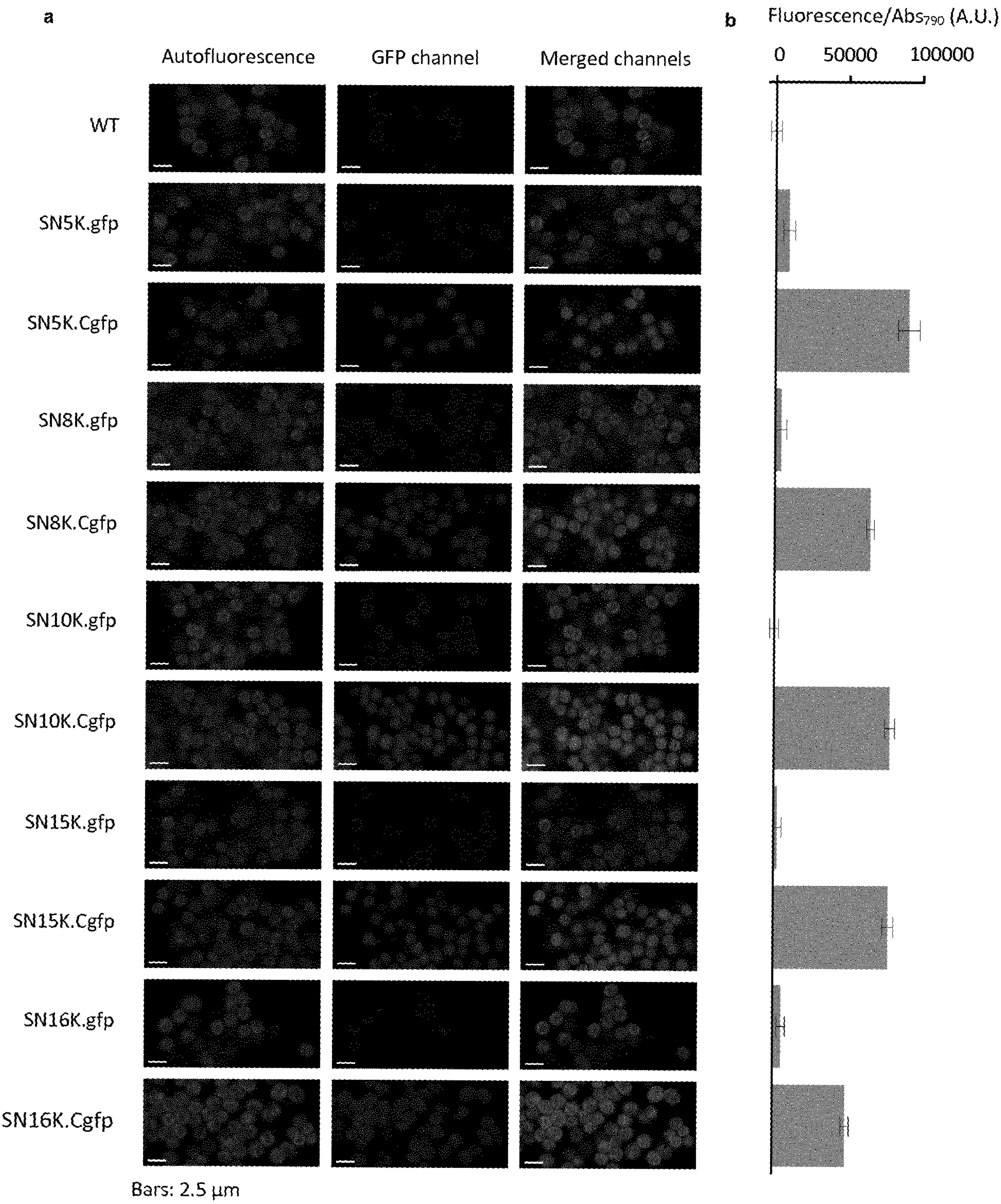
a
Autofluorescence
GFP channel
Merged channels
WT
SN5K.gfp
SN5K.Cgfp
SN8K.gfp
SN8K.Cgfp
SN10K.gfp
SN10K.Cgfp
SN15K.gfp
SN15K.Cgfp
SN16K.gfp
SN16K.Cgfp
Bars: 2.5 µm
b
Fluorescence/Abs790 (A.U.)
0
50000
100000

Fig. 4

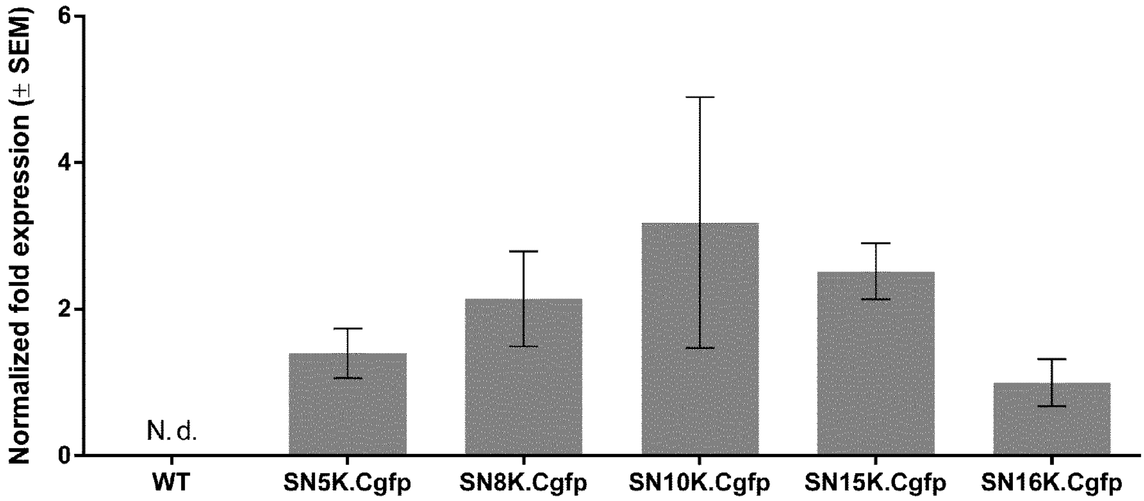
Normalized fold expression (± SEM)
6
4
2
0
N. d.
WT
SN5K.Cgfp
SN8K.Cgfp
SN10K.Cgfp
SN15K.Cgfp
SN16K.Cgfp

Fig. 5

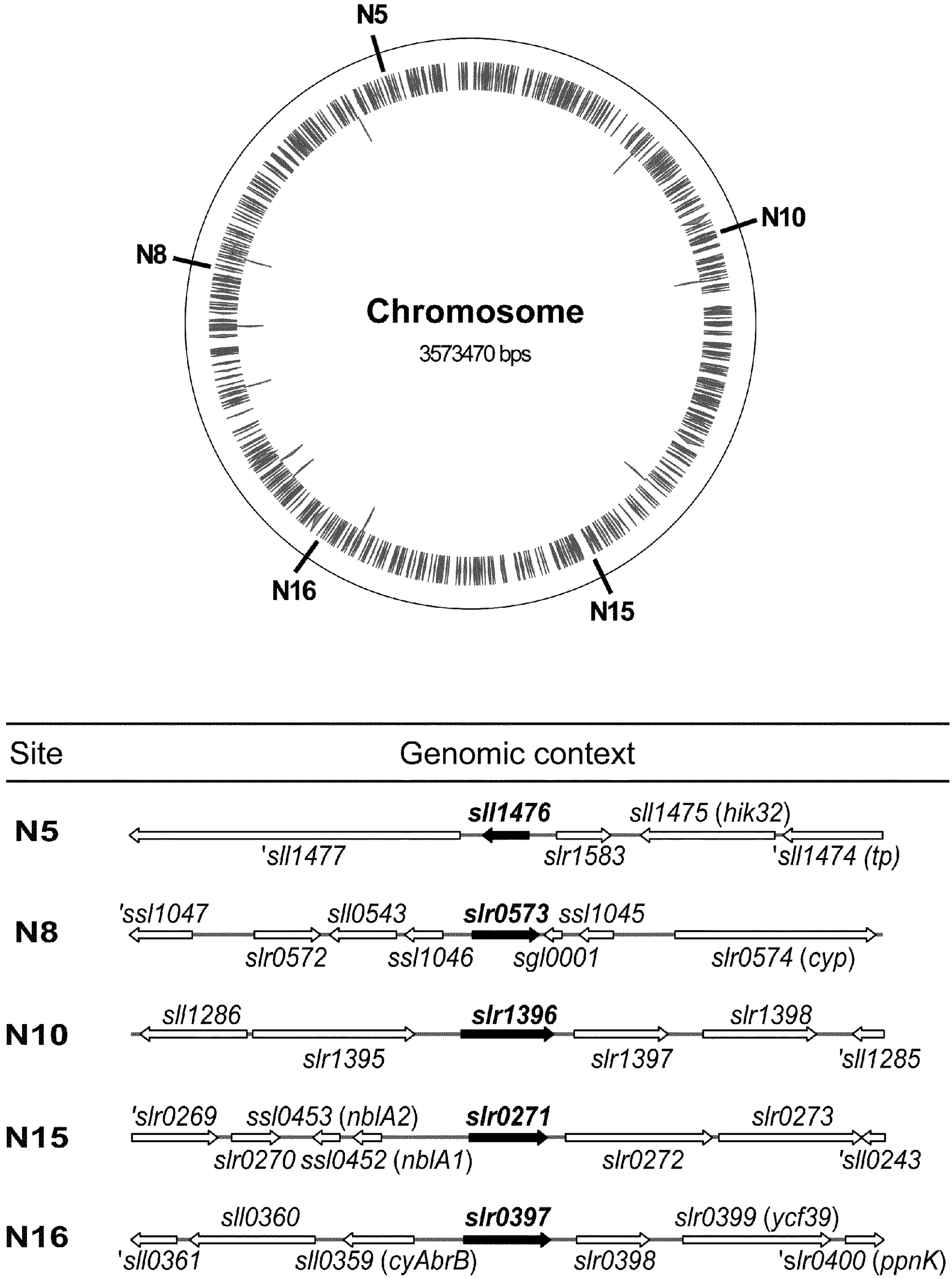
N5
N10
N8
Chromosome
3573470 bps
N16
N15
Site
Genomic context
N5
sll1476
sll1475 (hik32)
'sll1477
slr1583
'sll1474 (tp)
N8
'ssl1047
sll0543
slr0573
ssl1045
slr0572
ssl1046
sgl0001
slr0574 (cyp)
N10
sll1286
slr1396
slr1398
slr1395
slr1397
'sll1285
N15
'slr0269
ssl0453 (nblA2)
slr0271
slr0273
slr0270
ssl0452 (nblA1)
slr0272
'sll0243
N16
sll0360
slr0397
slr0399 (ycf39)
'sll0361
sll0359 (cyAbrB)
slr0398
'slr0400 (ppnK)

Fig. 6

| Site name | ORF ID | Chromosome position | Orientation* | Length of the putatively encoded protein (amino acids) |
|---|---|---|---|---|
| N1 | *ssl0606* | 2441925 - 2442083 | c | 52 |
| N2 | *slr0368* | 2365848 - 2366120 | d | 90 |
| N3 | *ssl0318* | 2302457 - 2302645 | c | 62 |
| N4 | *ssr0680* | 2684766 - 2684990 | d | 74 |
| N5 | *sll1476* | 3398409 - 3398717 | c | 102 |
| N6 | *slr1869* | 1212609 - 1213358 | d | 249 |
| N7 | *sll0494* | 3224429 - 3225334 | c | 301 |
| N8 | *slr0573* | 2816517 - 2816960 | d | 147 |
| N9 | *sll0181* | 2737929 - 2738552 | c | 207 |
| N10 | *slr1396* | 707437 - 708054 | d | 205 |
| N11 | *ssr1038* | 2950107 - 2950343 | d | 78 |
| N12 | *ssl3615* | 776058 - 776342 | c | 94 |
| N13 | *slr0587* | 3536090 - 3536422 | d | 110 |
| N14 | *sll0167* | 2317536 - 2318030 | c | 164 |
| N15 | *slr0271* | 1524568 - 1525086 | d | 172 |
| N16 | *slr0397* | 2146801 - 2147376 | d | 191 |

**Fig. 7**

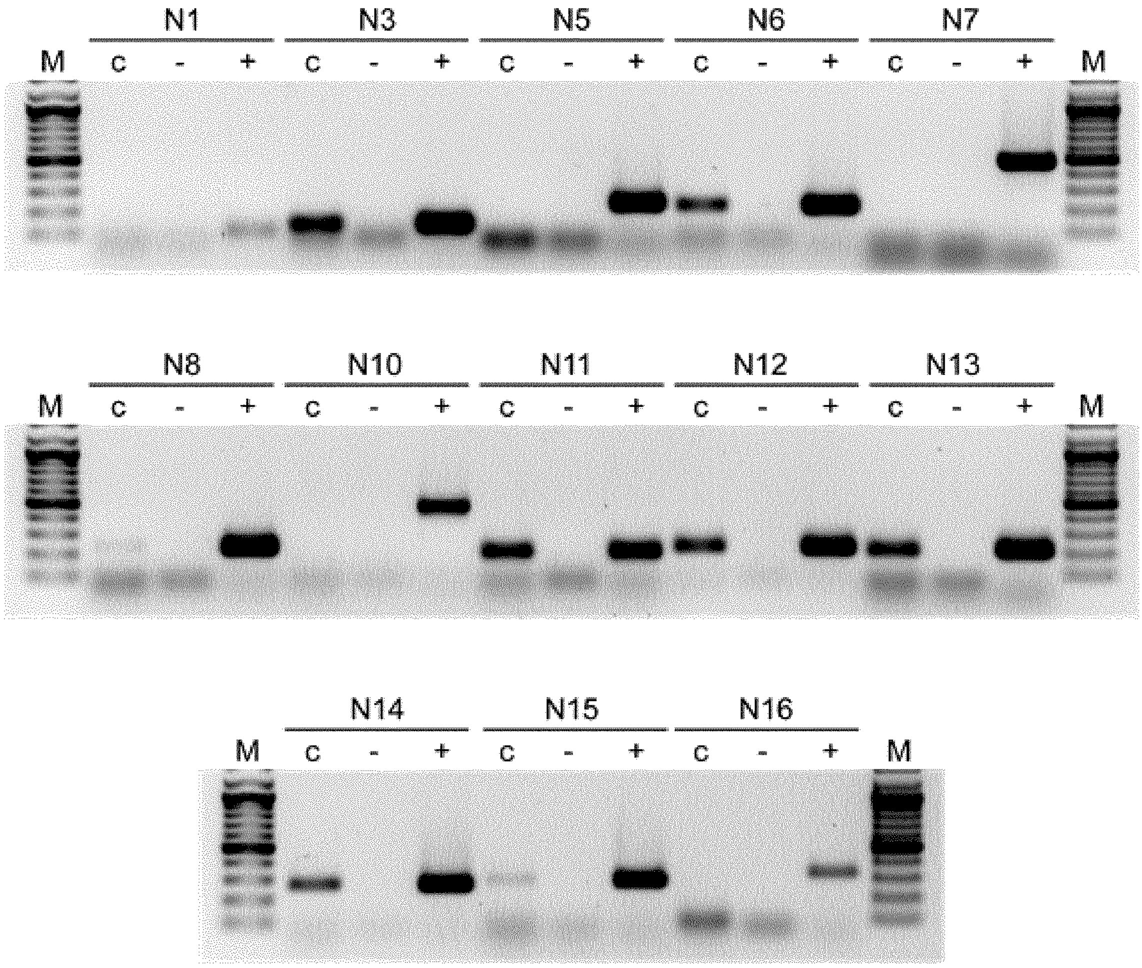
N1
N3
N5
N6
N7
M c - + c - + c - + c - + c - + M
N8
N10
N11
N12
N13
M c - + c - + c - + c - + c - + M
N14
N15
N16
M c - + c - + c - + M

Fig. 8

| Sequence ID Number | Primer name | Sequence 5' → 3' | Amplicon size (bp) |
|---|---|---|---|
| SEQ ID NO: 36: | N1F | CATGGATTGAAGGAAGGG | 100 |
| SEQ ID NO: 37: | N1R | TTTGGTGAACAATTAATGAAC | |
| SEQ ID NO: 38: | N3F | GCAGTCCCTCCTCCAAAC | 129 |
| SEQ ID NO: 39: | N3R | AAGCCGATATTCAGGTAAGATG | |
| SEQ ID NO: 40: | N5F | GGAAGTGTTGTTGCTGTC | 232 |
| SEQ ID NO: 41: | N5R | GTCCTTCGCCGTAGATTG | |
| SEQ ID NO: 42: | N6F | AAGGTAATCAAGCAGAAATG | 217 |
| SEQ ID NO: 43: | N6R | ATTGGTAACAGCACTTCC | |
| SEQ ID NO: 44: | N7F | TGACTGTAAGCAATCTACC | 500 |
| SEQ ID NO: 45: | N7R | ACTAACGATGGAATCTTGG | |
| SEQ ID NO: 46: | N8F | AACTTAACTTCTATTCGTGAG | 231 |
| SEQ ID NO: 47: | N8R | GAAACCTTGATAAGCAGTC | |
| SEQ ID NO: 48: | N10F | GATATTGATGTGTATTGC | 478 |
| SEQ ID NO: 49: | N10R | TTATTATCTTCTGTCTCC | |
| SEQ ID NO: 50: | N11F | TGAATACCACATCTCCCATTGAC | 200 |
| SEQ ID NO: 51: | N11R | TTTAACCTGCTGGCGTGAC | |
| SEQ ID NO: 52: | N12F | TTCTCACCGCTACTGTTAC | 227 |
| SEQ ID NO: 53: | N12R | CACTTCTCGCACTAATTCG | |
| SEQ ID NO: 54: | N13F | CCCAAATGATGACCGAAG | 216 |
| SEQ ID NO: 55: | N13R | CTAGTAACTGCTGTTCCTC | |
| SEQ ID NO: 56: | N14F | AAGTGTCCAAAGCCAAAC | 270 |
| SEQ ID NO: 57: | N14R | TCAACAATGCCATCTTCC | |
| SEQ ID NO: 58: | N15F | TTAATTGCCACTGCCTTAC | 293 |
| SEQ ID NO: 59: | N15R | TAGAGACAGCGGATATGC | |
| SEQ ID NO: 60: | N16F | TGATGTGGACGAGGATAC | 335 |
| SEQ ID NO: 61: | N16R | GGGCTAAGGGAATATGGG | |

**Fig. 9**

| Sequence ID Number | Primer name | Sequence 5' → 3'* | Purpose |
|---|---|---|---|
| SEQ ID NO: 62: | Km.KmScFwd | CTGACCCCGGGTGAATGTCAGCTACTGG | Selection cassettes amplification |
| SEQ ID NO: 63: | KmRev | CAAACCCGGGCGATTTACTTTTCGACCTC | |
| SEQ ID NO: 64: | KmScRev | ACAGACCCGGGCAAGCGGATGGCTGATG | |
| SEQ ID NO: 65: | N5.5O** | GCGGCCTCGAGTGGGGGCATCTGCTAGGCAATATTTG | Amplification of the DNA fragments flanking the neutral sites |
| SEQ ID NO: 66: | N5.5I | GATTACACCCGGGTGTAATCGGTTGATTATTTCAGTGGCCCGGCCGATGGATAATTAC | |
| SEQ ID NO: 67: | N5.3O** | GTCCAAATCAGCATTGCTCTGCCAGGTGAGAC | |
| SEQ ID NO: 68: | N5.3I | GATTACACCCGGGTGTAATCCTGAAGCCTTGACAATCCCCGTTGGTGATTTATACTTAG | |
| SEQ ID NO: 69: | N8.5O** | GAACACTCGAGTGCCAGCAACGACAATG | |
| SEQ ID NO: 70: | N8.5I | GATTACACCCGGGTGTAATCCTTCATTCCCCTATTGTTATTAATAGTGCTC | |
| SEQ ID NO: 71: | N8.3O** | GATGACCGCTGGCGGAGTTTAGTCCAG | |
| SEQ ID NO: 72: | N8.3I | GATTACACCCGGGTGTAATCTTGTCACCAATTTTTGTAGGGATGTTGGGCTAAATTG | |
| SEQ ID NO: 73: | N10.5O** | GATAACTCGAGTCGCCCGGTGGATTTAATGC | |
| SEQ ID NO: 74: | N10.5I | GATTACACCCGGGTGTAATCCCCATACTCAGCCTTCTAATGCTGAGAGAATG | |
| SEQ ID NO: 75: | N10.3O** | GGACTGACCCAAGATACAGTGGTAG | |
| SEQ ID NO: 76: | N10.3I | GATTACACCCGGGTGTAATCCGAGCGCAAACTACAATGCGCTTCGTTTGC | |
| SEQ ID NO: 77: | N15.5O** | GGTTCTCGAGCCGCTGAATTTAGTCAACATCG | |
| SEQ ID NO: 78: | N15.5I | GATTACACCCGGGTGTAATCCATTGGGCACGAGAGTTAGTAAGGCAGTG | |
| SEQ ID NO: 79: | N15.3O* | CTAAACTTACGGCATTGGCATCAACGGGAG | |
| SEQ ID NO: 80: | N15.3I | GATTACACCCGGGTGTAATCTCTTTACAATGGCCAGGTCTTTAGGGAGCGGTGAC | |
| SEQ ID NO: 81: | N16.5O** | GAACTCGAGTAGTAACCACAGGCTTTTG | |
| SEQ ID NO: 82: | N16.5I | GATTACACCCGGGTGTAATCGCTGGAGGCGAACTGGGTGAGAACCAT | |
| SEQ ID NO: 83: | N16.3O** | GTGAGCTTGATGGTGATGGTGGGTAAAG | |
| SEQ ID NO: 84: | N16.3I | GATTACACCCGGGTGTAATCTGCTGGCTTTGTTGCCCTGTCAACCAAAGTTC | |

| | | | |
|---|---|---|---|
| SEQ ID NO: 85: | N5_SDM | CGAGGCGATCGCCCAGTTGGAAGAATTGGCC | SDM[***] |
| SEQ ID NO: 86: | N10_SDM | CTAAAAAGACAAGTCTGTGGCTAGTTACTATGACGAGGC | SDM[***] |
| SEQ ID NO: 87: | N5.FO | TCCTGGTAACTCACGCTATC | Mutants confirmation by PCR |
| SEQ ID NO: 88: | N5.FI | AGCCGATCCAGGGAAGTGTTGTTG | |
| SEQ ID NO: 89: | N5.RI | CCATCGTCCTTCGCCGTAGATTGTG | |
| SEQ ID NO: 90: | N8.FO | CCCAGTTAAACTGCGAAAGG | |
| SEQ ID NO: 91: | N8.FI | TCGCCAAGCTTTCAGAAC | |
| SEQ ID NO: 92: | N8.RI | CAAACTCCAGCCGATAAC | Mutants confirmation by PCR |
| SEQ ID NO: 93: | N10.FO | CCGGTTGCCCTTATCGGAACCGATG | |
| SEQ ID NO: 94: | N10.FI | GCTATGGCGTCACTTGTAGC | |
| SEQ ID NO: 95: | N10.RI | TTTGCGACCCATCGGATTGC | |
| SEQ ID NO: 96: | N15.FO | CTCCAAGGCGACTACCTTTC | |
| SEQ ID NO: 97: | N15.FI | CCCAGTGGGAATGCGATCAG | |
| SEQ ID NO: 98: | N15.RI | TAGGAGGGCGATCACCGAAG | |
| SEQ ID NO: 99: | N16.FO | ACCCATTTCTTGGGTGTAGG | |
| SEQ ID NO: 100: | N16.FI | GGCCTTGGTTGCCCTGACTGATGTG | |
| SEQ ID NO: 101: | N16.RI | GACCGATCGCCGCAGTAGTTCTTGG | |
| SEQ ID NO: 102: | GFP.F | TCTTGTTGAATTAGATGGTG | RT-PCR/RT-qPCR |
| SEQ ID NO: 103: | GFP.R | TGTGAGTTATAGTTGTATTCC | |

**Fig. 10**

| **Organisms name*** | **Description** | **Source** |
|---|---|---|
| *Escherichia coli* DH5α | Transformation/cloning strain | Invitrogen |
| *Synechocystis* sp. PCC 6803 | Wild-type strain | Pasteur Culture Collection |
| SN*n*K | *Synechocystis* mutants with replacement of the neutral site N*n* by a kanamycin resistance cassette. | This work |
| SN*n*KS | *Synechocystis* mutants with replacement of the neutral site N*n* by a kanamycin resistance/sucrose sensitivity cassette. | This work |
| SN*n*K.gfp | *Synechocystis* mutants with replacement of the neutral site N*n* by a kanamycin resistance cassette and the promoterless GFP encoding gene. | This work |
| SN*n*K.Cgfp | *Synechocystis* mutants with replacement of the neutral site N*n* by a kanamycin resistance cassette and the GFP encoding gene under the control of a constitutive promoter ($P_{psbA2*}$). | This work |
| **Plamids name*** | **Description** | **Source** |
| pGEM®-T easy | T/A cloning vector | Promega |
| pK18mobsacB | Small mobilizable multi-purpose cloning vector derived from the *E. coli* plasmids pK18, conferring resistance to kanamycin (*nptII*), and sensitivity to sucrose (*sacB*). | National BioResource Project (NIG, Japan): E.coli |
| pSB1A2-E0240 | BioBrick™ vector containing the part BBa_E0240 (GFP generator). | Registry of Standard Biological Parts, MIT |
| pJ201-$P_{psbA2*}$ | Synthesized minimal *psbA2* promoter region, meeting the BioBrick RFC[10]standard specifications, cloned into the delivery vector pJ201 | DNA 2.0 Inc., this work |
| pBSK-FP300 | Synthetic interface containing a BioBrick compatible multiple cloning site( *Mun*I, *Not*I, *Xba*I, *Spe*I, *Not*I, *Pst*I) insulated with the transcriptional terminators BBa_B1006 and BBa_B0062 (upstream), and BBa_B0053 and BBa_B020 (downstream). | Epoch Life Science Inc., this work |
| pSN*n* | pGEM-T easy- based plasmid series containing the BioBrick compatible interface flanked by the two regions for double homologous recombination on neutral site *n*. | This work |

| | | |
|---|---|---|
| pSN*n*K | pSN*n* plasmid series with the kanamycin resistance cassette cloned upstream of the BioBrick compatible interface. | This work |
| pSN*n*KS | pSN*n* plasmid series with the kanamycin resistance/sucrose sensitivity cassette cloned upstream of the BioBrick compatible interface. | This work |
| pSN*n*K.gfp | pSN*n*K plasmid series with the GFP generator BioBrick BBa_E0240 cloned in the BioBrick compatible interface. | This work |
| pSN*n*K.Cgfp | pSN*n*K plasmid series with the GFP generator BioBrick BBa_E0240 under the control of a constitutive promoter ($P_{psbA2*}$), cloned in the BioBrick compatible interface. | This work |

**Fig. 11**

| Plasmid name | Description |
|---|---|
| pSN5 | pGEM-T easy vector containing the BioBrick compatible interface flanked by the two regions for double homologous recombination on neutral site N5. |
| pSN8 | pGEM-T easy vector containing the BioBrick compatible interface flanked by the two regions for double homologous recombination on neutral site N8. |
| pSN10 | pGEM-T easy vector containing the BioBrick compatible interface flanked by the two regions for double homologous recombination on neutral site N10. |
| pSN15 | pGEM-T easy vector containing the BioBrick compatible interface flanked by the two regions for double homologous recombination on neutral site N15. |
| pSN16 | pGEM-T easy vector containing the BioBrick compatible interface flanked by the two regions for double homologous recombination on neutral site N16. |
| pSN5K | pSN5 plasmid with the kanamycin resistance cassette cloned upstream of the BioBrick compatible interface. |
| pSN8K | pSN8 plasmid with the kanamycin resistance cassette cloned upstream of the BioBrick compatible interface. |
| pSN10K | pSN10 plasmid with the kanamycin resistance cassette cloned upstream of the BioBrick compatible interface. |
| pSN15K | pSN15 plasmid with the kanamycin resistance cassette cloned upstream of the BioBrick compatible interface. |
| pSN16K | pSN16 plasmid with the kanamycin resistance cassette cloned upstream of the BioBrick compatible interface. |

| | |
|---|---|
| pSN5KS | pSN5 plasmid with the kanamycin resistance/sucrose sensitivity cassette cloned upstream of the BioBrick compatible interface. |
| pSN8KS | pSN8 plasmid with the kanamycin resistance/sucrose sensitivity cassette cloned upstream of the BioBrick compatible interface. |
| pSN10KS | pSN10 plasmid with the kanamycin resistance/sucrose sensitivity cassette cloned upstream of the BioBrick compatible interface. |
| pSN15KS | pSN15 plasmid with the kanamycin resistance/sucrose sensitivity cassette cloned upstream of the BioBrick compatible interface. |
| pSN16KS | pSN16 plasmid with the kanamycin resistance/sucrose sensitivity cassette cloned upstream of the BioBrick compatible interface. |
| pSN5K.gfp | pSN5K plasmid with the GFP generator BioBrick BBa_E0240 cloned in the BioBrick compatible interface. |
| pSN8K.gfp | pSN8K plasmid with the GFP generator BioBrick BBa_E0240 cloned in the BioBrick compatible interface. |
| pSN10K.gfp | pSN10K plasmid with the GFP generator BioBrick BBa_E0240 cloned in the BioBrick compatible interface. |
| pSN15K.gfp | pSN15K plasmid with the GFP generator BioBrick BBa_E0240 cloned in the BioBrick compatible interface. |
| pSN16K.gfp | pSN16K plasmid with the GFP generator BioBrick BBa_E0240 cloned in the BioBrick compatible interface. |
| pSN5K.Cgfp | pSN5K plasmid with the GFP generator BioBrick BBa_E0240 under the control of a constitutive promoter ($P_{psbA2*}$), cloned in the BioBrick compatible interface. |
| pSN8K.Cgfp | pSN8K plasmid with the GFP generator BioBrick BBa_E0240 under the control of a constitutive promoter ($P_{psbA2*}$), cloned in the BioBrick compatible interface. |
| pSN10K.Cgfp | pSN10K plasmid with the GFP generator BioBrick BBa_E0240 under the control of a constitutive promoter ($P_{psbA2*}$), cloned in the BioBrick compatible interface. |
| pSN15K.Cgfp | pSN15K plasmid with the GFP generator BioBrick BBa_E0240 under the control of a constitutive promoter ($P_{psbA2*}$), cloned in the BioBrick compatible interface. |
| pSN16K.Cgfp | pSN16K plasmid with the GFP generator BioBrick BBa_E0240 under the control of a constitutive promoter ($P_{psbA2*}$), cloned in the BioBrick compatible interface. |

**Fig. 12**

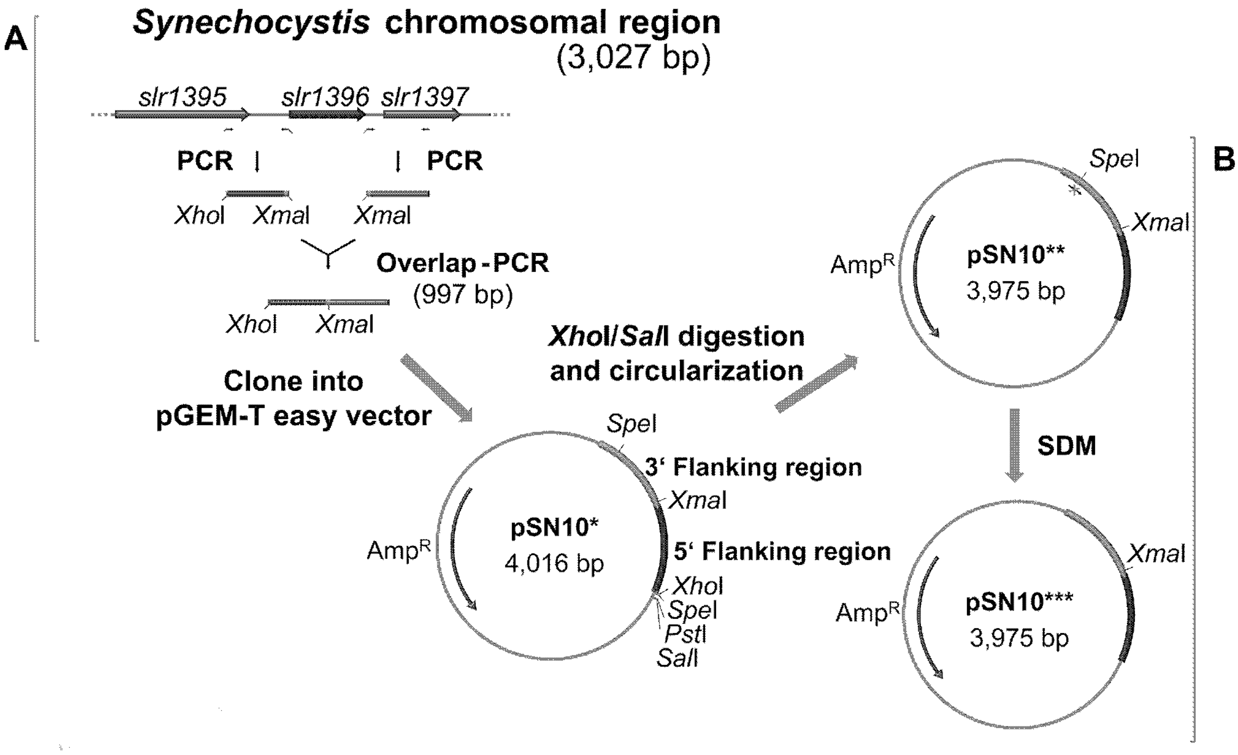


Fig. 13A-B

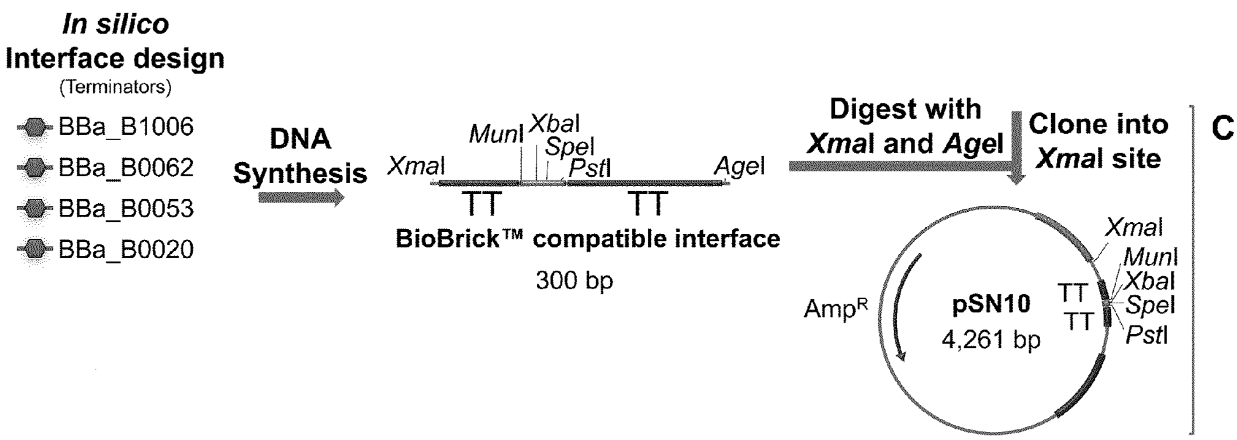


Fig. 13C

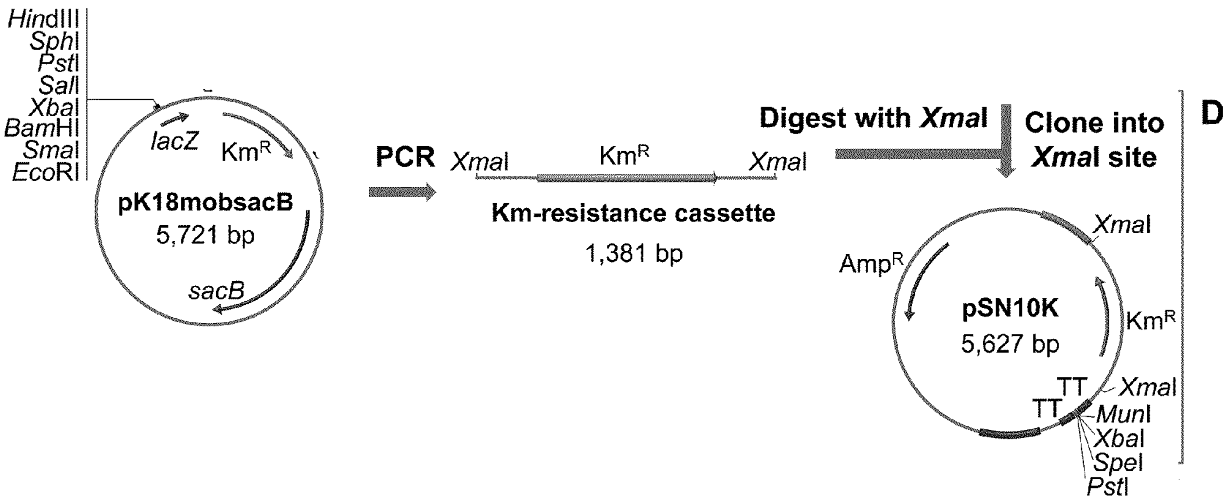


Fig. 13D

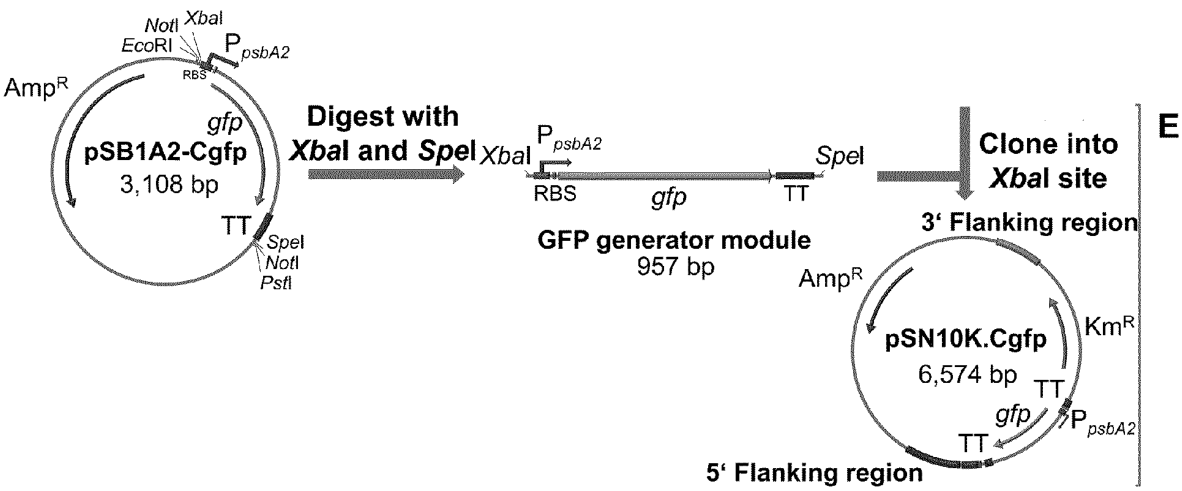


Fig. 13E

| Parameters | Mutants | | | | |
|---|---|---|---|---|---|
| | SN5K | SN8K | SN10K | SN15K | SN16K |
| Light | $1.61x10^{-38}$ | $3.47x10^{-37}$ | $8.70x10^{-34}$ | $1.58x10^{-33}$ | $5.28x10^{-39}$ |
| Glucose | $1.49x10^{-11}$ | $3.62x10^{-22}$ | $8.48x10^{-19}$ | $2.54x10^{-12}$ | $3.50x10^{-25}$ |
| Mutation* | $4.58x10^{-15}$ | $1.24x10^{-04}$ | $8.65x10^{-03}$ | $3.37x10^{-01}$ | $3.48x10^{-01}$ |
| Light + Glucose | $2.57x10^{-10}$ | $1.36x10^{-14}$ | $3.62x10^{-18}$ | $6.46x10^{-20}$ | $9.58x10^{-16}$ |
| Light + Mutation | $1.74x10^{-03}$ | $4.51x10^{-04}$ | $1.04x10^{-01}$ | $5.27x10^{-01}$ | $7.76x10^{-02}$ |
| Glucose + Mutation | $1.74x10^{-07}$ | $1.00x10^{-01}$ | $1.20x10^{-01}$ | $1.44x10^{-04}$ | $1.30x10^{-01}$ |

Fig. 14

| Mutant name | Description |
|---|---|
| SN5K | *Synechocystis* mutant with replacement of the neutral site N5 by a kanamycin resistance cassette. |
| SN8K | *Synechocystis* mutant with replacement of the neutral site N8 by a kanamycin resistance cassette. |
| SN10K | *Synechocystis* mutant with replacement of the neutral site N10 by a kanamycin resistance cassette. |
| SN15K | *Synechocystis* mutant with replacement of the neutral site N15 by a kanamycin resistance cassette. |
| SN16K | *Synechocystis* mutant with replacement of the neutral site N16 by a kanamycin resistance cassette. |
| SN5KS | *Synechocystis* mutant with replacement of the neutral site N5 by a kanamycin resistance/sucrose sensitivity cassette. |
| SN8KS | *Synechocystis* mutant with replacement of the neutral site N8 by a kanamycin resistance/sucrose sensitivity cassette. |
| SN10KS | *Synechocystis* mutant with replacement of the neutral site N10 by a kanamycin resistance/sucrose sensitivity cassette. |
| SN15KS | *Synechocystis* mutant with replacement of the neutral site N15 by a kanamycin resistance/sucrose sensitivity cassette. |
| SN16KS | *Synechocystis* mutant with replacement of the neutral site N16 by a kanamycin resistance/sucrose sensitivity cassette. |
| SN5K.gfp | *Synechocystis* mutant with replacement of the neutral site N5 by a kanamycin resistance cassette and the promoterless GFP encoding gene. |
| SN8K.gfp | *Synechocystis* mutant with replacement of the neutral site N8 by a kanamycin resistance cassette and the promoterless GFP encoding gene. |
| SN10K.gfp | *Synechocystis* mutant with replacement of the neutral site N10 by a kanamycin resistance cassette and the promoterless GFP encoding gene. |
| SN15K.gfp | *Synechocystis* mutant with replacement of the neutral site N15 by a kanamycin resistance cassette and the promoterless GFP encoding gene. |
| SN16K.gfp | *Synechocystis* mutant with replacement of the neutral site N16 by a kanamycin resistance cassette and the promoterless GFP encoding gene. |
| SN5K.Cgfp | *Synechocystis* mutant with replacement of the neutral site N5 by a kanamycin resistance cassette and the GFP encoding gene under the control of a constitutive promoter ($P_{psbA2*}$). |
| SN8K.Cgfp | *Synechocystis* mutant with replacement of the neutral site N8 by a kanamycin resistance cassette and the GFP encoding gene under the control of a constitutive promoter ($P_{psbA2*}$). |
| SN10K.Cgfp | *Synechocystis* mutant with replacement of the neutral site N10 by a kanamycin resistance cassette and the GFP encoding gene under the control of a constitutive promoter ($P_{psbA2*}$). |
| SN15K.Cgfp | *Synechocystis* mutant with replacement of the neutral site N15 by a kanamycin resistance cassette and the GFP encoding gene under the control of a constitutive promoter ($P_{psbA2*}$). |
| SN16K.Cgfp | *Synechocystis* mutant with replacement of the neutral site N16 by a kanamycin resistance cassette and the GFP encoding gene under the control of a constitutive promoter ($P_{psbA2*}$). |

**Fig. 15**

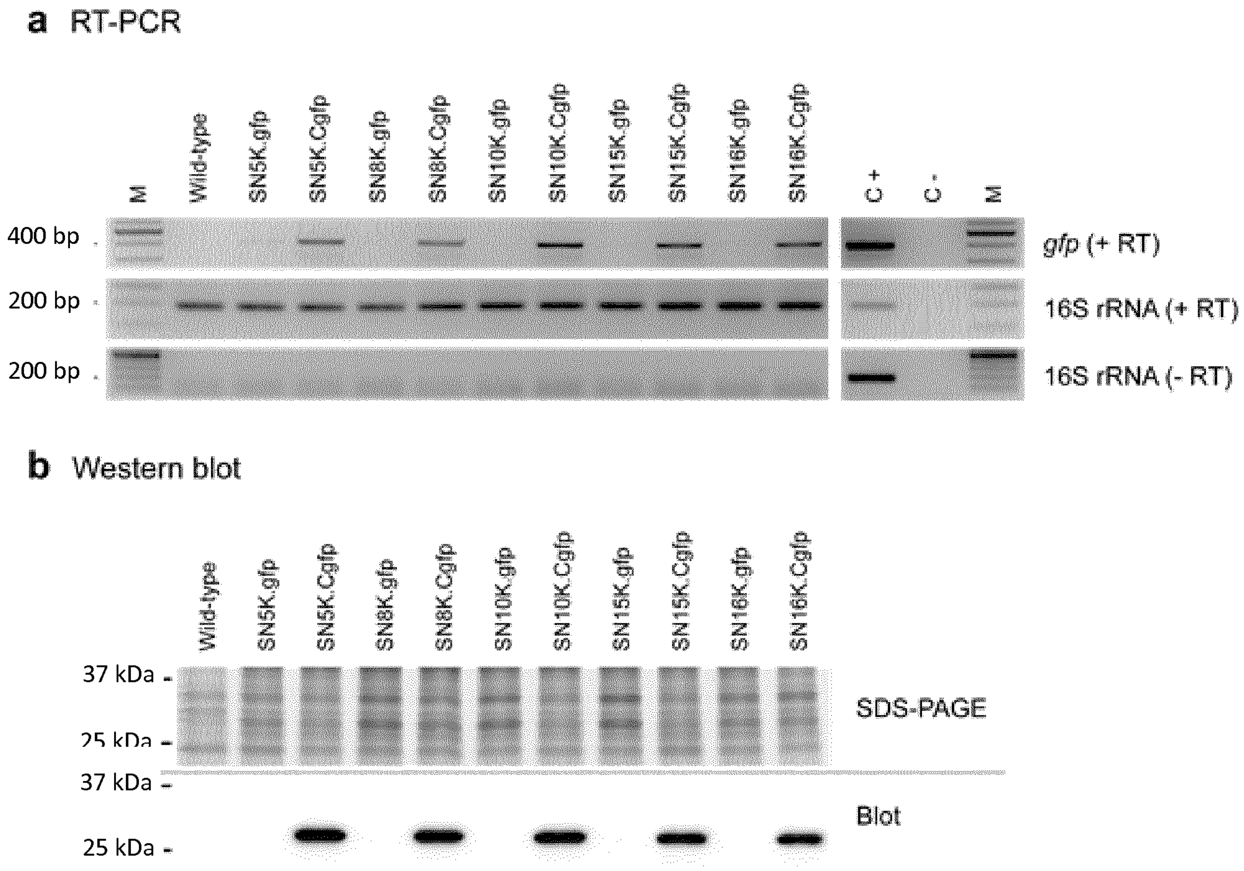
a RT-PCR
M
Wild-type
SN5K.gfp
SN5K.Cgfp
SN8K.gfp
SN8K.Cgfp
SN10K.gfp
SN10K.Cgfp
SN15K.gfp
SN15K.Cgfp
SN16K.gfp
SN16K.Cgfp
C +
C -
M
400 bp
200 bp
200 bp
gfp (+ RT)
16S rRNA (+ RT)
16S rRNA (- RT)
b Western blot
Wild-type
SN5K.gfp
SN5K.Cgfp
SN8K.gfp
SN8K.Cgfp
SN10K.gfp
SN10K.Cgfp
SN15K.gfp
SN15K.Cgfp
SN16K.gfp
SN16K.Cgfp
37 kDa
25 kDa
37 kDa
25 kDa
SDS-PAGE
Blot

Fig. 16

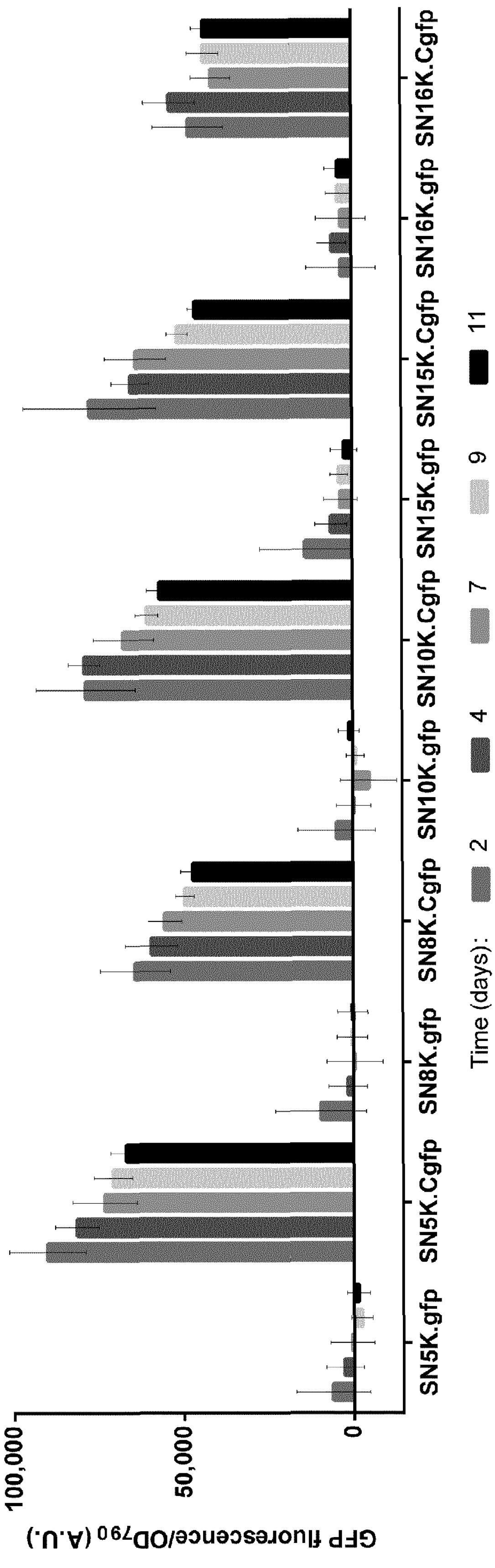
GFP fluorescence/OD790 (A.U.)
100,000
50,000
0
SN5K.gfp
SN5K.Cgfp
SN8K.gfp
SN8K.Cgfp
SN10K.gfp
SN10K.Cgfp
SN15K.gfp
SN15K.Cgfp
SN16K.gfp
SN16K.Cgfp
Time (days):
2
4
7
9
11

Fig. 17

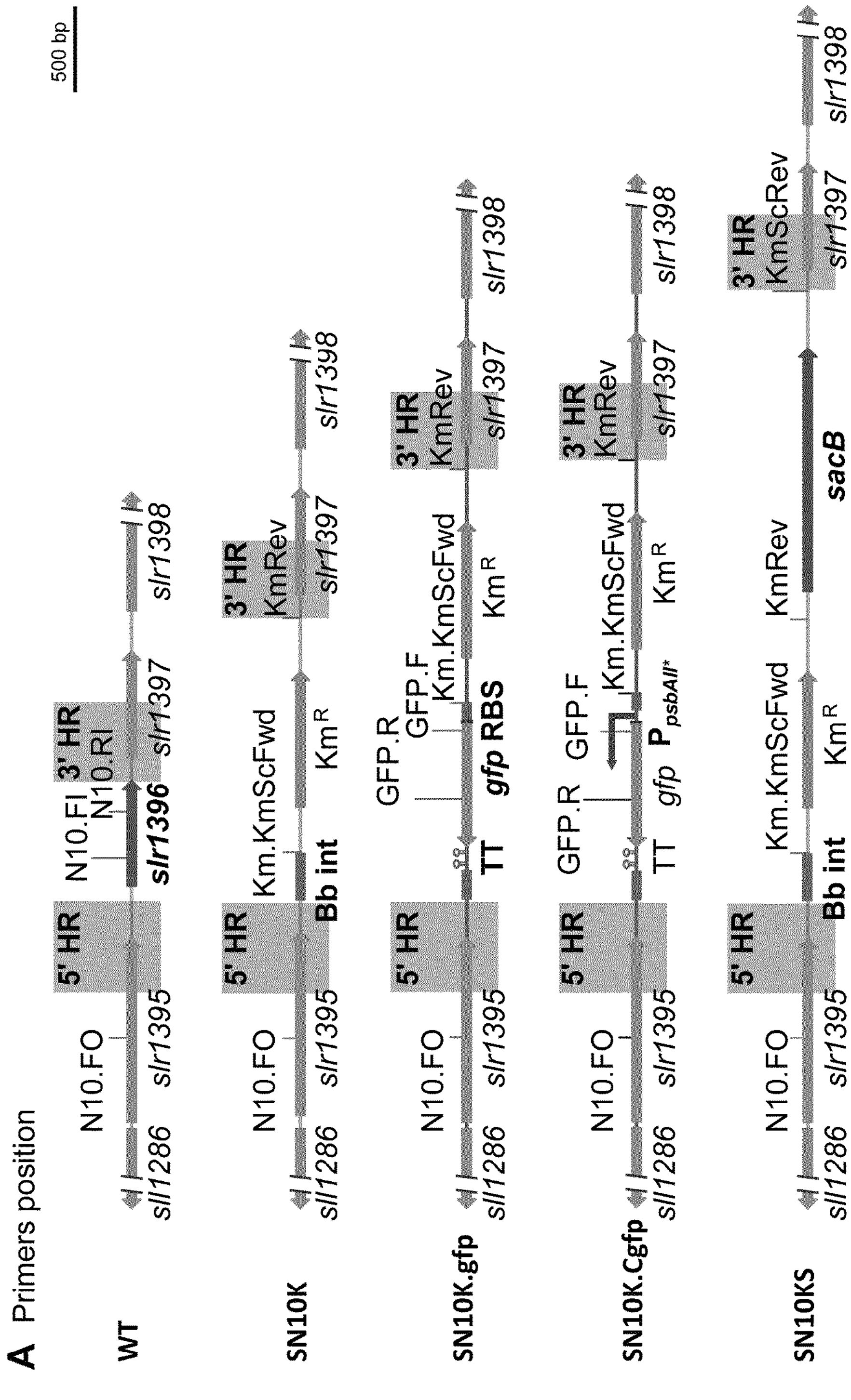
A Primers position
500 bp
WT
SN10K
SN10K.gfp
SN10K.Cgfp
SN10KS
sll1286
slr1395
N10.FO
5' HR
3' HR
slr1396
N10.FI
N10.RI
slr1397
slr1398
Bb int
Km.KmScFwd
KmR
KmRev
TT
gfp RBS
GFP.R
GFP.F
Km.KmScFwd
gfp
PpsbAII*
KmScRev
sacB

Fig. 18 A

**B** PCR with inner primers (N*n*.FI/N*n*.RI)

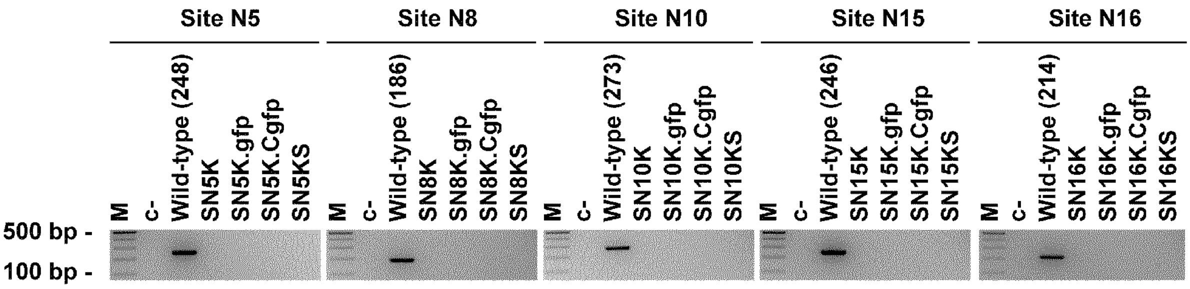


**C** PCR with outer and internal primers (N*n*.FO/KmRev)

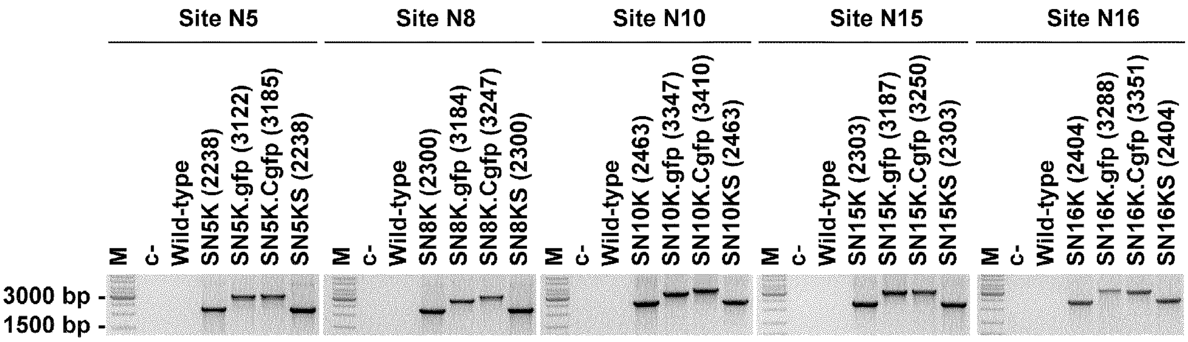


Fig. 18 B-C

**D** PCR with kanamycin primers (Km.KmScFwd/KmRev)

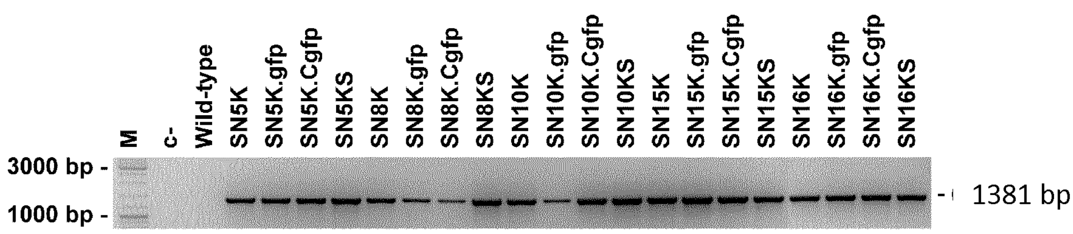


**E** PCR with *gfp* primers (GFP.F/GFP.R)

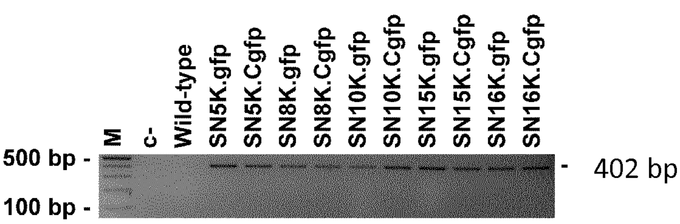


**F** PCR with kanamycin/*sacB* primers (Km.KmScFwd/KmScRev)

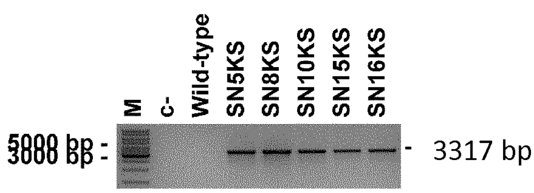


Fig. 18 D-F

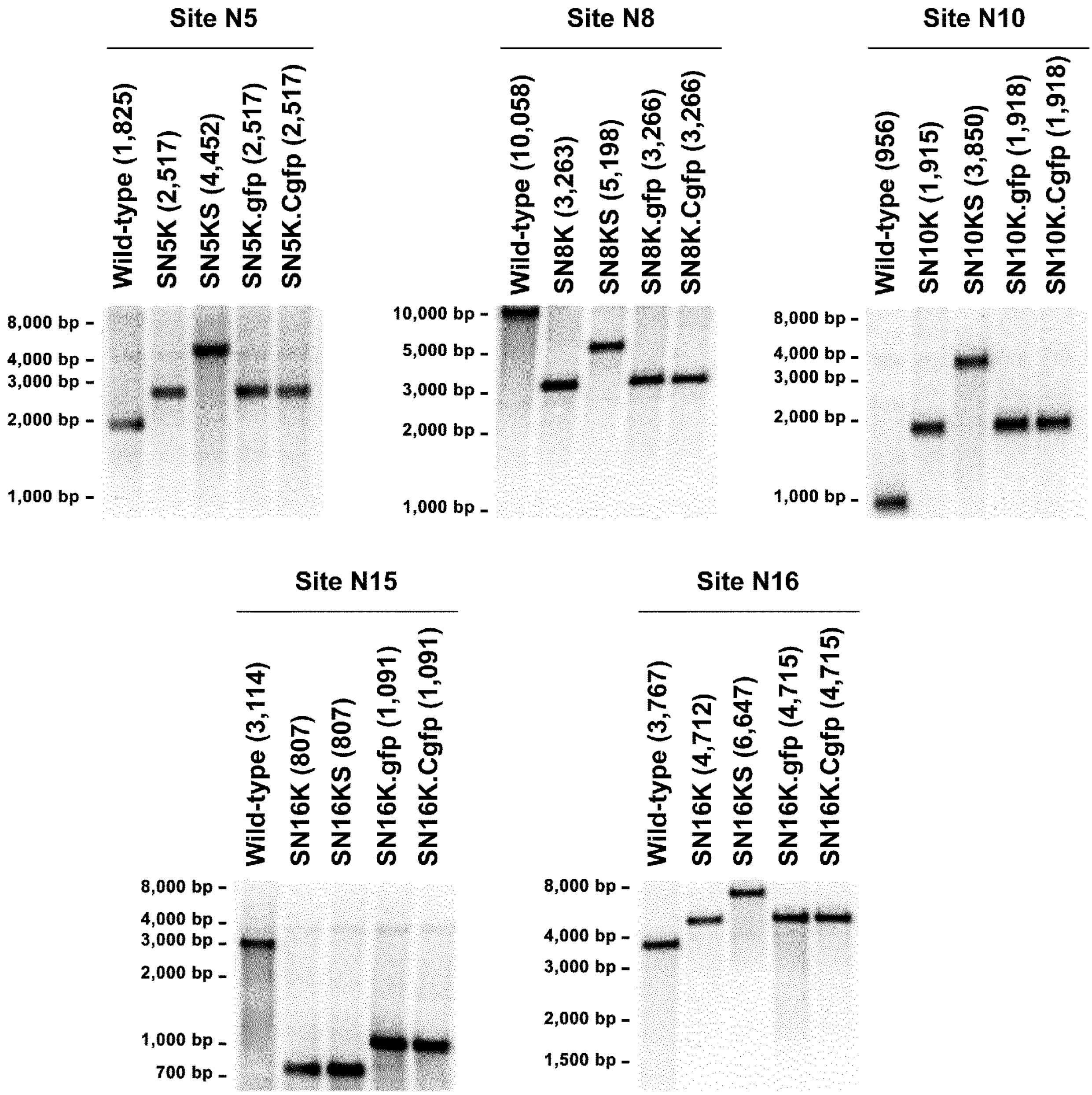
Site N5
Wild-type (1,825)
SN5K (2,517)
SN5KS (4,452)
SN5K.gfp (2,517)
SN5K.Cgfp (2,517)
8,000 bp
4,000 bp
3,000 bp
2,000 bp
1,000 bp
Site N8
Wild-type (10,058)
SN8K (3,263)
SN8KS (5,198)
SN8K.gfp (3,266)
SN8K.Cgfp (3,266)
10,000 bp
5,000 bp
3,000 bp
2,000 bp
1,000 bp
Site N10
Wild-type (956)
SN10K (1,915)
SN10KS (3,850)
SN10K.gfp (1,918)
SN10K.Cgfp (1,918)
8,000 bp
4,000 bp
3,000 bp
2,000 bp
1,000 bp
Site N15
Wild-type (3,114)
SN16K (807)
SN16KS (807)
SN16K.gfp (1,091)
SN16K.Cgfp (1,091)
8,000 bp
4,000 bp
3,000 bp
2,000 bp
1,000 bp
700 bp
Site N16
Wild-type (3,767)
SN16K (4,712)
SN16KS (6,647)
SN16K.gfp (4,715)
SN16K.Cgfp (4,715)
8,000 bp
4,000 bp
3,000 bp
2,000 bp
1,500 bp

Fig. 19

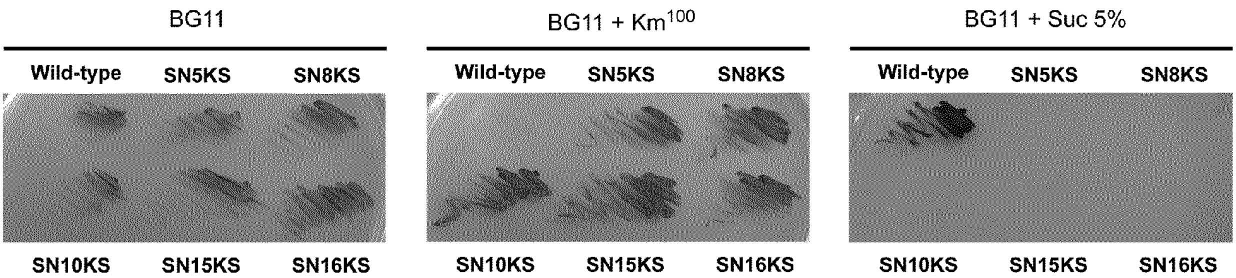
BG11
BG11 + Km100
BG11 + Suc 5%
Wild-type
SN5KS
SN8KS
Wild-type
SN5KS
SN8KS
Wild-type
SN5KS
SN8KS
SN10KS
SN15KS
SN16KS
SN10KS
SN15KS
SN16KS
SN10KS
SN15KS
SN16KS

Fig. 20

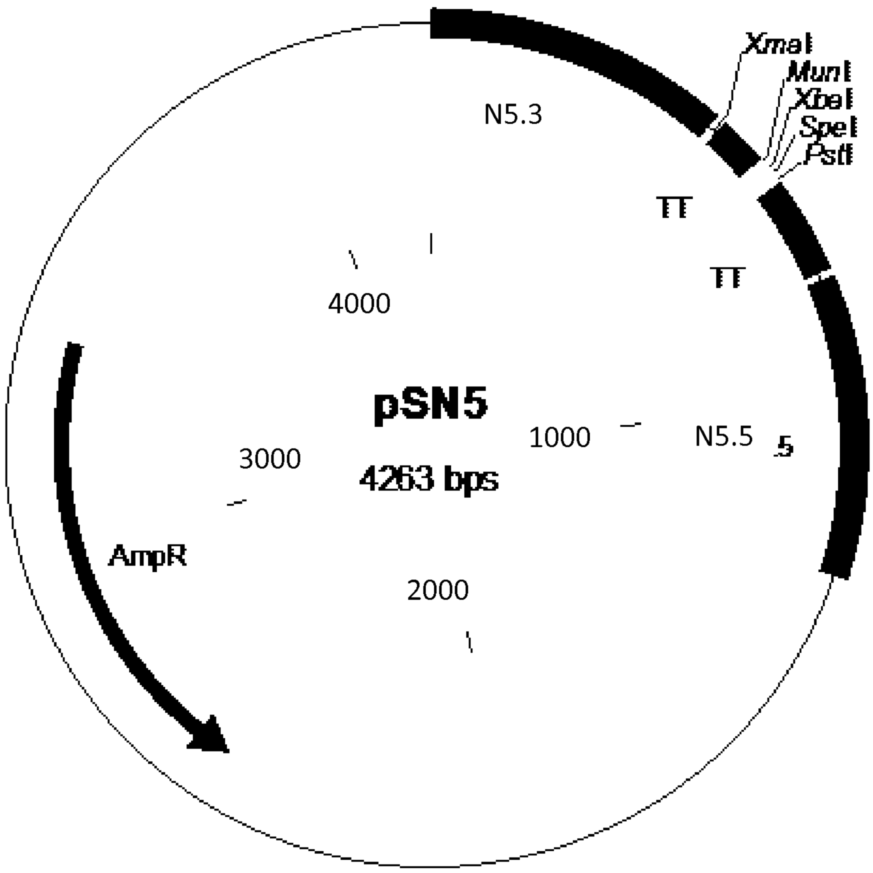
XmaI
MunI
XbaI
SpeI
PstI
N5.3
TT
TT
4000
pSN5
4263 bps
3000
1000
N5.5
AmpR
2000

Fig. 21

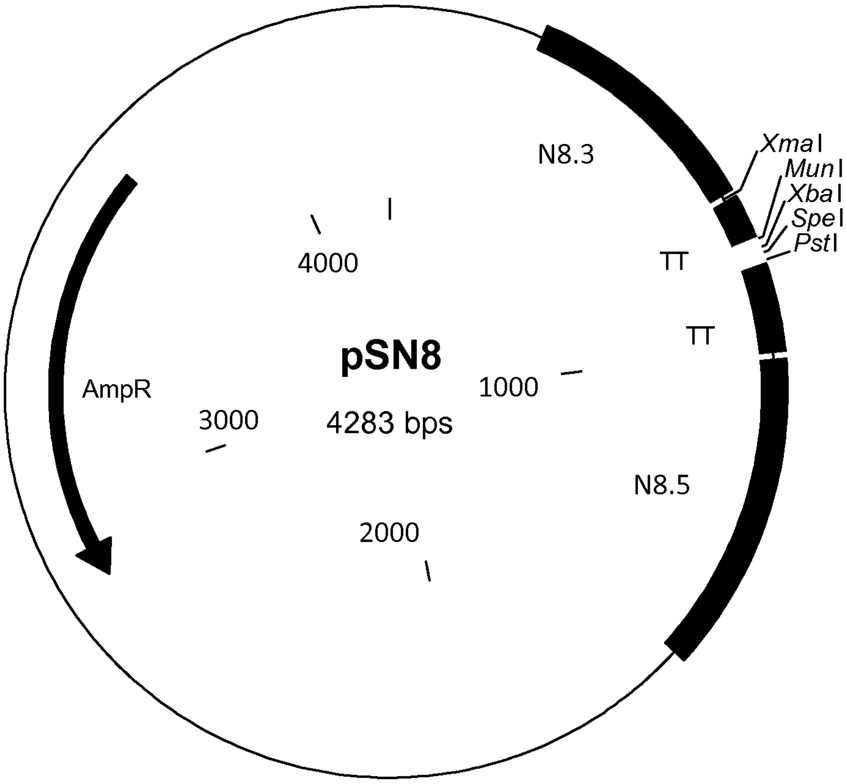
N8.3
XmaI
MunI
XbaI
SpeI
PstI
4000
TT
TT
pSN8
4283 bps
1000
AmpR
3000
N8.5
2000

Fig. 22

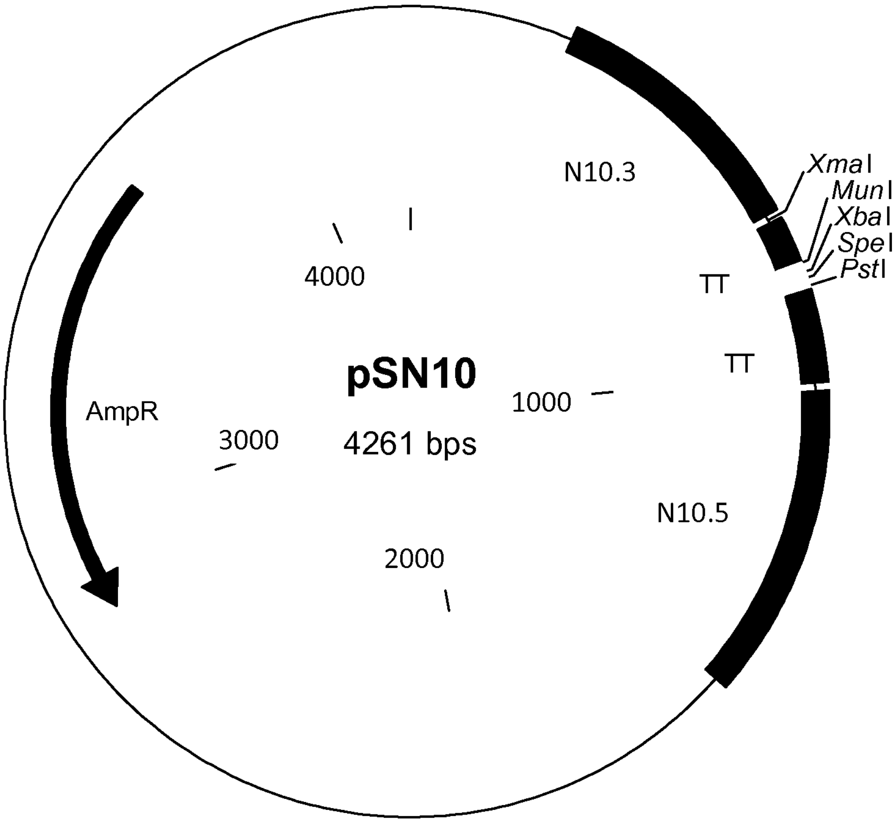
N10.3
XmaI
MunI
XbaI
SpeI
PstI
4000
TT
TT
pSN10
4261 bps
1000
AmpR
3000
N10.5
2000

Fig. 23

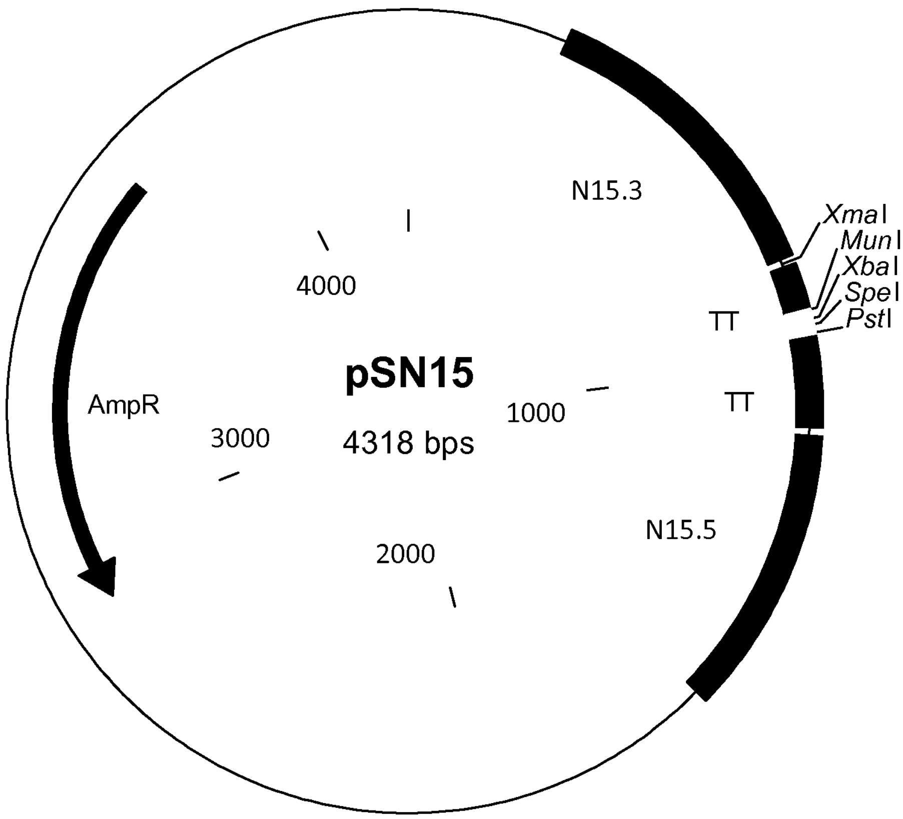
N15.3
XmaI
MunI
XbaI
SpeI
PstI
4000
TT
pSN15
AmpR
1000
TT
3000
4318 bps
N15.5
2000

Fig. 24

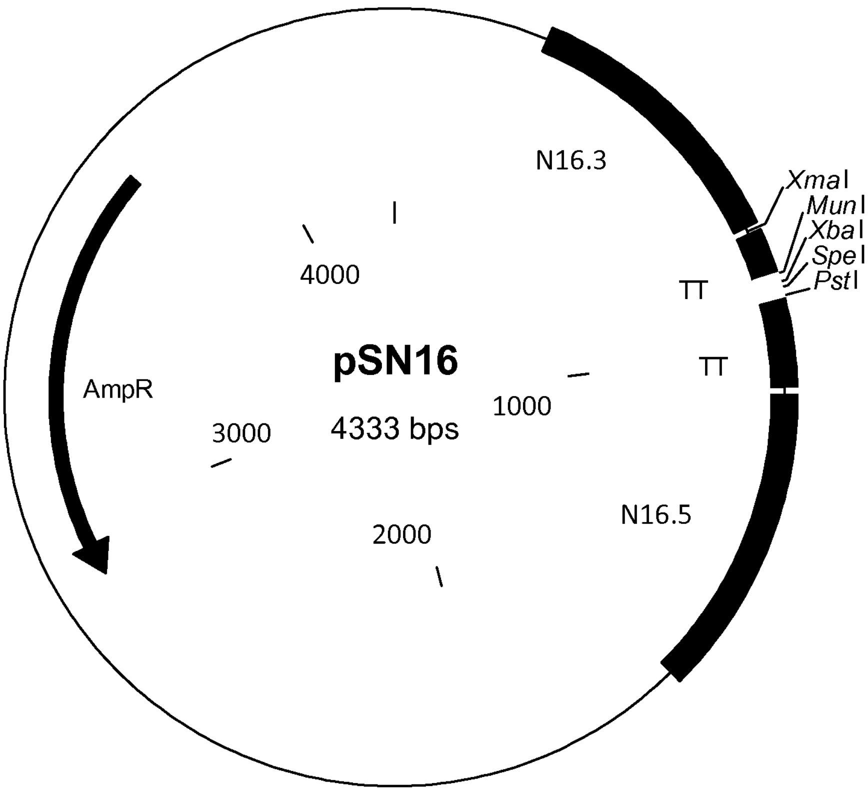
N16.3
XmaI
MunI
XbaI
SpeI
PstI
4000
TT
pSN16
TT
AmpR
1000
3000
4333 bps
N16.5
2000

Fig. 25

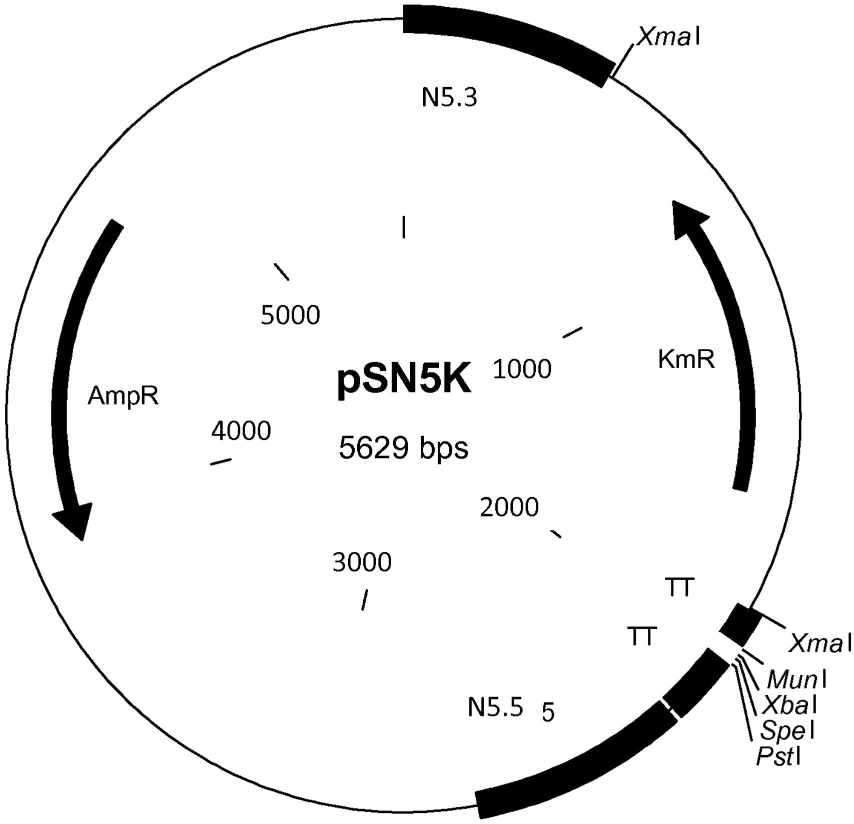
XmaI
N5.3
5000
1000
KmR
AmpR
pSN5K
4000
5629 bps
2000
3000
TT
TT
XmaI
MunI
N5.5
XbaI
SpeI
PstI

Fig. 26

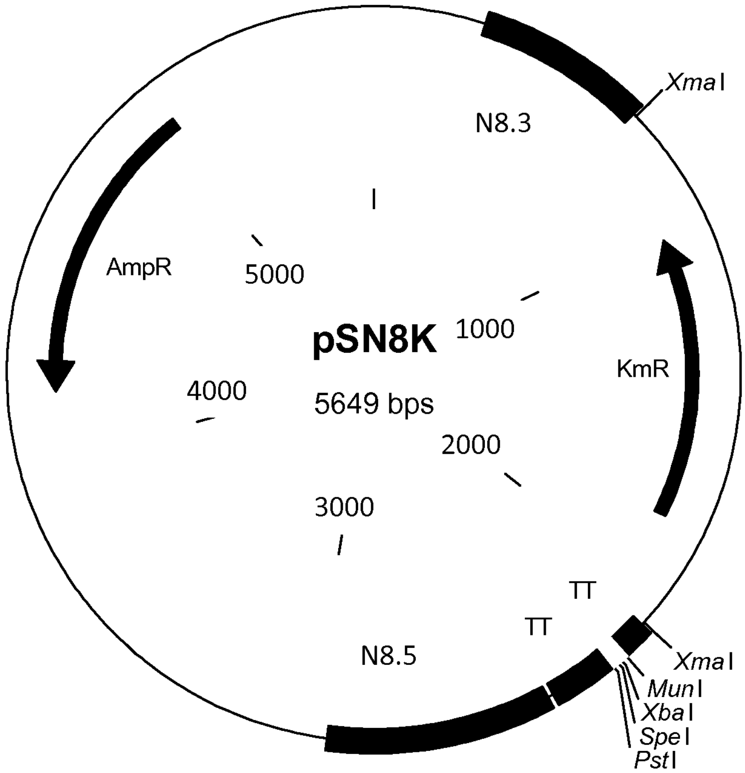
XmaI
N8.3
AmpR
5000
1000
pSN8K
KmR
4000
5649 bps
2000
3000
TT
TT
XmaI
N8.5
MunI
XbaI
SpeI
PstI

Fig. 27

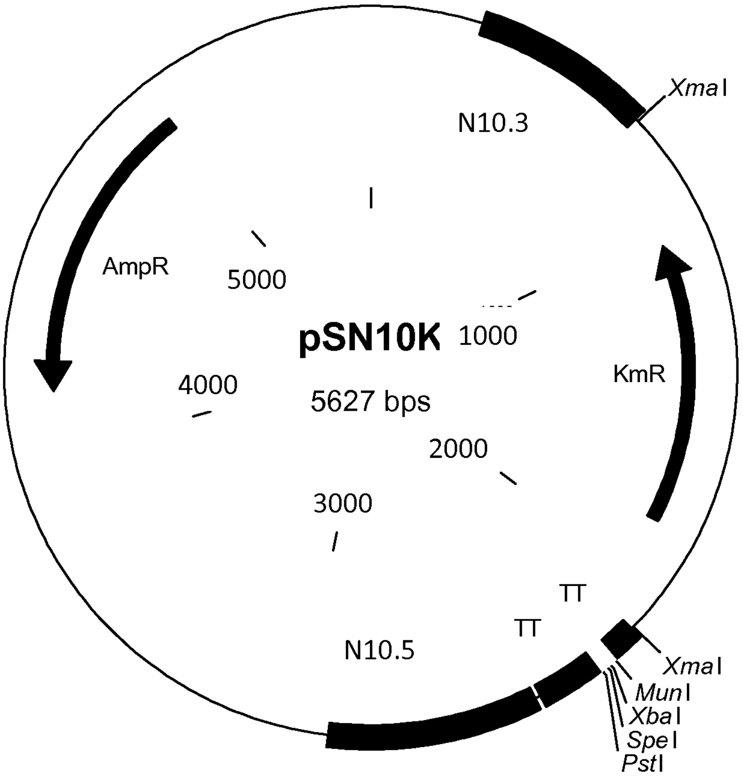
XmaI
N10.3
AmpR
5000
pSN10K
1000
5627 bps
4000
KmR
2000
3000
TT
TT
N10.5
XmaI
MunI
XbaI
SpeI
PstI

Fig. 28

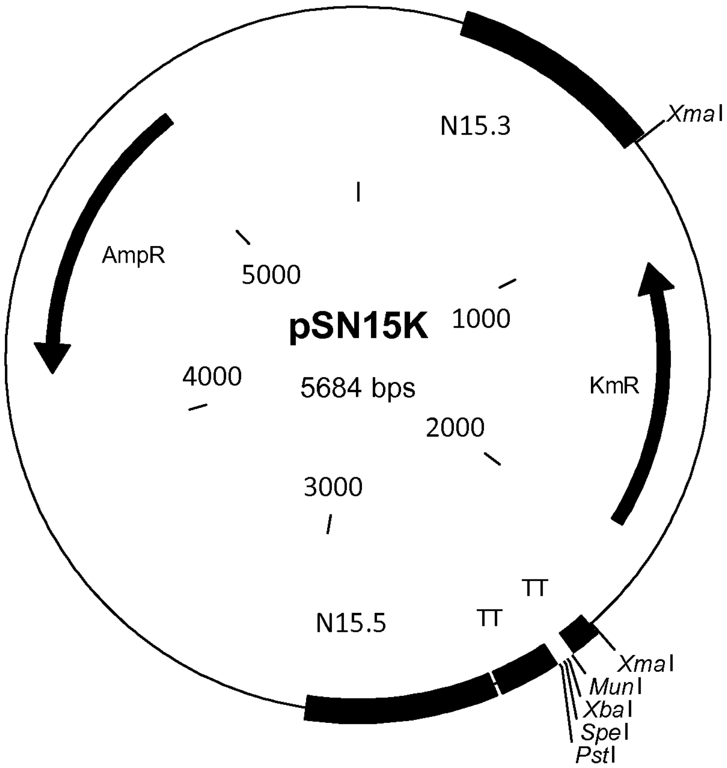
XmaI
N15.3
AmpR
5000
pSN15K
1000
4000
5684 bps
KmR
2000
3000
TT
TT
N15.5
XmaI
MunI
XbaI
SpeI
PstI

Fig. 29

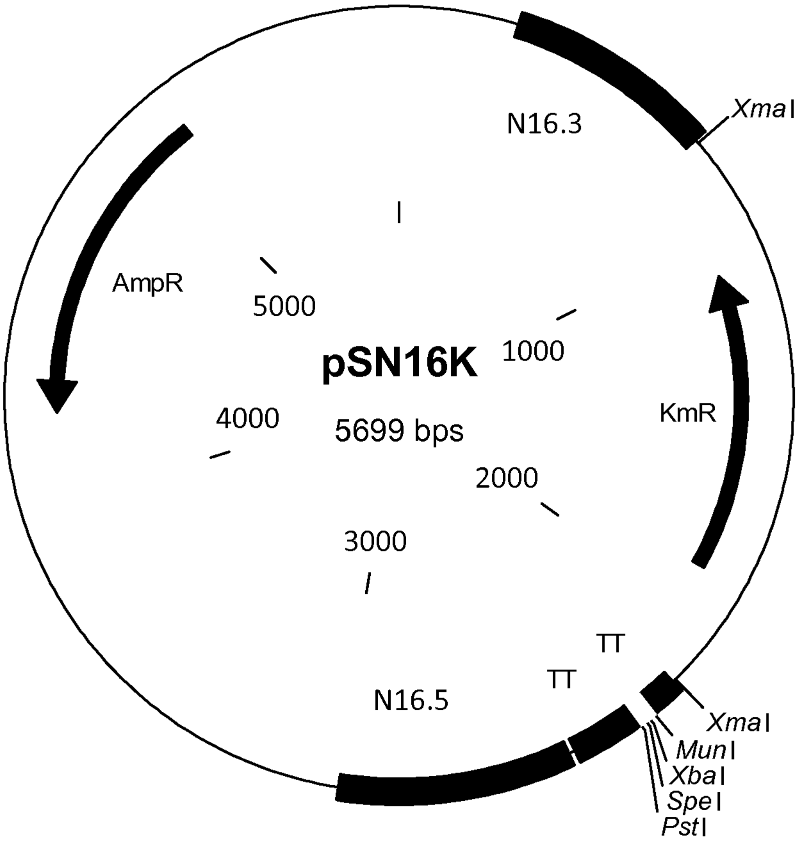
N16.3
XmaI
AmpR
5000
1000
pSN16K
5699 bps
4000
KmR
2000
3000
TT
TT
N16.5
XmaI
MunI
XbaI
SpeI
PstI

Fig. 30

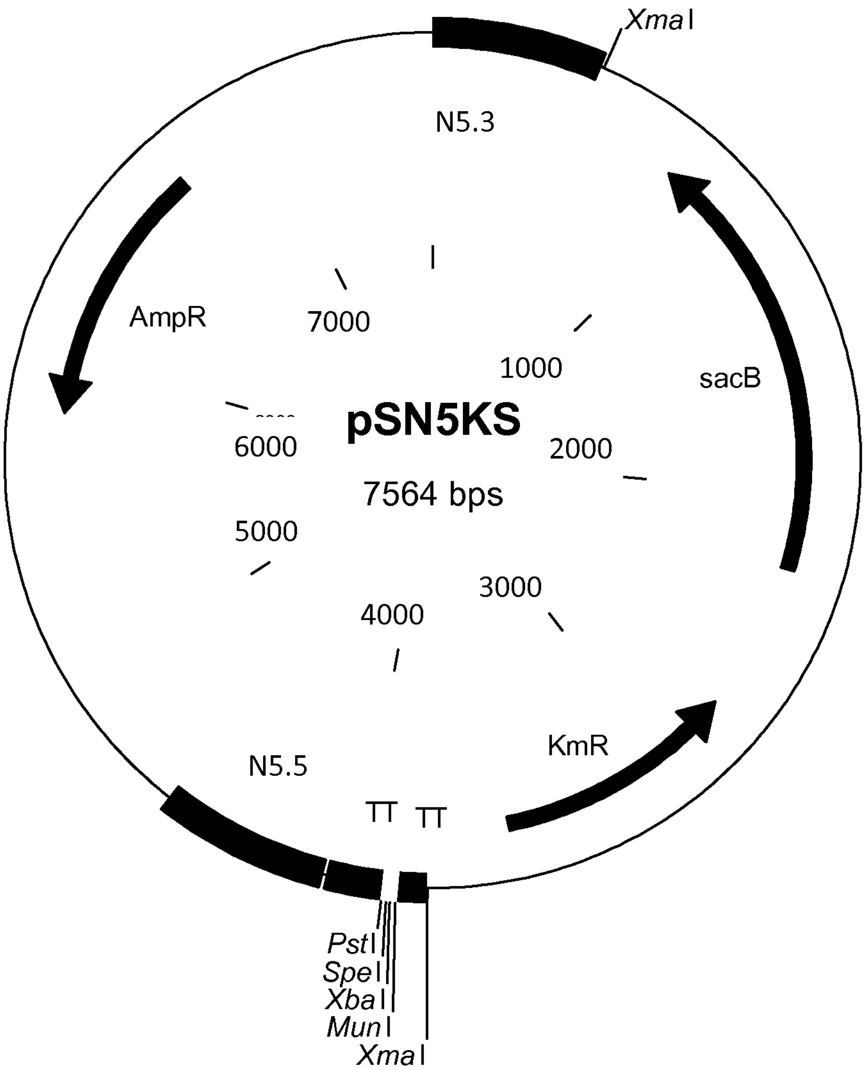
XmaI
N5.3
AmpR
7000
1000
sacB
pSN5KS
7564 bps
6000
2000
5000
3000
4000
KmR
N5.5
TT
TT
PstI
SpeI
XbaI
MunI
XmaI

Fig. 31

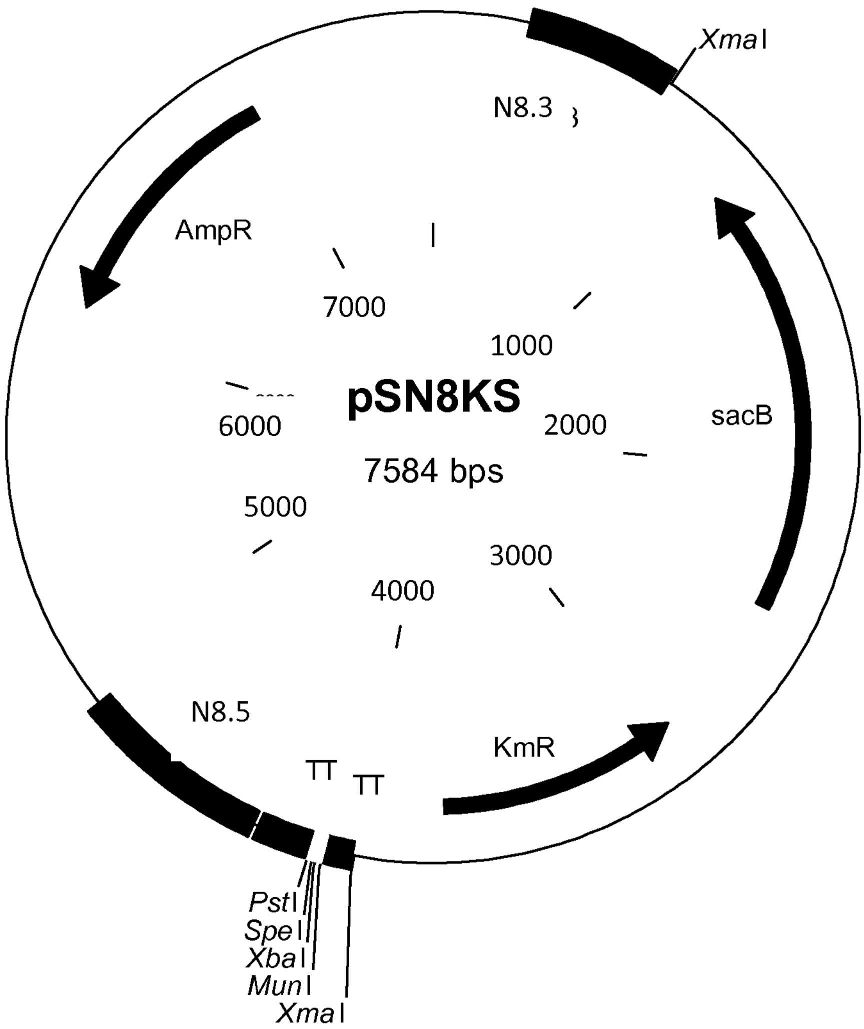
XmaI
N8.3
AmpR
7000
1000
pSN8KS
7584 bps
6000
2000
sacB
5000
3000
4000
N8.5
TT
TT
KmR
PstI
SpeI
XbaI
MunI
XmaI

Fig. 32

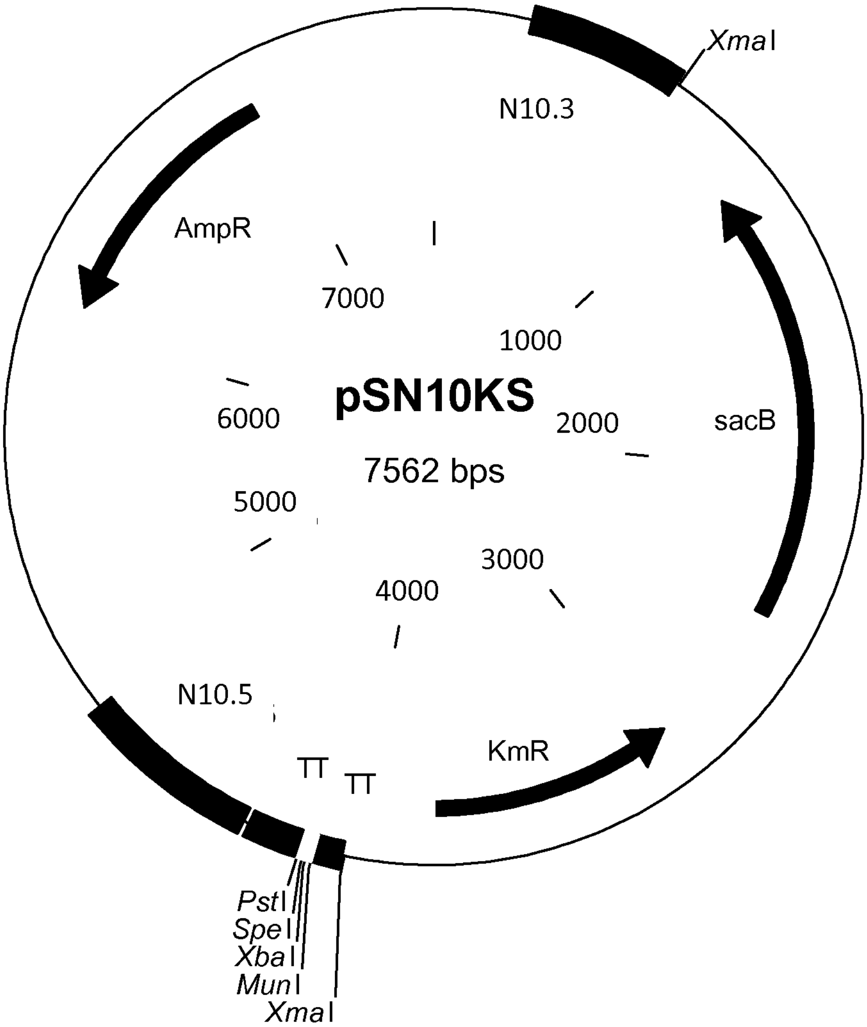
XmaI
N10.3
AmpR
7000
1000
pSN10KS
6000
2000
sacB
7562 bps
5000
3000
4000
N10.5
KmR
TT
TT
PstI
SpeI
XbaI
MunI
XmaI

Fig. 33

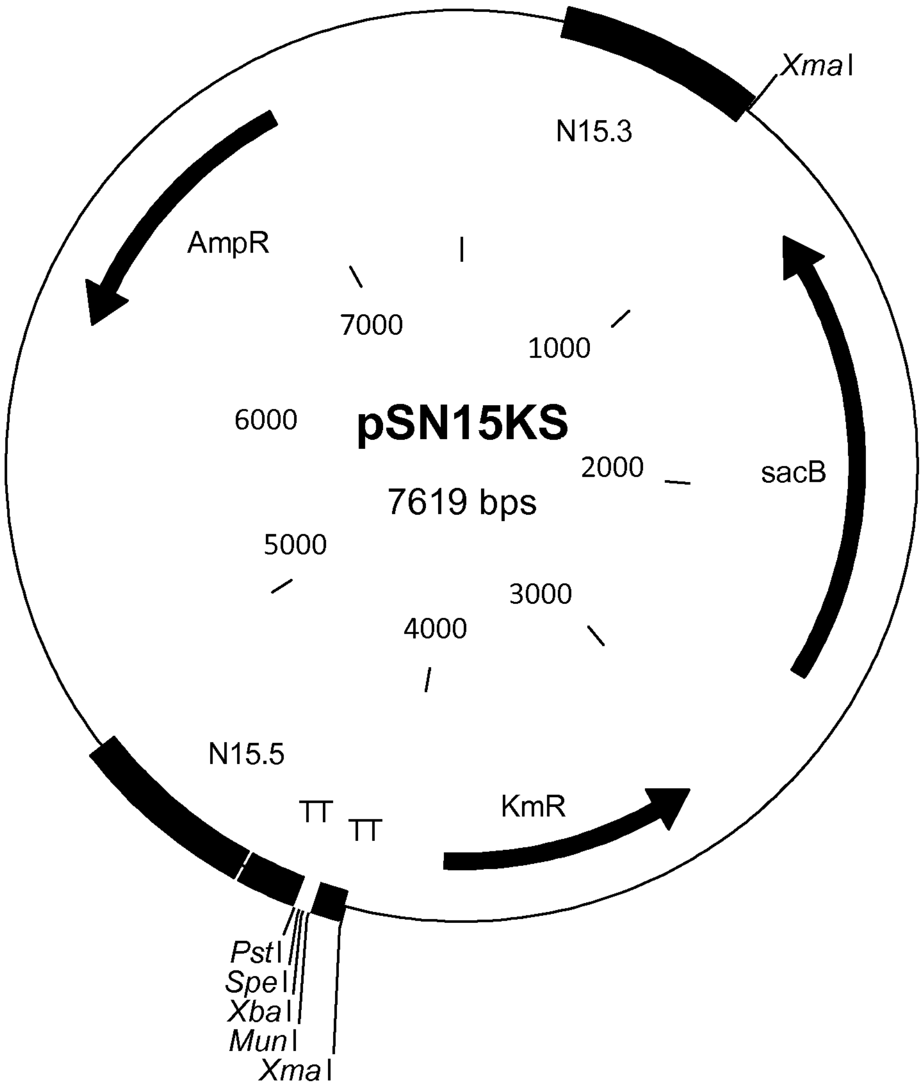
XmaI
N15.3
AmpR
7000
1000
6000
pSN15KS
7619 bps
2000
sacB
5000
3000
4000
N15.5
TT
TT
KmR
PstI
SpeI
XbaI
MunI
XmaI

Fig. 34

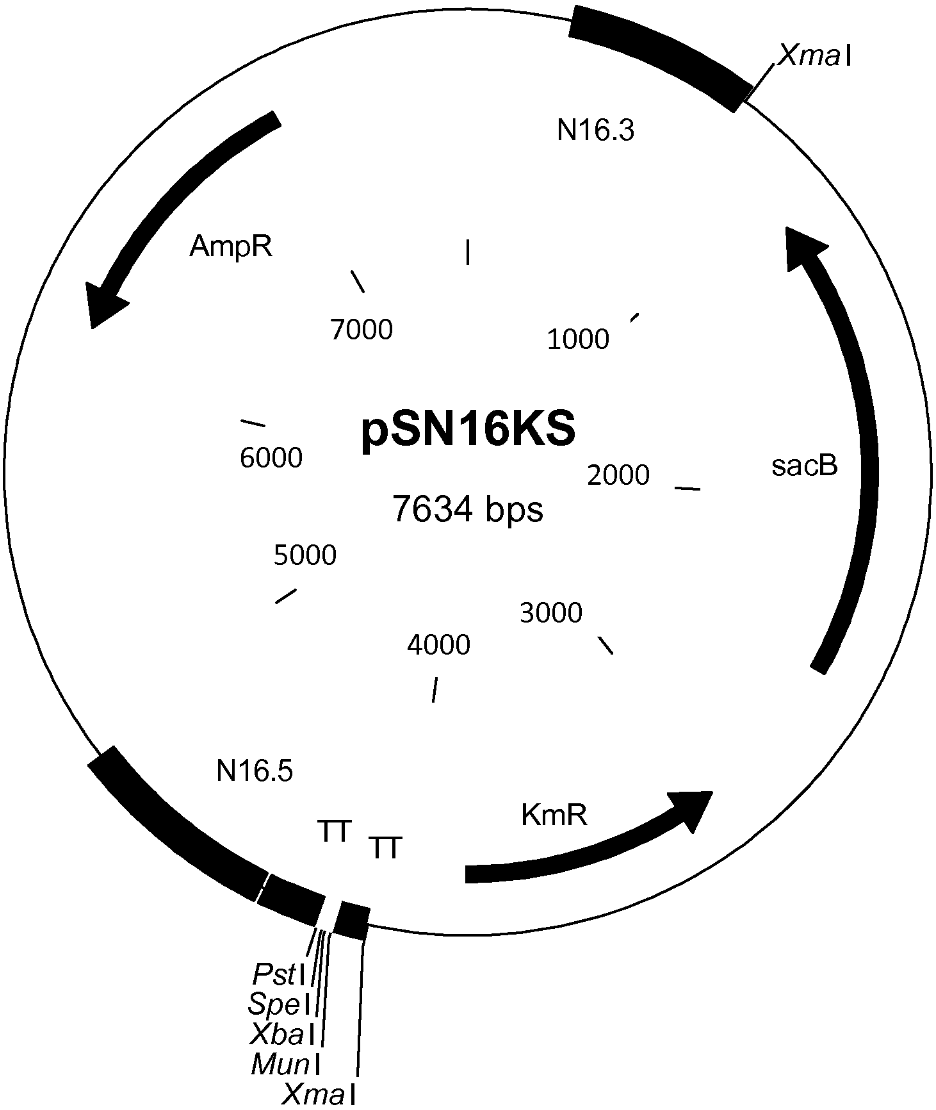
XmaI
N16.3
AmpR
7000
1000
pSN16KS
6000
2000
sacB
7634 bps
5000
3000
4000
N16.5
TT
TT
KmR
PstI
SpeI
XbaI
MunI
XmaI

Fig. 35

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **KUNERT A et al.** Construction of promoter probe vectors for Synechocystis sp. PCC 6803 using the light-emitting reporter systems Gfp and LuxAB. *JOURNAL OF NIICROBIOLOGICAL METHODS AUG 2000,* August 2000, vol. 41 (3
- **BENTLEY FIONA K et al.** Heterologous expression of the mevalonic acid pathway in cyanobacteria enhances endogenous carbon partitioning to isoprene. *MOLECULAR PLANT JAN 2014,* January 2014, vol. 7 (1